(19) Europäisches Patentamt
European Patent Office
Office européen des brevets

(11) EP 2 498 748 B1

(12) # EUROPEAN PATENT SPECIFICATION

(45) Date of publication and mention of the grant of the patent:
**05.09.2018 Bulletin 2018/36**

(21) Application number: **10773361.0**

(22) Date of filing: **09.11.2010**

(51) Int Cl.:
***A61K 8/49*** *(2006.01)* ***A61Q 5/06*** *(2006.01)*
***C09B 23/14*** *(2006.01)* ***C09B 26/04*** *(2006.01)*
***C09B 62/78*** *(2006.01)*

(86) International application number:
**PCT/EP2010/067117**

(87) International publication number:
**WO 2011/054966 (12.05.2011 Gazette 2011/19)**

(54) **NEW DYES WITH HETEROCYCLIC DISULPHIDE UNIT, COLOURING COMPOSITION COMPRISING THEM, AND METHOD FOR DYEING HUMAN KERATINOUS FIBRES ON THE BASIS OF THESE DYES**

NEUE FARBSTOFFE MIT EINEM HETEROZYKLISCHEN DISULFID, ENTSPRECHENDE HAARFÄRBEZUSAMMENSETZUNGEN UND VERFAHREN ZUM FÄRBEN VON KERATINFASERN

NOUVEAUX COLORANTS AVEC UN MOTIF DISULFURE HÉTÉROCYCLIQUE, COMPOSITION COLORANTE COMPRENANT CEUX-CI, ET PROCÉDÉ POUR COLORER DES FIBRES KÉRATINIQUES HUMAINES BASÉ SUR CES COLORANTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.11.2009 FR 0957910**
**10.11.2009 US 259762 P**

(43) Date of publication of application:
**19.09.2012 Bulletin 2012/38**

(73) Proprietor: **L'Oréal**
**75008 Paris (FR)**

(72) Inventors:
- **GREAVES, Andrew**
**F-77700 Magny-le-hongre (FR)**
- **DAUBRESSE, Nicolas**
**F-78170 La Celle Saint Cloud (FR)**

(74) Representative: **Wattremez, Catherine**
**L'Oréal**
**Service DIPI**
**9 Rue Pierre Dreyfus**
**92110 Clichy (FR)**

(56) References cited:
**EP-A1- 1 647 580** **EP-A2- 0 279 461**
**WO-A2-03/006491** **WO-A2-2008/018894**
**JP-A- 2008 156 449** **US-A- 6 013 663**
**US-A1- 2004 053 222** **US-A1- 2005 142 068**
**US-A1- 2006 030 699**

- **TOMASULO MASSIMILIANO ET AL: "Luminescence Modulation with Semiconductor Quantum Dots and Photochromic Ligands", AUSTRALIAN JOURNAL OF CHEMISTRY, CSIRO, AU, vol. 59, no. 3, 1 January 2006 (2006-01-01), pages 175-178, XP009137463, ISSN: 0004-9425 cited in the application**
- **"Fluoreszenz" In: Dr. Elisabeth Hillen: "Römpp Chemie Lexikon, 9. Auflage", 1995, Georg Thieme Verlag, Stuttgart, XP002596512, vol. 2, pages 1403-1405, page 1403 - page 1404**
- **J.W. BRIDGES & R.T. WILLIAMS: "The Fluorescence of Indoles and Anilines Derivatives", BIOCHEMICAL JOURNAL, vol. 107, no. 2 March 1968 (1968-03), pages 225-237, XP002596513, Great Britain Retrieved from the Internet: URL:http://www.ncbi.nlm.nih.gov/pmc/articles/PMC1198649/pdf/biochemj00730-0098.pdf [retrieved on 2010-08-13]**
- **"Chromophore" In: Dr. Elisabeth Hillen: "Römpp Chemie Lexikon, 9. Auflage", 1995, Georg Thieme Verlag, Stuttgart, XP002686023, pages 727-727, the whole document**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 498 748 B1

- KOUFAKI M ET AL: "Design and synthesis of novel neuroprotective 1,2-dithiolane/chroman hybrids", BIOORGANIC & MEDICINAL CHEMISTRY, PERGAMON, GB, vol. 17, no. 17, 1 September 2009 (2009-09-01), pages 6432-6441, XP026471124, ISSN: 0968-0896, DOI: 10.1016/J.BMC.2009.07.010 [retrieved on 2009-07-09]
- Tobias Weidner, Frauke Bretthauer, Nirmalya Ballav, Hubert Motschmann, Horst Orendi, Clemens Bruhn,Ulrich Siemerling, M. Zharnikov: "Correlation between the Molecular Structure and Photoresponse in Aliphatic Self-Assembled Monolayers with Azobenzene Tailgroups", Langmuir, vol. 24 27 September 2008 (2008-09-27), pages 11691-11700, XP055042180, DOI: 10.1021/la802454w Retrieved from the Internet: URL:http://pubs.acs.org/doi/abs/10.1021/la 802454w [retrieved on 2012-10-24]
- Ibrahim Yildiz, Shuvasree Ray, Tiziana Benelli and Francisco M. Raymo: "Dithiolane ligands for semiconductor quantum dots", Journal of Materials Chemistry, vol. 18 14 July 2008 (2008-07-14), pages 3940-3947, XP055042181, DOI: 10.1039/b806247a Retrieved from the Internet: URL:http://pubs.rsc.org/en/Content/Article Landing/2008/JM/b806247a [retrieved on 2012-10-24]
- U. Siemeling, C.Bruhn, F. Bretthauer, M. Borg, F. Träger, F. Vogel, W. Azzam, M. Badin, T. Strunskus, C. Wölld: "Photoresponsive SAMs on gold fabricated from azobenzene-functionalisedasparagusic acid derivatives", RSC Dalton Transactions, 18 August 2009 (2009-08-18), pages 8593-8604, XP055042182, DOI: 10.1039/b905025f Retrieved from the Internet: URL:http://pubs.rsc.org/en/Content/Article Landing/2009/DT/b905025f [retrieved on 2012-10-24]
- Isao Takahashi, Yuichiro Honda, and Shun Hirota: "Regulating Copper-Binding Affinity with PhotoisomerizableAzobenzene Ligand by Construction of a Self-Assembled Monolayer", Angewandte Chemie Int. Ed., vol. 48 3 August 2009 (2009-08-03), pages 6065-6068, XP055042183, DOI: 10.1002/anie.200902048 Retrieved from the Internet: URL:http://onlinelibrary.wiley.com/doi/10. 1002/anie.200902048/abstract [retrieved on 2012-10-24]
- JAN MARTEN ET AL: "Self-Assembled Layers Based on Isomerizable Stilbene and Diketoarylhydrazone Moieties", LANGMUIR, vol. 24, no. 6, 1 March 2008 (2008-03-01), pages 2479-2486, XP55042316, ISSN: 0743-7463, DOI: 10.1021/la703109x
- KOUFAKI ET AL: "Design and synthesis of 1,2-dithiolane derivatives and evaluation of their neuroprotective activity", BIOORGANIC & MEDICINAL CHEMISTRY LETTERS, PERGAMON, ELSEVIER SCIENCE, GB, vol. 17, no. 15, 1 August 2007 (2007-08-01), pages 4223-4227, XP022144678, ISSN: 0960-894X, DOI: 10.1016/J.BMCL.2007.05.036
- Yasuhiko Tada, Tomoyuki Morita, Junzo Umemura, Mitsumasa Iwamoto, Shunsaku Kimura: "Photoresponsive Change of the Surface Potential Generated by Helical Peptide Self-Assembled Monolayers", Polymer Journal, vol. 37, no. 8 2005, pages 599-607, XP002686024, DOI: 10.1295/polymj.37.599 Retrieved from the Internet: URL:https://www.jstage.jst.go.jp/article/p olymj/37/8/37_8_599/_article [retrieved on 2005-10-26]
- FIONA MCKENZIE ET AL: "SERRS coded nanoparticles for biomolecular labelling with wavelength-tunable discrimination", THE ANALYST, vol. 134, no. 3, 1 January 2009 (2009-01-01), page 549, XP55042335, ISSN: 0003-2654, DOI: 10.1039/b813821d
- SIEMELING U ET AL: "COOH-terminated SAMs on gold fabricated from an azobenzene derivative with a 1,2-dithiolane headgroup", APPLIED SURFACE SCIENCE, ELSEVIER, AMSTERDAM, NL, vol. 256, no. 6, 14 October 2009 (2009-10-14), pages 1832-1836, XP026820516, ISSN: 0169-4332 [retrieved on 2009-10-14]

## Description

The invention relates to the dyeing of keratinous materials using dyes especially fluorescent, having a heterocyclic disulphide group, of formula **(I).**

The colouring of keratinous fibres, especially human keratinous fibres, by direct dyeing is known. The direct dyeing method conventionally used involves applying, to the keratinous fibres, direct dyes, which are coloured and colouring molecules with an affinity for the fibres, allowing them to diffuse, and then rinsing the fibres.

The direct dyes which are conventionally used are, for example, nitrobenzene dyes, anthraquinonoid dyes, nitropyridine dyes, and azo, xanthene, acridine, azine or triarylmethane dyes.

Colorations which result from the use of direct dyes are temporary or semipermanent colorations because the nature of the interactions which bind the direct dyes to the keratin fibre, and their desorption from the surface and/or from the core of the fibre are responsible for their weak dyeing power and their poor fastness to washing and to perspiration.

To increase the fastness of direct dyes, it is known to fix the direct dyes *via* covalent bonding to the hair. For example, it is known to cause dyes with reactive groups to react with the cystine or cysteine residues which are present in very large numbers in the hair fibres. Also described are some dyes bearing Bunte salt and isothiouronium functional groups, or other thiol-protecting groups. However, the production of the reactive form of the dye generally requires the use of strongly basic media. Furthermore, the thiol functional groups are generally generated in excess, which makes a post-neutralizing step following the dyeing necessary.

Other disulphide dyes known for dyeing keratin fibres are disulphide derivatives of aminothiophenol derivatives. Such dyes are described for example in the French Patent FR 1 156 407. These dyes may be used under relatively mild conditions, in the presence of a slightly reducing medium or after a reducing pretreatment of the hair. However, these dyes can cause colour variations during application.

Other documents WO 2005/097051 or EP 1647580 describe aza-imidazolium disulphide dyes for the direct dyeing of keratin fibres.

Nevertheless, for effective fixing of these disulphide dyes on the surface of the keratinous fibres, it is generally necessary at the same time, before or after the application of disulphide dyes, to carry out treatment with a reducing agent. In general the use of disulfide direct dyes provides a foul-smelling or unpleasant odour especially in the presence of reducing agent.

Therefore, one of the aims of the present invention is to provide direct dyeing systems which make it possible to obtain chromatic, and vivid (intense) colorations which are very fast, in particular towards successive shampooings without an unpleasant odour..

On the other hand, dyeing keratinous fibres on the basis of these conventional direct dyes do not produce significant lightening of the keratinous fibres.

Lightening the colour of dark keratinous fibres towards lighter shades, by possibly modifying the shade of the fibres, is a substantial requirement.

Conventionally, in order to obtain a lighter coloration, a process of chemical bleaching is employed. This process involves treating the keratinous fibres, such as the hair, with a strong oxidizing system, generally composed of hydrogen peroxide alone or in combination with persalts, usually in an alkaline medium.
This bleaching system has the drawback of degrading the keratinous fibres and impairing their cosmetic properties. Indeed, the fibres have a tendency to become rough, less easy to disentangle, and weaker. Lastly, the lightening or bleaching of keratinous fibres with oxidizing agents is incompatible with treatments to modify the shape of said fibres, particularly in hair straightening treatments.

Another technique for lightening involves applying fluorescent direct dyes to dark hair. This technique, described in particular in documents WO 03/028685 and WO 2004/091473, respects the quality of the keratinous fibre in the course of the treatment. These fluorescent direct dyes, however, do not possess satisfactory tenacity with regard to extrinsic agents.

Known for the purpose of enhancing remanence is the use of dyes containing fluorescent chromophores which are joined to one another by a spacer or linker which contains a disulphide function (see, for example, WO 2005/097051, EP 1647580, WO 2006/134043, WO 2006/136617, WO 2007/110533, WO 2007/110534, WO 2007/110535, WO 2007/110537, WO 2007/110542). Nevertheless, for effective fixing of these disulphide dyes on the surface of the keratinous fibres, it is generally necessary at the same time, before or after the application of disulphide dyes, to carry out treatment with a reducing agent. The use of significant quantities of reducing agents may have the drawback of giving off an unpleasant odour during the treatment of the keratinous fibres, such that there is a real need to reduce the level of reducing agent. Moreover, it is generally necessary to carry out an additional oxidative treatment using a chemical oxidizing agent in order to fix the dyes on the keratinous fibres and thus to enhance the tenacity of the coloration.

Furthermore, fluorescent compounds comprising a disulphide heterocycle **(A), (B)** and **(C)** (see formulae hereinafter) have been used as ligands, modifiers of the luminescence of quantum dots semiconductors, and for evaluating the energy transfer mechanism with the aim of "switching" the luminescence of said semiconductors - Australian Journal

of Chemistry, 2006, 59(3), 175-178; WO 2008/18894, Journal of Materials Chemistry, 2008, 18(46), 5577-5584. Compound (D) is disclosed as acid 7 in Analyst, 2009, 134, 549-556. These fluorescent compounds have not been used for cosmetics purposes.

The aim of the present invention is to provide new systems for lightening and dyeing keratinous materials, especially dark keratinous materials and more particularly human keratinous fibres, such as the hair, which are powerful and durable and which do not automatically necessitate a reducing treatment on the fibres.

In particular, one aim of the invention is to provide direct dyeing systems that product lightening effects, especially on naturally or artificially dark keratinous fibres, that are tenacious on exposure to successive shampooings, which do not degrade the keratinous fibres and which do not impair their cosmetic properties.

Another aim of the invention is to dye the keratinous materials in a way which is chromatic and is remanent towards extrinsic exposures. The invention also aims to provide compounds which dye the keratinous fibres, such as the hair, with a low dyeing selectivity between root and end, whether on natural fibres or on permed fibres.

These aims are achieved with the present invention, which provides a method for dyeing and/or lightening keratinous materials, particularly dark keratinous fibres, wherein said materials have applied to them an appropriate colouring composition comprising one or more dyes, especially fluorescent dyes, containing a heterocyclic disulphide group, of formula **(I)** below:

$$\mathbf{A\text{-}(X)_p\text{-}C_{sat}\text{-}(X')_{p'}\text{-}Het} \qquad \mathbf{(I)}$$

organic or inorganic acid salts, optical isomers and geometric isomers thereof, and solvates thereof such as hydrates; in which formula **(I):**

- **A** represents a radical containing at least one cationic or non-cationic chromophore especially the said chromophore is fluorescent ;
- **Het** represents a heterocyclic disulphide radical such as that of formula **(II)** which is connected to the rest of the molecule by one of the substituents $R_5$, $R_6$, $R_7$, $R_8$, $R_9$ or $R_{10}$, or said radical is connected to the rest of the molecule directly by one of the carbon atoms of the heterocyclic radical in alpha or beta position or, when q is 2 or 3, in gamma position, in which case one of the substituents $R_5$, $R_6$, $R_7$, $R_8$, $R_9$ or $R_{10}$ is absent:

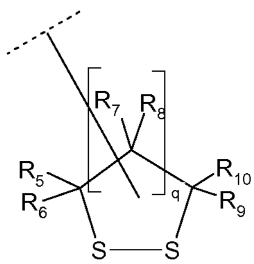

**(II)**

in which formula **(II):**

- **$R_5$, $R_6$, $R_9$** and **$R_{10}$,** which are identical or different, represent a hydrogen atom or a group selected from: i) ($C_1$-$C_8$)alkyl, ii) aryl, iii) hydroxyl, iv) (di)($C_1$-$C_8$)(alkyl)amino, v) ($C_1$-$C_8$)alkoxy, vi) (poly)hydroxy($C_1$-$C_8$)alkyl, vii) (di)($C_1$-$C_8$)(alkyl)amino($C_1$-$C_8$)alkyl, viii) carboxyl, ix) carboxy($C_1$-$C_3$)alkyl, x) (di)($C_1$-$C_8$)(alkyl)aminocarbonyl($C_1$-$C_8$)alkyl, and xii) ($C_1$-$C_8$)(alkyl)carbonyl($C_1$-$C_8$)(alkyl)amino($C_1$-$C_8$)alkyl;
- **$R_7$** and **$R_8$,** which are identical or different, represent a hydrogen atom or a group selected from: i) ($C_1$-$C_8$)alkyl, ii) aryl, iii) hydroxyl, iv) (di)($C_1$-$C_8$)(alkyl)amino, v) ($C_1$-$C_8$)alkoxy, vi) (poly)hydroxy($C_1$-$C_8$)alkyl, vii) (di)($C_1$-$C_8$)(alkyl)amino($C_1$-$C_8$)alkyl, viii) carboxyl, ix) carboxy($C_1$-$C_8$)alkyl, x) (di)($C_1$-$C_8$)(alkyl)aminocarbonyl($C_1$-$C_8$)alkyl, and xii) ($C_1$-$C_8$)(alkyl)-carbonyl($C_1$-$C_8$)(alkyl)amino($C_1$-$C_8$)alkyl; particularly, **$R_7$** and **$R_8$** are selected from a hydrogen atom and a ($C_1$-$C_4$) alkyl group such as methyl;
- **q** represents an integer between 1 and 3 inclusive, particularly between 1 and 2;

with the proviso that when **q** is 2 or 3, the groups **$R_7$** and **$R_8$** may be identical to or different from one another; preferably q is 1; and more particularly all of the substituents $R_1$ to $R_6$ represent a hydrogen atom;

- **X** and **X'**, which are identical or different, represent:

◦ a saturated or unsaturated, linear or branched $C_1$-$C_{30}$ hydrocarbon chain which is optionally interrupted and/or optionally terminated at one or both of its ends by one or more divalent groups or combinations thereof selected from:

- -N(R)-; -N$^+$(R)(R')-, Q$^-$; -O-; -S-; -C(O)-; -S(O)$_2$-, where R and R', which are identical or different, are selected from a hydrogen atom and a $C_1$-$C_4$ alkyl, hydroxyalkyl and aminoalkyl radical, and Q$^-$ represents an anionic counterion;
- a condensed or non-condensed, saturated or unsaturated, aromatic or non-aromatic (hetero)cyclic radical optionally comprising one or more identical or non-identical heteroatoms and optionally substituted;

◦ a divalent group or combination thereof selected from: -N(R)-; -N$^+$(R)(R')-, Q$^-$; -O-; -S-; -C(O)-; -S(O)$_2$-, where R, R' and Q$^-$ are as defined above;
preferably the divalent group or groups or combinations thereof are selected from -O-; -N(R)-; -C(O)-, where R is selected from a hydrogen atom and a $C_1$-$C_4$ alkyl radical;

➢ **p** and **p'**, which are identical or different, represent an integer 0 or 1; and
➢ $\mathbf{C_{sat}}$ represents an optionally cyclic, optionally substituted, linear or branched C1-C18 alkylene chain.

Another subject of the invention relates to dyes containing a heterocyclic disulphide group, of formula **(I),** especially to fluorescent dyes containing a heterocyclic disulphide group of formula **(I),** as defined above, where **Het** represents a heterocyclic disulphide radical of formula **(II)** as defined above, the radical **A** of the formula (I) contains at least one cationic radical which is carried by or included in at least one of the chromophores and with the provisos that:

- the compound of formula **(I)** cannot comprise fluorescent riboflavin-5-monophosphate chromophore **(A);**
- the compound of formula **(I)** cannot comprise fluorescent chromophore **(A)** which represents an indol-3-yl or 5-methoxyindol-3-yl group; and
- the compound of formula **(I)** cannot represent the compounds **(A), (B), (C)** or **(D)** :

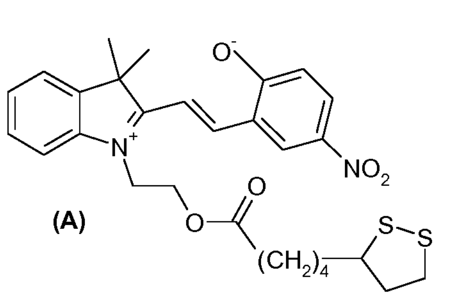

**(A)**

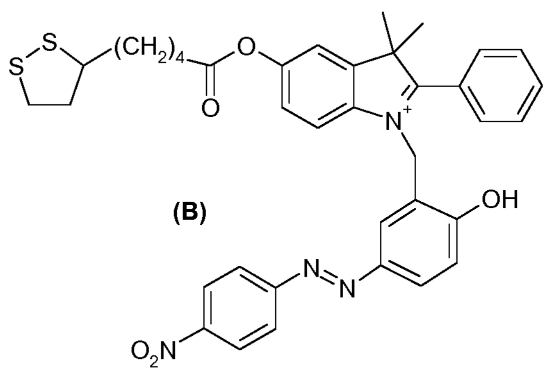

**(B)**

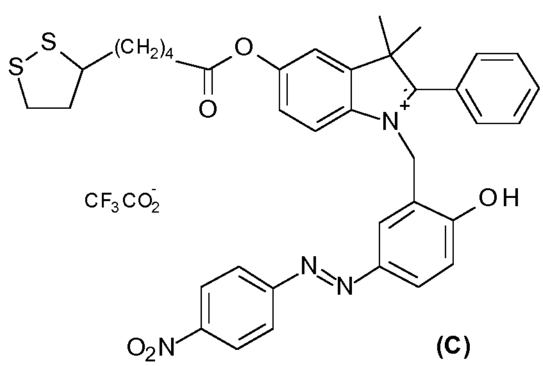

**(C)**

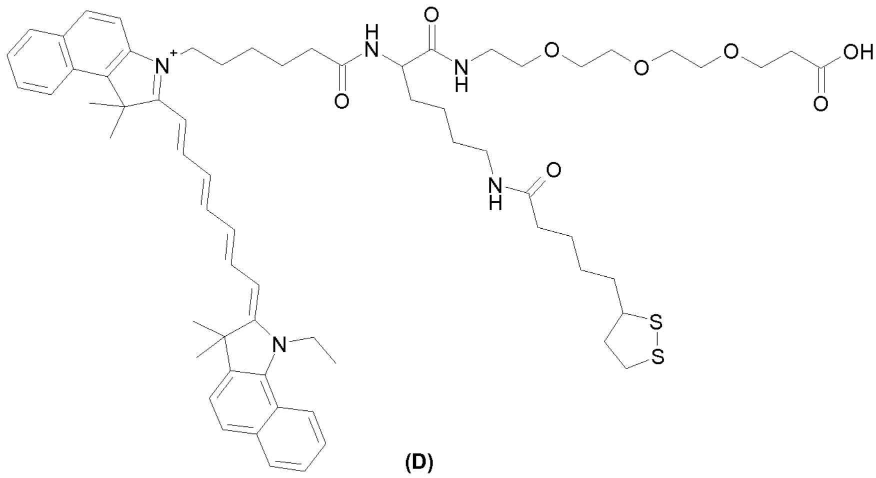

(D)

where **(B)** is combined or not combined with a CdSe-ZnS, CdTe-ZnS, CdSe-ZnSe or CdTe-ZnSe core-shell quantum dot; **(C)** is combined or not combined with a CdSe-ZnS core-shell quantum dot..

Another subject of the invention is a colouring composition comprising, in an appropriate cosmetic medium, at least one dye containing a heterocyclic disulphide group of formula **(I),** especially at least one fluorescent dye containing a heterocyclic disulphide group of formula **(I),** where **Het** represents a heterocyclic disulphide radical of formula **(II)** as defined above, the radical **A** of the formula (I) contains at least one cationic radical which is carried by or included in at least one of the chromophores and with the provisos that:

- the compound of formula **(I)** cannot comprise fluorescent riboflavin-5-monophosphate chromophore **(A);**
- the compound of formula **(I)** cannot comprise fluorescent chromophore **(A)** which represents an indol-3-yl or 5-methoxyindol-3-yl group; and
- the compound of formula **(I)** cannot represent the compounds **(A), (B), (C)** or **(D)** as defined above where **(B)** is combined or not combined with a CdSe-ZnS, CdTe-ZnS, CdSe-ZnSe or CdTe-ZnSe core-shell quantum dot; **(C)** is combined or not combined with a CdSe-ZnS core-shell quantum dot.

The method of the invention produces a visible colouring on keratinous materials, even dark keratinous materials, especially human keratinous fibres, particularly the hair, without degrading said material, the colouring being remanent towards shampooings, towards common exposures (sun, perspiration) and towards other hair treatments, even in the absence of reducing agent. The method of the invention also produces a lightening of the keratinous materials such as the keratinous fibres, particularly dark keratinous fibres, and more particularly dark hair.

Furthermore, the dyes of the invention are stable towards oxidizing agents, and have satisfactory solubility in cosmetic colouring media. These dyes extend the colouristic range to yellows and oranges, to red, violet and blue. Following application to keratinous fibres, the dyes of formula **(I)** dye the keratinous materials in a manner which is chromatic and remanent towards extrinsic exposures, with a low dyeing selectivity between root and end, and on different types of fibres.

For the purposes of the invention, a dark keratinous material is one which has a lightness L* with a number in the C.I.E. L*a*b* system of less than or equal to 45 and preferably less than or equal to 40, where L*=0 is equivalent to black and L*=100 to white.

For the purposes of the invention, naturally or artificially dark hair is hair whose tone level is less than or equal to 6 (dark blond) and preferably less than or equal to 4 (chestnut-brown).

The lightening of the hair is evaluated by the change in "tone level" before and after application of the compound of formula **(I).** The "tone" concept is based on the classification of natural shades, with one tone separating each shade from its immediate precursor or successor. This definition and the classification of the natural shades are well known to haircare professionals, and are published in "Science des traitements capillaires" by Charles ZVIAK, 1988, published by Masson, pp. 215 and 278.

The tone levels range from 1 (black) to 10 (very light blonde), with one unit corresponding to one tone; the higher the number, the lighter the shade.

For the purposes of the invention, bleached hair is hair whose tone level is greater than 4 (chestnut-brown)

and preferably greater than 6 (dark blonde).

One way of measuring the lightening effect imparted to the hair after application of the fluorescent dyes of the invention is to utilize the phenomenon of the hair's reflectance which is made as below:

- Interest is taken in the reflectance performance levels of the hair when it is irradiated with visible light in the wavelength range from 400 to 700 nanometres.
- The curves of reflectance as a function of wavelength for hair treated with the composition of the invention and for untreated hair are then compared.
- The curve corresponding to the treated hair should show a reflectance in the wavelength range from 500 to 700 nanometres which is higher than the curve corresponding to the untreated hair.
- This means that, in the wavelength range from 500 to 700 nanometres, there is at least one section where the reflectance curve corresponding to the treated hair is higher than the reflectance curve corresponding to the untreated hair. The term "higher" is intended to mean a difference of at least 0.05% in reflectance, preferably of at least 0.1%. All the same, there may be, in the wavelength range from 540 to 700 nanometres, at least one section where the reflectance curve corresponding to the treated hair is superimposable on or lower than the reflectance curve corresponding to the untreated hair.

Preferably, the wavelength where the difference is at a maximum between the reflectance curve of the treated hair and that of the untreated hair is within the wavelength range from 500 to 650 nanometres, and preferably within the wavelength range from 550 to 620 nanometres.

For the purposes of the present invention, and unless otherwise indicated :

- the "aryl" or "heteroaryl" radicals or the aryl or heteroaryl moiety of a radical may be substituted by at least one substituent carried by a carbon atom, selected from:

  - a $C_1$-$C_{16}$, preferably $C_1$-$C_8$, alkyl radical optionally substituted by one or more radicals selected from the radicals hydroxyl, $C_1$-$C_2$ alkoxy, $C_2$-$C_4$(poly)hydroxyalkoxy, acylamino and amino substituted with two identical or different $C_1$-$C_4$ alkyl radicals, which optionally carry at least one hydroxyl group, or the two radicals possibly forming, with the nitrogen atom to which they are attached, a heterocycle comprising from 5 to 7 members, preferably 5 or 6 members, which is saturated or unsaturated, which is optionally substituted, and which optionally comprises another heteroatom, which may be identical to or different from nitrogen;
  - a halogen atom such as chlorine, fluorine or bromine;
  - a hydroxyl group;
  - $C_1$-$C_2$ alkoxy radical;
  - a $C_2$-$C_4$ (poly)hydroxyalkoxy radical;
  - an amino radical;
  - a 5- or 6-membered heterocycloalkyl radical;
  - an optionally cationic 5- or 6-membered heteroaryl radical, preferably imidazolium, optionally substituted by a $C_1$-$C_4$ alkyl radical, preferably methyl;
  - an amino radical substituted by one or two identical or different $C_1$-$C_6$ alkyl radicals, which carry optionally at least:

    i) one hydroxyl group,
    ii) one amino group optionally substituted by one or two optionally substituted $C_1$-$C_3$ alkyl radicals, said alkyl radicals possibly forming, with the nitrogen atom to which they are attached, a heterocycle comprising from 5 to 7 members, which is saturated or unsaturated, which is optionally substituted, and which optionally comprises at least one other heteroatom, which may or may not be different from nitrogen,
    iii) one quaternary ammonium group $-N^+R'R''R'''$, $M^-$, for which R', R" and R"', which are identical or different, represent a hydrogen atom or a $C_1$-$C_4$ alkyl group; and $M^-$ represents the counterion of the organic or inorganic acid or of the corresponding halide,
    iv) or one 5- or 6-membered heteroaryl radical which is optionally cationic, preferably imidazolium, and is optionally substituted by a $C_1$-$C_4$ alkyl radical, preferably methyl;

  - an acylamino radical (-N(R)-C(O)R') in which the radical R is a hydrogen atom or a $C_1$-$C_4$ alkyl radical which optionally carries at least one hydroxyl group, and the radical R' is a $C_1$-$C_2$ alkyl radical;
  - a carbamoyl radical ($(R)_2N$-C(O)-) in which the radicals R, which may be identical or not, represent a hydrogen atom or a $C_1$-$C_4$ alkyl radical which optionally carries at least one hydroxyl group;
  - a carboxylic acid or ester radical, (-O-C(O)R') or (-C(O)OR'), in which the radical R' is a hydrogen atom or a $C_1$-$C_4$ alkyl radical which optionally carries at least one hydroxyl group, and more particularly radical R' is a

$C_1$-$C_2$ alkyl radical;

- the carboxylic radical may be in acid form or salified form (preferably with an alkali metal or an ammonium which is substituted or unsubstituted);
- an alkylsulphonylamino radical (R'S(O)$_2$-N(R)-) in which the radical R represents a hydrogen atom or a $C_1$-$C_4$ alkyl radical which optionally carries at least one hydroxyl group, and the radical R' represents a $C_1$-$C_4$ alkyl radical or a phenyl radical;
- an aminosulphonyl radical ((R)$_2$N-S(O)$_2$-) in which the radicals R, which may be identical or not, represent a hydrogen atom or a $C_1$-$C_4$ alkyl radical which optionally carries at least one hydroxyl group;
- a cyano group (CN);
- a polyhaloalkyl group, preferably trifluoromethyl ($CF_3$);

- the cyclic or heterocyclic moiety of a non-aromatic radical may be substituted by at least one substituent carried by a carbon atom and selected from the following groups:

  - hydroxyl,
  - $C_1$-$C_4$ alkoxy, $C_2$-$C_4$(poly)hydroxyalkoxy,
  - alkylcarbonylamino (RC(O)-N(R')-) in which the radical R' is a hydrogen atom or a $C_1$-$C_4$ alkyl radical which optionally carries at least one hydroxyl group, and the radical R is a $C_1$-$C_2$ alkyl radical or amino radical which is substituted by two identical or different $C_1$-$C_4$ alkyl groups which optionally carry at least one hydroxyl group, said alkyl radicals possibly forming, with the nitrogen atom to which they are attached, a heterocycle comprising from 5 to 7 members which is saturated or unsaturated, which is optionally substituted, and which optionally comprises at least one other heteroatom, which may or may not be different from nitrogen;
  - alkylcarbonyloxy (RC(O)-O-) in which the radical R is a $C_1$-$C_4$ alkyl radical, amino substituted by two identical or different $C_1$-$C_4$ alkyl groups which optionally carry at least one hydroxyl group, said alkyl radicals possibly forming, with the nitrogen atom to which they are attached, a heterocycle comprising from 5 to 7 members which is saturated or unsaturated, which is optionally substituted, and which optionally comprises at least one other heteroatom, which may or may not be different from nitrogen;
  - alkoxycarbonyl (RO-C(O)-) in which the radical R is a $C_1$-$C_4$ alkyl radical, amino substituted by two identical or different $C_1$-$C_4$ alkyl groups which optionally carry at least one hydroxyl group, said alkyl radicals possibly forming, with the nitrogen atom to which they are attached, a heterocycle comprising from 5 to 7 members which is saturated or unsaturated, which is optionally substituted, and which optionally comprises at least one other heteroatom, which may or may not be different from nitrogen;

- a cyclic or heterocyclic radical, or a non-aromatic moiety of an aryl or heteroaryl radical, may also be substituted by one or more oxo groups;

- a hydrocarbon chain is unsaturated when it comprises one or more double bonds and/or one or more triple bonds;

- an "aryl" radical represents a condensed or non-condensed polycyclic or monocyclic group comprising from 6 to 22 carbon atoms, in which at least one ring is aromatic; the aryl radical is preferably a phenyl, biphenyl, naphthyl, indenyl, anthracenyl or tetrahydronaphthyl;

- a "heteroaryl radical" represents a condensed or non-condensed polycyclic or monocyclic group which is optionally cationic and comprises from 5 to 22 members, from 1 to 6 heteroatoms selected from nitrogen, oxygen, sulphur and selenium, and in which at least one ring is aromatic; a heteroaryl radical is preferably selected from acridinyl, benzimidazolyl, benzobistriazolyl, benzopyrazolyl, benzopyridazinyl, benzoquinolyl, benzothiazolyl, benzotriazolyl, benzoxazolyl, pyridinyl, tetrazolyl, dihydrothiazolyl, imidazopyridinyl, imidazolyl, indolyl, isoquinolyl, naphthoimidazolyl, naphthooxazolyl, naphthopyrazolyl, oxadiazolyl, oxazolyl, oxazolopyridyl, phenazinyl, phenooxazolyl, pyrazinyl, pyrazolyl, pyrilyl, pyrazoyltriazyl, pyridyl, pyridinoimidazolyl, pyrrolyl, quinolyl, tetrazolyl, thiadiazolyl, thiazolyl, thiazolopyridinyl, thiazoylimidazolyl, thiopyrylyl, triazolyl, xanthylyl and its ammonium salt;

- a "cationic heteroaryl radical" represents a "heteroaryl radical" as defined herein before which bears an endocyclic or exocyclic cationic group :

  ◦ when the cationic charge is endocyclic said charge is electronically delocalised by mesomeric effect for eg. such group is selected from pyridinium, imidazolium and indolinium :

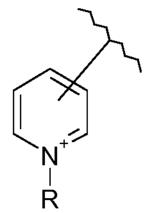 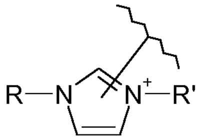 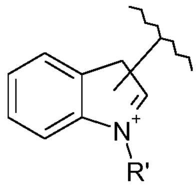

wherein R and R' being a usual substituent of heteroaryl radical as defined herein before for substituted heteroaryl group such as (hydroxy)($C_1$-$C_8$)alkyle especially a methyl group ;

◦ when the cationic charge is exocyclic, said charge is not electronically delocalised by mesomeric effect, for eg. It is an ammonium or phosphonium substituant $R^+$, such as trimethylammonium, said charge being outside of the heteroaryl group such as pyridine, indole, imidazole :

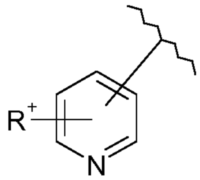 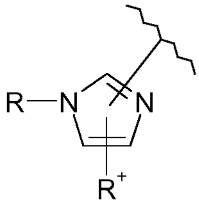 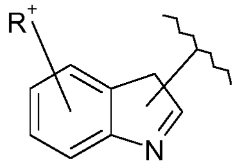

wherein R is a substituant of heteroaryl radical as defined herein before and $R^+$ represents an ammonium $R_aR_bR_cN^+$- or phosphonium $R_aR_bR_cP^+$- group wherein $R_a$, $R_b$ and $R_c$ which are identical or different, representing and hydrogen atom or an ($C_1$-$C_8$)alkyl group such as methyl;

- a "cationic aryl group with exocyclic charge" is an aryl radical in which the cationic group is outside of said aryl, for eg. it an ammonium or phosphonium $R^+$ such as trimethylammonium, in which the cationic charge is outside of the phenyl or naphtyl group :

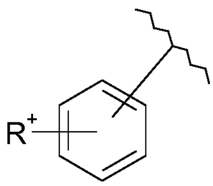 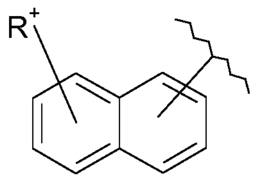 ;

wherein $R^+$ is as defined herein before;

- a "cyclic radical" is a condensed or non-condensed polycyclic or monocyclic, non-aromatic cycloalkyl radical which contains from 5 to 22 carbon atoms and may comprise from 1 to a plurality of unsaturations;

- a "heterocyclic radical" is a condensed or non-condensed polycyclic or monocyclic, non-aromatic radical which contains from 5 to 22 members and comprises from 1 to 6 heteroatoms selected from nitrogen, oxygen, sulphur and selenium, for example morpholinyl, thiomorpholinyl, piperidinyl, piperazinyl, pyrrolidinyl, tetrahydrofuranyl, tetrahydrothiophenyl, azepanyl, thioazepanyl; preferably pyrrolidinyl and morpholino;

- a "heterocyclic disulphide radical" is a heterocyclic radical comprising in its ring, i.e. in the same ring and not two different rings, a disulphide sequence -S-S-between two carbon atoms, it being possible for said heterocycle to be substituted, and said heterocycle preferably not comprising an amido sequence in the ring ; the heterocyclic radical may be illustrated by the formula below:

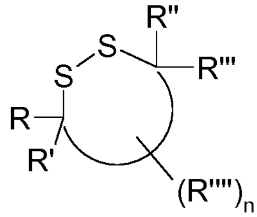

where **R, R', R"** and **R"'**, which are identical or different, represent substituents as defined for $\mathbf{R_5}$, $\mathbf{R_6}$, $\mathbf{R_9}$ and $\mathbf{R_{10}}$ above, **n** representing an integer between 1 and 4, and **R""**, identical or different at each occurrence, represents a substituent as defined for $\mathbf{R_7}$ and $\mathbf{R_8}$ above;

- an "alkyl radical" is a linear or branched $C_1$-$C_{16}$ hydrocarbon radical, preferably $C_1$-$C_8$;

- the expression "optionally substituted" attributed to the alkyl radical implies that said alkyl radical may be substituted by one or more radicals selected from the following radicals: i) hydroxyl, ii) $C_1$-$C_4$ alkoxy, iii) acylamino, iv) amino which is optionally substituted by one or two identical or different $C_1$-$C_4$ alkyl radicals, it being possible for said alkyl radicals to form, with the nitrogen atom which carries them, a heterocycle comprising from 5 to 7 members, optionally comprising another heteroatom, which may or may not be different from nitrogen; v) or a quaternary ammonium group $-N^+R'R''R'''$, $M^-$, for which R', R" and R"', which are identical or different, represent a hydrogen atom or a $C_1$-$C_4$ alkyl group, or $-N^+R'R''R'''$ forms a heteroaryl such as imidazolium which is optionally substituted by a $C_1$-$C_4$ alkyl group, and $M^-$ represents the counterion of the organic or inorganic acid or of the corresponding halide;

- an "alkoxy radical" is an alkyl-oxy radical for which the alkyl radical is a $C_1$-$C_{16}$, preferably $C_1$-$C_8$, linear or branched hydrocarbon radical; when the alkoxy group is optionally substituted, this implies that the alkyl group is optionally substituted as defined above.

The compounds of formula **(I)** according to the invention may be anionic, cationic or neutral;

- when the compounds are anionic or cationic, a counterion, two or more counterions or a mixture of counterions is or are present in order to provide electroneutrality, or one or more groups in the compound provide electroneutrality, for example, if the rest of the compound is cationic, by means of $-O^-$ (oxalate), $-COO^-$ (carboxylate), $R_3N^+$- (ammonium), or $R_3P^+$- (phosphonium) groups;

- when the compound is anionic, the counterion or counterions are cationic, preferably selected from alkali metal cations or alkaline earth metal cations such as Na, Mg, K and Ca, and organic cations such as ammonium $NH_4^+$ or (di/tri)alkylammonium; when the compound is cationic, the counterion or counterions are anionic and are preferably selected from i) halides such as chloride and bromide, ii) nitrates; iii) sulphonates, including $C_1$-$C_6$ alkylsulphonates: $Alk-S(O)_2O^-$ such as methylsulphonate or mesylate and ethylsulphonate; iv) arylsulphonates: $Ar-S(O)_2O^-$ such as benzenesulphonate and toluenesulphonate or tosylate; v) citrate; vi) succinate; vii) tartrate; viii) lactate; ix) alkylsulphites: $Alk-O-S(O)O^-$ such as methylsulphite and ethylsulphite; x) arylsulphites: $Ar-O-S(O)O^-$ such as benzenesulphite and toluenesulphite; xi) alkylsulphates: $Alk-O-S(O)_2O^-$ such as methylsulphate and ethylsulphate; xii) arylsulphates: $Ar-O-S(O)_2O^-$, xiii) phosphate; xiv) acetate; xv) triflate; and xvi) borates such as tetrafluoroborate;

- a "salt of organic or inorganic acid" is more particularly selected from a salt derived from i) hydrochloric acid HCl, ii) hydrobromic acid HBr, iii) sulphuric acid $H_2SO_4$, iv) alkylsulphonic acids: $Alk-S(O)_2OH$ such as methylsulphonic acid and ethylsulphonic acid; v) arylsulphonic acids: $Ar-S(O)_2OH$ such as benzenesulphonic acid and toluenesulphonic acid; vi) citric acid; vii) succinic acid; viii) tartaric acid; ix) lactic acid, x) alkoxysulphinic acids: Alk-O-S(O)OH such as methoxysulphinic acid and ethoxysulphinic acid; xi) aryloxysulphinic acid such as tolueneoxysulphinic acid and phenoxysulphinic acid; xii) phosphoric acid $H_3PO_4$; xiii) acetic acid $CH_3COOH$; xiv) triflic acid $CF_3SO_3H$; and xv) tetrafluoroboric acid $HBF_4$.

Moreover, unless indicated otherwise, the end points delimiting the extent of a range of values are included in that range of values.

According to the present invention, a *"chromophore"* is a radical resulting from a dye i.e. which is coloured and its colour is visible with naked eyes. Another words a dye is a compound which is capable of absorption in UV and visible, or in visible radiation at a wavelength $\lambda_{abs}$ of between 250 and 800 nm. Preferably, the chromophore are preferably capable of absorbing in the visible region at a $\lambda_{abs}$ of between 400 and 800 nm. More preferably chromophore derives

from dye capable of absorbing at a $\lambda_{abs}$ of between 420 nm and 550 nm.

According to the present invention, a *"fluorescent chromophore"* is a radical resulting from a fluorescent dye. A fluorescent dye is a compound which is capable of absorption in UV and visible radiation or in visible radiation at a wavelength $\lambda_{abs}$ of between 250 and 800 nm, and capable of re-emission in the visible region at an emission wavelength $\lambda_{em}$ of between 400 and 800 nm. Preferably, the fluorescent compounds are preferably dyes capable of absorbing in the visible region at a $\lambda_{abs}$ of between 400 and 800 nm and of re-emitting in the visible region at a $\lambda_{em}$ of between 400 and 800 nm. More preferably the fluorescent dyes are dyes capable of absorbing at a $\lambda_{abs}$ of between 420 nm and 550 nm and of re-emitting in the visible region at a $\lambda_{em}$ of between 470 and 600 nm.

*I. Dyes of formula* ***(I)***

The radical **A** of formula **(I)** contain one or more chromophores which are identical or different, with the provisos that at least one of chromophore is colored;

The dyes of formula **(I)** according to the invention are therefore compounds which are capable of absorbing light in the visible region. The dyes of the invention are therefore not colourless, but are visibly coloured, and this property can be observed with the naked eye.

Some of them are, furthermore, fluorescent, which is to say that they have the capacity to re-emit at least some of the wavelength absorbed at a wavelength greater than that absorbed.

*I.1. Chromophore* ***A***

As dyed or colored chromophores according to the invention we can mention acridines ; acridones ; anthranthrones ; anthrapyrimidines ; anthraquinones ; azines ; (poly)azoiques, hydrazono or hydrazones, especially arylhydrazones ; azomethines ; benzanthrones ; benzimidazoles ; benzimidazolones ; benzindoles ; benzoxazoles ; benzopyranes; benzothiazoles; benzoquinones; bisazines ; bis isoindolines ; carboxanilides ; coumarines ; cyanines such as azacarbocyanines, diazacarbocyanines, diazahemicyanines, hemicyanines, or tetraazacarbocyanines ; diazines ; dicetopyrrolopyrroles ; dioxazines ; diphenylamines ; diphenylmethanes ; dithiazines ; flavonoides such as flavanthrones and flavones ; fluorindines ; formazans ; hydroxycetones ; indamines ; indanthrones ; indigoides and pseudo-indigoides; indophenols ; indoanilines ; isoindolines ; isoindolinones ; isoviolanthrones ; lactones ; (poly)methines such as dimethines of stilbenes or styryles types ; naphthalimides ; naphthanilides ; naphtholactames ; naphthoquinones ; nitro, especially nitro(hetero)aromatiques ; oxadiazoles ; oxazines ; perilones ; perinones ; perylenes ; phenazines ; phenoxazine ; phenothiazines ; phthalocyanine ; polyenes/carotenoides ; porphyrines ; pyranthrones ; pyrazolanthrones ; pyrazolones ; pyrimidinoanthrones ; pyronines ; quinacridones ; quinolines ; quinophthalones ; squaranes ; tetrazoliums ; thiazines, thioindigo ; thiopyronines ; triarylmethanes, ou xanthenes.

In the case of azoiques we can mention especially the ones disclosed in Kirk Othmer Encyclopedia of Chemical Technology, "Dyes, Azo", J. Wiley & sons, updated 19/04/2010.

Particulary chromophore **A** is selected from the ones derived from (poly)azoiques dyes such as (di)azoiques, hydrazono, (poly)methines such as styryles and anthraquinones.

In accordance with one particular embodiment of the invention, the coloured or dyed chromophore **A** is chosen from cationic chromophores and more particularly the ones so called « *basic dyes »*.

More particularly we can mention the hydrazono of formulae **(III)** and **(III')**, azoiques **(IV)** and **(IV')** and diazoiques **(V)** cationic chromophores:

**$Hét^{+}$-C($R^a$)=N-N($R^b$)-Ar, $Q^-$** **(II),**

**$Hét^{+}$-N($R^a$)-N=C($R^b$)-Ar, $Q^-$** **(III),**

**$Hét^{+}$-N=N-Ar, $Q^-$** **(IV)**

**$Ar^{+}$-N=N-Ar", $Q^-$** **(IV')**

and

**$Hét^{+}$-N=N-Ar'-N=N-Ar, $Q^-$** **(V)**

formulas **(III), (III'), (IV), (IV')** and **(V)** wherein :

- **Hét⁺** representing a cationic heteroaryl radical, especially an endocyclic cationic radical such as imidazolium, indolium, or pyridinium, potentially substituted especially by at least one ($C_1$-$C_8$)alkyl group such as methyl;

- **Ar⁺** representing an aryl radical, such as phenyl or naphtyl group, with exocyclic cationic charge especially ammonium more especially tri($C_1$-$C_8$)alkyl-ammonium such as trimethylammonium ;
- **Ar** representing an aryl group, especially phenyl, potentially substituted, preferably by at least one electrodonor such as i) ($C_1$-$C_8$)alkyle potentially substituted, ii) ($C_1$-$C_8$)alcoxy potentially substituted, iii) (di)($C_1$-$C_8$)(alkyl)amino potentially substituted on alkyl group(s) by one or more hydroxy groups, iv) aryl($C_1$-$C_8$)alkylamino, v) *N*-($C_1$-$C_8$)alkyl-*N*-aryl($C_1$-$C_8$)alkylamino potentially substituted or **Ar** represents a julolidine group ;
- **Ar'** representing a divalent (hetero)arylene potentially substituted such as phenylene especially para-phenylene, or naphtalene, potentially substituted, especially by one or more groups selected from($C_1$-$C_8$)alkyl, hydroxy, and ($C_1$-$C_8$)alcoxy ;
- **Ar"** representing an (hetero)aryl group potentially substituted such as phenyl or pyrazolyl potentially substituted, preferably by one or more groups selected from ($C_1$-$C_8$)alkyl, hydroxy, (di)($C_1$-$C_8$)(alkyl)amino, ($C_1$-$C_8$)alcoxy and phenyl group ;
- **$R^a$** and **$R^b$,** which are identical or different, representing an hydrogen or a ($C_1$-$C_8$)alkyl group potentially substituted, especially by an hydroxy group,
  or substituant **$R^a$** with one of substituant of **Het⁺** radical and/or **$R^b$** with one substituant of **Ar** radical possibly forming, with atom to which they are attached, a (hetero)cycloalkyle ;
  particularly **$R^a$** and **$R^b$,** representing a hydrogen atom or a ($C_1$-$C_4$)alkyl group possibly or potentially substituted by hydroxy group(s) ;
- **$Q^-$** representing an organic or inorganic anionic counterion such as halogen or alkylsulfate ;

With the proviso that **(III), (III'), (IV), (IV')** or **(V)** is attached to the rest of molecule of formula (I) by **Hét⁺, Ar⁺, Ar** or **Ar".**

The preferred chromophores with an endocyclic charge are azoic chromophores or hydrazono chromphores of formula **(III), (III')** or **(IV)** as defined herein before. More particularly the preferred ones are those of formula **(III), (III')** and **(IV)** derived from the dyes disclosed in the international publication WO 95/15144, WO 95/01772 and EP-714954.

In accordance with one particular embodiment of the invention, chromophores **A** are derived from (III-1) and (IV-1) :

| | |
|---|---|
| 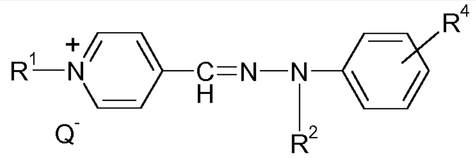 **(III-1)** | 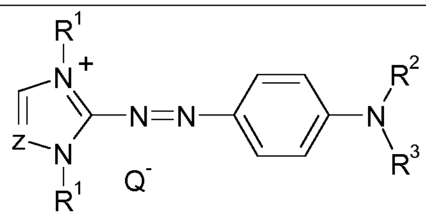 **(IV-1)** |

formulas **(III-1)** and **(IV-1)** wherein :

- **$R^1$** represents an ($C_1$-$C_4$)alkyl group such as a methyl group ;
- **$R^2$** and **$R^3$** which are identical or different, represent a hydrogen atom or a ($C_1$-$C_4$)alkyl group such as methyl ; and
- **$R^4$** represents a hydrogen atom or an electrodonor group such as ($C_1$-$C_8$)alkyle potentially substituted, ($C_1$-$C_8$)alcoxy potentially substituted, (di)($C_1$-$C_8$)(alkyl)amino potentially substituted on alkyle(s) group(s) by hydroxyl group(s) ; particularly **$R^4$** represents a hydrogen atom,
- **Z** represents a CH group or a nitrogen atom, preferably CH,
- **$Q^-$** as defined herein before ;

With the proviso that chromophores **(III-1)** or **(IV-1)** are attached the dye of formula **(I)** by **$R^1$** or **$R^4$** and in the latter case one of the hydrogen atoms of **$R^1$** or **$R^4$** is substituted by **X'** when p = 1 or **$C_{sat}$** when p = 0.

Particularly chromophores **(III-1)** and **(IV-1)** are derived from the dyes selected from Basic Red 51, Basic Yellow 87 and Basic Orange 31 :

| | | |
|---|---|---|
| 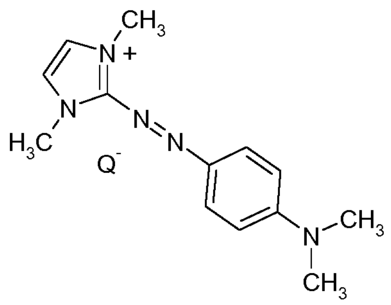 | 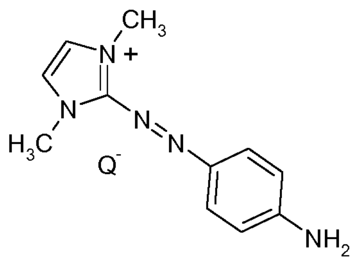 | 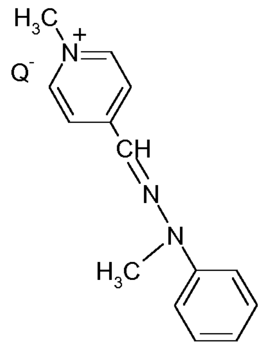 |
| Basic Red 51 | Basic Orange 31 | Basic Yellow 87 |

wherein **Q'** as defined herein before, partucularly halogen such as chloride or alkylsulfate such as methylsulfate or mesytyle,

More particularly, non-fluorescent dyes of formula **(I)** are selected from:

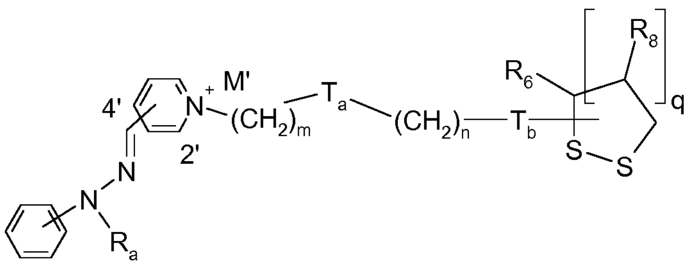

**(I'g)**

in which formulae **(I'g):**

- ◦ $\mathbf{R_a}$ represent a $C_1$-$C_4$ alkyl group such as methyl;
- ◦ $\mathbf{T_a}$ and $\mathbf{T_b}$, which are identical or different, represent i) a covalent σ bond; or ii) a group selected from a radical -O-, -C(O)-, -N(R)- or a combination thereof such as -N(R)-C(O)-, -C(O)-N(R)-, -O-C(O)-, -C(O)-O- or -C(O)-N(R)-C(O)-, where R represents a hydrogen atom or a $C_1$-$C_4$ alkyl group; particularly R is a hydrogen atom or a methyl;
- ◦ $\mathbf{R_6}$ and $\mathbf{R_7}$ represent a hydrogen atom or a $C_1$-$C_4$ alkyl group such as methyl, $\mathbf{T_b}$ being connected directly on the carbon atom in alpha or beta position to the disulphide function of the heterocycle;
- ◦ **m** and **n,** which are identical or different, represent an integer between 0 and 7 inclusive, where m is between 0 and 3 and where n is 0, 1, 2, 3 or 4;
- ◦ **q** is 1, 2 or 3; particularly q is 1;
- ◦ **M'** represents an organic or inorganic anionic counterion;

with the proviso that the pyridinium group is connected to the hydrazono by the carbon atom in position 2' (ortho) or 4' (para), preferably 4'.

Examples of non-fluorescent dyes containing a heterocyclic disulphide group according to formula **(I'g)** include in particular the following compounds **69** :

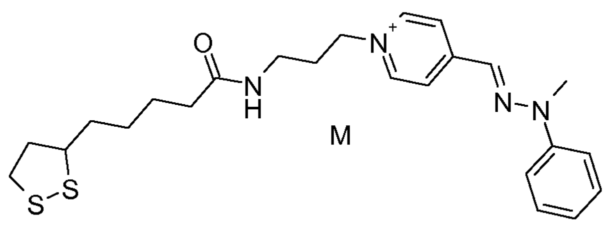

**69**

where M represents an anionic counterion.

According to a very interesting embodiment of the invention, the radical **A** of formula **(I)** contain one or more identical or different fluorescent chromophores, especially for enlightening dark keratin fibers even in the absence of oxidation agent.

*I.1. Fluorescent chromophore **A***

Fluorescent chromophores which are useful in the present invention include the radicals resulting from acridine, acridone, benzanthrone, benzimidazole, benzimidazolone, benzindole, benzoxazole, benzopyran, benzothiazol, coumarin, difluoro{2-[(2*H*-pyrrol-2-ylidene-kN)methyl]-1*H*-pyrrolato-kN}boron (BODIPY®), dipyrrinone, diketopyrrolopyrrole, fluorindine, (poly)methine (especially cyanine and stilbene or styryl/hemicyanine), naphthalimide, naphthanilide, naphthylamine (such as dansyl), oxadiazole, oxazine, perilone, perinone, perylene, polyene/carotenoid, squarane, or xanthene dyes.

Mention may also be made of the fluorescent dyes described in documents EP 1133975, WO 03/029359, EP 860636, WO 95/01772, WO 95/15144 and EP 714954 and those listed in the Encyclopaedia "The chemistry of synthetic dyes" by K. Venkataraman, 1952, Academic Press vols. 1 to 7, in the Encyclopaedia "Kirk Othmer" "Chemical Technology", chapter on "Dyes and Dye Intermediates", 1993, Wiley and Sons, and in various chapters of the Encyclopaedia "ULLMANN's ENCYCLOPEDIA of Industrial Chemistry" 7th edition, Wiley and Sons, in The Handbook - A Guide to Fluorescent Probes and Labeling Technologies, 10th ed. Molecular Probes/Invitrogen - Oregon, 2005, disseminated via the Internet or in the preceding printed editions.

The chromophores are preferably selected from those resulting from coumarin, (poly)methine (especially cyanine and styryl/hemicyanine dyes) and naphthalimide dyes.

According to one variant, the radical **A** of the formula **(I)** contains at least one cationic radical which is carried by or included in at least one of the chromophores.

The cationic radical is preferably a quaternary ammonium.

These cationic radicals are, for example, a (poly)alkylammonium, acridinium, benzimidazolium, benzobistriazolium, benzopyrazolium, benzopyridazinium, benzoquinolium, benzothiazolium, benzotriazolium, benzoxazolium, bipyridinium, bistetrazolium, dihydrothiazolium, imidazopyridinium, imidazolium, indolium, isoquinolium, naphthoimidazolium, naphthooxazolium, naphthopyrazolium, oxadiazolium, oxazolium, oxazolopyridinium, oxonium, phenazinium, phenooxazolium, pyrazinium, pyrazolium, pyrazoyltriazolium, pyridinium, pyridinoimidazolium, pyrrolium, pyrylium, quinolium, tetrazolium, thiadiazolium, thiazolium, thiazolopyridinium, thiazoylimidazolium, thiopyrylium, triazolium or xanthylium radical.

*I.2. $C_{sat}$:*

As indicated above, in the formula **(I),** $C_{sat}$ represents a divalent, linear or branched $C_1$-$C_{18}$ alkylene chain which is optionally substituted and optionally cyclic. Possible substituents include amino, ($C_1$-$C_4$)alkylamino or ($C_1$-$C_4$)dialkylamino groups, or the group $R^a$-$Z^a$-C($Z^b$)- (in which $Z^a$ and $Z^b$, which are identical or different, represent an oxygen or sulphur atom or a group $NR^{a'}$, and $R^a$ represents an alkali metal, a hydrogen atom or a $C_1$-$C_4$ alkyl group, and $R^{a'}$ represents a hydrogen atom or a $C_1$-$C_4$ alkyl group) present preferably on the carbon in beta or gamma position to the atoms of the disulphide sequence -S-S- in the heterocycle **Het.**

Preferably, in the case of formulae **(I),** $C_{sat}$ represents a chain $-(CH_2)k-$ where k is an integer between 1 and 8 inclusive.

*1.3. **X** and **X'**:*

In accordance with one particular embodiment of the invention, in the formula (I) above, when p is 1, X and X', which are identical or different, represent the following sequence:

$$-(T)_t-(Z)_z-(T')_{t'}-$$

said sequence being connected in the formulae **(I)** as follows:

$$-\ C_{sat}-(T')_{t'}-(Z)_z-(T)_t-(A\ \text{or}\ Het);$$

in which sequence:

- **T** and **T'**, which are identical or different, represent one or more radicals or combinations thereof selected from:

$-S(O)_2-$; -O-; -S-; -N(R)-; $-N^+(R)(R^\circ)-$, $Q^-$; -C(O)-; where R and R°, which are identical or different, represent a hydrogen atom or a $C_1$-$C_4$ alkyl, $C_1$-$C_4$ hydroxyalkyl or aryl($C_1$-$C_4$)alkyl radical and Q' represents an anionic counterion; and a cationic or non-cationic heterocycloalkyl or heteroaryl radical, which is preferably monocyclic and preferably contains two heteroatoms (more preferably two nitrogen atoms) and comprises preferably from 5 to 7 members, more preferably imidazolium, piperazinyl or piperidinyl;
preferably **T** and **T'** represent one or more radicals or combinations thereof selected from -O-, -N(R)-, -C(O)-, where R is selected from a hydrogen atom and a $C_1$-$C_4$ alkyl radical;

- the indices **t** and **t'**, which are identical or different, are 0 or 1;
- **Z** represents:

  - $-(CR_1R_2)_m-$ where m is an integer between 1 and 8 and $R_1$ and $R_2$, which are identical or different, represent a hydrogen atom or a $C_1$-$C_4$ alkyl, $C_1$-$C_{12}$ alkoxy, hydroxyl, cyano, carboxyl, amino, $C_1$-$C_4$ alkylamino or $C_1$-$C_4$ dialkylamino group, it being possible for said alkyl radicals to form, with the nitrogen atom carrying them, a heterocycle containing from 5 to 7 members, optionally comprising another heteroatom different or not from nitrogen;
  - $-(CH_2CH_2O)_q-$ or $-(OCH_2CH_2)_q-$ in which q is an integer between 1 and 15, or
  - an aryl, alkylaryl or arylalkyl radical in which the alkyl radical is $C_1$-$C_4$ and the aryl radical is preferably $C_6$, being optionally substituted by at least one group $SO_3M$, where M represents a hydrogen atom, an alkali metal or an ammonium group which is substituted by one or more identical or non-identical, linear or branched $C_1$-$C_{18}$ alkyl radicals which optionally carry at least one hydroxyl; and

- **z** is 0 or 1.

Furthermore, according to one particular embodiment of the invention, Z represents:

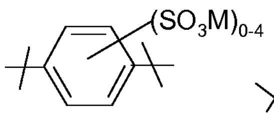 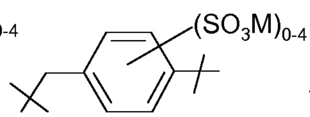 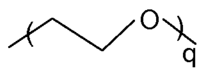 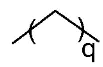

Wherein q represents 1, 2, 3 or 4.

*1.4. Preferred fluorescent dyes of formula **(I)** or **(I')**:*

A further subject of the invention relates to the fluorescent dyes containing a heterocyclic disulphide group, of the formula **(I),** as defined, with the proviso that in the compounds of formula **(I), Het** represents a heterocyclic disulphide radical of formula **(II)** as defined above, and with the provisos that:

- the compound of formula **(I)** cannot comprise fluorescent riboflavin-5-monophosphate chromophore **(A);**
- the compound of formula **(I)** cannot comprise fluorescent chromophore **(A)** which represents an indol-3-yl or 5-methoxyindol-3-yl group; and
- the compound of formula **(I)** cannot represent the compounds **(A)** or **(B)** as drawn below, or **(C)** or **(D)** as defined above,

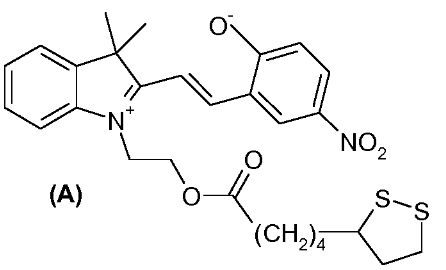
**(A)**

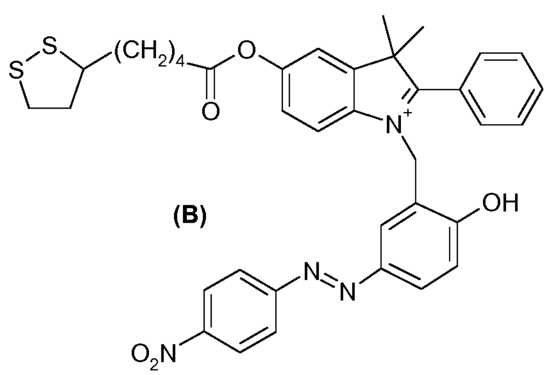
**(B)**

where **(B)** is combined or not combined with a CdSe-ZnS, CdTe-ZnS, CdSe-ZnSe or CdTe-ZnSe core-shell quantum

and (C) is combined or not combined with a CdSe-ZnS core-shell quantum dot.

According to one preferred variant of the invention, the disulphide dye is a cationic fluorescent dye comprising at least one quaternary ammonium radical and is such that, in the formula **(I),** with p being equal to 1:

- **A** represents **W-C($R^c$)=C($R^d$)-Ar'-** or **-W'-C($R^c$)=C($R^d$)-Ar,** where
- **W** is a monovalent radical which represents a heterocycle or a heteroaryl, comprising a quaternary ammonium which is optionally substituted by a $C_1$-$C_8$ alkyl which is optionally substituted in particular by one or more hydroxyl groups;
- **W'** is a divalent radical which represents a heterocycle or heteroaryl as defined for **W**;
- **Ar** is a monovalent radical that represents a 5- or 6-membered aryl radical of phenyl type or a bicyclic aromatic system of naphthyl type, optionally substituted i) by one or more halogen atoms, preferably chlorine or fluorine; ii) by one or more alkyl groups, preferably $C_1$-$C_4$ alkyl groups; iii) by one or more hydroxyl groups; iv) by one or more alkoxy groups; v) by one or more hydroxyalkyl groups; vi) by one or more amino or (di)alkylamino groups, preferably where the $C_1$-$C_4$ alkyl moiety is optionally substituted by one or more hydroxyls, such as (di)hydroxylethylamino; vii) by one or more acylamino groups; or viii) by one or more 5- or 6-membered heterocycloalkyl or heteroaryl groups, preferably selected from pyrrolidinyl, piperazinyl, piperidinyl and imidazolinyl;
- **Ar'** is a divalent radical which represents an aryl as defined for **Ar;** and
- **$R^c$** and **$R^d$,** which are identical or different, represent a hydrogen atom or a $C_1$-$C_4$ alkyl group.

According to one preferred variant, the compounds of formula **(I)** are such that they conform to the formula **(I'):**

$$\mathbf{A\text{-}(T)_t\text{-}[(CR_1R_2)_m]_z\text{-}T_a\text{-}C_{sat}\text{-}(T_b)_{t'}\text{-}Het} \qquad \mathbf{(I')}$$

in which formula **(I'):**

- **Het** represents a heterocyclic disulphide radical of formula **(II)** as defined above;
- **A** is derived from hydrazono of formulae **(III)** and **(III'),** azoiques **(IV)** and **(IV')** and diazoiques **(V)** cationic chromophores as defined herin before or represents a fluorescent chromophore **W-C($R^c$)=C($R^d$)-Ar'-** or **-W'-C($R^c$)=C($R^d$)-Ar,** as defined herein before;
- **z** is 0 or 1;
- **t** and **t',** which are identical or different, are 0 or 1; and
- **T** represents i) an amino group -N(R)- where R represents a hydrogen atom, a $C_1$-$C_4$ alkyl, $C_1$-$C_4$ hydroxyalkyl or aryl($C_1$-$C_4$)alkyl radical, or ii) an optionally substituted divalent heterocycloalkyl group;
- **$T_a$** and **$T_b$**, which are identical or different, represent one or more radicals or combinations thereof selected from -$S(O)_2$-, -O-, -S-, -N(R)-, -$N^+$(R)(R°)-, $M^-$, -C(O)-, where R and R°, which are identical or different, represent a hydrogen atom, a $C_1$-$C_4$ alkyl or $C_1$-$C_4$ hydroxyalkyl radical; or an aryl($C_1$-$C_4$)alkyl radical, and $M^-$ represents an organic or inorganic anionic counterion such as halide, preferably with $T_a$ representing a covalent $\sigma$ bond or a group -N(R)- and $T_b$ representing a covalent $\sigma$ bond or a group selected from a radical -O-, -C(O)-, -N(R)-, or a combination thereof, where R represents a hydrogen atom or a $C_1$-$C_4$ alkyl group such as methyl;
  And
- **$T_a$** being preferably in para position on Ar or Ar', relative to the olefin function -C($R^c$)=C($R^d$)-.

According to one more preferred variant, the compounds of formula **(I)** are such that they conform to the formula **(I'):**

$$\mathbf{A\text{-}(T)_t\text{-}[(CR_1R_2)_m]_z\text{-}T_a\text{-}C_{sat}\text{-}(T_b)_{t'}\text{-}Het} \qquad \mathbf{(I')}$$

in which formula **(I'):**

- **Het** represents a heterocyclic disulphide radical of formula **(II)** as defined above;
- **A** represents **W-C($R^c$)=C($R^d$)-Ar'-** or **-W'-C($R^c$)=C($R^d$)-Ar,** as defined above,
- **z** is 0 or 1;
- **t** and **t'**, which are identical or different, are 0 or 1; and
- **T** represents i) an amino group -N(R)- where R represents a hydrogen atom, a $C_1$-$C_4$ alkyl, $C_1$-$C_4$ hydroxyalkyl or aryl($C_1$-$C_4$)alkyl radical, or ii) an optionally substituted divalent heterocycloalkyl group;
- **$T_a$** and **$T_b$**, which are identical or different, represent one or more radicals or combinations thereof selected from -$S(O)_2$-, -O-, -S-, -N(R)-, -$N^+$(R)(R°)-, $M^-$, -C(O)-, where R and R°, which are identical or different, represent a hydrogen

atom, a $C_1$-$C_4$ alkyl or $C_1$-$C_4$ hydroxyalkyl radical; or an aryl($C_1$-$C_4$)alkyl radical, and $M^-$ represents an organic or inorganic anionic counterion such as halide, preferably with $T_a$ representing a covalent σ bond or a group -N(R)- and $T_b$ representing a covalent σ bond or a group selected from a radical -O-, -C(O)-, -N(R)-, or a combination thereof, where R represents a hydrogen atom or a $C_1$-$C_4$ alkyl group such as methyl;
and

- **$T_a$** being preferably in para position on Ar or Ar', relative to the olefin function $-C(R^c)=C(R^d)-$.

Especially, **W** is an imidazolium, pyridinium, benzopyridinium, benzimidazolium, quinolinium, indolinium and pyrazolium, which are optionally substituted particularly by one or more identical or non-identical $C_1$-$C_4$ alkyl radicals. More particularly, W is selected from pyridinium and indolinium groups which are optionally substituted by one or more identical or non-identical $C_1$-$C_4$ alkyl radicals such as methyl.

Preferably, the dye containing a heterocyclic disulphide group is a dye selected from the compounds of formula **(Ia)** to **(Ig)** below:

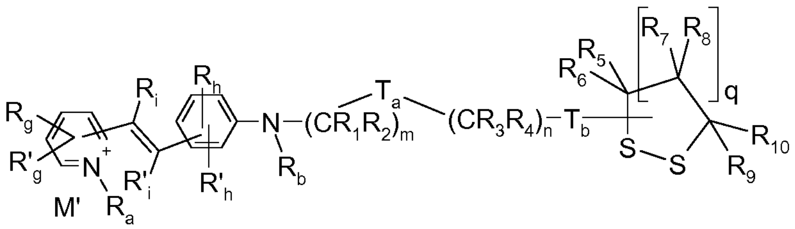

**(Ia)**

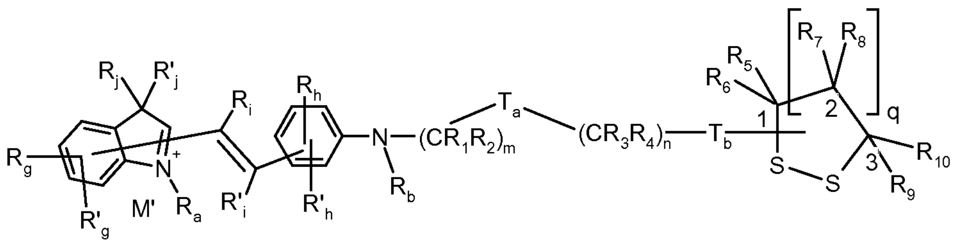

**(Ib)**

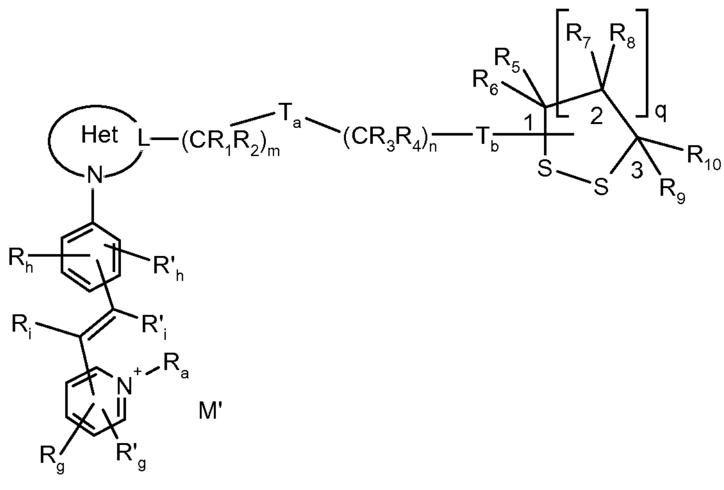

**(Ic)**

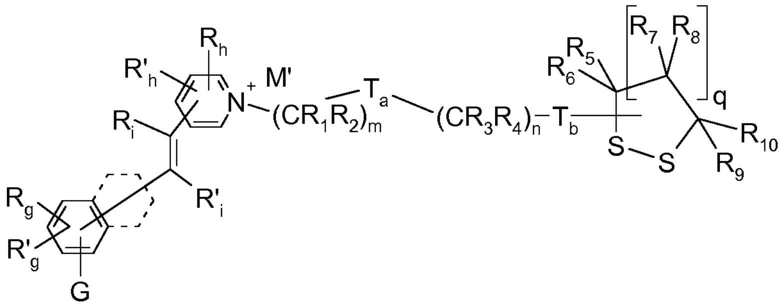

**(Id)**

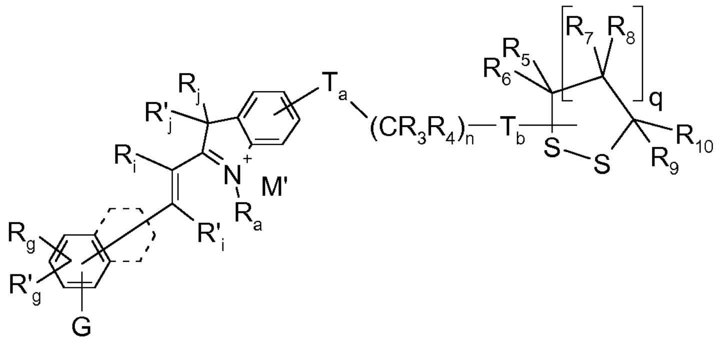

**(Ie)**

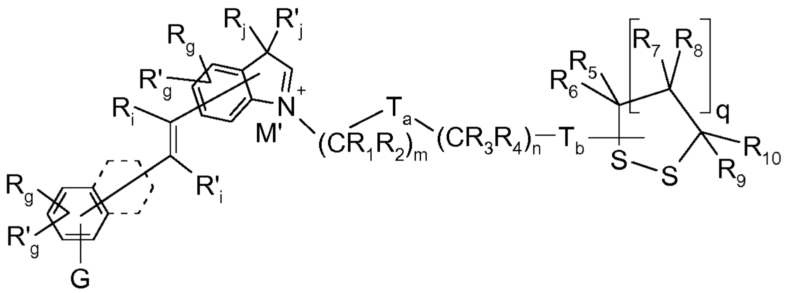

**(If)**

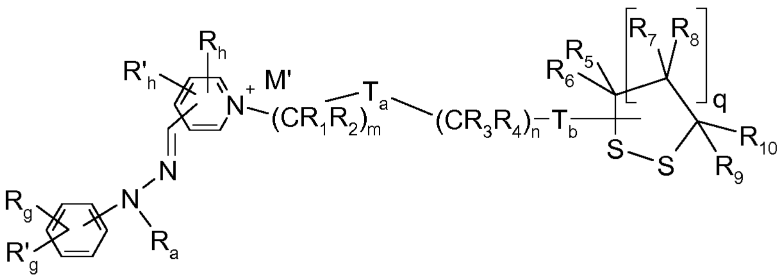

**(Ig)**

and their organic or inorganic acid salts, optical isomers and geometric isomers, and the solvates such as hydrates; in which formulae **(Ia)** to **(Ig):**

- **G** represents a group $-NR_cR_d$ or a $C_1$-$C_6$ alkoxy group; preferably, G represents a group $-NR_cR_d$, which is preferably positioned para to the styryl group;
- $\mathbf{R_a}$ represents an optionally substituted $C_1$-$C_6$ alkyl group; preferably $R_a$ represents a $C_1$-$C_3$ alkyl group which is optionally substituted by a hydroxyl group, but preferably unsubstituted, such as methyl;
- $\mathbf{R_b}$ represents a hydrogen atom or an optionally substituted $C_1$-$C_6$ alkyl group; preferably unsubstituted, such as methyl;
- $\mathbf{R_c}$ and $\mathbf{R_d}$**,** which are identical or different, represent a hydrogen atom, an aryl($C_1$-$C_4$)alkyl group, $C_1$-$C_6$ alkoxy or an optionally substituted $C_1$-$C_6$ alkyl group; $R_c$ and $R_d$ preferably represent a hydrogen atom or a $C_1$-$C_3$ alkyl group which is optionally substituted by i) a hydroxyl group, ii) amino, iii) $C_1$-$C_3$ (di)alkylamino, or iv) quaternary ammonium (R")(R"')(R"")$N^+$-;

  or two adjacent radicals $\mathbf{R_c}$ and $\mathbf{R_d}$ carried by the same nitrogen atom together form a heterocyclic or heteroaryl

group; preferably the heterocycle or heteroaryl is monocyclic and comprises between 5 and 7 members; more preferably the groups are selected from imidazolyl and pyrrolidinyl;

particularly $\mathbf{R_c}$ and $\mathbf{R_d}$ represent identical groups, and preferably $R_c$ and $R_d$ represent a $C_1$-$C_3$ alkyl which is optionally substituted by a hydroxyl group, such as methyl, hydroxyethyl and 2-hydroxypropyl;

- $\mathbf{R_g}$, $\mathbf{R'_g}$, $\mathbf{R_h}$ and $\mathbf{R'_h}$, which are identical or different, represent a hydrogen atom, a halogen atom, an amino, $C_1$-$C_4$ alkylamino, $C_1$-$C_4$ dialkylamino, cyano, carboxyl, hydroxyl or trifluoromethyl group, an acylamino, $C_1$-$C_4$ alkoxy, $C_2$-$C_4$ (poly)hydroxyalkoxy, alkylcarbonyloxy, alkoxycarbonyl or alkylcarbonylamino radical, an acylamino, carbamoyl or alkylsulphonylamino radical, an aminosulphonyl radical, or a $C_1$-$C_{16}$ alkyl radical which is optionally substituted by a group selected from $C_1$-$C_{12}$ alkoxy, hydroxyl, cyano, carboxyl, amino, $C_1$-$C_4$ alkylamino and $C_1$-$C_4$ dialkylamino, or the two alkyl radicals carried by the nitrogen atom of the amino group form a heterocycle containing from 5 to 7 members and optionally comprising another heteroatom, which is identical to or different from the nitrogen atom; preferably $R_g$, $R'_g$, $R_h$ and $R'_h$ represent a hydrogen or halogen atom or a $C_1$-$C_3$ alkyl group; more preferably $R_g$, $R'_g$, $R_h$ and $R'_h$ represent a hydrogen atom;
- or two groups $\mathbf{R_g}$ and $\mathbf{R'_g}$; $\mathbf{R_h}$ and $\mathbf{R'_h}$; carried by two adjacent carbon atoms together form a benzo or indeno ring system or a fused heterocyclo alkyl or fused heteroaryl group; the benzo, indeno, heterocycloalkyl or heteroaryl ring system being optionally substituted by a halogen atom, an amino, $C_1$-$C_4$ alkylamino, $C_1$-$C_4$ dialkylamino, nitro, cyano, carboxyl, hydroxyl or trifluoromethyl group, an acylamino, $C_1$-$C_4$ alkoxy, $C_2$-$C_4$ (poly)hydroxyalkoxy, alkylcarbonyloxy alkoxycarbonyl or alkylcarbonylamino radical, an acylamino, carbamoyl or alkylsulphonylamino radical, an aminosulphonyl radical, or a $C_1$-$C_{16}$ alkyl radical which is optionally substituted by: a group selected from C1-C12 alkoxy, hydroxyl, cyano, carboxyl, amino, $C_1$-$C_4$ alkylamino, $C_1$-$C_4$ dialkylamino, or the two alkyl radicals carried by the nitrogen atom of the amino group form a heterocycle comprising from 5 to 7 members and optionally comprising another heteroatom, which is identical to or different from the nitrogen atom; preferably $\mathbf{R_g}$ and $\mathbf{R'_g}$ together form a benzo group;
- or, when **G** represents $-NR_cR_d$, two groups $R_c$ and $R'_g$; $R_d$ and $R_g$; together form a saturated heterocycle or heteroaryl which is optionally substituted by one or more $C_1$-$C_6$ alkyl groups, preferably a heterocycle containing one or two heteroatoms selected from nitrogen and oxygen and comprising between 5 and 7 members; more preferably the heterocycle is selected from morpholinyl, piperazinyl, piperidinyl and pyrrolidinyl groups;
- $\mathbf{R_i}$, $\mathbf{R_j}$, $\mathbf{R'_j}$ and $\mathbf{R'_i}$, which are identical or different, represent a hydrogen atom or a $C_1$-$C_4$ alkyl group; preferably $\mathbf{R_i}$, $\mathbf{R_j}$, $\mathbf{R'_j}$ and $\mathbf{R'_i}$ represent a hydrogen atom;
- $\mathbf{R_1}$, $\mathbf{R_2}$, $\mathbf{R_3}$ and $\mathbf{R_4}$, which are identical or different, represent a hydrogen atom or a $C_1$-$C_4$ alkyl, $C_1$-$C_{12}$ alkoxy, hydroxyl, cyano, carboxyl, amino, $C_1$-$C_4$ alkylamino or $C_1$-$C_4$ dialkylamino group, it being possible for said alkyl radicals to form, with the nitrogen atom carrying them, a heterocycle comprising from 5 to 7 members, optionally comprising another heteroatom, which is different or not from nitrogen; preferably $R_1$, $R_2$, $R_3$ and $R_4$ are hydrogen atoms or an amino group; more preferably $R_1$, $R_2$, $R_3$ and $R_4$ represent a hydrogen atom;
- $\mathbf{T_a}$ and $\mathbf{T_b}$, which are identical or different, represent:

  i) either a covalent $\sigma$ bond;

  ii) or one or more radicals or combinations thereof selected from $-SO_2$-, -O-, -S-, -N(R)-, $-N^+(R)(R°)$- $M^-$, -C(O)-, where R and R°, which are identical or different, represent a hydrogen atom or a $C_1$-$C_4$ alkyl or $C_1$-$C_4$ hydroxyalkyl radical; or an aryl($C_1$-$C_4$)alkyl, and $M^-$ represents an organic or inorganic anionic counterion such as halide; preferably $T_a$ represents a covalent $\sigma$ bond or a group -N(R)- and $T_b$ represents a covalent $\sigma$ bond or a group selected from a radical -O-, -C(O)-, -N(R)- or a combination thereof, where R represents a hydrogen atom or a $C_1$-$C_4$ alkyl group such as methyl;

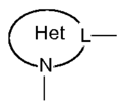

represents an optionally substituted heterocyclic group; L represents a carbon atom or a nitrogen atom, the heterocycle is preferably monocyclic and saturated and comprises a total of two nitrogen atoms and from 5 to 8 members;

- 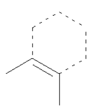

represents an aryl or heteroaryl group which is fused to the phenyl ring; or is absent from the phenyl ring; when the ring is present, the ring is preferably a benzo; more preferably the ring is absent;

- **m** and **n,** which are identical or different, represent an integer between 0 and 10, preferably between 0 and 7, inclusive, where m is preferably between 0 and 5, more particularly between 0 and 3, inclusive and where n is between 0 and 8 inclusive; more particularly n is 0, 1, 2, 3 or 4;
- $\mathbf{R_5}$, $\mathbf{R_6}$, $\mathbf{R_9}$ and $\mathbf{R_{10}}$, which are identical or different, represent a hydrogen atom or a group selected from: i) ($C_1$-$C_8$)alkyl, iii) hydroxyl, iv) (di)($C_1$-$C_8$)(alkyl)amino, v) ($C_1$-$C_8$)alkoxy, vi) (poly)hydroxy($C_1$-$C_8$)alkyl, vii) (di)($C_1$-$C_8$)(alkyl)amino($C_1$-$C_8$)alkyl, viii) carboxyl, ix) carboxy($C_1$-$C_3$)alkyl, x) (di)($C_1$-$C_8$)(alkyl)aminocarbonyl($C_1$-$C_8$)alkyl and xii) ($C_1$-$C_8$)(alkyl)carbonyl($C_1$-$C_8$)(alkyl)amino($C_1$-$C_8$)alkyl;
  preferably $\mathbf{R_5}$ is an atom or an alkyl group such as methyl and $\mathbf{R_6}$, $\mathbf{R_9}$ and $\mathbf{R_{10}}$ represent hydrogen atoms;
- $\mathbf{R_7}$ and $\mathbf{R_8}$, which are identical or different, represent a hydrogen atom or a group selected from: i) ($C_1$-$C_8$)alkyl, iii) hydroxyl, iv) (di)($C_1$-$C_8$)(alkyl)amino, v) ($C_1$-$C_8$)alkoxy, vi) (poly)hydroxy($C_1$-$C_8$)alkyl, vii) (di)($C_1$-$C_8$)(alkyl)amino($C_1$-$C_8$)alkyl, viii) carboxyl, ix) carboxy($C_1$-$C_8$)alkyl, x) (di)($C_1$-$C_8$)(alkyl)amino-carbonyl($C_1$-$C_8$)alkyl and xii) ($C_1$-$C_8$)(alkyl)carbonyl($C_1$-$C_8$)(alkyl)amino($C_1$-$C_8$)alkyl;
  preferably $\mathbf{R_7}$ and $\mathbf{R_8}$ represent a hydrogen atom or a $C_1$-$C_4$ alkyl group such as methyl;
- q represents an integer between 1 and 3 inclusive; preferably q = 1; and
- **M'** represents an organic or inorganic anionic counterion with the proviso that the disulphide heterocycle is connected to the rest of the molecule directly by the carbon atom, preferably in the beta position to the disulphide function of said heterocycle.

More preferably, the dye containing a heterocyclic disulphide group is a fluorescent dye selected from the compounds of formula **(Ia)** to **(If)** herein before.

More particularly, the fluorescent dyes according to the invention are selected from:

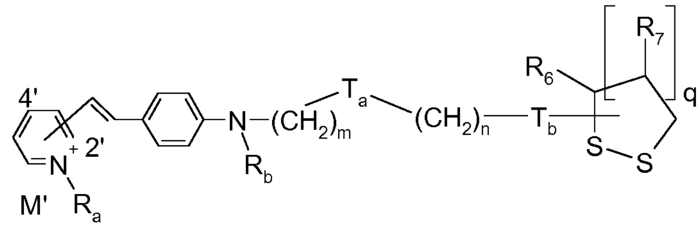

**(I'a)**

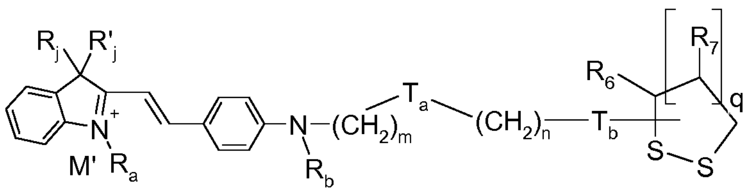

**(I'b)**

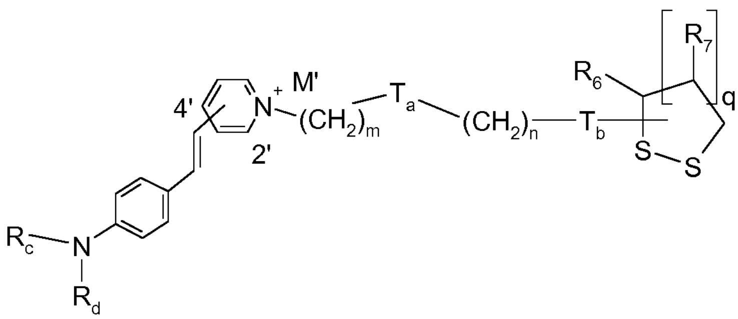

**(I'd)**

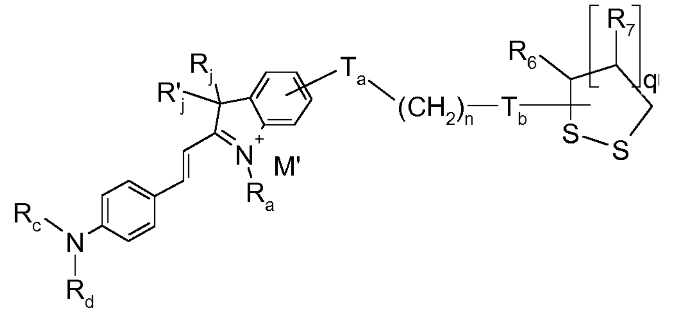

(I'e)

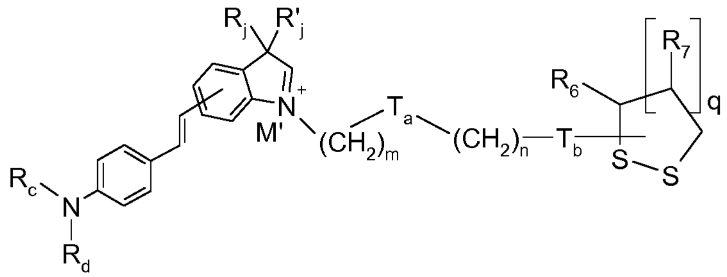

(I'f)

in which formulae **(I'a), (I'b), (I'd), (I'e)** and **(I'f):**

- **$R_a$, $R_b$**, **$R_j$** and **$R'_j$** represent a $C_1$-$C_4$ alkyl group such as methyl;
- **$R_6$** and **$R_7$** represent a hydrogen atom or a $C_1$-$C_4$ alkyl group such as methyl, **$T_b$** being connected directly on the carbon atom in alpha or beta position to the disulphide function of the heterocycle;
- **m** and **n,** which are identical or different, represent an integer between 0 and 7 inclusive, where m is between 0 and 3 and where n is 0, 1, 2, 3 or 4;
- **$T_a$** and **$T_b$,** which are identical or different, represent i) a covalent $\sigma$ bond; or ii) a group selected from a radical -O-, -C(O)-, -N(R)- or a combination thereof such as -N(R)-C(O)-, -C(O)-N(R)-, -O-C(O)-, -C(O)-O- or -C(O)-N(R)-C(O)-, where R represents a hydrogen atom or a $C_1$-$C_4$ alkyl group; particularly R is a hydrogen atom or a methyl;
- **q** is 1, 2 or 3; particularly q is 1;
- **$R_c$** and **$R_d$** represent identical $C_1$-$C_3$ alkyl groups optionally substituted by a hydroxyl group, such as methyl, hydroxyethyl and 2-hydroxypropyl;

and
- **M'** represents an organic or inorganic anionic counterion;

with the proviso that in the formula **(I'a)** and **(I'd)** the pyridinium group is connected to the styryl by the carbon atom in position 2' (ortho) or 4' (para).

Examples of fluorescent dyes containing a heterocyclic disulphide group include in particular the following compounds 1 to 68 :

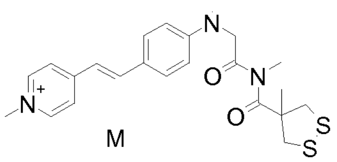

**1**

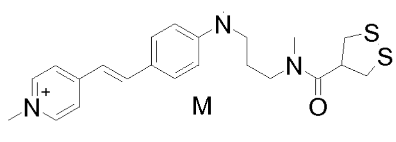

**2**

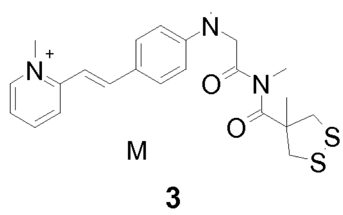

**3**

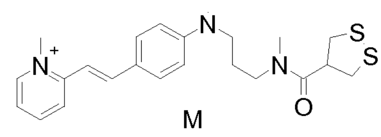

**4**

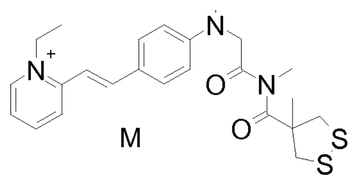

**5**

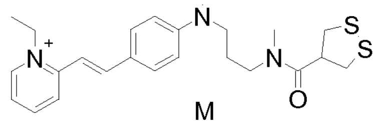

**6**

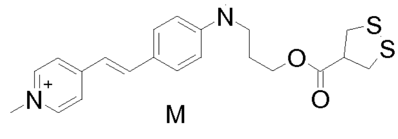

**7**

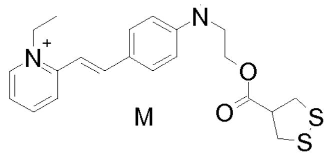

**8**

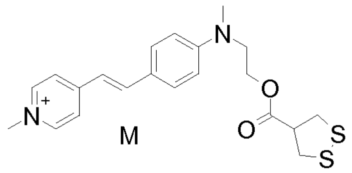

**9**

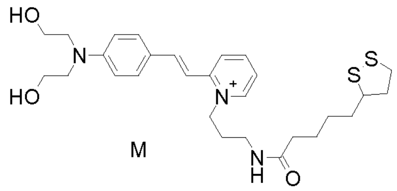

**10**

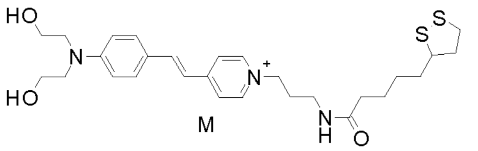

**11**

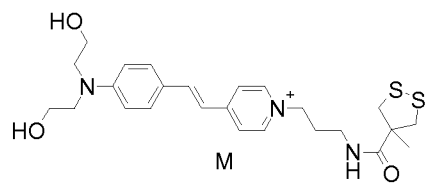

**12**

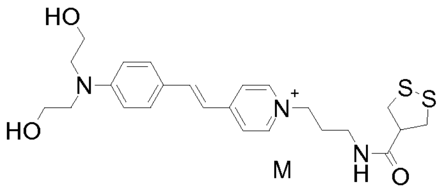

**13**

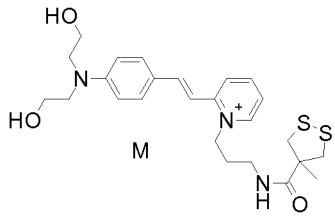

**14**

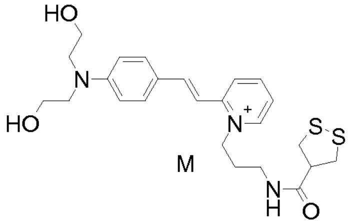

**15**

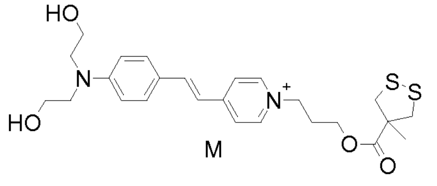

**16**

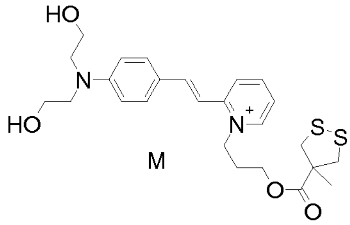

**17**

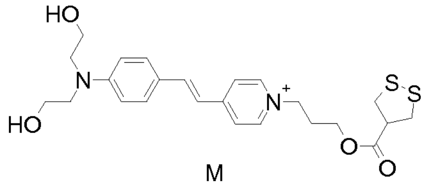

**18**

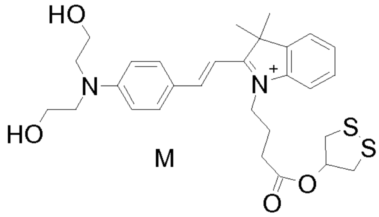

**19**

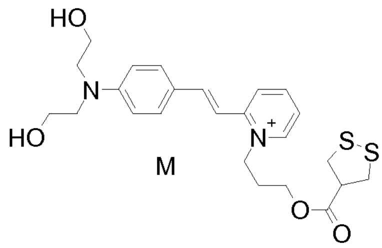

**20**

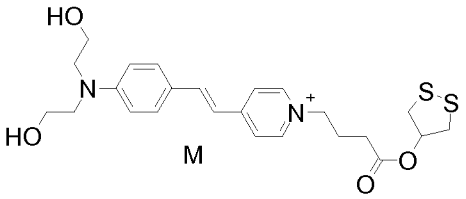

**21**

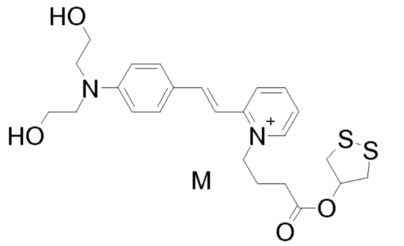

**22**

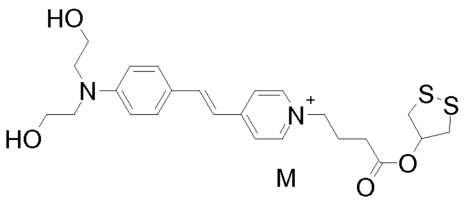

**23**

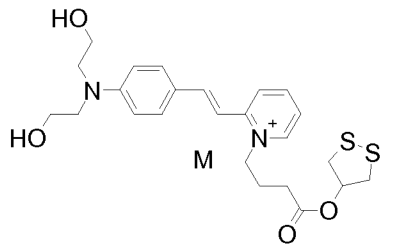

**24**

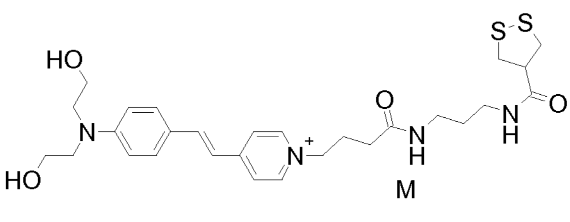

**25**

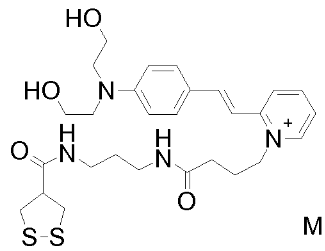

**26**

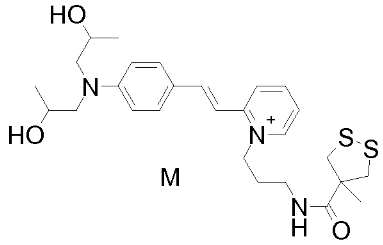

**27**

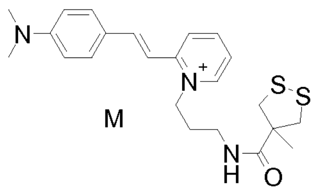

**28**

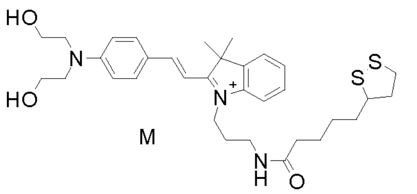

**29**

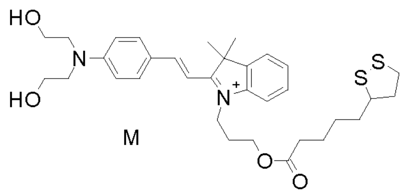

**30**

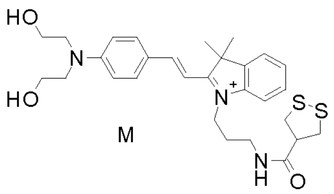

**31**

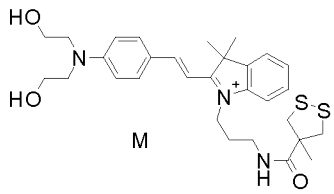

**32**

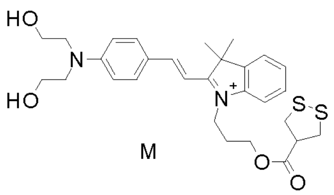

**33**

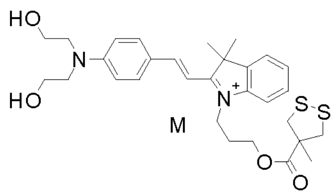

**34**

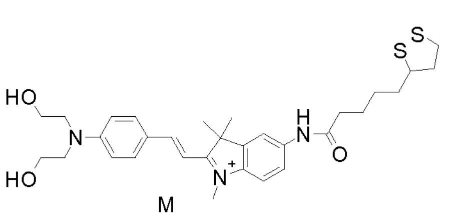

**35**

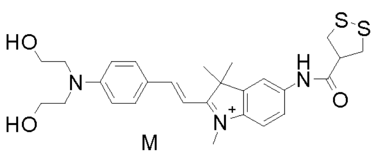

**36**

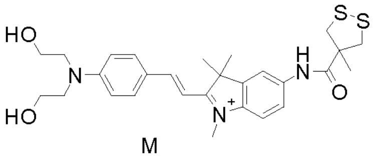

**37**

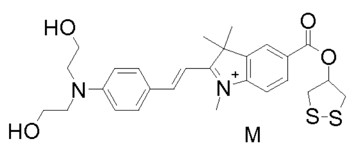

**38**

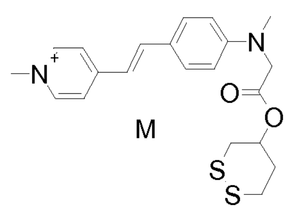

39

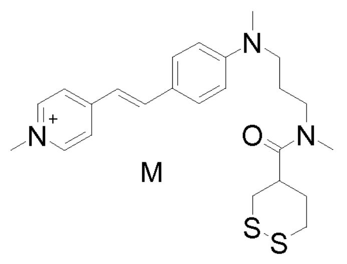

40

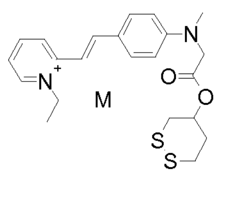

41

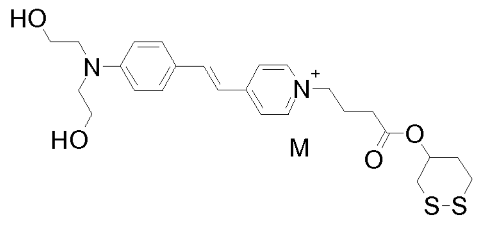

42

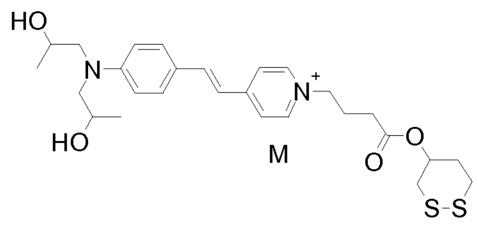

43

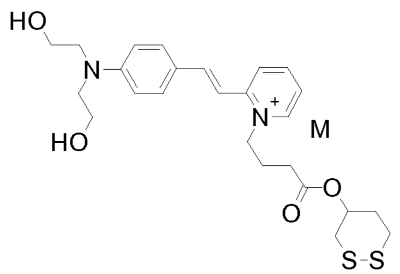

44

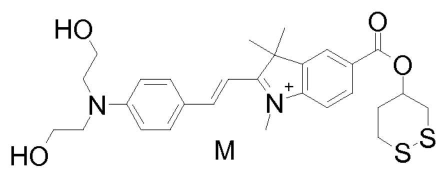

45

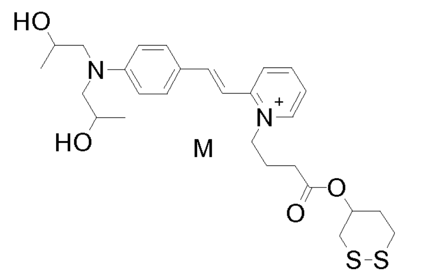

46

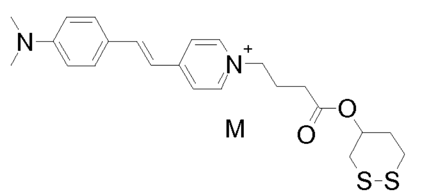

47

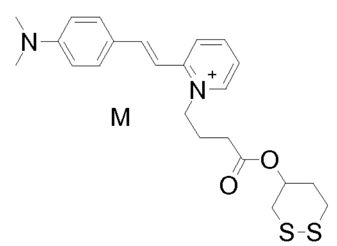

48

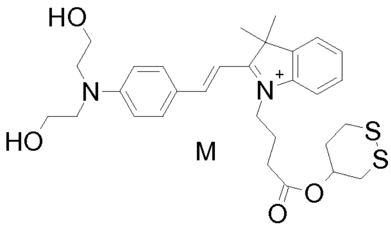

**49**

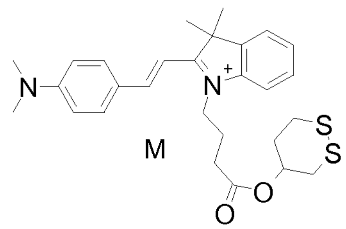

**50**

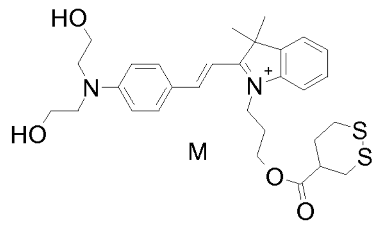

**51**

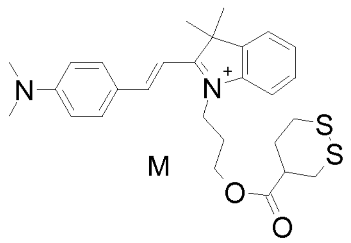

**52**

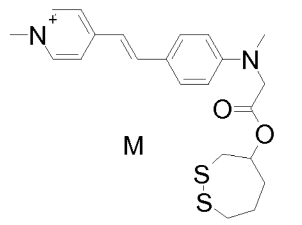

**53**

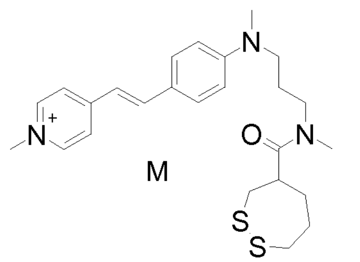

**54**

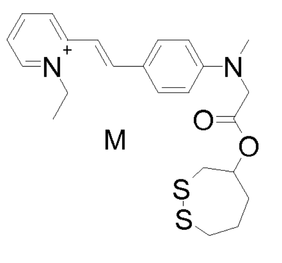

**55**

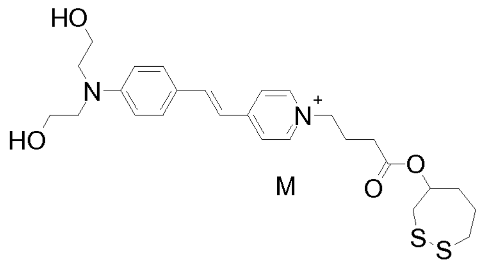

**56**

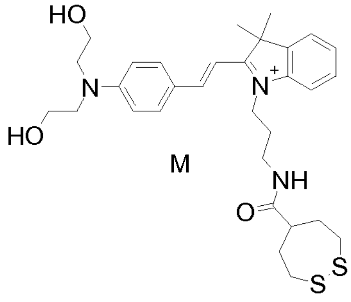

**57**

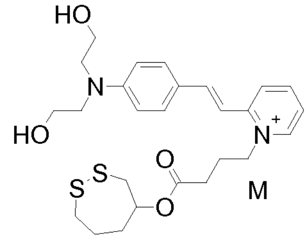

**58**

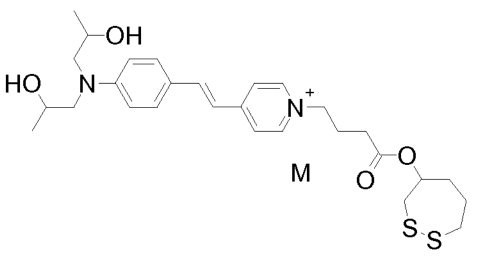

**59**

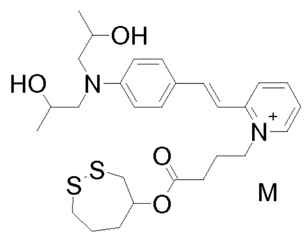

**60**

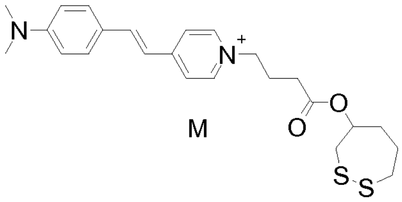

**61**

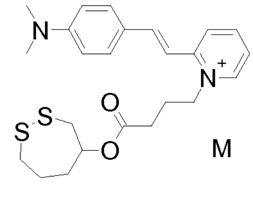

**62**

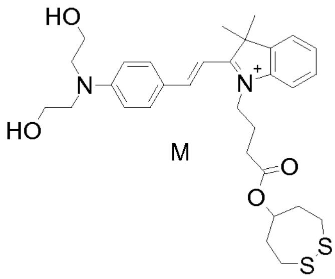

**63**

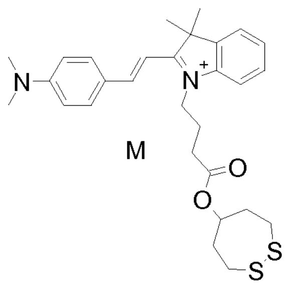

**64**

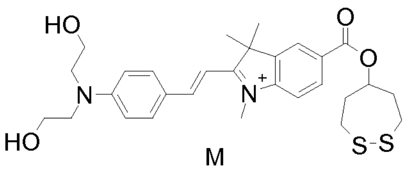

**65**

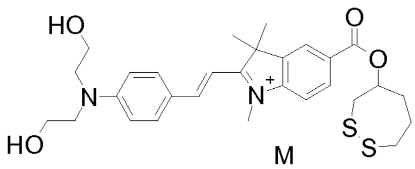

**66**

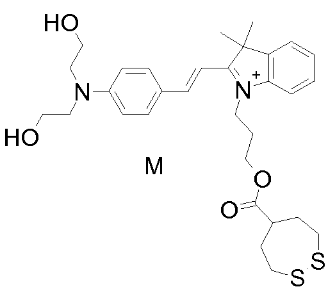

**67**

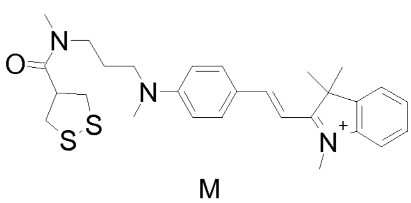

**68**

where M represents an anionic counterion.

The dyes of formula **(Ia)** may be synthesized by a condensation reaction between a compound **(a1)** and a compound **(a2).** This reaction is well known to the skilled person. It is called a Knoevenagel reaction and involves condensing an aldehyde with a nucleophile of activated methyl type. This reaction is described in the literature. Reference

may be made, for example, to the book Advanced Organic Chemistry (ISBN 0-471-60180-2). This reaction is exemplified in the scheme below:

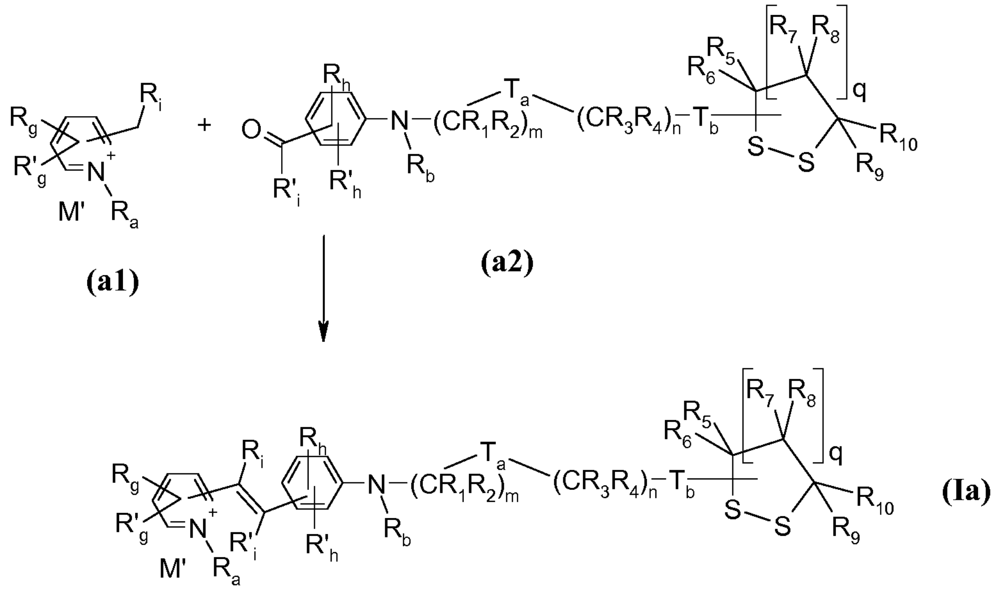

(a1) (a2) (Ia)

where $R_1$ to $R_{10}$, $R_a$, $R_b$, G, m, n, $T_a$, $T_b$, $R_g$, $R'_g$, $R_i$, $R'_i$, $R_h$, $R'_h$, M' and q are as defined above.

The compounds **(a1)** are known to the skilled person and are described in the literature. Some of them are available commercially.

The compounds **(a2)** may be synthesized by a nucleophilic substitution reaction between a compound **(a3)** and a compound **(a4).** This reaction is known to the skilled person and is described in the literature. By way of example, reference may be made to the book Advanced Organic Chemistry (ISBN 0-471-60180-2). This reaction is exemplified in the scheme below:

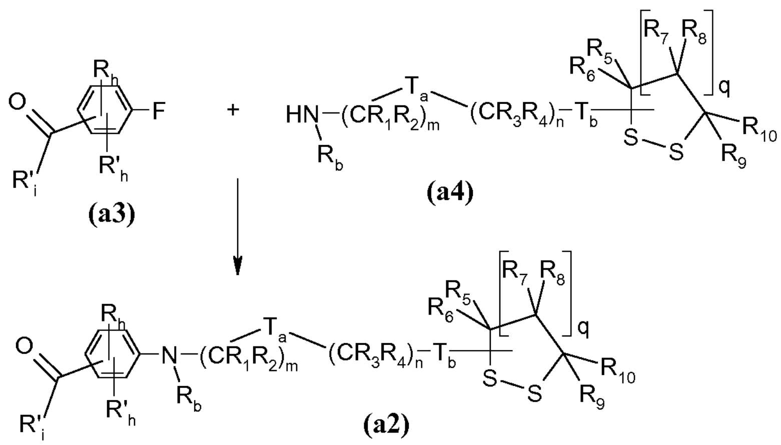

(a3) (a4) (a2)

where $R_1$ to $R_{10}$, $R_a$, $R_b$, G, m, n, $T_a$, $T_b$, $R_i$, $R'_i$, $R_h$, $R'_h$ and q are as defined above.

The fluorobenzaldehyde compounds **(a3)** are known to the skilled person and/or are available commercially.

The compounds **(a4)** may be synthesized by a reaction between a compound **(a5)** and a compound **(a6).** This reaction is exemplified in the scheme below:

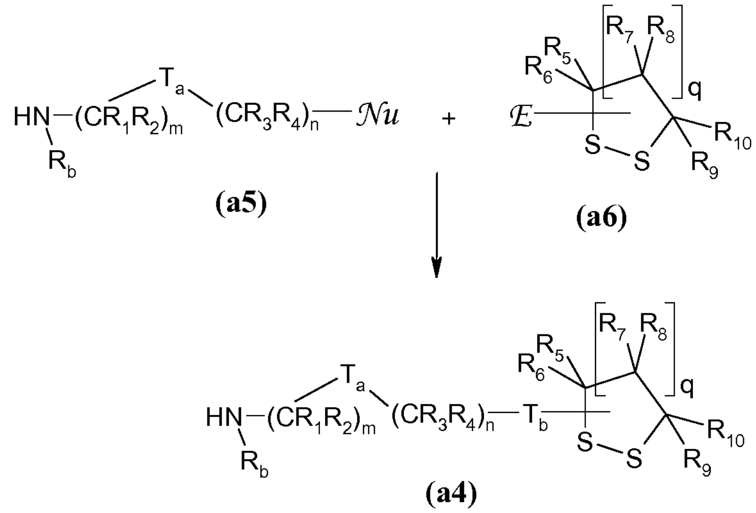

(a5) (a6) (a4)

where $R_1$ to $R_{10}$, $R_a$, $R_b$, G, m, n, $T_a$, $T_b$ and q are as defined above; Nu represents a nucleophilic group; E represents an electrophilic group; and $T_b$ represents the bond generated following attack of the nucleophile on the electrophile.

By way of example, the covalent bonds $T_b$ that may be generated are listed in Table A below, on the basis of condensation of electrophiles with nucleophiles:

Table A

| Electrophiles E | Nucleophiles Nu | Covalent bonds $T_b$ |
|---|---|---|
| Activated esters* | Amines | Carboxamides |
| Acyl azides** | Amines | Carboxamides |
| Acyl halides | Amines | Carboxamides |
| Acyl halides | Alcohols | Esters |
| Acyl cyanides | Alcohols | Esters |
| Acyl cyanides | Amines | Carboxamides |
| Alkyl halides | Amines | Alkylamines |
| Alkyl halides | Carboxylic acids | Esters |
| Alkyl halides | Thiols | Thioesters |
| Alkyl halides | Alcohols | Ethers |
| Sulphonic acids and their salts | Thiols | Thioethers |
| Sulphonic acids and their salts | Carboxylic acids | Esters |
| Sulphonic acids and their salts | Alcohols | Ethers |
| Anhydrides | Alcohols | Esters |
| Anhydrides | Amines | Carboxamides |
| Aryl halides | Thiols | Thioethers |
| Aryl halides | Amines | Arylamines |
| Aziridines | Thiols | Thioethers |
| Carboxylic acids | Amines | Carboxamides |
| Carboxylic acids | Alcohols | Esters |
| Carbodiimides | Carboxylic acids | N-acylureas or anhydrides |
| Diazoalkanes | Carboxylic acids | Esters |

(continued)

| Electrophiles E | Nucleophiles Nu | Covalent bonds $T_b$ |
|---|---|---|
| Epoxides | Thiols | Thioethers |
| Haloacetamides | Thiols | Thioethers |
| Imide esters | Amines | Amidines |
| Isocyanates | Amines | Ureas |
| Isocyanates | Alcohols | Urethanes |
| Isothiocyanates | Amines | Thioureas |
| Maleimides | Thiols | Thioethers |
| Sulphonic esters | Amines | Alkylamines |
| Sulphonic esters | Thiols | Thioethers |
| Sulphonic esters | Carboxylic acids | Esters |
| Sulphonic esters | Alcohols | Ethers |
| Sulphonyl halides | Amines | Sulphonamides |
| ** the activated esters of general formula -CO-LG where LG represents a leaving group such as oxysuccinimidyl, oxybenzotriazolyl or optionally substituted aryloxy;<br>** the acyl azides may undergo rearrangement to give isocyanates.* | | |

These reactions are known to the skilled person and are described in the literature. Reference may be made to the book Advanced Organic Chemistry (ISBN 0-471-60180-2).

One variant to this method is to use a fluorescent chromophore possessing an electrophilic acrylate function (-OCO-C=C-) on which an addition reaction is performed, generating a bond $T_b$.

In certain cases it is necessary to protect the amine carrying the group $R_b$ before the reaction, and then to deprotect it following the reaction between the compounds **(a5)** and **(a6).** The protecting groups on the amines are well known to the skilled person, including the reaction conditions required for the protection and deprotection steps. Reference may be made to the book Protecting groups in Organic Chemistry (ISBN 0-471-05764-9) for the conditions for protection and deprotection.

The compounds **(a5)** are known to the skilled person and are available commercially.

The compounds **(a6)** are known to the skilled person and their synthesis is described in Comprehensive Heterocyclic Chemistry III, 2008, Chapter 4.11, pages 893-954 and Comprehensive Heterocyclic Chemistry III, 2008, Chapter 13.10, pages 299-319. Some compounds are available commercially.

Alternatively, the compounds **(a4)** may be synthesized by a reaction between a compound **(a7)** and a compound **(a8).** This reaction is exemplified in the scheme below:

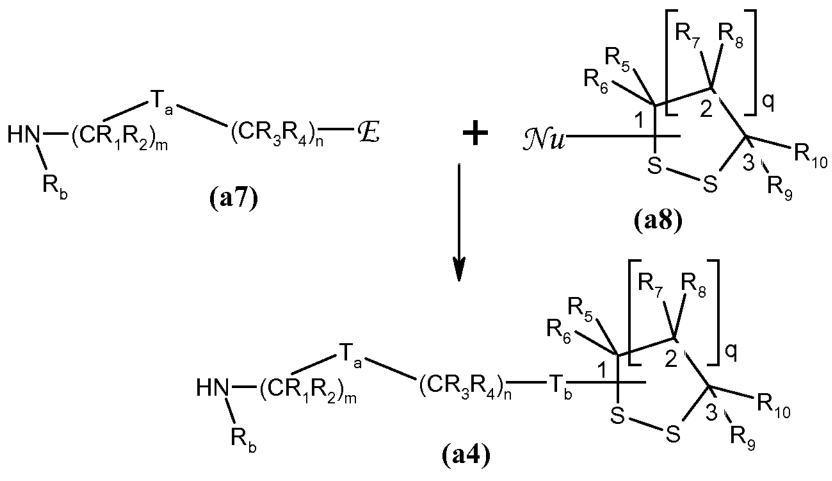

(a7) (a8) (a4)

where $R_1$ to $R_{10}$, $R_a$, $R_b$, G, m, n, $T_a$, $T_b$, E, Nu, $R_h$, $R'_h$ and q are as defined above.

The compounds **(a7)** are known to the skilled person and are described in the literature. Some of them are available commercially.

The compounds **(a8)** are known to the skilled person and their synthesis is described in Comprehensive Heterocyclic Chemistry III, 2008, Chapter 4.11, pages 893-954 and pages 299-319. Some compounds are available commercially.

In certain cases it is necessary to protect the amine carrying the group $R_b$ before the reaction, and then to deprotect it following the reaction between the compounds **(a7)** and **(a8).** The protecting groups on the amines are well known to the skilled person, including the reaction conditions required for the protection and deprotection steps. Reference may be made to the book Protecting groups in Organic Chemistry (ISBN 0-471-05764-9) for the conditions for protection and deprotection.

Alternatively, the compounds **(a2)** may be synthesized by a reaction between a compound **(a9)** and a compound **(a10).** This reaction is exemplified in the scheme below:

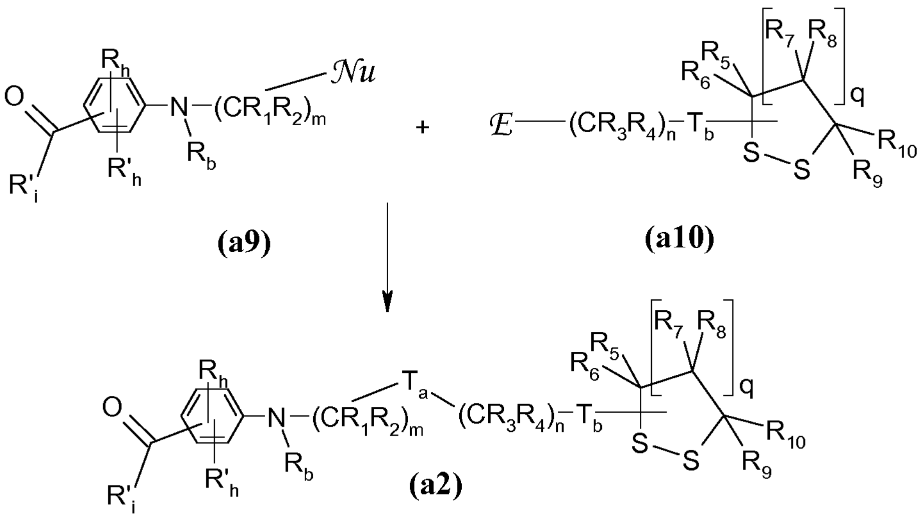

**(a9)** **(a10)** **(a2)**

where $R_1$ to $R_{10}$, $R_a$, $R_b$, G, m, n, $T_a$, $T_b$, $R_i$, $R'_i$, $R_h$, $R'_h$ and q are as defined above; Nu represents a nucleophilic group, E represents an electrophilic group; and $T_a$ represents the bond generated following attack of the nucleophile on the electrophile. By way of example, the covalent bonds $T_a$ which may be generated are listed in Table B, on the basis of condensation of electrophiles with nucleophiles:

Table B

| Electrophiles E | Nucleophiles Nu | Covalent bonds $T_a$ |
|---|---|---|
| Activated esters* | Amines | Carboxamides |
| Acyl azides** | Amines | Carboxamides |
| Acyl halides | Amines | Carboxamides |
| Acyl halides | Alcohols | Esters |
| Acyl cyanides | Alcohols | Esters |
| Acyl cyanides | Amines | Carboxamides |
| Alkyl halides | Amines | Alkylamines |
| Alkyl halides | Carboxylic acids | Esters |
| Alkyl halides | Thiols | Thioesters |
| Alkyl halides | Alcohols | Ethers |
| Sulphonic acids and their salts | Thiols | Thioethers |
| Sulphonic acids and their salts | Carboxylic acids | Esters |
| Sulphonic acids and their salts | Alcohols | Ethers |

(continued)

| Electrophiles E | Nucleophiles Nu | Covalent bonds $T_a$ |
|---|---|---|
| Anhydrides | Alcohols | Esters |
| Anhydrides | Amines | Carboxamides |
| Aryl halides | Thiols | Thioethers |
| Aryl halides | Amines | Arylamines |
| Aziridines | Thiols | Thioethers |
| Carboxylic acids | Amines | Carboxamides |
| Carboxylic acids | Alcohols | Esters |
| Carbodiimides | Carboxylic acids | N-acylureas or anhydrides |
| Diazoalkanes | Carboxylic acids | Esters |
| Epoxides | Thiols | Thioethers |
| Haloacetamides | Thiols | Thioethers |
| Imide esters | Amines | Amidines |
| Isocyanates | Amines | Ureas |
| Isocyanates | Alcohols | Urethanes |
| Isothiocyanates | Amines | Thioureas |
| Maleimides | Thiols | Thioethers |
| Sulphonic esters | Amines | Alkylamines |
| Sulphonic esters | Thiols | Thioethers |
| Sulphonic esters | Carboxylic acids | Esters |
| Sulphonic esters | Alcohols | Ethers |
| Sulphonyl halides | Amines | Sulphonamides |
| ** the activated esters of general formula -CO-LG where LG represents a leaving group such as oxysuccinimidyl, oxybenzotriazolyl or optionally substituted aryloxy;* <br> *** the acyl azides may undergo rearrangement to give isocyanates.* | | |

These reactions are known to the skilled person and are described in the literature. Reference may be made to the book Advanced Organic Chemistry (ISBN 0-471-60180-2).

The compounds **(a10)** are known to the skilled person and are described in the literature. Mention may be made of the publication Comprehensive Heterocyclic Chemistry III, 2008, chapter 4.11, pages 893-954 and chapter 13.10, pages 299-319. Certain compounds are available commercially.

The compounds **(a9)** are known to the skilled person and are described in the literature. Some of them are available commercially. Their synthesis involves reacting a primary or secondary amine (compounds **(a11)**) with a fluorobenzaldehyde (compounds **(a3)**). This reaction is exemplified in the scheme below:

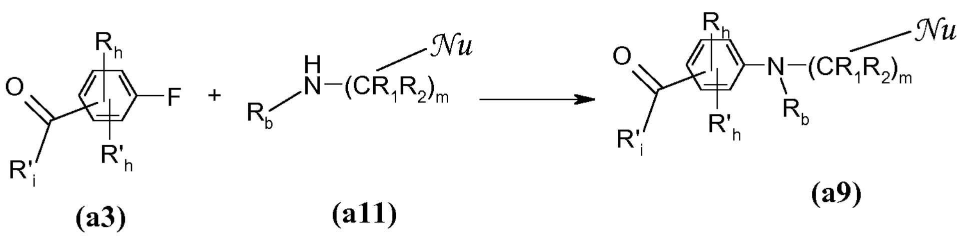

**(a3)** **(a11)** **(a9)**

where $R_1$, $R_2$, $R_b$, m, Nu, $R_h$, $R'_h$ and q are as defined above.

The compounds **(a3)** and **(a11)** are known to the skilled person and are described in the literature. Some of

them are available commercially.

Another variant of the synthesis involves reacting a compound **(a12)** with a heterocyclic compound **(a13).** This reaction is exemplified in the scheme below:

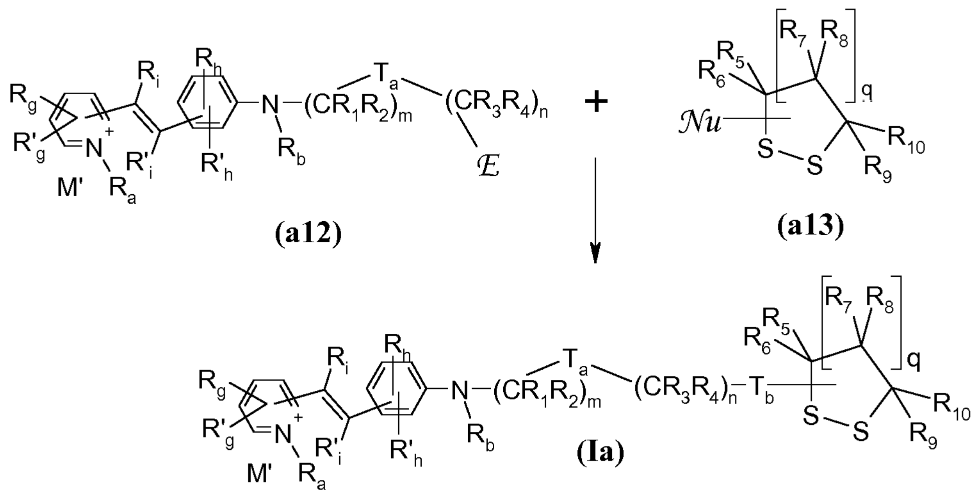

(a12) (a13) (Ia)

where $R_1$ to $R_{10}$, $R_a$, $R_b$, G, m, n, $T_a$, $T_b$, E, Nu, $R_g$, $R'_g$, $R_i$, $R'_i$, $R_h$, $R'_h$, M' and q are as defined above.

The compounds **(a13)** are known to the skilled person and their synthesis is described in Comprehensive Heterocyclic Chemistry III, 2008, chapter 4.11, pages 893-954 and chapter 13.10, pages 299-319. Some compounds are also available commercially.

The dyes of formula **(Ib)** may be synthesized by the synthesis routes described above for the dyes of formula **(Ia).** The main difference is that the compounds **(a14),** compounds containing an indolinium unit, are used in place of the compounds **(a1),** compounds containing a pyridinium unit. The reaction with the compounds **(a14)** is exemplified in the scheme below:

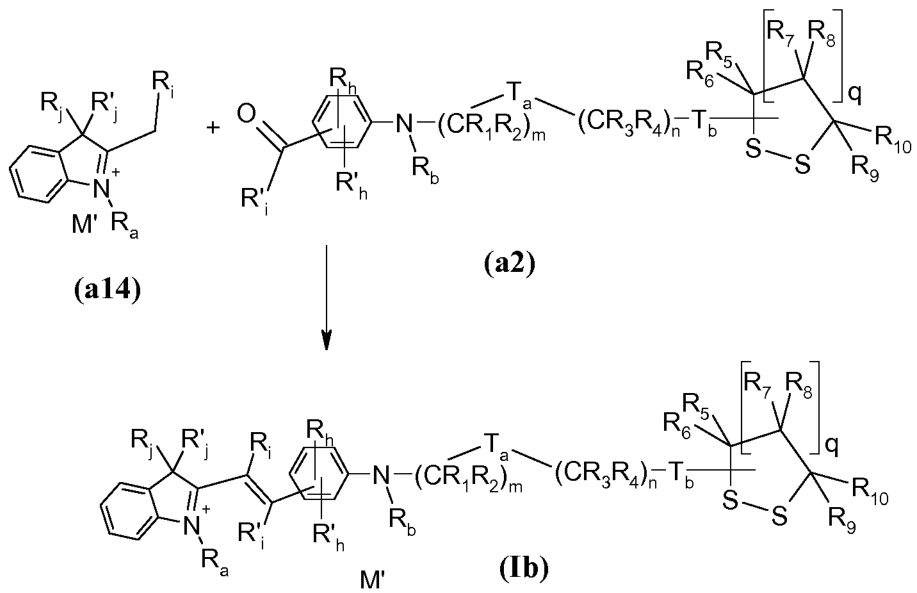

(a14) (a2) (Ib)

where $R_1$ to $R_{10}$, $R_a$, $R_b$, G, m, n, $T_a$, $T_b$, E, Nu, $R_g$, $R'_g$, $R_i$, $R'_i$, $R_h$, $R'_h$, M' and q are as defined above.

The compounds **(a14)** are known to the skilled person and are described in the literature. Some of them are available commercially

Alternatively, the compounds **(Ib)** may be synthesized by a condensation reaction between a compound **(a15)** and a compound **(a13).** The reaction with the compounds **(a15)** is exemplified in the scheme below:

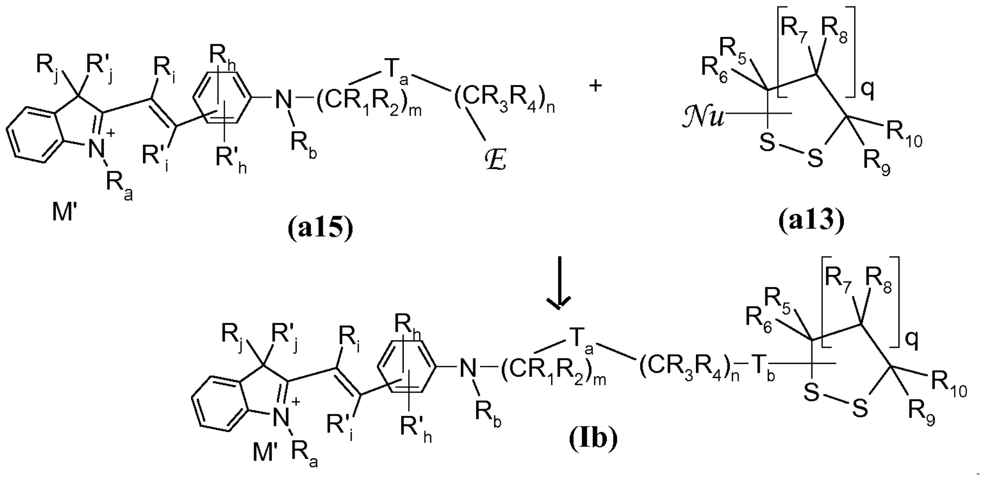

(a15) (a13)

(Ib)

where $R_1$ to $R_{10}$, $R_a$, $R_b$, G, m, n, $T_a$, $T_b$, E, Nu, $R_g$, $R'_g$, $R_i$, $R'_i$, $R_h$, $R'_h$, M' and q are as defined above.

One variant of this synthesis is a condensation reaction between a compound **(a15')** and a compound **(a6).** The reaction with the compounds **(a15')** is exemplified in the scheme below:

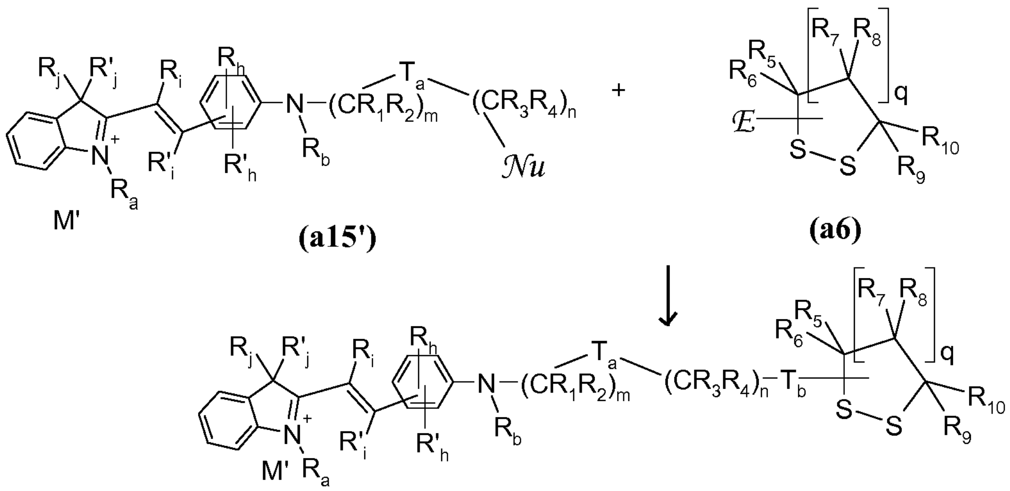

(a15') (a6)

(Ib)

where $R_1$ to $R_{10}$, $R_a$, $R_b$, G, m, n, $T_a$, $T_b$, E, Nu, $R_g$, $R'_g$, $R_i$, $R'_i$, $R_h$, $R'_h$, M' and q are as defined above.

The dyes of formula **(Ic)** may be synthesized by a condensation reaction between a compound **(a1)** and a compound **(a16).** This reaction is well known to the skilled person, and is a Knoevenagel reaction, which involves condensing an aldehyde with a nucleophile of activated methyl type. This reaction is exemplified in the scheme below:

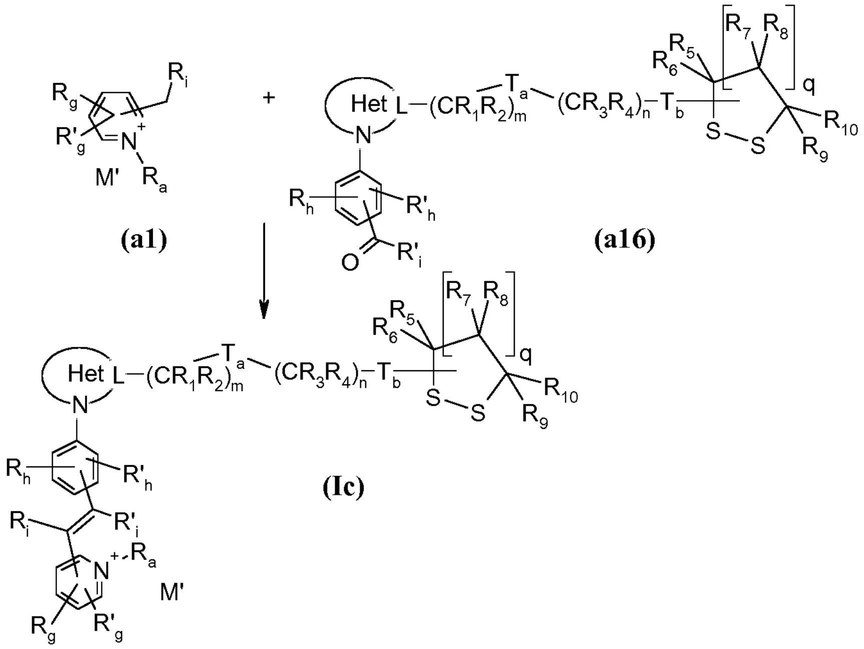

(a1) (a16) (Ic)

where $R_1$ to $R_{10}$, $R_a$, $R_b$, G, m, n, $T_a$, $T_b$, E, Nu, $R_g$, $R'_g$, $R_i$, $R'_i$, $R_h$, $R'_h$, M', Het, L and q are as defined above.

The compounds **(a1)** are known to the skilled person and are described in the literature. Some of them are available commercially.

The compounds **(a16)** may be synthesized by a nucleophilic substitution reaction between a compound **(a3)** and a compound **(a17)**. This reaction is well known to the skilled person.

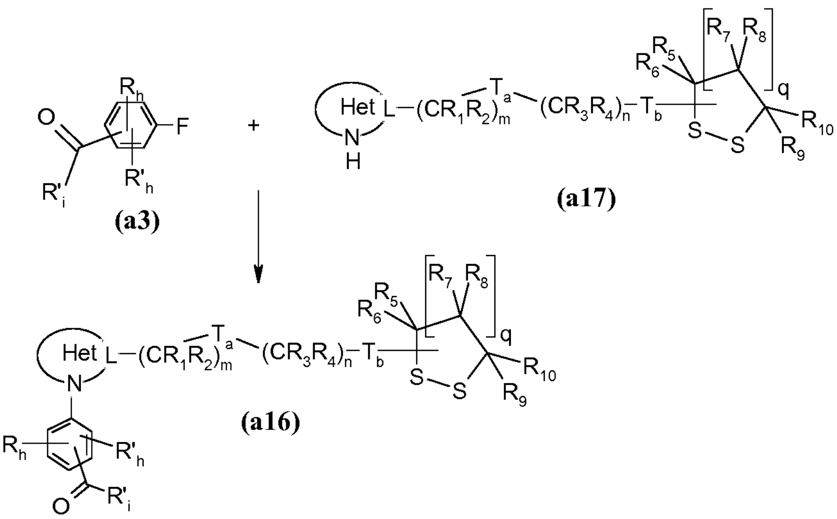

(a3) (a17) (a16)

where $R_1$ to $R_{10}$, $R_b$, G, m, n, $T_a$, $T_b$, E, Nu, $R_i$, $R'_i$, $R_h$, $R'_h$, M, Het, L and q are as defined above.

The fluorobenzaldehyde compounds **(a3)** are known to the skilled person and/or are available commercially.

The compounds **(a17)** may be synthesized by a reaction between a compound **(a18)** and a compound **(a6)**. This reaction is exemplified in the scheme below:

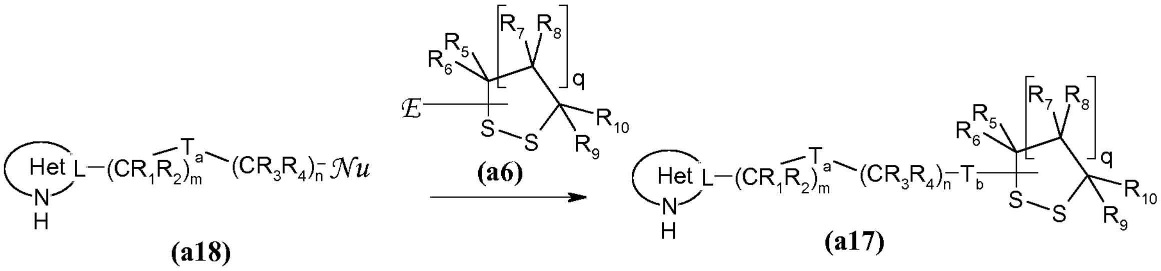

(a18) (a6) (a17)

where $R_1$ to $R_{10}$, G, m, n, $T_a$, $T_b$, E, Nu, Het, L and q are as defined above.

The compounds **(a18)** are known to the skilled person and are described in the literature. Some of them are available commercially, and the compounds **(a6)** are as described above.

Alternatively, the compounds **(a17)** may be synthesized by a reaction between a compound **(a19)** and a compound **(a8).** This reaction is exemplified by the scheme below:

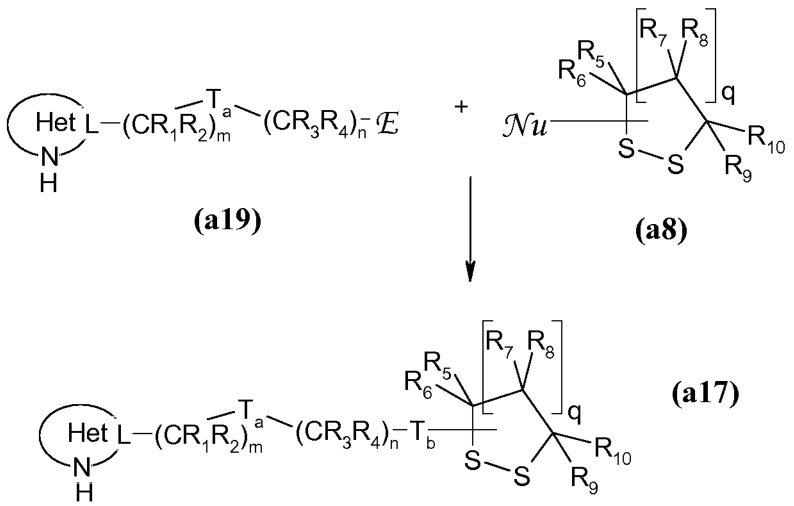

(a19) (a8) (a17)

where $R_1$ to $R_{10}$, G, m, n, $T_a$, $T_b$, E, Nu, Het, L and q are as defined above.

The compounds **(a19)** are known to the skilled person and are described in the literature. Some of them are available commercially, and the compounds **(a8)** are as described above.

Alternatively, the compounds **(a16)** may be synthesized by a reaction between a compound **(a20)** and a compound **(a10).** This reaction is exemplified by the scheme below:

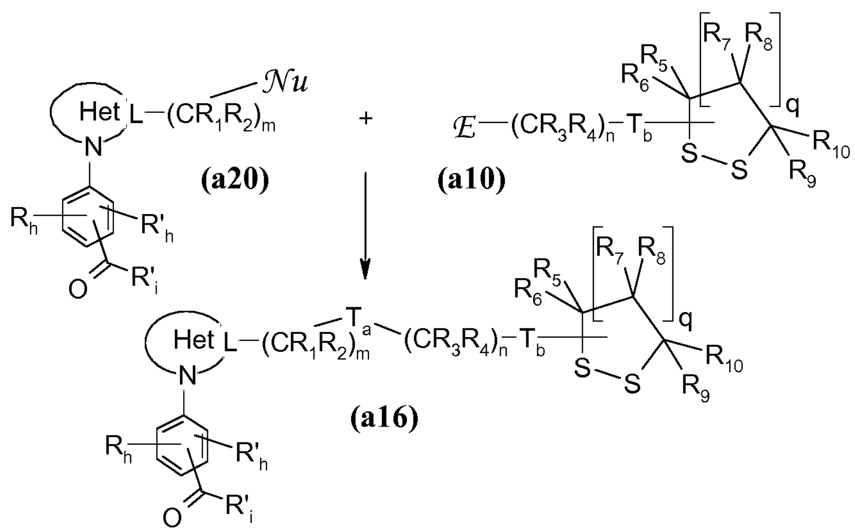

(a20) (a10) (a16)

where $R_1$ to $R_{10}$, $R_a$, $R_b$, G, m, n, $T_a$, $T_b$, E, Nu, $R_i$, $R'_i$, $R_h$, $R'_h$, Het, L and q are as defined above.

The compounds **(a20)** may be synthesized by reacting a secondary amine with a fluorobenzaldehyde. This reaction is exemplified in the scheme below:

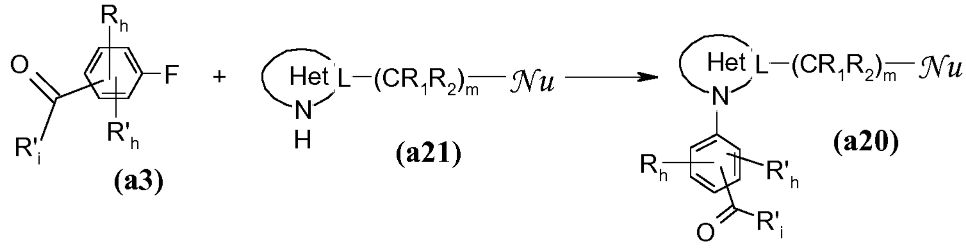

(a3) (a21) (a20)

where $R_1$, $R_2$, $R_a$, $R_b$, m, n, $T_a$, $T_b$, Nu, $R_i$, $R'_i$, $R_h$, $R'_h$, Het, L and q are as defined above.

The compounds **(a3)** and **(a21)** are known to the skilled person and are described in the literature. Some of them are available commercially.

Another variant of the synthesis of the compounds **(Ic)** involves reacting a compound **(a22)** with a compound **(a13)** according to the following scheme:

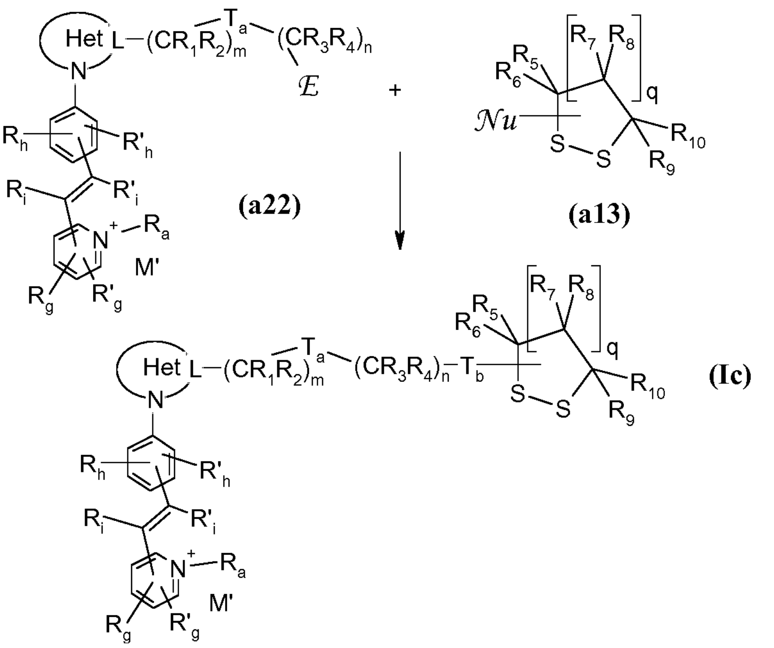

(a22) (a13) (Ic)

where $R_1$ to $R_{10}$, $R_a$, $R_b$, G, m, n, $T_a$, $T_b$, E, Nu, $R_g$, $R'_g$, $R_i$, $R'_i$, $R_h$, $R'_h$, M', Het, L and q are as defined above; the compounds **(a13)** are as described above.

The dyes of formula **(Id)** may be synthesized by a condensation reaction between a compound **(a23)** and a compound **(a24)**. This reaction is well known to the skilled person, and is a Knoevenagel reaction, which involves condensing an aldehyde with a nucleophile of activated methyl type. This reaction is exemplified in the scheme below:

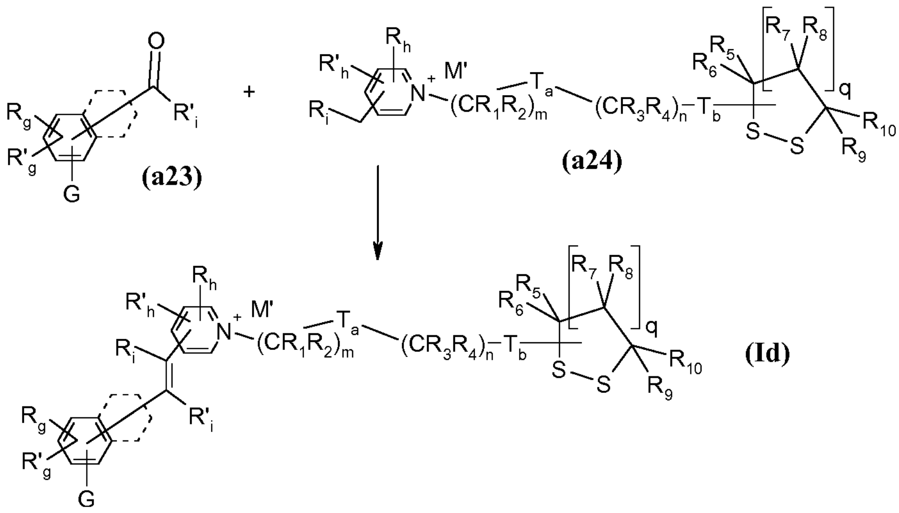

(a23) (a24) (Id)

where $R_1$ to $R_{10}$, $R_a$, $R_b$, G, m, n, $T_a$, $T_b$, E, Nu, $R_g$, $R'_g$, $R_i$, $R'_i$, $R_h$, $R'_h$, M' and q are as defined above.

The compounds **(a23)** are known to the skilled person and are described in the literature. Some of them are available commercially.

The compounds **(a24)** may be synthesized by a quaternization reaction between an electrophilic compound **(a25)** and a pyridine compound **(a26).** This reaction is well known to the skilled person.

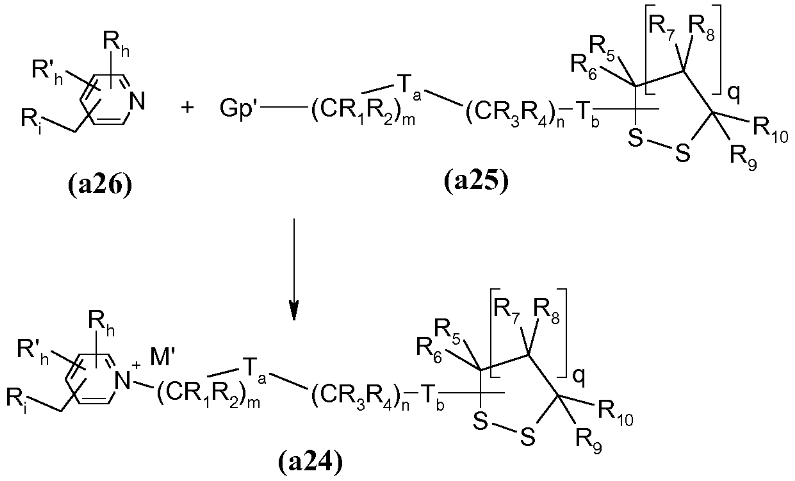

(a26) (a25) (a24)

where $R_1$ to $R_{10}$, $R_a$, $R_b$, m, n, $T_a$, $T_b$, E, Nu, $R_i$, $R'_i$, M' and q are as defined above.

The group Gp' represents a leaving group which when it has left gives rise to an anionic counterion. Mention may be made of halides such as iodide, bromide and chloride, and of the mesylate, sulphate, phosphate, triflate or tosylate group.

The compounds **(a26)** are known to the skilled person and are available commercially.

The compounds **(a25)** may be synthesized by a reaction between a compound **(a27)** and a compound **(a6).** This reaction is exemplified in the scheme below:

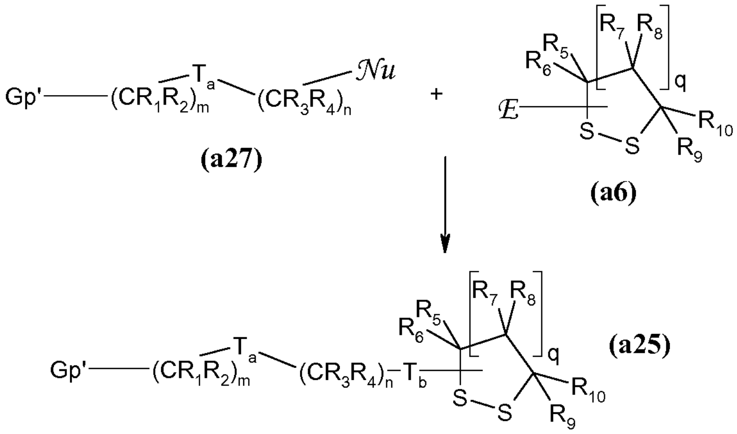

(a27) (a6) (a25)

where $R_1$ to $R_{10}$, $R_a$, $R_b$, m, n, $T_a$, $T_b$, E, Nu, M and q are as defined above.

The compounds **(a6)** and **(a27)** are known to the skilled person and are available commercially.

Alternatively, the compounds **(a25)** may be synthesized by a reaction between a compound **(a28)** and a compound **(a8).** This reaction is exemplified in the scheme below:

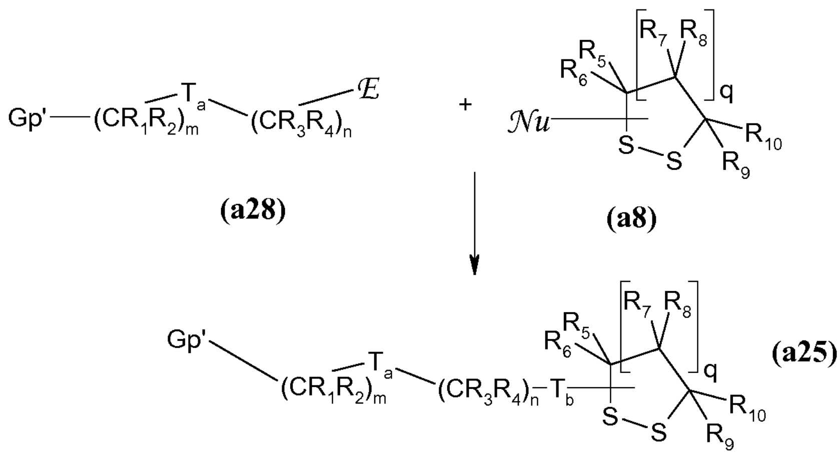

(a28) (a8) (a25)

where $R_1$ to $R_{10}$, $R_a$, $R_b$, G, m, n, $T_a$, $T_b$, E, Nu, $R_g$, $R'_g$, $R_i$, $R'_i$, $R_h$, $R'_h$, M and q are as defined above.

The compounds **(a28)** are known to the skilled person and are described in the literature. Some of them are available commercially.

The compounds **(a8)** are described above.

Alternatively, the compounds **(Id)** may be synthesized by a reaction between a compound **(a29)** and a compound **(a6).** This reaction is exemplified in the scheme below:

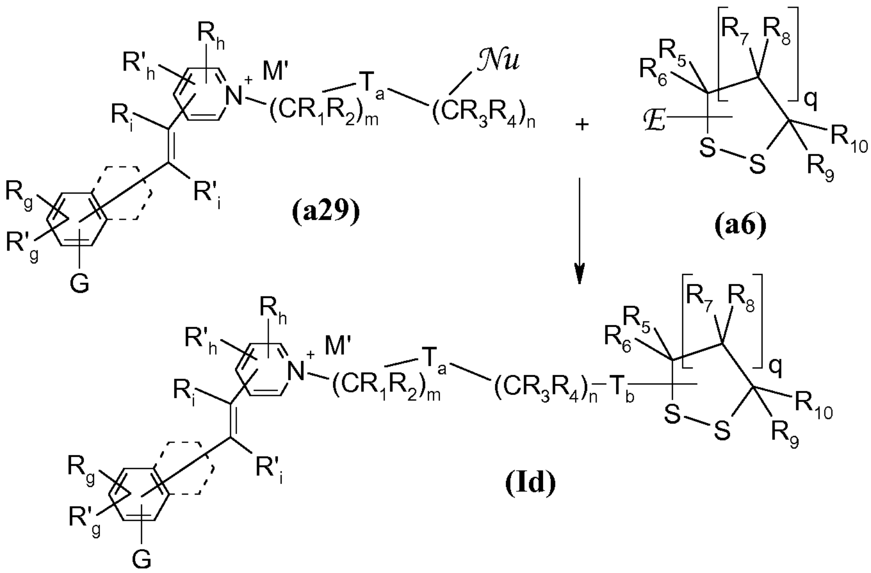

(a29) (a6) (Id)

where $R_1$ to $R_{10}$, $R_a$, $R_b$, G, m, n, $T_a$, $T_b$, E, Nu, $R_g$, $R'_g$, $R_i$, $R'_i$, $R_h$, $R'_h$, M and q are as defined above; and the compounds **(a6)** are as described above.

The compounds **(a29)** may be synthesized by a reaction between a compound **(a30)** and a compound **(a23).** This reaction is exemplified in the scheme below:

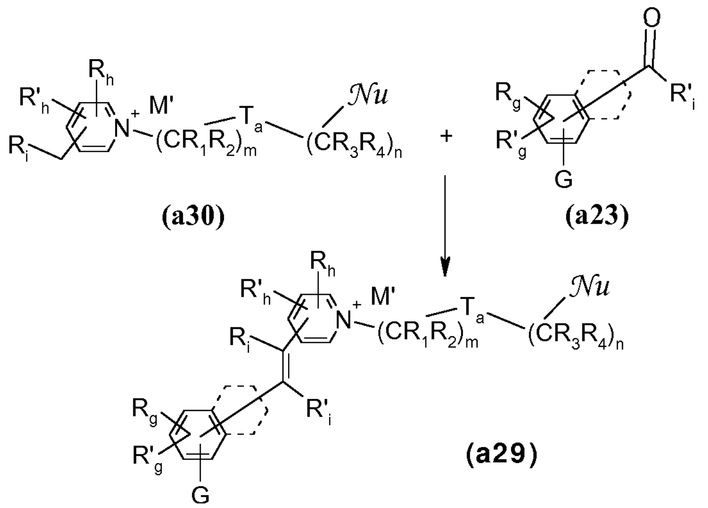

(a30) (a23) (a29)

where $R_1$ to $R_{10}$, $R_a$, $R_b$, G, m, n, $T_a$, E, Nu, $R_g$, $R'_g$, $R_i$, $R'_i$, $R_h$, $R'_h$, M and q are as defined above.

The compounds **(a23)** are as defined above.

The compounds **(a30)** may be synthesized by a reaction between a compound **(a27)** and a pyridine derivative **(a26).** This reaction is exemplified by the scheme below:

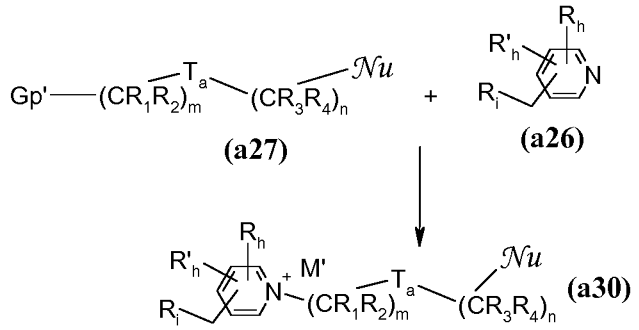

(a27) (a26) (a30)

where $R_1$ to $R_{10}$, $R_a$, $R_b$, G, m, n, $T_a$, E, Nu, $R_g$, $R'_g$, $R_i$, $R'_i$, $R_h$, $R'_h$, M and q are as defined above.

The compounds **(a27)** and **(a26)** are as defined above. They are known to the skilled person and are described in the literature. Some of them are available commercially.

Another variant of the synthesis involves reacting a compound **(a31)** with the compounds **(a8)**

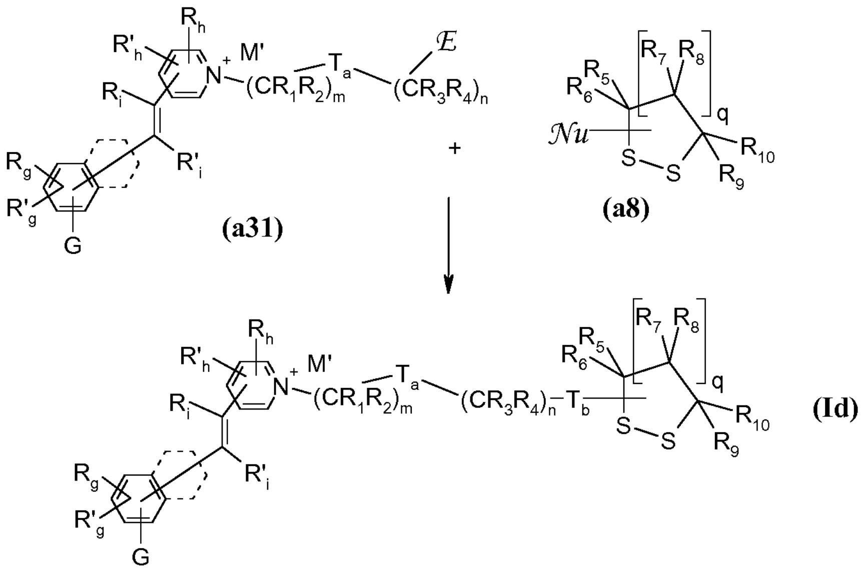

(a31) (a8) (Id)

where $R_1$ to $R_{10}$, $R_a$, $R_b$, G, m, n, $T_a$, $T_b$, E, Nu, $R_g$, $R'_g$, $R_i$, $R'_i$, $R_h$, $R'_h$, M and q are as defined above; and the compounds **(a8)** are as described above.

The compounds **(a31)** may be synthesized by a reaction between a compound **(a32)** and a compound **(a23).** This reaction is exemplified in the scheme below:

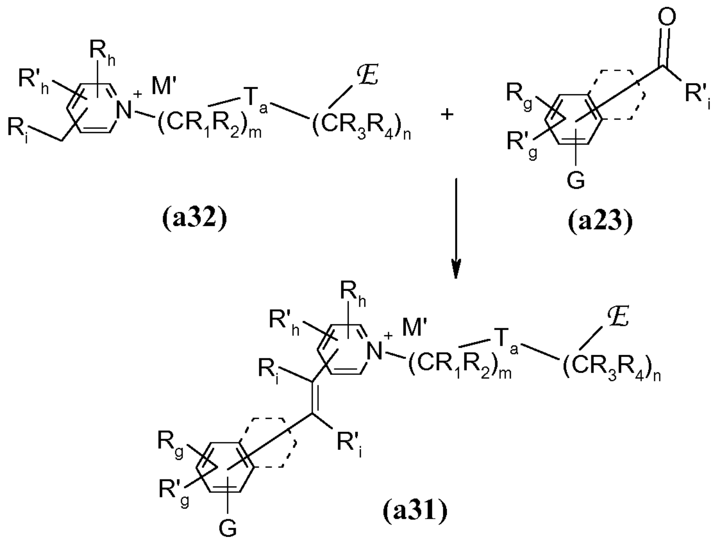

(a32) (a23) (a31)

where $R_1$ to $R_4$, $R_a$, $R_b$, G, m, n, $T_a$, E, $R_g$, $R'_g$, $R_i$, $R'_i$, $R_h$, $R'_h$, M and q are as defined above; and the compounds **(a23)** are as defined above.

The compounds **(a32)** may be synthesized by a reaction between a compound **(a28)** and a compound **(a26).** This reaction is exemplified by the scheme below:

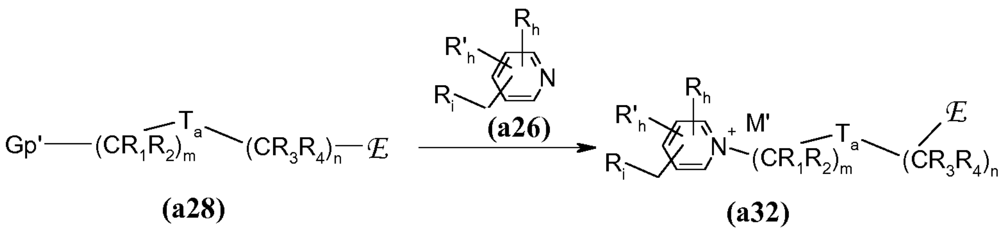

(a26) (a28) (a32)

where $R_1$ to $R_4$, $R_a$, $R_b$, m, n, $T_a$, E, $R_i$, $R'_i$, $R_h$, $R'_h$, M' and Gp' are as defined above; and the compounds **(a28)** and **(a26)** are described above. They are known to the skilled person and are described in the literature. Some of them are available commercially.

The compounds **(Ie)** may be synthesized by a reaction between a compound **(a33)** and a compound **(a8).** This reaction is exemplified in the scheme below:

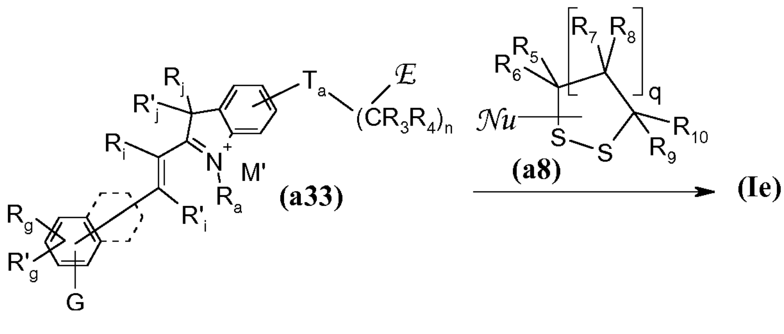

(a33) (a8) (Ie)

where $R_1$ to $R_{10}$, $R_a$, $R_b$, G, m, n, $T_a$, $T_b$, E, Nu, $R_g$, $R'_g$, $R_i$, $R'_i$, $R_j$, $R'_j$, $R_h$, $R'_h$, M' and q are as defined above; and the compounds **(a8)** are described above.

The compounds **(a33)** may be synthesized by a reaction between a compound **(a34)** and a compound **(a34')**. This reaction is exemplified in the scheme below:

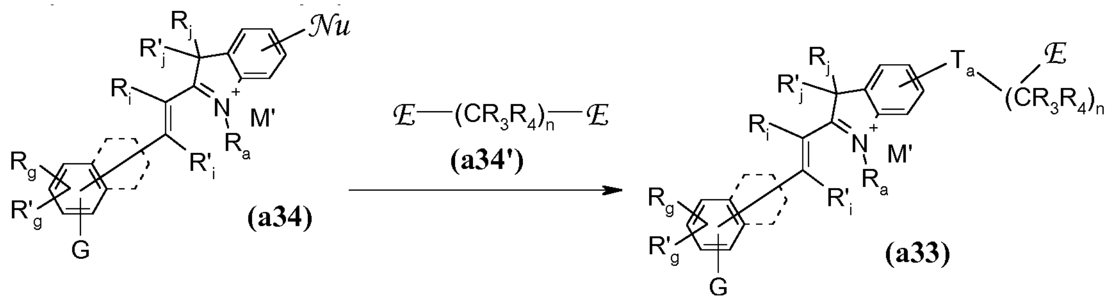
(a34) (a34') (a33)

where $R_3$, $R_4$, $R_a$, $R_b$, G, m, n, $T_a$, E, Nu, $R_g$, $R'_g$, $R_i$, $R'_i$, $R_j$, $R'_j$ and M' are as defined above; the compounds **(a34)** are known to the skilled person and are described in the literature. Some of them are available commercially.

The compounds **(a34)** may be synthesized by a reaction between a compound **(a35)** and a compound **(a23).** This reaction is exemplified with the scheme below:

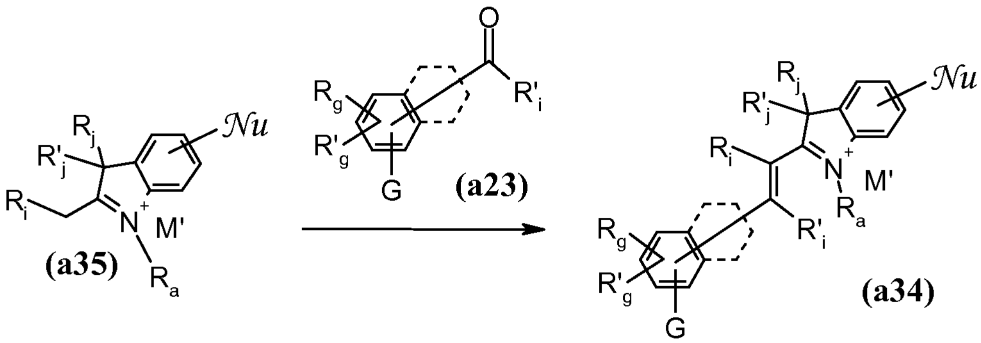
(a35) (a23) (a34)

where **(Ie),** $R_a$, G, m, n, Nu, $R_g$, $R'_g$, $R_i$, $R'_i$, $R_j$, $R'_j$ and M' are as defined above.

The compounds **(a35)** are known to the skilled person and are described in the literature. Some of them are available commercially, and the compounds **(a23)** are as defined above.

Alternatively, the compounds **(Ie)** may be synthesized by a reaction between a compound **(a36)** and a compound **(a6).** This reaction is exemplified with the scheme below:

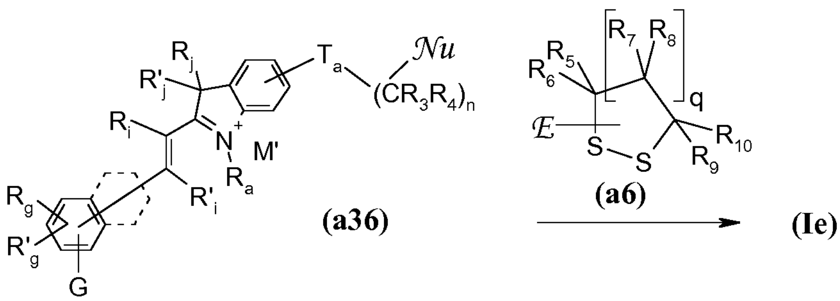
(a36) (a6) (Ie)

where **(Ie),** $R_1$ to $R_{10}$, $R_a$, $R_b$, G, m, n, $T_a$, $T_b$, E, Nu, $R_g$, $R'_g$, $R_i$, $R'_i$, $R_j$, $R'_j$, $R_h$, $R'_h$, M' and q are as defined above; and the compounds **(a6)** are as described above.

The compounds **(a36)** may be synthesized by a reaction between a compound **(a37)** and a compound **(a38).** This reaction is exemplified with the scheme below:

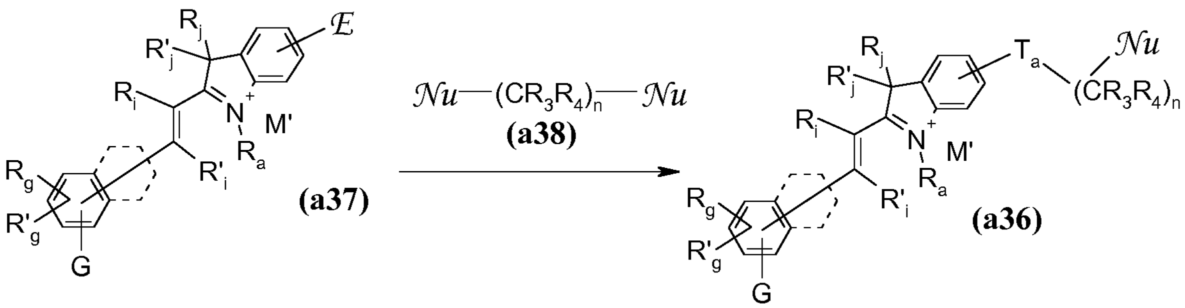


(a37) (a36)

where $R_3$, $R_4$, $R_a$, $R_b$, G, m, n, $T_a$, E, Nu, $R_g$, $R'_g$, $R_i$, $R'_i$, $R_j$, $R'_j$, M' and q are as defined above.

The compounds **(a38)** are known to the skilled person and are described in the literature. Some of them are available commercially.

The compounds **(a37)** may be synthesized by a reaction between an indolinium derivative **(a37')** and a compound containing a carbonyl group **(a23).** This reaction is exemplified in the scheme below:

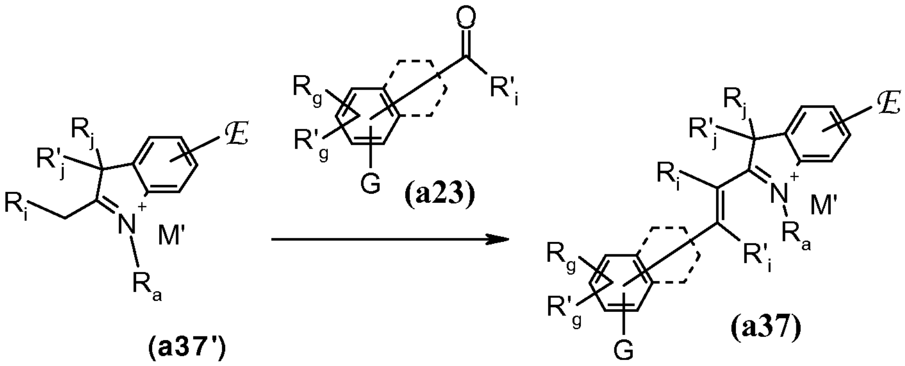

(a23) (a37') (a37)

where $R_a$, G, m, n, E, $R_g$, $R'_g$, $R_i$, $R'_i$, $R_j$, $R'_j$, $R_h$, $R'_h$ and M' are as defined above.

The compounds **(a37')** are known to the skilled person and are described in the literature. Some of them are available commercially and the compounds **(a23)** are as described above.

The compounds **(Ie)** may also be synthesized by a reaction between a compound containing a carbonyl group **(a23)** and a compound **(a39).** This reaction is exemplified with the scheme below:

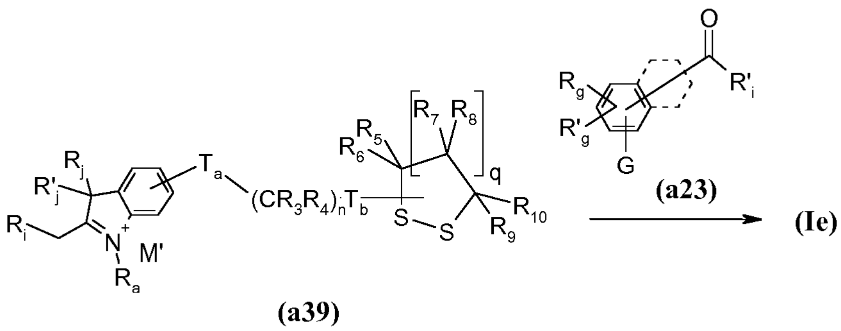

(a23) (Ie) (a39)

where **(Ie),** $R_1$ to $R_{10}$, $R_a$, $R_b$, G, m, n, $T_a$, $T_b$, E, Nu, $R_g$, $R'_g$, $R_i$, $R'_i$, $R_j$, $R'_j$, $R_h$, $R'_h$, M' and q are as defined above; the compounds **(a23)** are described above.

The compounds **(a39)** may be synthesized by a reaction between a compound **(a40)** and a compound **(a35).** This reaction is exemplified with the scheme below:

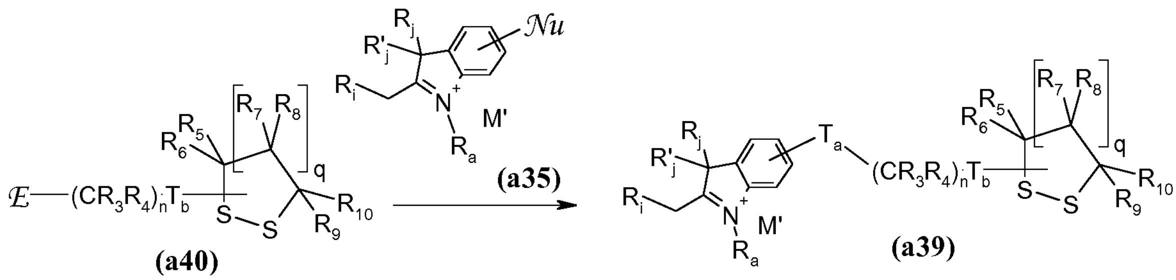

(a40) (a35) (a39)

where $R_1$ to $R_{10}$, $R_a$, $R_b$, G, m, n, $T_a$, $T_b$, E, Nu, $R_g$, $R'_g$, $R_i$, $R'_i$, $R_j$, $R'_j$, $R_h$, $R'_h$, M' and q are as defined above.

The compounds **(a35)** are known to the skilled person and are described in the literature. Some of them are available commercially. The compounds **(a40)** may be synthesized by a reaction between a compound **(a34)** and a compound **(a8).** This reaction is exemplified in the scheme below:

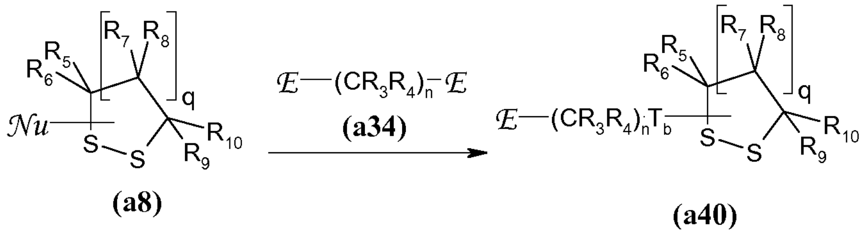

(a8) (a34) (a40)

where $R_1$ to $R_{10}$, $R_a$, $R_b$, G, m, n, $T_a$, $T_b$, E, Nu, $R_g$, $R'_g$, $R_i$, $R'_i$, $R_h$, $R'_h$ and q are as defined above.

The compounds **(a8)** and **(a34)** are known to the skilled person, and some of them are available commercially.

Alternatively, the compounds **(a39)** may be synthesized by a reaction between a compound **(a41)** and a compound **(a38).** This reaction is exemplified in the scheme below:

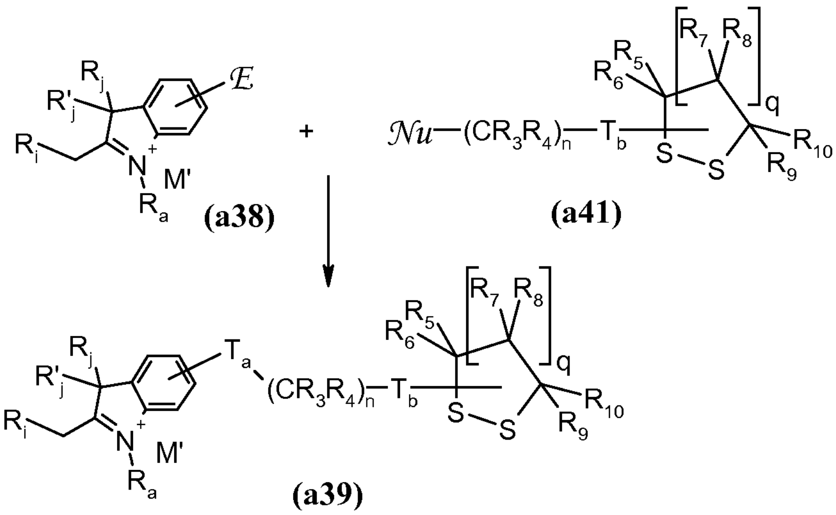

(a38) + (a41) (a39)

where $R_1$ to $R_{10}$, $R_a$, $R_b$, G, m, n, $T_a$, $T_b$, E, Nu, $R_g$, $R'_g$, $R_i$, $R'_i$, $R_j$, $R'_j$, $R_h$, $R'_h$, M and q are as defined above.

The compounds **(a38)** are known to the skilled person and are available commercially.

The compounds **(a41)** may be synthesized by a reaction between a compound **(a6)** and a compound **(a38).** This reaction is exemplified in the scheme below:

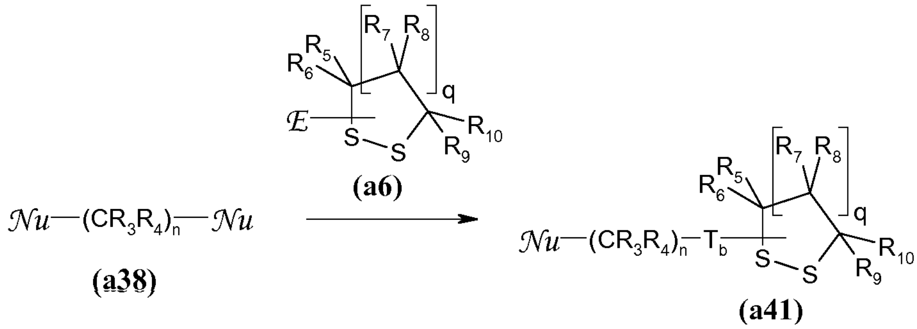

(a6) (a38) (a41)

where $R_1$ to $R_{10}$, $R_a$, $R_b$, G, m, n, $T_a$, $T_b$, E, Nu, $R_g$, $R'_g$, $R_i$, $R'_i$, $R_h$, $R'_h$, M and q are as defined above.

The compounds **(a6)** are described above. The compounds **(a38)** are known to the skilled person and are available commercially.

The compounds **(If)** may be synthesized by a reaction between a compound **(a23)** and a compound **(a42).** This reaction is exemplified in the scheme below:

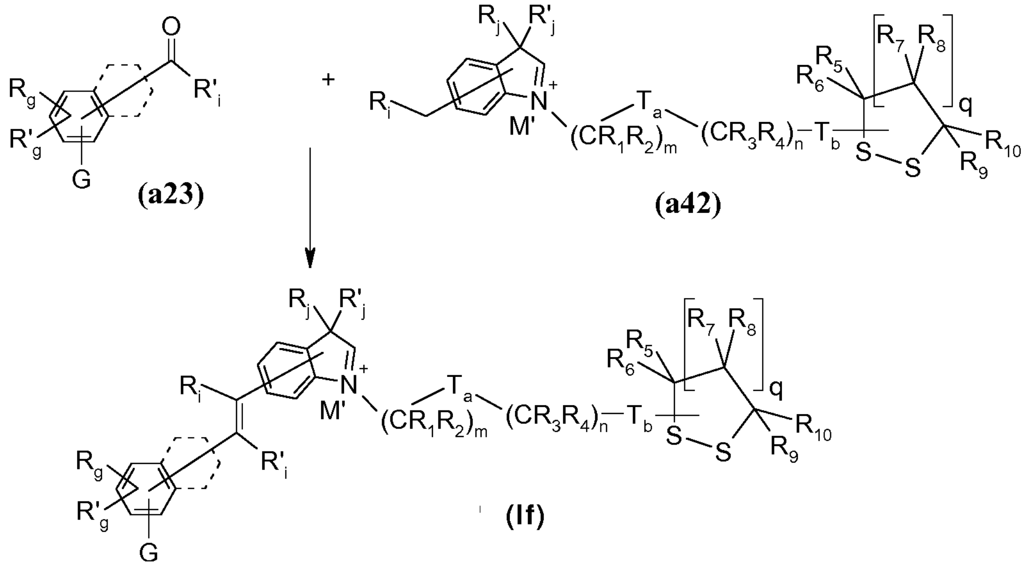

(a23) (a42) (If)

where $R_1$ to $R_{10}$, $R_a$, $R_b$, G, m, n, $T_a$, $T_b$, E, Nu, $R_g$, $R'_g$, $R_i$, $R'_i$, $R_j$, $R'_j$, $R_h$, $R'_h$, M and q are as defined above. The compounds **(a23)** are as described above.

The compounds **(a42)** may be synthesized by a reaction between a compound **(a43)** and a compound **(a25).** This reaction is exemplified in the scheme below:

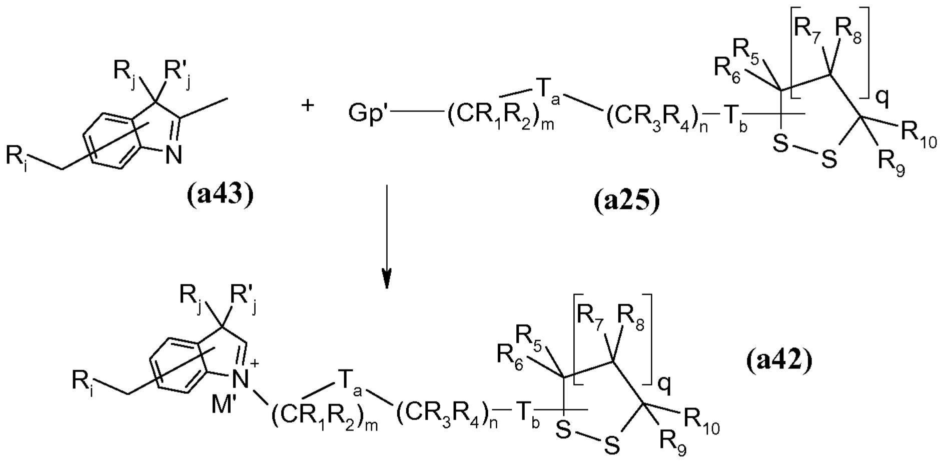

(a43) (a25) (a42)

where $R_1$ to $R_{10}$, $R_a$, $R_b$, G, m, n, $T_a$, $T_b$, E, Nu, $R_g$, $R'_g$, $R_i$, $R'_i$, $R_j$, $R'_j$, $R_h$, $R'_h$, M and q are as defined above.

The group Gp' represents a leaving group which, once liberated, will generate an anionic counterion M'. Mention may be made of halides such as iodide, bromide and chloride, and of the triflate, mesylate, sulphate, phosphate or tosylate group.

The compounds **(a43)** are known to the skilled person and are available commercially. The compounds **(a25)** are as described above.

Alternatively, the compounds **(If)** may be synthesized by a reaction between a compound **(a44)** and a compound **(a6).** This reaction is exemplified in the scheme below:

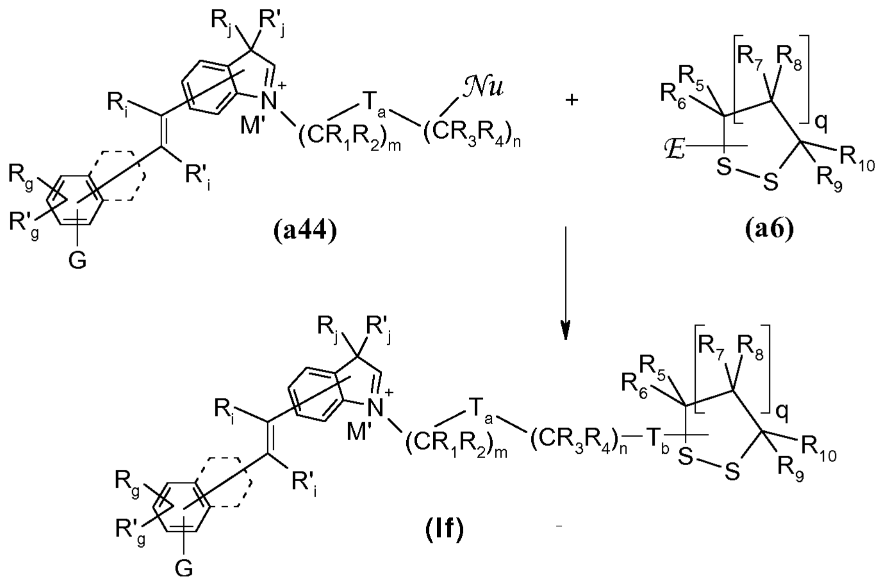

(a44) (a6) (If)

where $R_1$ to $R_{10}$, $R_a$, $R_b$, G, m, n, $T_a$, $T_b$, E, Nu, $R_g$, $R'_g$, $R_i$, $R'_i$, $R_j$, $R'_j$, $R_h$, $R'_h$, M and q are as defined above. The compounds **(a6)** are as described above.

The compounds **(a44)** may be synthesized by a reaction between a compound **(a45)** and a compound **(a23).** This reaction is exemplified in the scheme below:

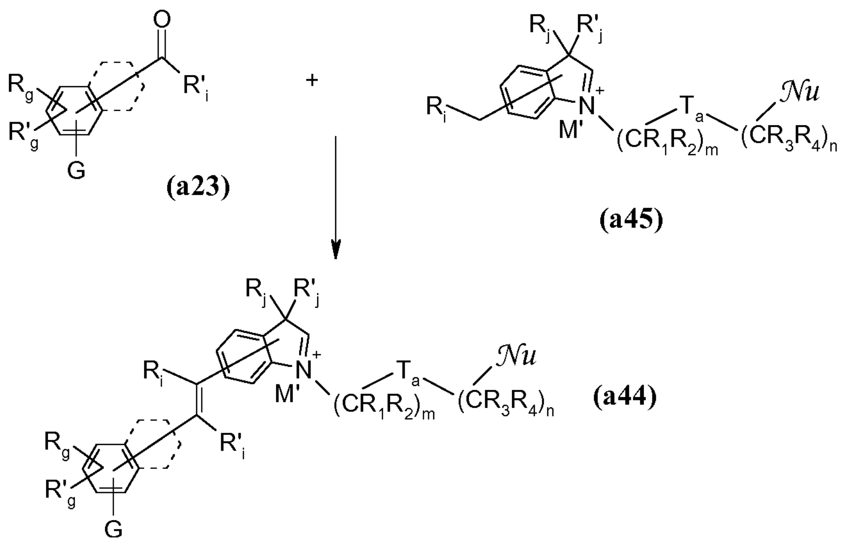

(a23) (a45) (a44)

where $R_1$ to $R_{10}$, $R_a$, $R_b$, G, m, n, $T_a$, $T_b$, E, Nu, $R_g$, $R'_g$, $R_i$, $R'_i$, $R_j$, $R'_j$, $R_h$, $R'_h$, M and q are as defined above. The compounds **(a23)** are as described above.

The compounds **(a45)** may be synthesized by a reaction between a compound **(a46)** and a compound **(a27).** This reaction is exemplified in the scheme below:

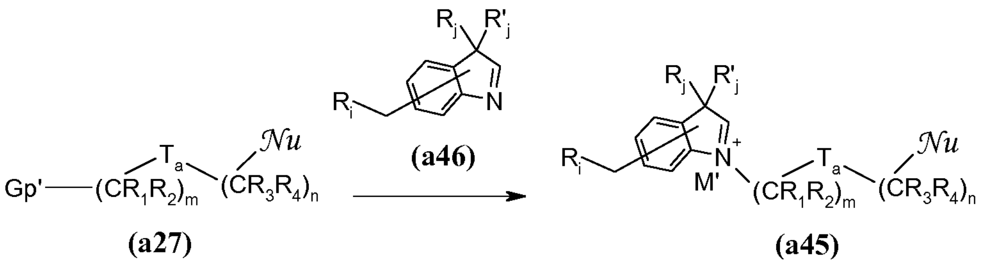

(a27) (a46) (a45)

where $R_1$ to $R_4$, G, m, n, $T_a$, Nu, $R_i$, $R'_i$, $R_j$, $R'_j$, and M are as defined above; the compounds **(a27)** are as described above. The compounds **(a46)** are known to the skilled person and are described in the literature.

Alternatively, the compounds **(If)** may be synthesized by a reaction between a compound **(a44')** and a compound **(a8).** This reaction is exemplified in the scheme below:

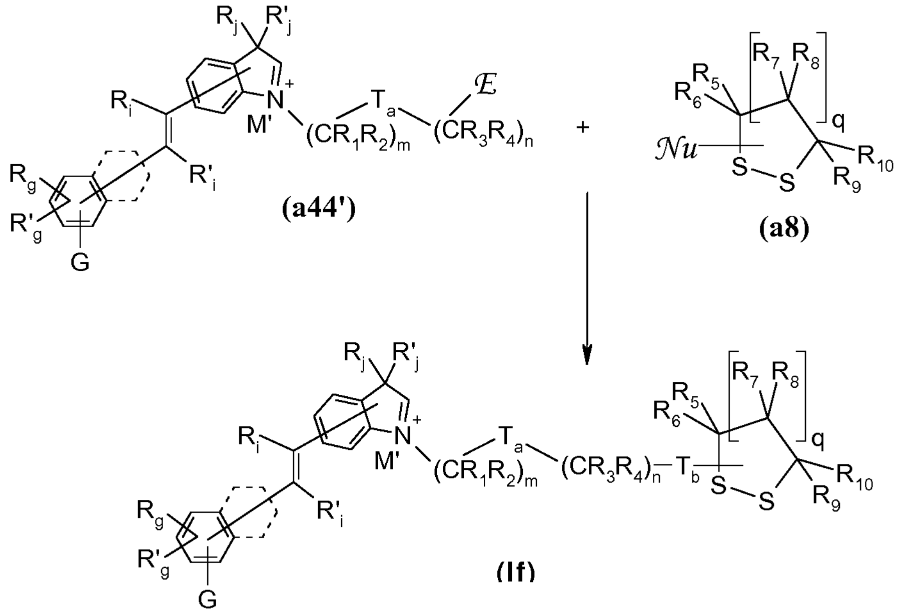

(a44') + (a8)

(If)

where $R_1$ to $R_{10}$, $R_a$, $R_b$, G, m, n, $T_a$, $T_b$, E, Nu, $R_g$, $R'_g$, $R_i$, $R'_i$, $R_j$, $R'_j$, M and q are as defined above. The compounds **(a8)** are as described above.

The compounds **(a44')** may be synthesized by a reaction between a compound **(a45')** and a compound **(a23).** This reaction is exemplified in the scheme below:

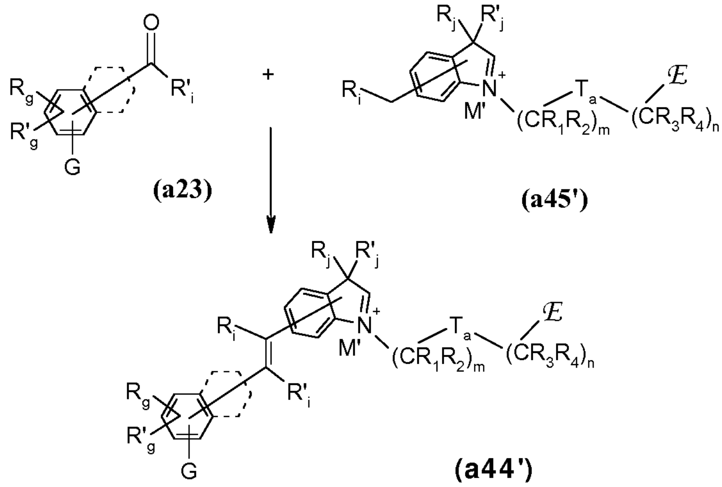

(a23) + (a45')

(a44')

where $R_1$ to $R_4$, G, m, n, $T_a$, $T_b$, E, $R_g$, $R'_g$, $R_i$, $R'_i$, $R_j$, $R'_j$ and M are as defined above. The compounds **(a23)** are as described above.

The compounds **(a45')** may be synthesized by a reaction between a compound **(a46)** and a compound **(a28).** This reaction is exemplified in the scheme below:

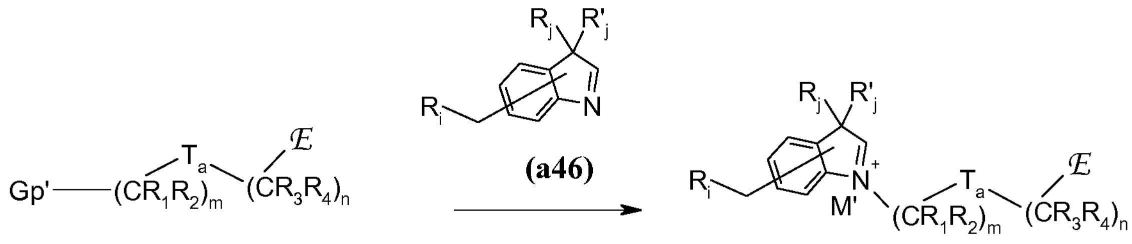

**(a28)** **(a46)** **(a45')**

where $R_1$ to $R_4$, G, m, n, $T_a$, E, $R_i$, $R'_i$, $R_j$, $R'_j$ and M are as defined above. The compounds **(a28)** and **(a46)** are as described above.

*II. Composition comprising the dye of formulae (I)*

A further subject of the invention is a colouring composition as defined in claim 17. In an embodiment, the dye of formula (I) is a fluorescent dye. Further to the presence of at least one fluorescent dye of formula **(I),** the composition of the invention may also include a reducing agent.
Preferably, the composition does not contain a reducing agent.

The colouring composition that can be used in the invention generally comprises an amount of dye of formula **(I)** of between 0.001% and 50%, relative to the total weight of the composition. Preferably this amount is between 0.005% and 20% by weight and more preferably still between 0.01% and 5% by weight, relative to the total weight of the composition.

The colouring composition may further comprise additional direct dyes. These direct dyes are selected, for example, from neutral, acidic or cationic nitrobenzene direct dyes, neutral, acidic or cationic azo direct dyes, tetraazapentamethine dyes, neutral, acidic or cationic quinione dyes and especially anthraquinone dyes, azine direct dyes, triarylmethane direct dyes, indoamine direct dyes and natural direct dyes.

The natural direct dyes include lawsone, juglone, alizarin, purpurin, carminic acid, kermesic acid, purpurogallin, protocatechaldehyde, indigo, isatin, curcumin, spinulosin and apigenidin. It is also possible to use extracts or decoctions containing these natural dyes, and especially poultices or extracts based on henna.

The colouring composition may comprise one or more oxidation bases and/or one or more couplers which are conventionally used for the dyeing of keratinous fibres.

The oxidation bases include para-phenylenediamines, bisphenylalkylenediamines, para-aminophenols, bis-para-aminophenols, ortho-aminophenols, heterocyclic bases and the addition salts thereof.

These couplers include in particular meta-phenylenediamines, meta-aminophenols, meta-diphenols, naphthalenic couplers, heterocyclic couplers and the addition salts thereof.

The coupler or couplers are each generally present in an amount of between 0.001% and 10% by weight of the total weight of the colouring composition, preferably between 0.005% and 6%.

The oxidation base or bases present in the colouring composition are generally each present in an amount of between 0.001% to 10% by weight of the total weight of the colouring composition, preferably between 0.005% and 6% by weight.

Generally speaking, the addition salts of the oxidation bases and of the couplers that can be used in the context of the invention are selected particularly from the addition salts with an acid, such as hydrochlorides, hydrobromides, sulphates, citrates, succinates, tartrates, lactates, tosylates, benzenesulphonates, phosphates and acetates, and the addition salts with a base, such as alkali metal hydroxides, for instance aqueous sodium hydroxide, aqueous potassium hydroxide, aqueous ammonia, amines or alkanolamines.

The medium appropriate for dyeing, also referred to as dyeing vehicle, is a cosmetic medium which is generally composed of water or of a mixture of water and at least one organic solvent. Organic solvent includes, for example, $C_1$-$C_4$ lower alkanols, such as ethanol and isopropanol; polyols and polyol ethers such as 2-butoxyethanol, propylene glycol, propylene glycol monomethyl ether, diethylene glycol monoethyl ether and monomethyl ether, and also aromatic alcohols such as benzyl alcohol or phenoxyethanol, and mixtures thereof.

The solvents, when present, are preferably present in proportions of preferably between 1% and 99% by weight,

approximately, relative to the total weight of the colouring composition, and even more preferably between 5% and 95% by weight, approximately.

The colouring composition may further comprise various adjuvants that are conventionally used in hair-dyeing compositions, such as anionic, cationic, nonionic, amphoteric, and zwitterionic surfactants or mixtures thereof, anionic, cationic, nonionic, amphoteric and zwitterionic polymers or mixtures thereof, organic or inorganic thickeners, and especially anionic, cationic, nonionic and amphoteric polymeric associative thickeners, antioxidants, penetrants, sequestrants, fragrances, buffers, dispersants, conditioning agents such as, for example, volatile or non-volatile silicones with or without modification, such as amino silicones, and film formers, ceramides, preservatives, opacifiers and conductive polymers.

The adjuvants above are present generally in an amount, for each of them, of between 0.01% and 20% by weight, relative to the weight of the composition.

The skilled person will of course ensure that this or these possible further compounds are selected in such a way that the advantageous properties intrinsically attached to the colouring composition in accordance with the invention are not, or not substantially, adversely affected by the intended addition or additions.

The pH of the colouring composition is generally between 3 and 14 approximately, and preferably between 5 and 11 approximately. It may be adjusted to the desired value using acidifying or alkalifying agents that are commonly used in dyeing of keratinous fibres, or else with the aid of conventional buffer systems.

The acidifying agents include, by way of example, organic or inorganic acids such as hydrochloric acid, orthophosphoric acid, sulphuric acid, carboxylic acids such as acetic acid, tartaric acid, citric acid and lactic acid, and sulphonic acids.

The alkalifying agents include, by way of example, aqueous ammonia, alkali metal carbonates, alkanolamines such as mono-, di- and triethanolamines, and derivatives thereof, sodium hydroxide or potassium hydroxide, and the compounds of formula ($\gamma$) below:

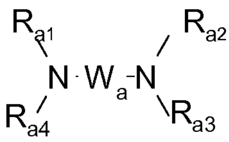

($\gamma$)

in which formula ($\gamma$):

- $W_a$ is a ($C_1$-$C_6$)alkylene residue optionally interrupted by one or more heteroatoms such as O or N, and/or optionally substituted by one or more ($C_1$-$C_6$)alkyl groups or hydroxyl; in particular, $W_a$ represents a propylene group which is optionally substituted by a hydroxyl group or a $C_1$-$C_4$ alkyl radical;
- $R_{a1}$, $R_{a2}$, $R_{a3}$ and $R_{a4}$, which are identical or different, represent a hydrogen atom or a $C_1$-$C_4$ alkyl or $C_1$-$C_4$ hydroxyalkyl radical.

The colouring composition may take any of various forms, such as the form of a liquid, cream, gel or any other appropriate form for bringing about the dyeing of keratinous fibres, and especially the hair.

*III. Method of dyeing and/or lightening using dye of formula (I)*

Another subject of the invention is a method for dyeing and/or lightening keratinous material, especially dark keratinous material, which involves applying to said material a composition comprising at least one dye of formula **(I).**

According to one particular embodiment, in the method of the invention, a reducing agent may also be applied before, at the same time as or after application of the composition comprising at least one fluorescent dye of formula **(I).**

Preferably, no reducing agent is employed in the method according to the invention.

According to one variant, when the composition comprising at least one fluorescent dye of formula **(I)** has been applied to the keratinous materials, the composition is left for a certain time and then the keratinous materials are rinsed, and/or are wrung and/or undergo a heat treatment and/or are exposed to ultraviolet (UV) light.

The time of the treatment after application of the composition comprising at least one fluorescent dye of formula **(I)** may be short, for example from 0.1 second to 1 hour, in particular between 5 minutes and 50 minutes, more particularly between 10 and 45 minutes, and preferably the leave-in time is 30 minutes.

The time of the subsequent treatment with heat may be short, for example from 0.1 to 30 minutes. The temperature is between 40°C and 250°C, more particularly between 50°C and 200°C, and preferably from 80°C to 180°C. According to one particular embodiment, the heat treatment of the keratinous fibres is carried out using a straightening tool for the keratin fibres, or by means of a hairstyling hood, a hairdryer or an infrared emitter.

According to one preferred variant of the invention, the method for dyeing and/or lightening according to the

invention involves applying to the keratinous materials the composition according to the invention, and then wringing them and/or subjecting them to a heat treatment, in particular at a temperature of between 50 and 200°C, more preferably between 80 and 180°C, with, for example, a hair straightener. In this variant of the method, the thermal treatment may be followed by rinsing.

The time of the subsequent treatment with UV is between 1 second and 2 hours, preferably 30 minutes.

One particular embodiment of the invention relates to a method in which the compound of formula **(I)** may be applied directly to the hair without reducing agent, free from reductive preliminary or subsequent treatments.

A treatment with an oxidizing agent may also be used in addition. Any type of oxidizing agent conventional in the art may be used. Hence it may be selected from hydrogen peroxide, urea peroxide, alkali metal bromates, persalts such as perborates and persulphates, and also enzymes, among which mention may be made of peroxidases, 2-electron oxidoreductases such as uricases, and 4-electron oxygenases such as laccases. The use of hydrogen peroxide is particularly preferred.

This oxidizing agent may be applied to the fibres before or after the application of the composition comprising at least one compound of formula **(I).**

The invention further provides a multi-compartment dyeing device or kit, in which a first compartment contains a colouring composition comprising at least one compound of formula **(I)** and a second compartment contains an oxidizing agent.

One of these compartments may further comprise one or more other dyes of direct dye or oxidation dye type.

The invention additionally provides a multi-compartment dyeing device or kit in which a first compartment contains a colouring composition comprising at least one compound of formula **(I)** and a second compartment contains a reducing agent.

One of these compartments may further comprise one or more other dyes of direct dye or oxidation dye type.

Each of the devices referred to above may be equipped with a means allowing the desired mixture to be delivered to the hair, such as, for example, the devices described in patent FR2 586 913.

The examples which follow serve to illustrate the invention, but do not have any limitative character. The thiol fluorescent dyes in the examples below were characterized in their entirety by conventional spectroscopic and spectrometric methods.

**I- SYNTHESIS EXAMPLES**

**Example 1:**

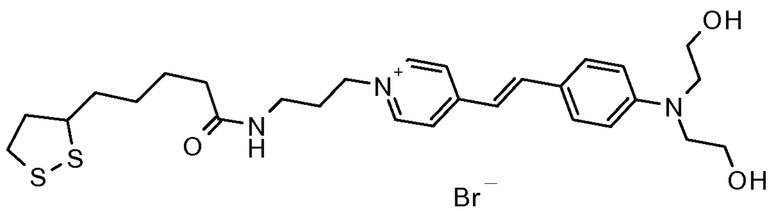

**[1]**

Step 1: Reaction scheme

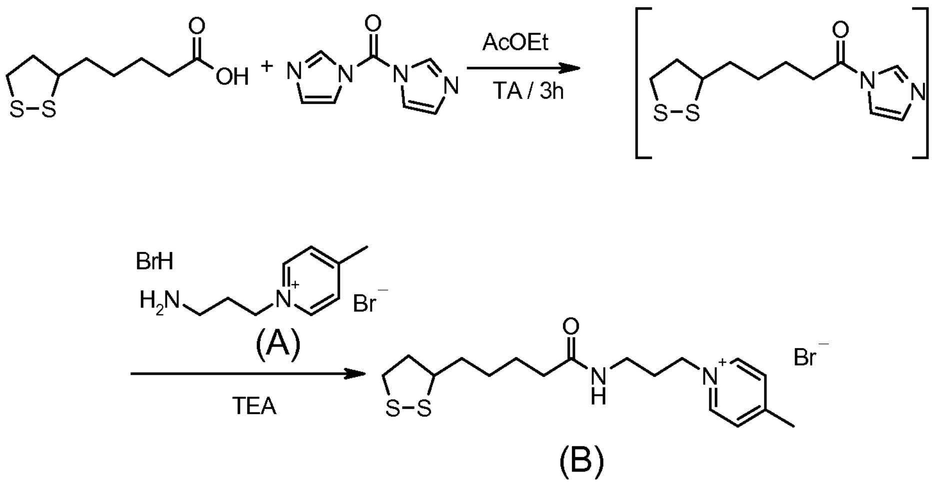


(A)

(B)

Procedure:

In a 600 ml beaker equipped with a stirrer, and in the absence of light, 19.8 g (1.5 eq) of lipoic acid are dissolved at ambient temperature in 100 ml of AcOEt.

16 g (1.5 eq) of carbonyldiimidazole are introduced as is in small fractions over 5 minutes. Evolution of gas is observed (for 15 minutes). The reaction mixture is poured, in the absence of light, on to a solution composed of 20.11 g (1 eq) of compound (A) in suspension in 150 ml of methanol, and then neutralized with 6.47 g (1 eq) of triethylamine. A slight exotherm is observed at the end of the addition (20°C to 25°C). The reaction mixture is diluted with 1.5 l of diisopropyl ether, to give white crystals of triethylamine hydrobromide (TEAHBr) in a mixture with a (yellowish) oil. The diisopropyl ether is decanted and then destroyed, leaving the oil and the crystals (of TEAHBr) in the flask. The oil and the crystals are suspended at ambient temperature in 50 ml of isopropanol (iPrOH) and then the mixture is filtered. The insoluble product is washed with 2 x 20 ml of IPrOH. The filtrate is evaporated to dryness to give the product (B), which is characterized by mass spectrometry (amu = 339) and then used as it is for the following reaction.

Step 2: Reaction scheme

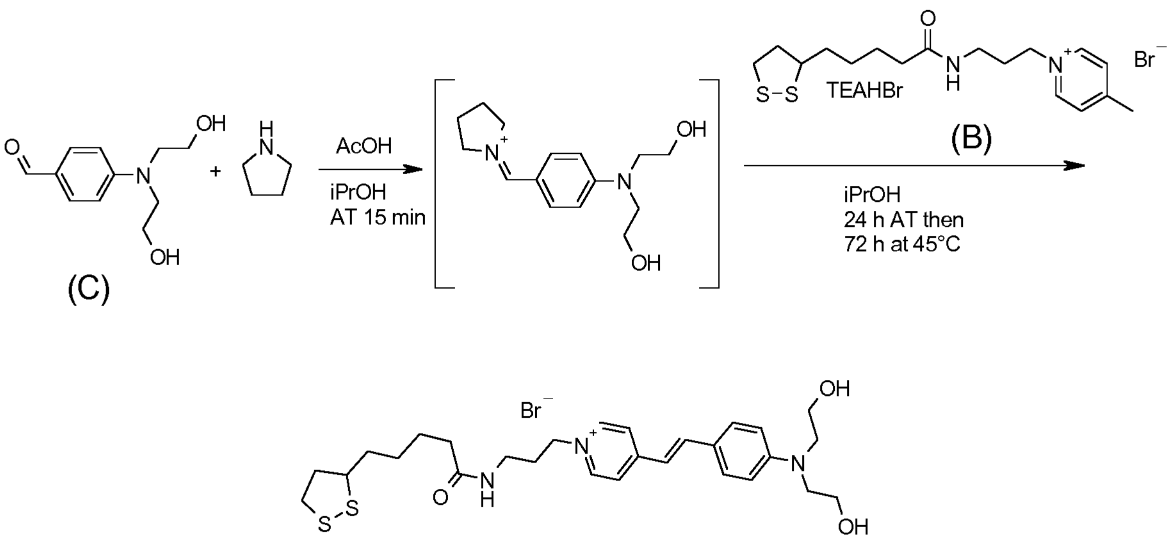


(C)

(B)

**(1)**

In a 250 ml three-necked flask, covered with aluminium foil in order to protect it from the light, and equipped with a thermometer, 5.83 g of aldehyde (C) (1 eq) are dissolved with 16 ml of isopropanol, with stirring. 1.98 g of pyrrolidine

are introduced at ambient temperature. The reaction mixture is stirred for 10 minutes and then 1.89 g (1.13 eq) of acetic acid are added.

9.6 g of compound (B), obtained in step 1 (2 eq), are added to the reaction mixture. After 10 minutes of reaction, the solution becomes red. The reaction mixture is stirred at ambient temperature overnight and then heated at 60°C for 72 hours. The reaction mixture is cooled, triturated (10 minutes) and then filtered. The precipitate is washed with 2 x 70 ml of AcOEt and then dried under high vacuum at 35°C over $P_2O_5$ to constant mass, to give a mass of m = 12.2 g, in the form of matt red crystals.

The analyses are in agreement with the expected product **(1)**

LC/MS m/:z 530, $\lambda_{max}$ = 474 nm

Melting point = 137°C

**Example 2 : Dyeing method - compound [2]**

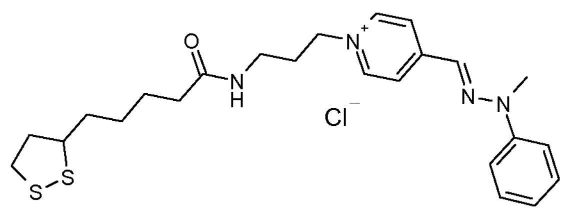

**[2]**

Step 1: Reaction scheme

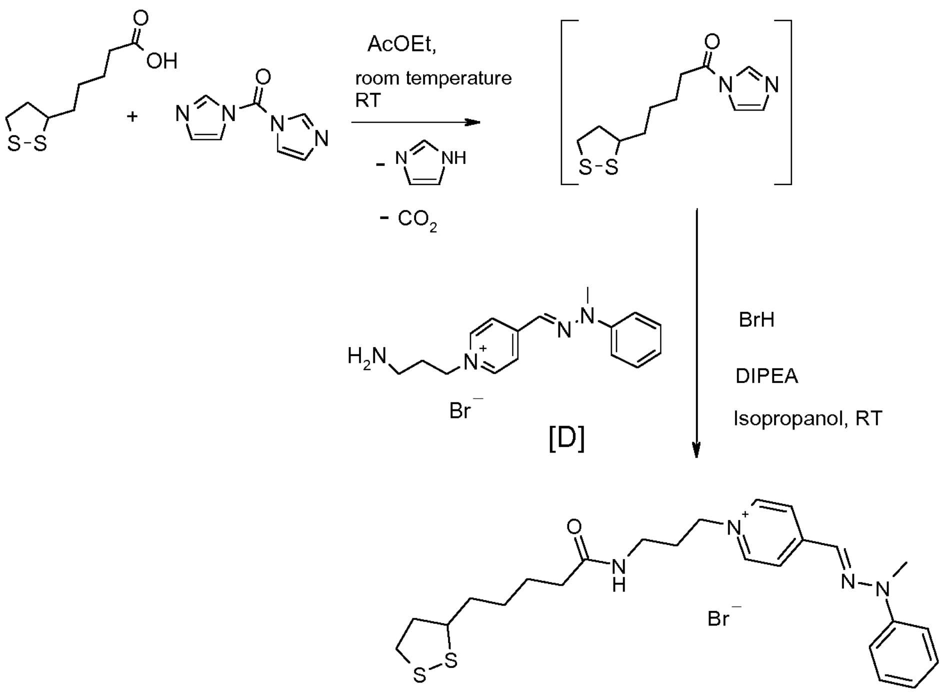


Conter ion exchange

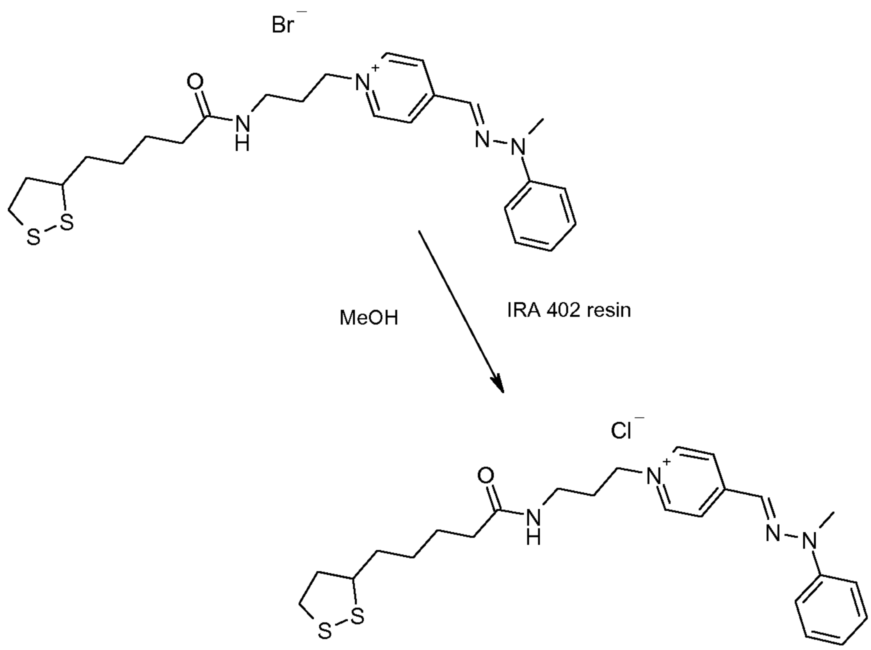


Procedure:

Into a 500 ml reaction vessel equipped with a stirrer are introduced 8.83 g of lipoic acid followed by 100 ml of ethyl acetate (AcOEt). The reaction vessel is protected from light by covering with aluminium foil. 6.94g (1 eq) of carbonyldiimidazole are introduced in small fractions over 5 minutes. Evolution of gas is observed (for 15 minutes).

The reaction mixture is poured, in the absence of light, on to a solution composed of 1 eq of compound [D] in suspension in 200 ml of d'isopropanol, and then neutralized with N,N-Diisopropylethylamine (DIPEA). Following the addition, 600 mL of methanol is added containing 2 mL of DIPEA.

The solution is then evaporated with a ROTAVAP® to give a crude product. EtOAc is added to form a precipitate. The precipitate is collected by filtration and vacuum dried at room to give the desired product with bromide counter-anion (14.6g, yellow powder).

10g of the yellow powder is dissolved in 1 litre of methanol. 100 g of IRA 402 resin prewashed with methanol is added under stirring at room temperature. The mixture is stirred in the absence of light for one hour then filtered and rinsed with methanol. The product is dried under vacuum at room temperature to give the desired product with chloride counter-anion (8, yellow powder). The analyses are in agreement with the expected product **(2)**

**II- DYEING EXAMPLE**

**Example 1: Dyeing method - compound [1]**

4 dyeing baths are prepared by adding 0.1 mmol of compound **(1)** dissolved in 18 g of the dyeing vehicle 1 formula, and then 2 g of solution 2:

| Dyeing vehicle 1 | |
|---|---|
| Natrosol 250MR hydroxyethylcellulose | 0.72 g |
| C8/C10 CG110 alkyl (50:50) hydroxylethylcellulose | 5 g |
| Benzyl alcohol | 4 g |
| Polyethylene glycol 400 | 4 g |
| Water | qs 100 g |

| Solution 2 | | |
|---|---|---|
| | Water | DULCIA™ DV2* |
| Baths 1, 2 and 4 | 2 g | |
| Bath 3 | | 2 g |
| ** contains a reducing agent: thioglycolic acid in a proportion of 9.9 g%* | | |

Two 1 g swatches are added to each bath (natural chestnut-brown hair of Japanese origin, with a tone level of 4), at 5 g of composition for 1 g of hair. The swatches are maintained in the baths at ambient temperature for 30 minutes.

- The swatches from bath 1 are rinsed with water and then dried in air (control).
- The swatches from bath 2 are wrung and then passed through a hair straightener (5 applications of 5 seconds over the whole length of the swatch, repeated twice), rinsed with water and then dried in air (control).
- The swatches from bath 3 are bathed in a bath (20 ml) of DULCIA® fixative (L'Oréal) for 5 minutes, rinsed with water and then dried in air (control).
- The swatches from bath 4 are wrung and then subjected to UV-A light exposure for 1 hour, rinsed with water and then dried in air (control).

## II-1- Shampooing resistance test

A shampooing resistance test was carried out on the swatches or locks of hair dyed with the dye and obtained under the conditions described above.

The dyed swatches were subjected to 5 shampoo washes in accordance with a cycle which comprises wetting of the swatches in water, washing with shampoo (L'Oréal Elsève), and rinsing with water followed by drying with a hairdryer.

### *II-1a- Visual evaluation*

The results are set out in the tables below.

Results

A visual evaluation was made at the time of the first rinse, in order to estimate the fixing performance. We consider that, the greater the exhaustion, the better the fixation.

| | Exhaustion during first shampoo wash |
|---|---|
| Swatch dyed with bath 1 | Highly coloured liquid |
| Swatch dyed with bath 2, heat | Lightly coloured liquid |
| Swatch dyed with bath 3, DULCIA™ DV2 | Lightly coloured liquid |
| Swatch dyed with bath 4, photo UV | Coloured liquid |

Moreover, no visual difference was noted with regard to the cosmetic appearance of the hair after treatment with the methods according to the invention, relative to before treatment.

### *II-1b- Spectrocolorimetric evaluation*

The colour of the swatches was evaluated before dyeing; then after dyeing/before washing; then after dyeing/after successive washes. The colour of the swatches is measured using the Minolta CM2600d spectrocolorimeter (specular components included, 10° angle, D65 illuminant) in the CIEL*a*b* system. In this system, L* represents the

intensity of the colour, a* indicates the green/red colour axis, and b* indicates the blue/yellow colour axis.
The lower the value of L, the darker or more intense the colour. The higher the value of a*, the redder the shade, and the higher the value of b*, the yellower the shade.

The variation in dyeing between the swatches before and after successive washes is measured by (ΔE) according to the following equation:

$$\Delta E = \sqrt{(L^* - L_o^*)^2 + (a^* - a_o^*)^2 + (b^* - b_o^*)^2}$$

In this equation, L*, a* and b* represent the values measured after successive washes, and $L_0$*, $a_0$* and $b_0$* represent the values measured before washing.
The higher the value of ΔE, the greater the difference in colour between the swatch before and after washing, which shows a low washing resistance.

The colorimetric results obtained are given in the table below.

| | L*(D65) | a*(D65) | b*(D65) | ΔE* before/ after shampoo washes |
|---|---|---|---|---|
| | | | | |
| **Dark, TL 4 reference swatch (control)** | 17.69 | 2.38 | 2.47 | - |
| TL4 swatch from bath 1/1 wash | 18.72 | 5.99 | 5.29 | 1.28 |
| TL4 swatch from bath 1/5 washes | 18.77 | 5.13 | 4.33 | |
| TL4 swatch from bath 3/1 wash | 19.41 | 8.33 | 7.05 | 0.72 |
| TL4 swatch from bath 3/5 washes | 19.47 | 7.71 | 6.69 | |
| TL4 swatch from bath 2/1 wash | 18.29 | 5.94 | 5.58 | 1.41 |
| TL4 swatch from bath 2/5 washes | 19.25 | 5.53 | 4.63 | |

The ΔE results from the table above show that the variation in colour between the swatches treated with the compounds of formula **(I)** according to the invention is not significant even after 5 successive washes. The compounds of formula **(I)** are therefore very tenacious. These results were confirmed by the reflectance results (see below).

**II-2- Evaluation of optical lightening by reflectance**

The lightening performance of the compositions according to the invention was expressed as a function of the reflectance of the hair. This reflectance is compared with the reflectance of a swatch of untreated hair with a tone level TL4. The reflectance is measured by means of a KONIKA-MINOLTA® CM 3600d spectrophotocolorimeter, and after irradiation of the hair with visible light in the wavelength range from 400 to 700 nanometres.

Graph showing the optical lightening effect (and remanence effect after 5 washes) (see Fig 1).

It is noted, first, that the reflectance of a swatch of hair treated with a composition according to the invention is markedly greater than that of the untreated hair in the wavelength sector above 560 nm. The swatches treated with the compounds of the invention therefore appear lighter than the TL4 control swatch, even after 5 shampoo washes. These results show that the dyes exhibit high tenacity with respect to shampoo washes.

**Example 2: Dyeing method - compound [2]**

| 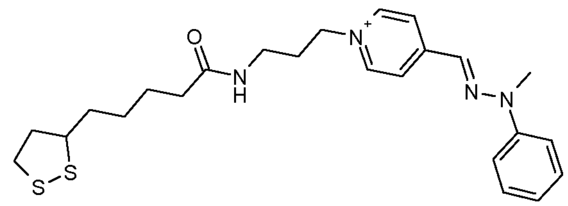 (2) | 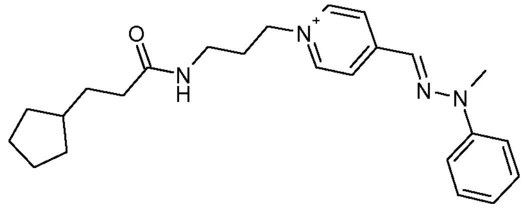 (3) |
|---|---|
| According to the invention | comparative |

2 dyeing baths are prepared by adding 0.1 mmol of compound **[2]** ou compound (3) (comparative) dissolved in 18 g of the dyeing vehicle 1 formula, and then 2 g of solution 2:

| Dyeing vehicle 1 | |
|---|---|
| Natrosol 250MR hydroxyethylcellulose | 0.72 g |
| C8/C10 CG110 alkyl (50:50) hydroxylethylcellulose | 5 g |
| Benzyl alcohol | 4 g |
| Polyethylene glycol 400 | 4 g |
| Water | qs 100 g |

| Solution 2 | | |
|---|---|---|
| Bath 4 | | 2 g $H_2O$ |

Two 1 g swatches are added to each bath (90 % of white natural hair), at 5 g of composition for 1 g of hair. The swatches are maintained in the baths at ambient temperature for 30 minutes and during the coloration are exposed to UV light. The swatches are rinsed with water and then dried in air before shampooing.

**Shampooing resistance test**

A shampooing resistance test was carried out as described herein before, but with dyes [2] & [3] on natural white hair.

***Spectrocolorimetric results***

| | **ΔE (after 5 washes)** |
|---|---|
| Compound [2] According to the invention | 4.47 |
| Comparative | 7.45 |

The ΔE results from the table above show that the variation in colour between the swatches treated with the compound (2) according to the invention is significantly lower after 5 successive washes. The compound (2) is therefore more resistant to shampoo washing.

**Claims**

**1.** Method for dyeing keratinous materials, wherein said materials have applied to them an appropriate colouring

composition comprising one or more dyes of formula **(I)** below:

$$\mathbf{A\text{-}(X)_p\text{-}C_{sat}\text{-}(X')_{p'}\text{-}Het} \qquad \mathbf{(I)}$$

organic or inorganic acid salts, optical isomers and geometric isomers thereof, and solvates thereof such as hydrates; in which formula **(I):**

- **A** represents a radical containing at least one cationic or non-cationic chromophore;
- **Het** represents a heterocyclic disulphide radical such as that of formula **(II)** which is connected to the rest of the molecule by one of the substituents $R_5$, $R_6$, $R_7$, $R_8$, $R_9$ or $R_{10}$, or said radical is connected to the rest of the molecule directly by one of the carbon atoms of the heterocyclic radical in alpha or beta position or, when q is 2 or 3, in gamma position, in which case one of the substituents $R_5$, $R_6$, $R_7$, $R_8$, $R_9$ or $R_{10}$ is absent: in which formula **(II):**

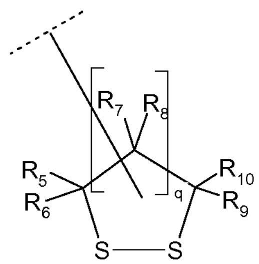

**(II)**

  - $\mathbf{R_5}$, $\mathbf{R_6}$, $\mathbf{R_9}$ and $\mathbf{R_{10}}$, which are identical or different, represent a hydrogen atom or a group selected from: i) $(C_1\text{-}C_8)$alkyl, ii) aryl, iii) hydroxyl, iv) (di)$(C_1\text{-}C_8)$(alkyl)amino, v) $(C_1\text{-}C_8)$alkoxy, vi) (poly)hydroxy$(C_1\text{-}C_8)$alkyl, vii) (di)$(C_1\text{-}C_8)$(alkyl)amino$(C_1\text{-}C_8)$alkyl, viii) carboxyl, ix) carboxy$(C_1\text{-}C_3)$alkyl, x) (di)$(C_1\text{-}C_8)$(alkyl)aminocarbonyl$(C_1\text{-}C_8)$alkyl, and xii) $(C_1\text{-}C_8)$(alkyl) carbonyl$(C_1\text{-}C_8)$(alkyl)amino$(C_1\text{-}C_8)$alkyl;
  - $\mathbf{R_7}$ and $\mathbf{R_8}$, which are identical or different, represent a hydrogen atom or a group selected from: i) $(C_1\text{-}C_8)$alkyl, ii) aryl, iii) hydroxyl, iv) (di)$(C_1\text{-}C_8)$(alkyl)amino, v) $(C_1\text{-}C_8)$alkoxy, vi) (poly)hydroxy$(C_1\text{-}C_8)$alkyl, vii) (di)$(C_1\text{-}C_8)$(alkyl)amino$(C_1\text{-}C_8)$alkyl, viii) carboxyl, ix) carboxy$(C_1\text{-}C_8)$alkyl, x) (di)$(C_1\text{-}C_8)$(alkyl)aminocarbonyl$(C_1\text{-}C_8)$alkyl, and xii) $(C_1\text{-}C_8)$(alkyl)-carbonyl$(C_1\text{-}C_8)$(alkyl)amino$(C_1\text{-}C_8)$alkyl;
  - **q** represents an integer between 1 and 3 inclusive;

  with the proviso that when **q** is 2 or 3, the groups $\mathbf{R_7}$ and $\mathbf{R_8}$ may be identical to or different from one another;

- **X** and **X'**, which are identical or different, represent:

  ◦ a saturated or unsaturated, linear or branched $C_1\text{-}C_{30}$ hydrocarbon chain which is optionally interrupted and/or optionally terminated at one or both of its ends by one or more divalent groups or combinations thereof selected from:

    - -N(R)-; $-N^+(R)(R')$, $Q^-$; -O-; -S-; -C(O)-; $-S(O)_2-$, where R and R', which are identical or different, are selected from a hydrogen atom and a $C_1\text{-}C_4$ alkyl, hydroxyalkyl and aminoalkyl radical, and $Q^-$ represents an anionic counterion;
    - a condensed or non-condensed, saturated or unsaturated, aromatic or non-aromatic (hetero)cyclic radical optionally comprising one or more identical or non-identical heteroatoms and optionally substituted;

  ◦ a divalent group or combination thereof selected from: -N(R)-; $-N^+(R)(R')-$, $Q^-$; -O-; -S-; -C(O)-; $-S(O)_2-$, where R, R' and $Q^-$ are as defined above;

- **p** and **p'**, which are identical or different, represent an integer 0 or 1; and
- $\mathbf{C_{sat}}$ represents an optionally cyclic, optionally substituted, linear or branched C1-C18 alkylene chain.

**2.** Method for dyeing according to the preceding claim, wherein the dye of formula **(I)** is such that **p** is 1, **X** and **X'**,

which are identical or different, represent the following sequence:

**$-(T)_t-(Z)_z-(T')_{t'}-$**

said sequence being connected in the formulae **(I)** as follows:

- **$C_{sat}-(T')_{t'}-(Z)_z-(T)_t-$(A** or **Het);**

in which sequence:

- **T** and **T'**, which are identical or different, represent one or more radicals or combinations thereof selected from: $-S(O)_2-$; -O-; -S-; -N(R)-; $-N^+(R)(R^\circ)-$, $Q^-$; -CO-; where R and R°, which are identical or different, represent a hydrogen atom or a $C_1$-$C_4$ alkyl, $C_1$-$C_4$ hydroxyalkyl or aryl($C_1$-$C_4$)alkyl radical and Q' represents an anionic counterion; and a cationic or non-cationic heterocycloalkyl or heteroaryl radical;
- the indices **t** and **t'**, which are identical or different, are 0 or 1;
- **Z** represents:

  - $-(CR_1R_2)_m-$ where m is an integer between 1 and 8 and $R_1$ and $R_2$, which are identical or different, represent a hydrogen atom or a $C_1$-$C_4$ alkyl, $C_1$-$C_{12}$ alkoxy, hydroxyl, cyano, carboxyl, amino, $C_1$-$C_4$ alkylamino or $C_1$-$C_4$ dialkylamino group, it being possible for said alkyl radicals to form, with the nitrogen atom carrying them, a heterocycle containing from 5 to 7 members, optionally comprising another heteroatom different or not from nitrogen;
  - $-(CH_2CH_2O)_q-$ or $-(OCH_2CH_2)_q-$ in which q is an integer between 1 and 15, or
  - an aryl, alkylaryl or arylalkyl radical in which the alkyl radical is $C_1$-$C_4$ and the aryl radical is preferably $C_6$, being optionally substituted by at least one group $SO_3M$, where M represents a hydrogen atom, an alkali metal or an ammonium group which is substituted by one or more identical or non-identical, linear or branched $C_1$-$C_{18}$ alkyl radicals which optionally carry at least one hydroxyl; and

- **z** is 0 or 1.

**3.** Method for dyeing according to either of the preceding claims, wherein the dye of formula **(I)** is such that **A** is selected from the chromophores derived from i) (poly)azoiques dyes such as (di)azoiques dyes ; ii) hydrazono dyes ; iii) (poly)methines dyes such as styryles and iv) anthraquinones dyes.

**4.** Method for dyeing according to either of the preceding claims, wherein the dye of formula **(I)** is such that **A** is derived from hydrazono of formulae **(III)** and **(III'),** azoiques **(IV)** and **(IV')** and diazoiques **(V)** cationic chromophores:

**$Hét^+-C(R^a)=N-N(R^b)-Ar, Q^-$ (II),**

**$Hét^+-N(R^a)-N=C(R^b)-Ar, Q^-$ (III),**

**$Hét^+-N=N-Ar, Q^-$ (IV)**

**$Ar^+-N=N-Ar'', Q^-$ (IV')**

and

**$Het^+-N=N-Ar'-N=N-Ar, Q^-$ (V)**

formulas **(III), (III'), (IV), (IV')** and **(V)** wherein :

- **Hét⁺** representing a cationic heteroaryl radical, especially an endocyclic cationic radical such as imidazolium, indolium, or pyridinium, potentially substituted especially by at least one ($C_1$-$C_8$)alkyl group such as methyl;
- **Ar⁺** representing an aryl radical, such as phenyl or naphtyl group, with exocyclic cationic charge especially ammonium more especially tri($C_1$-$C_8$)alkyl-ammonium such as trimethylammonium ;
- **Ar** representing an aryl group, especially phenyl, potentially substituted, preferably by at least one electrodonor such as i) ($C_1$-$C_8$)alkyle potentially substituted, ii) ($C_1$-$C_8$)alcoxy potentially substituted, iii) (di)($C_1$-$C_8$)(alkyl)ami-

no potentially substituted on alkyl group(s) by one or more hydroxyl groups, iv) aryl($C_1$-$C_8$)alkylamino, v) *N*-($C_1$-$C_8$)alkyl-*N*-aryl($C_1$-$C_8$)alkylamino potentially substituted or **Ar** represents a julolidine group ;

- **Ar'** representing a divalent (hetero)arylene potentially substituted such as phenylene especially para-phenylene, or naphtalene, potentially substituted, especially by one or more groups selected from($C_1$-$C_8$)alkyl, hydroxy, and ($C_1$-$C_8$)alcoxy ;
- **Ar''** representing an (hetero)aryl group potentially substituted such as phenyl or pyrazolyl potentially substituted, preferably by one or more groups selected from ($C_1$-$C_8$)alkyl, hydroxy, (di)($C_1$-$C_8$)(alkyl)amino, ($C_1$-$C_8$)alcoxy and phenyle ;
- $\mathbf{R^a}$ and $\mathbf{R^b}$, which are identical or different, representing an hydrogen or a ($C_1$-$C_8$)alkyl group potentially substituted, especially by an hydroxy group,

or substituant $\mathbf{R^a}$ with one of substituant of $\mathbf{Het^+}$radical and/or $\mathbf{R^b}$ with one substituant of **Ar** radical possibly forming, with atom to which they are attached, a (hetero)cycloalkyle ;

particularly $\mathbf{R^a}$ and $\mathbf{R^b}$, representing a hydrogen atom or a ($C_1$-$C_4$)alkyl group possibly or potentially substituted by hydroxy group(s) ;

- $\mathbf{Q^-}$ representing an organic or inorganic anionic counterion such as halogen or alkylsulfate ;
- With the proviso that **(III), (III'), (IV), (IV')** or **(V)** is attached on the dye of formula (I) by $\mathbf{Het^+}$, $\mathbf{Ar^+}$, **Ar** or **Ar''.**

**5.** Method for dyeing according to either of the claims 1 to 3, wherein the dye of formula **(I)** is such that **p** is 1, and **A** represents a fluorescent chromophore such as : **W-C(R^c^)=C(R^d^)-Ar'-** or **-W'-C(R^c^)=C(R^d^)-Ar,** where:

- **W** is a monovalent radical which represents a heterocycle or a heteroaryl, comprising a quaternary ammonium which is optionally substituted by a $C_1$-$C_8$ alkyl which is optionally substituted in particular by one or more hydroxyl groups;
- **W'** is a divalent radical which represents a heterocycle or heteroaryl as defined **for W;**
- **Ar** represents a 5- or 6-membered aryl radical of phenyl type or a bicyclic aromatic system of naphthyl type, optionally substituted i) by one or more halogen atoms; ii) by one or more alkyl groups; iii) by one or more hydroxyl groups; iv) by one or more alkoxy groups; v) by one or more hydroxyalkyl groups; vi) by one or more amino or (di)alkylamino groups, where the $C_1$-$C_4$ alkyl moiety is optionally substituted by one or more hydroxyls, such as (di)hydroxylethylamino; vii) by one or more acylamino groups; or viii) by one or more 5- or 6-membered heterocycloalkyl or heteroaryl groups;
- **Ar'** is a divalent radical which represents an aryl radical as defined **for Ar;** and
- $\mathbf{R^c}$ and $\mathbf{R^d}$, which are identical or different, represent a hydrogen atom or a $C_1$-$C_4$ alkyl group.

**6.** Method for dyeing according to any of the preceding claims, wherein the dye of formula **(I)** is of formula **(I'):**

$$\mathbf{A\text{-}(T)_t\text{-}[(CR_1R_2)_m]_z\text{-}T_{a}\text{-}C_{sat}\text{-}(T_b)_{t'}\text{-}Het} \qquad \mathbf{(I')}$$

in which formula **(I'):**

- **Het** represents a heterocyclic disulphide radical of formula **(II)** as defined in claim 1;
- **A** is derived from hydrazono of formulae **(III)** and **(III'),** azoiques **(IV)** and **(IV')** and diazoiques **(V)** cationic chromophores as defined in claim 4 or represents a fluorescent chromophore $\mathbf{W\text{-}C(R^c)\text{=}C(R^d)\text{-}Ar'\text{-}}$ or $\mathbf{\text{-}W'\text{-}C(R^c)\text{=}C(R^d)\text{-}Ar}$, as defined in claim 5,
- **z** is 0 or 1;
- **t** and **t',** which are identical or different, are 0 or 1; and
- **T** represents i) an amino group -N(R)- where R represents a hydrogen atom, a $C_1$-$C_4$ alkyl, $C_1$-$C_4$ hydroxyalkyl or aryl($C_1$-$C_4$)alkyl radical, or ii) an optionally substituted divalent heterocycloalkyl group;
- $\mathbf{T_a}$ and $\mathbf{T_b}$, which are identical or different, represent one or more radicals or combinations thereof selected from $-S(O)_2-$, -O-, -S-, -N(R)-, $-N^+(R)(R°)-$, $M^-$, -C(O)-, where R and R°, which are identical or different, represent a hydrogen atom, a $C_1$-$C_4$ alkyl or $C_1$-$C_4$ hydroxyalkyl radical; or an aryl($C_1$-$C_4$)alkyl radical, and $M^-$ represents an organic or inorganic anionic counterion such as halide, preferably with $T_a$ representing a covalent $\sigma$ bond or a group -N(R)- and $T_b$ representing a covalent $\sigma$ bond or a group selected from a radical -O-, -C(O)-, -N(R)-, or a combination thereof, where R represents a hydrogen atom or a $C_1$-$C_4$ alkyl group such as methyl;

and

- $\mathbf{T_a}$ being preferably in para position on Ar or Ar', relative to the olefin function $-C(R^c)=C(R^d)-$.

**7.** Method for dyeing according to either of Claims 5 and 6, wherein the dye of formula **(I)** is such that **W** represents a group selected from imidazolium, pyridinium, benzopyridinium, benzimidazolium, quinolinium, indolinium and pyrazolium, which are optionally substituted.

**8.** Method for dyeing according to any of the preceding claims, wherein the dye of the formula **(I)** is selected from the compounds **(Ia)** to **(Ig)** below:

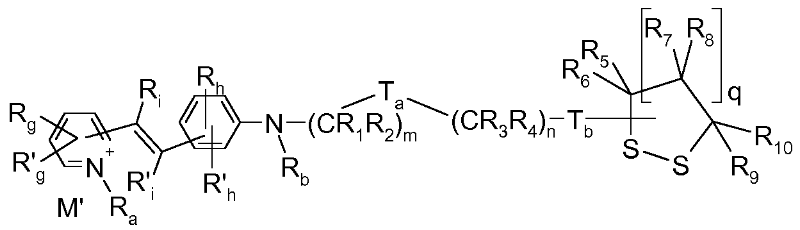

**(Ia)**

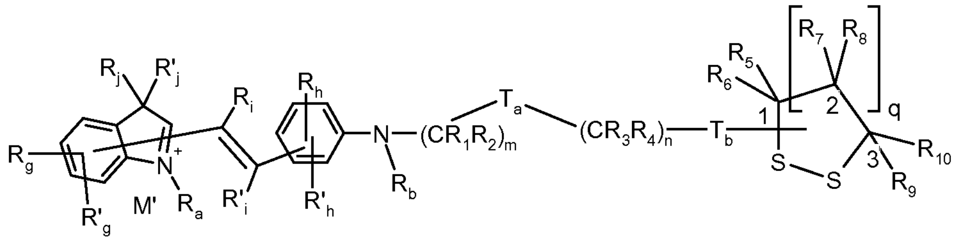

**(Ib)**

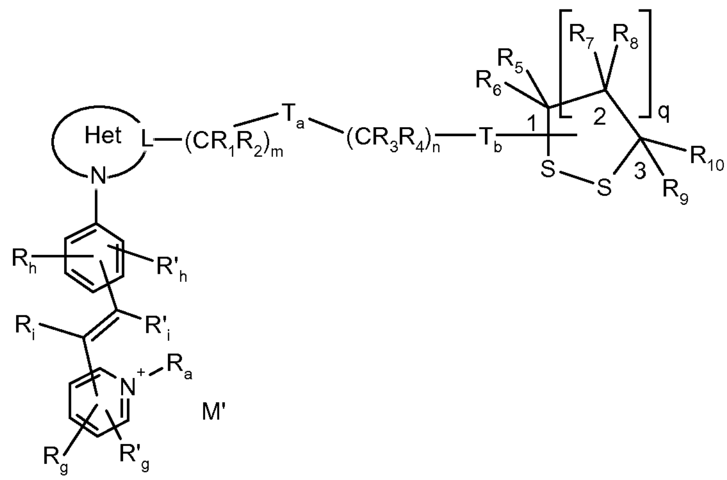

**(Ic)**

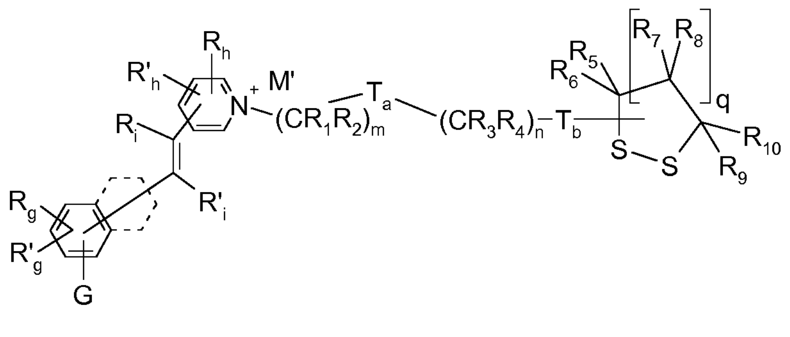

**(Id)**

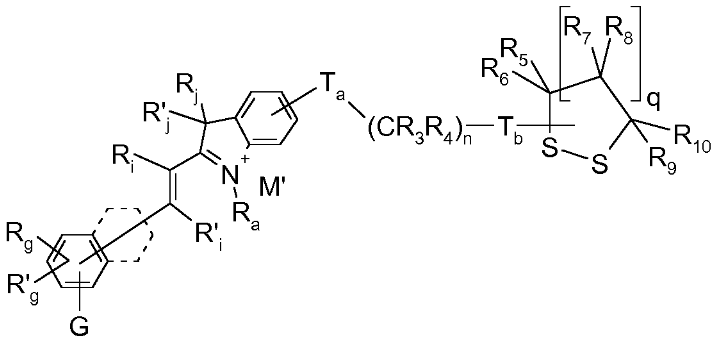

**(Ie)**

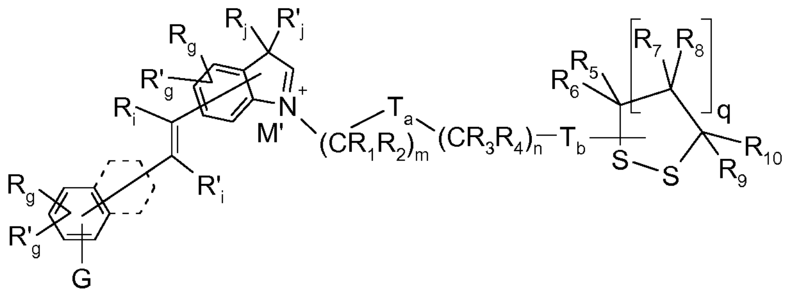

**(If)**

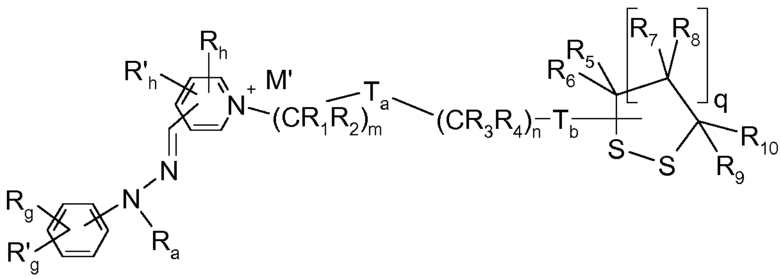

**(Ig)**

and their organic or inorganic acid salts, optical isomers and geometric isomers, and the solvates such as hydrates; in which formulae **(Ia)** to **(Ig):**

- **G** represents a group $-NR_cR_d$ or a $C_1$-$C_6$ alkoxy group;
- $\mathbf{R_a}$ represents an optionally substituted $C_1$-$C_6$ alkyl group;
- $\mathbf{R_b}$ represents a hydrogen atom or an optionally substituted $C_1$-$C_6$ alkyl group;
- $\mathbf{R_c}$ and $\mathbf{R_d}$, which are identical or different, represent a hydrogen atom, an aryl($C_1$-$C_4$)alkyl group, $C_1$-$C_6$ alkoxy or an optionally substituted $C_1$-$C_6$ alkyl group; $R_c$ and $R_d$ preferably represent a hydrogen atom or a $C_1$-$C_3$ alkyl group which is optionally substituted by i) a hydroxyl group, ii) amino, iii) $C_1$-$C_3$ (di)alkylamino, or iv) quaternary ammonium (R")(R"')(R"")$N^+$-;
  or two adjacent radicals $\mathbf{R_c}$ and $\mathbf{R_d}$ carried by the same nitrogen atom together form a heterocyclic or heteroaryl group;
  particularly $\mathbf{R_c}$ and $\mathbf{R_d}$ represent identical groups, and preferably $R_c$ and $R_d$ represent a $C_1$-$C_3$ alkyl which is optionally substituted by a hydroxyl group, such as methyl, hydroxyethyl and 2-hydroxypropyl;
- $\mathbf{R_g}$, $\mathbf{R'_g}$, $\mathbf{R_h}$ and $\mathbf{R'_h}$, which are identical or different, represent a hydrogen atom, a halogen atom, an amino, $C_1$-$C_4$ alkylamino, $C_1$-$C_4$ dialkylamino, cyano, carboxyl, hydroxyl or trifluoromethyl group, an acylamino, $C_1$-$C_4$ alkoxy, $C_2$-$C_4$ (poly)hydroxyalkoxy, alkylcarbonyloxy, alkoxycarbonyl or alkylcarbonylamino radical, an acylamino, carbamoyl or alkylsulphonylamino radical, an aminosulphonyl radical, or a $C_1$-$C_{16}$ alkyl radical which is optionally substituted by a group selected from $C_1$-$C_{12}$ alkoxy, hydroxyl, cyano, carboxyl, amino, $C_1$-$C_4$ alkylamino and $C_1$-$C_4$ dialkylamino, or the two alkyl radicals carried by the nitrogen atom of the amino group form a heterocycle containing from 5 to 7 members and optionally comprising another heteroatom, which is identical to or different from the nitrogen atom; preferably $R_g$, $R'_g$, $R_h$ and $R'_h$ represent a hydrogen or halogen atom or a $C_1$-$C_3$ alkyl group;
- or two groups $\mathbf{R_g}$ and $\mathbf{R'_g}$; $\mathbf{R_h}$ and $\mathbf{R'_h}$; carried by two adjacent carbon atoms together form a benzo or indeno ring system or a fused heterocyclo alkyl or fused heteroaryl group; the benzo, indeno, heterocycloalkyl or

heteroaryl ring system being optionally substituted by a halogen atom, an amino, $C_1$-$C_4$ alkylamino, $C_1$-$C_4$ dialkylamino, nitro, cyano, carboxyl, hydroxyl or trifluoromethyl group, an acylamino, $C_1$-$C_4$ alkoxy, $C_2$-$C_4$ (poly)hydroxyalkoxy, alkylcarbonyloxy alkoxycarbonyl or alkylcarbonylamino radical, an acylamino, carbamoyl or alkylsulphonylamino radical, an aminosulphonyl radical, or a $C_1$-$C_{16}$ alkyl radical which is optionally substituted by: a group selected from C1-C12 alkoxy, hydroxyl, cyano, carboxyl, amino, $C_1$-$C_4$ alkylamino, $C_1$-$C_4$ dialkylamino, or the two alkyl radicals carried by the nitrogen atom of the amino group form a heterocycle comprising from 5 to 7 members and optionally comprising another heteroatom, which is identical to or different from the nitrogen atom; preferably $\mathbf{R_g}$ and $\mathbf{R'_g}$ together form a benzo group;

• or, when **G** represents $-NR_cR_d$, two groups $R_c$ and $R'_g$; $R_d$ and $R_g$; together form a saturated heterocycle or heteroaryl which is optionally substituted by one or more $C_1$-$C_6$ alkyl groups, preferably a heterocycle containing one or two heteroatoms selected from nitrogen and oxygen and comprising between 5 and 7 members;

• $\mathbf{R_i}$, $\mathbf{R_j}$, $\mathbf{R'_j}$ and $\mathbf{R'_i}$, which are identical or different, represent a hydrogen atom or a $C_1$-$C_4$ alkyl group; preferably $\mathbf{R_i}$, $\mathbf{R_j}$, $\mathbf{R'_j}$ and $\mathbf{R'_i}$ represent a hydrogen atom;

• $\mathbf{R_1}$, $\mathbf{R_2}$, $\mathbf{R_3}$ and $\mathbf{R_4}$, which are identical or different, represent a hydrogen atom or a $C_1$-$C_4$ alkyl, $C_1$-$C_{12}$ alkoxy, hydroxyl, cyano, carboxyl, amino, $C_1$-$C_4$ alkylamino or $C_1$-$C_4$ dialkylamino group, it being possible for said alkyl radicals to form, with the nitrogen atom carrying them, a heterocycle comprising from 5 to 7 members, optionally comprising another heteroatom, which is different or not from nitrogen; preferably $R_1$, $R_2$, $R_3$ and $R_4$ are hydrogen atoms or an amino group; more preferably $R_1$, $R_2$, $R_3$ and $R_4$ represent a hydrogen atom;

• $\mathbf{T_a}$ and $\mathbf{T_b}$, which are identical or different, represent:

i) either a covalent $\sigma$ bond;
ii) or one or more radicals or combinations thereof selected from $-S(O)_2-$,

- O-, -S-, -N(R)-, $-N^+(R)(R^\circ)-\ M^-$, -C(O)-, where R and R°, which are identical or different, represent a hydrogen atom or a $C_1$-$C_4$ alkyl or $C_1$-$C_4$ hydroxyalkyl radical; or an aryl($C_1$-$C_4$)alkyl, and $M^-$ represents an organic or inorganic anionic counterion such as halide; preferably $T_a$ represents a covalent $\sigma$ bond or a group -N(R)- and $T_b$ represents a covalent $\sigma$ bond or a group selected from a radical -O-, -C(O)-, -N(R)- or a combination thereof, where R represents a hydrogen atom or a $C_1$-$C_4$ alkyl group such as methyl;

•

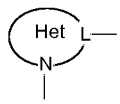

represents an optionally substituted heterocyclic group; L represents a carbon atom or a nitrogen atom, the heterocycle is preferably monocyclic and saturated and comprises a total of two nitrogen atoms and from 5 to 8 members;

•

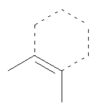

represents an aryl or heteroaryl group which is fused to the phenyl ring; or is absent from the phenyl ring; when the ring is present, the ring is preferably a benzo; more preferably the ring is absent;

• **m** and **n,** which are identical or different, represent an integer between 0 and 10, preferably between 0 and 7, inclusive, where m is preferably between 0 and 5, more particularly between 0 and 3, inclusive and where n is between 0 and 8 inclusive; more particularly n is 0, 1, 2, 3 or 4;

• $\mathbf{R_5}$, $\mathbf{R_6}$, $\mathbf{R_9}$ and $\mathbf{R_{10}}$, which are identical or different, represent a hydrogen atom or a group selected from: i) ($C_1$-$C_8$)alkyl, iii) hydroxyl, iv) (di)($C_1$-$C_8$)(alkyl)amino, v) ($C_1$-$C_8$)alkoxy, vi) (poly)hydroxy($C_1$-$C_8$)alkyl, vii) (di)($C_1$-$C_8$)(alkyl)amino($C_1$-$C_8$)alkyl, viii) carboxyl, ix) carboxy($C_1$-$C_3$)alkyl, x) (di)($C_1$-$C_8$)(alkyl)aminocarbonyl($C_1$-$C_8$)alkyl and xii) ($C_1$-$C_8$)(alkyl)carbonyl($C_1$-$C_8$)(alkyl)amino($C_1$-$C_8$)alkyl;

preferably $\mathbf{R_5}$ is an atom or an alkyl group such as methyl and $\mathbf{R_6}$, $\mathbf{R_9}$ and $\mathbf{R_{10}}$ represent hydrogen atoms;

• $\mathbf{R_7}$ and $\mathbf{R_8}$, which are identical or different, represent a hydrogen atom or a group selected from: i) ($C_1$-$C_8$)alkyl,

iii) hydroxyl, iv) (di)($C_1$-$C_8$)(alkyl)amino, v) ($C_1$-$C_8$)alkoxy, vi) (poly)hydroxy($C_1$-$C_8$)alkyl, vii) (di)($C_1$-$C_8$)(alkyl)amino($C_1$-$C_8$)alkyl, viii) carboxyl, ix) carboxy($C_1$-$C_8$)alkyl, x) (di)($C_1$-$C_8$)(alkyl)amino-carbonyl($C_1$-$C_8$)alkyl and xii) ($C_1$-$C_8$)(alkyl)carbonyl($C_1$-$C_8$)(alkyl)amino($C_1$-$C_8$)alkyl;
preferably $\mathbf{R_7}$ and $\mathbf{R_8}$ represent a hydrogen atom or a $C_1$-$C_4$ alkyl group such as methyl;

- **q** represents an integer between 1 and 3 inclusive; preferably q = 1; and
- **M'** represents an organic or inorganic anionic counterion;

with the proviso that the disulphide heterocycle is connected to the rest of the molecule directly by the carbon atom, preferably in the beta position to the disulphide function of said heterocycle.

**9.** Method for dyeing according to any of the claims 1 to 3 and 5 to 8, wherein the fluorescent dye of formula **(I)** is selected from the compounds **(I'a), (I'b), (I'd), (I'e),** and **(I'f)** below:

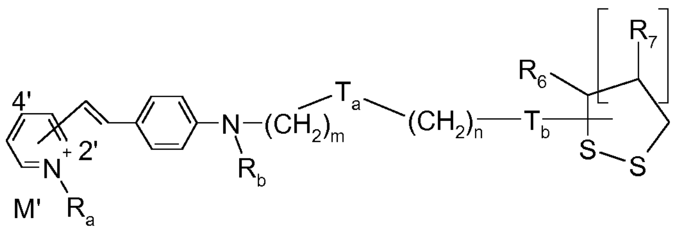

**(I'a)**

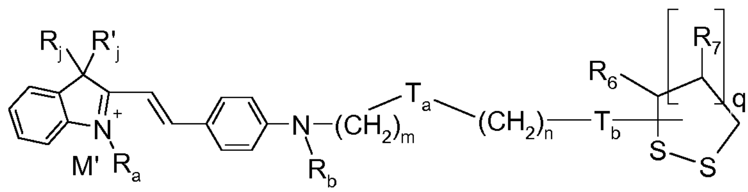

**(I'b)**

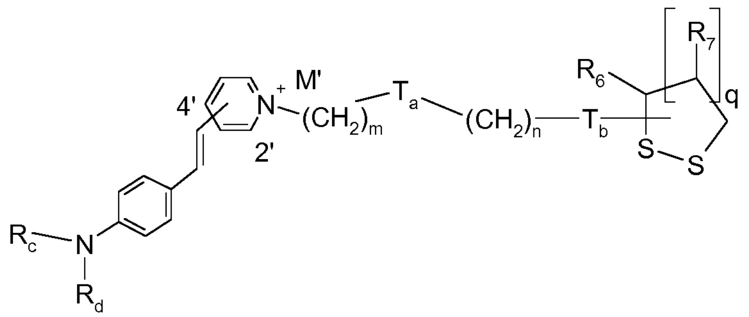

**(I'd)**

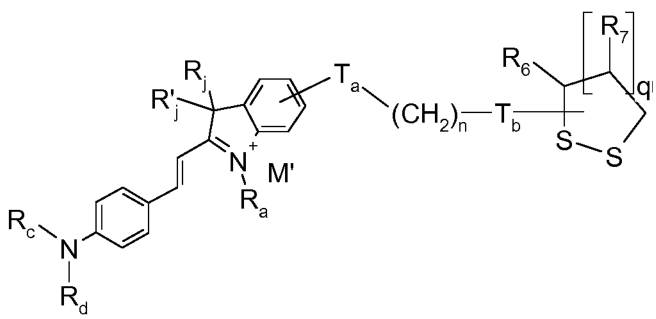

**(I'e)**

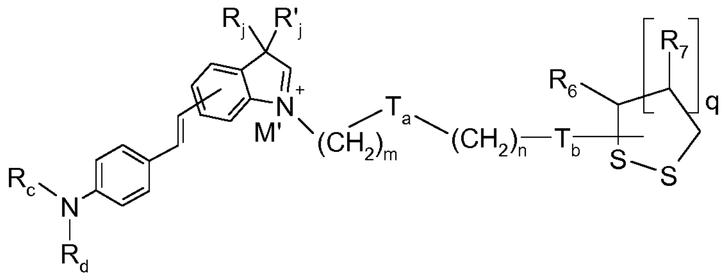

(I'f)

in which formulae **(I'a), (I'b), (I'd), (I'e)** and **(I'f):**

- ◦ **$R_a$, $R_b$, $R_j$** and **$R'_j$** represent a $C_1$-$C_4$ alkyl group such as methyl;
- ◦ **$R_6$** and **$R_7$** represent a hydrogen atom or a $C_1$-$C_4$ alkyl group such as methyl, **$T_b$** being connected directly on the carbon atom in alpha or beta position to the disulphide function of the heterocycle;
- ◦ **m** and **n,** which are identical or different, represent an integer between 0 and 7 inclusive, where m is between 0 and 3 and where n is 0, 1, 2, 3 or 4;
- ◦ **$T_a$** and **$T_b$,** which are identical or different, represent i) a covalent σ bond; or ii) a group selected from a radical -O-, -C(O)-, -N(R)- or a combination thereof such as -N(R)-C(O)-, -C(O)-N(R)-, -O-C(O)-, -C(O)-O- or -C(O)-N(R)-C(O)-, where R represents a hydrogen atom or a $C_1$-$C_4$ alkyl group; particularly R is a hydrogen atom or a methyl;
- ◦ **q** is 1, 2 or 3; particularly q is 1;
- ◦ **$R_c$** and **$R_d$** represent identical $C_1$-$C_3$ alkyl groups optionally substituted by a hydroxyl group, such as methyl, hydroxyethyl and 2-hydroxypropyl;
and
- ◦ **M'** represents an organic or inorganic anionic counterion;

with the proviso that in the formula **(I'a)** and **(I'd)** the pyridinium group is connected to the styryl by the carbon atom in position 2' (ortho) or 4' (para).

**10.** Method for dyeing according to any of the claims 1 to 4, 6 and 8, wherein the dye of formula **(I)** is selected from **(I'g)** below:

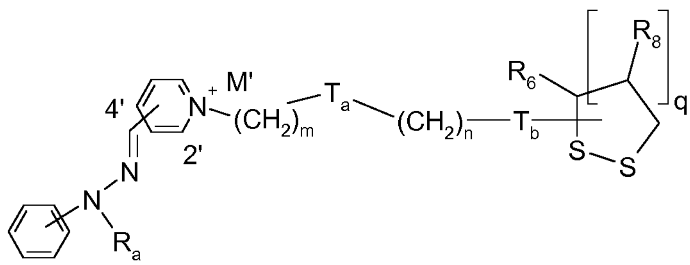

(I'g)

in which formulae **(I'g):**

- ◦ **$R_a$** represent a $C_1$-$C_4$ alkyl group such as methyl;
- ◦ **$T_a$** and **$T_b$,** which are identical or different, represent i) a covalent σ bond; or ii) a group selected from a radical -O-, -C(O)-, -N(R)- or a combination thereof such as -N(R)-C(O)-, -C(O)-N(R)-, -O-C(O)-, -C(O)-O- or -C(O)-N(R)-C(O)-, where R represents a hydrogen atom or a $C_1$-$C_4$ alkyl group; particularly R is a hydrogen atom or a methyl;
- ◦ **$R_6$** and **$R_7$** represent a hydrogen atom or a $C_1$-$C_4$ alkyl group such as methyl, **$T_b$** being connected directly on the carbon atom in alpha or beta position to the disulphide function of the heterocycle;
- ◦ **m** and **n,** which are identical or different, represent an integer between 0 and 7 inclusive, where m is between 0 and 3 and where n is 0, 1, 2, 3 or 4;
- ◦ **q** is 1, 2 or 3; particularly q is 1;
- ◦ **M'** represents an organic or inorganic anionic counterion;

with the proviso that the pyridinium group is connected to the hydrazono by the carbon atom in position 2' (ortho)

or 4' (para), preferably 4'.

**11.** Method for dyeing according to any of the claims 1 to 3 and 5 to 9, wherein the fluorescent dye of formula **(I)** is selected from compounds **(1)** to **(68)** below:

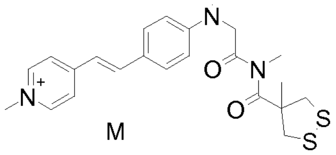

**1**

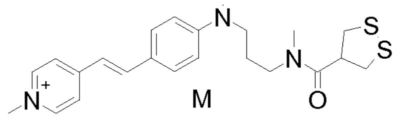

**2**

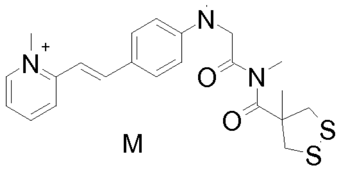

**3**

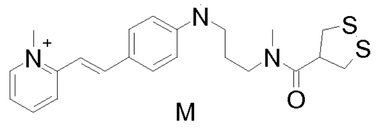

**4**

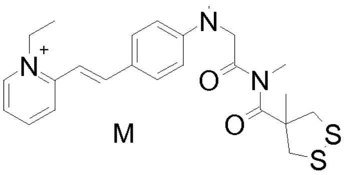

**5**

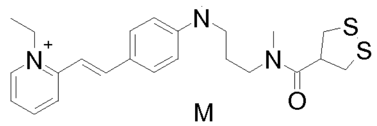

**6**

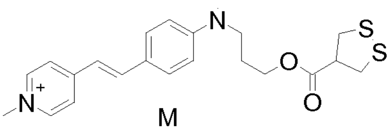

**7**

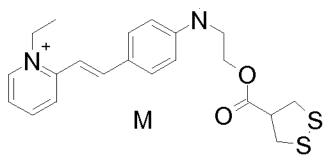

**8**

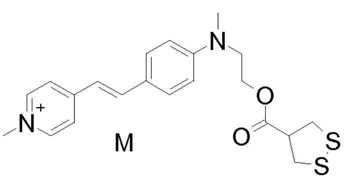

**9**

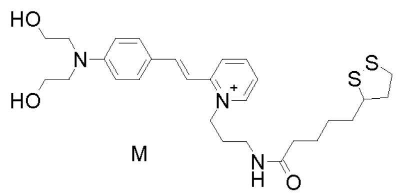

**10**

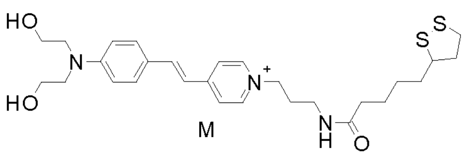

**11**

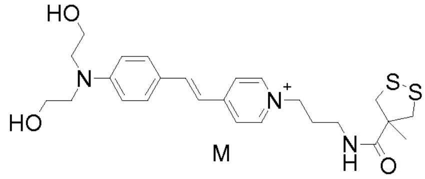

**12**

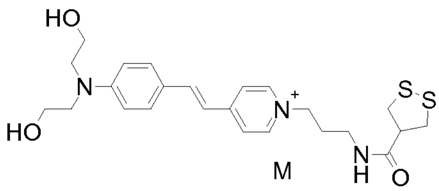

**13**

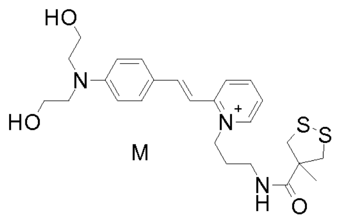

**14**

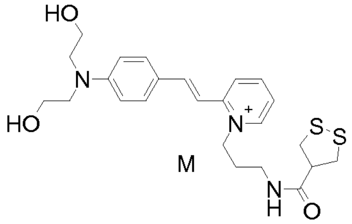

**15**

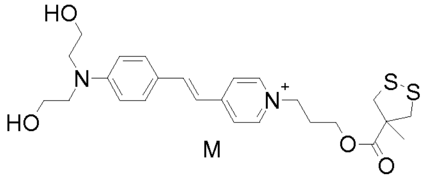

**16**

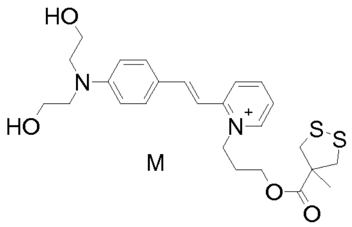

**17**

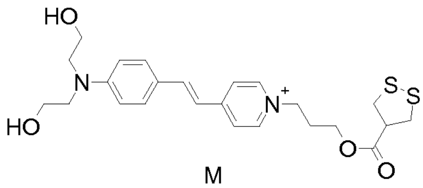

**18**

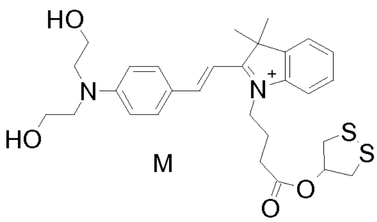

**19**

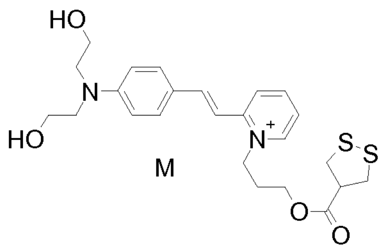

**20**

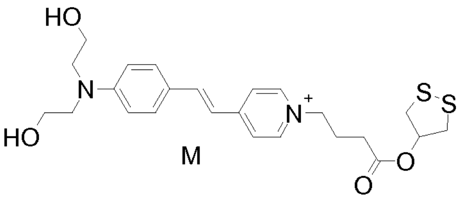

**21**

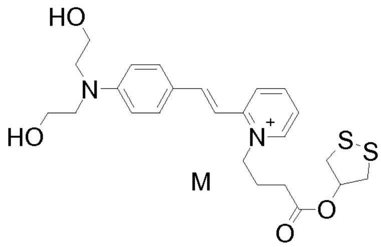

**22**

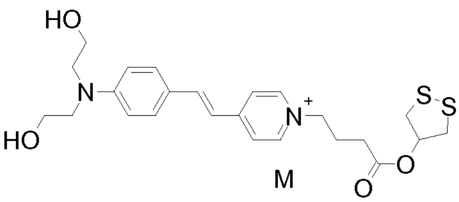

**23**

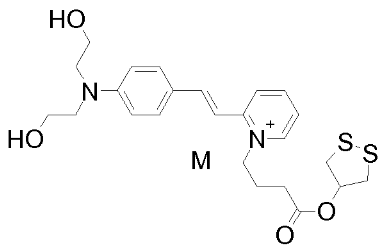

**24**

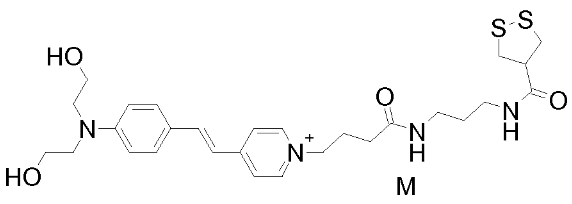

**25**

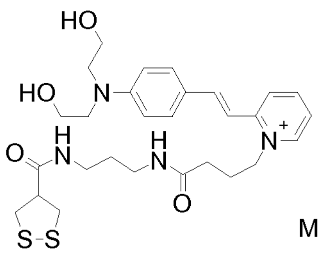

**26**

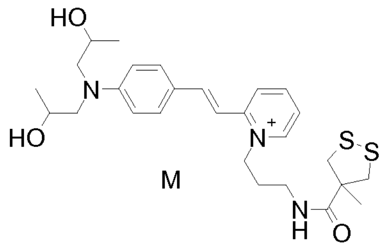

**27**

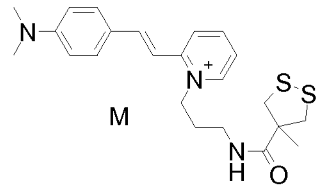

**28**

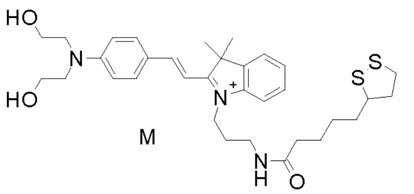

**29**

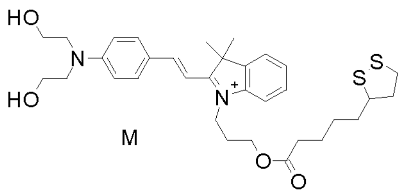

**30**

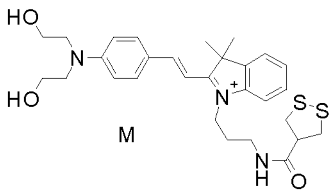

**31**

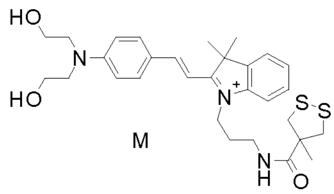

**32**

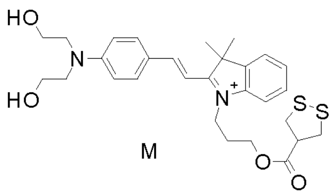

**33**

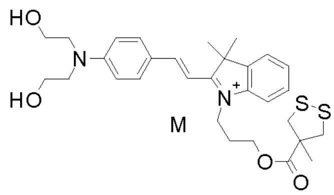

**34**

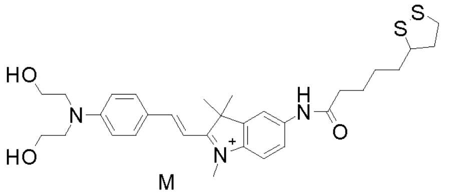

**35**

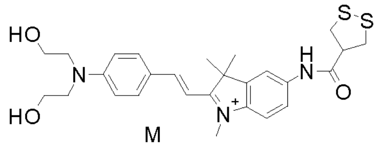

**36**

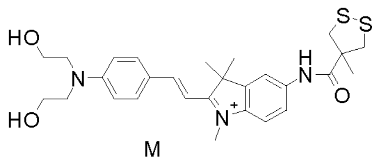

**37**

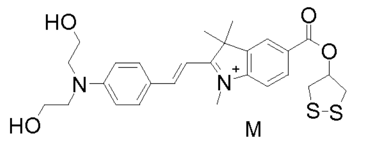

**38**

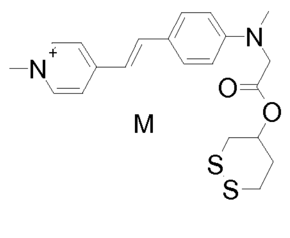

**39**

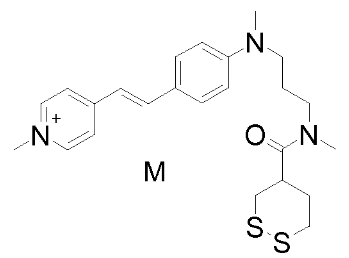

**40**

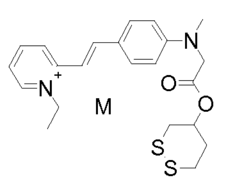

**41**

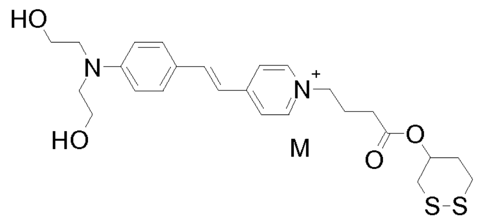

**42**

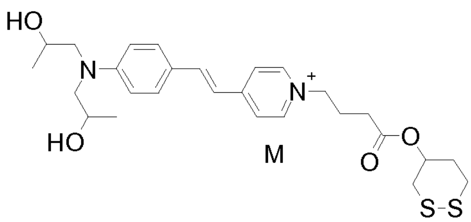

**43**

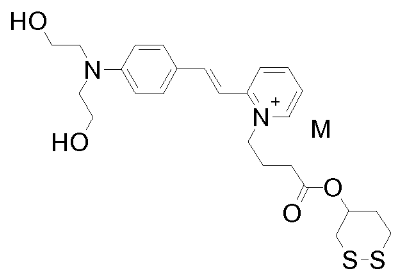

**44**

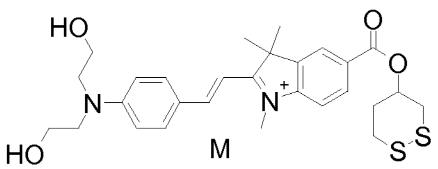

**45**

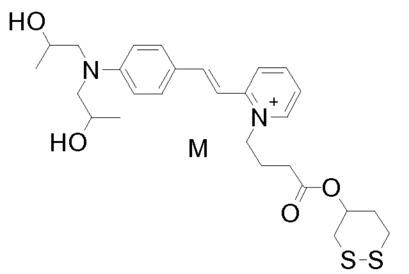

**46**

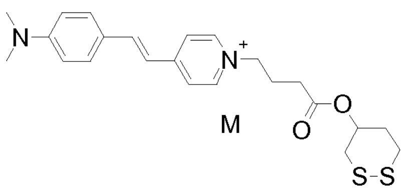

**47**

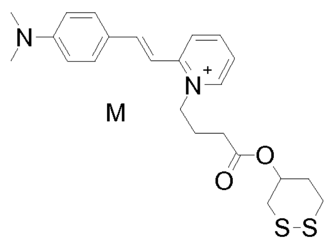

**48**

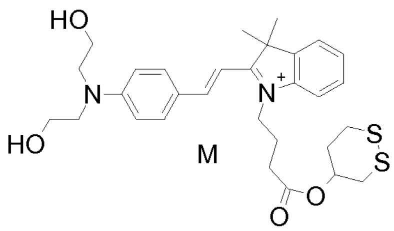

**49**

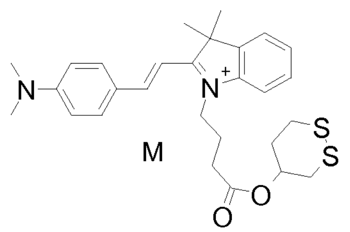

**50**

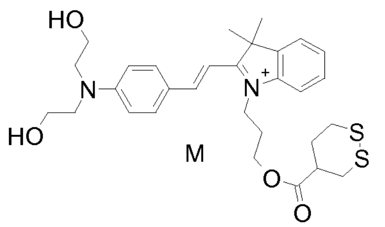

**51**

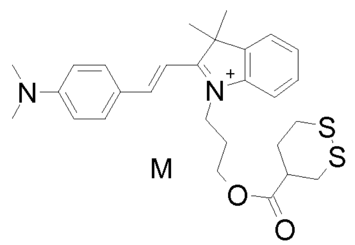

**52**

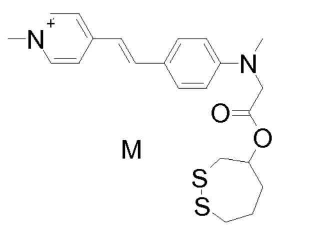

**53**

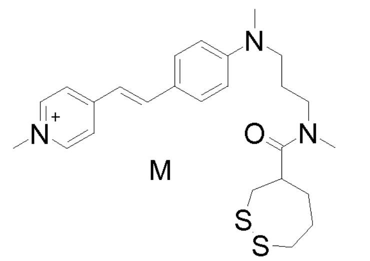

**54**

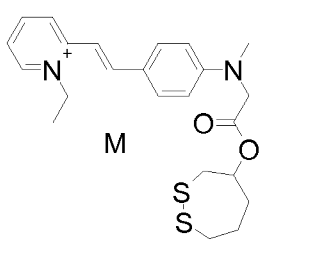

**55**

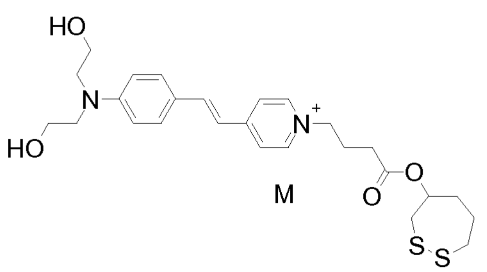

**56**

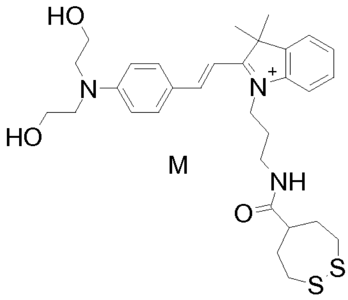

57

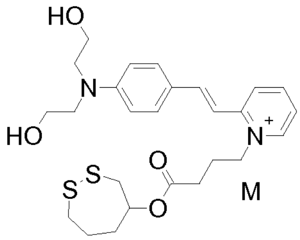

58

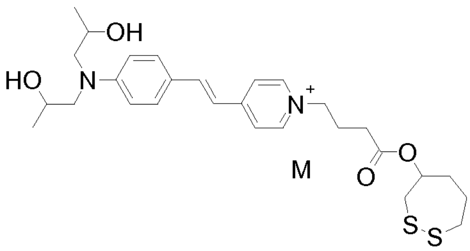

59

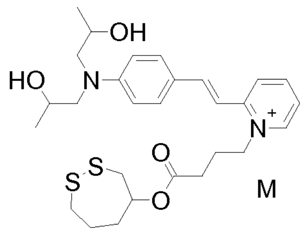

60

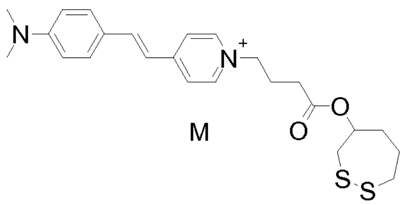

61

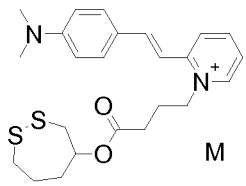

62

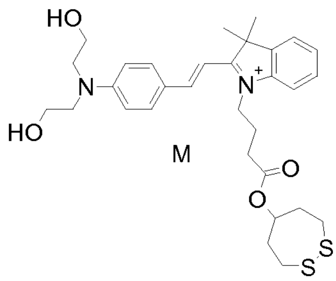

63

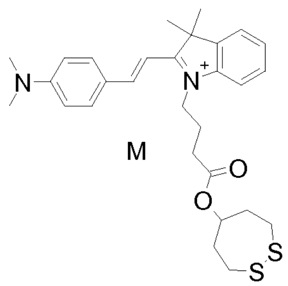

64

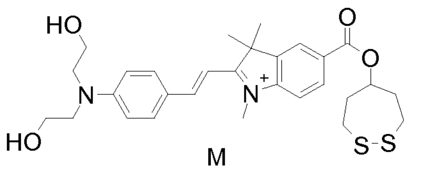

65

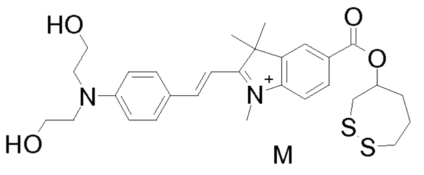

66

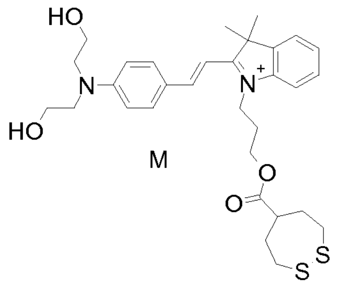

**67**

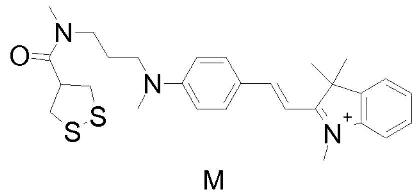

**68**

where M represents an anionic counterion.

**12.** Method for dyeing according to any of the claims 1 to 4, 6, 8 and 10, wherein the dye of formula **(I)** is selected from compound **69** below:

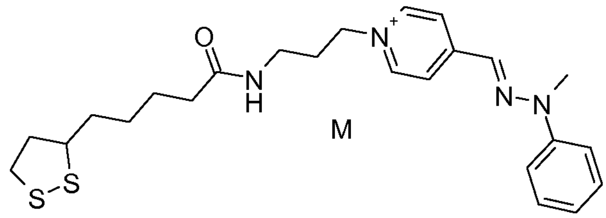

**69**

where M represents an anionic counterion.

**13.** Method for dyeing keratinous materials according to any of the preceding claims, wherein the composition comprising the compound or compounds of formula **(I)** is left to act and then the keratinous materials are rinsed, and/or are wrung and/or undergo a heat treatment and/or are exposed to ultraviolet (UV) light.

**14.** Method for dyeing keratinous materials according to the preceding claim, wherein the composition comprising the compound or compounds of formula **(I)** is left to act and then the keratinous materials are wrung and/or undergo a heat treatment in particular at a temperature of between 50 and 200°C, preferably between 80 and 180°C.

**15.** Method according to any of the preceding claims, comprising an additional step of applying an oxidizing agent to the keratinous fibres.

**16.** Method for dyeing and/or lightening according to any of the preceding claims, wherein the keratinous materials are dark keratinous fibres possessing a tone level of less than or equal to 6, particularly less than or equal to 4.

**17.** Colouring composition comprising, in an appropriate cosmetic medium, a dye of formula **(I)** as defined in any of Claims 1 to 12, where **Het** represents a heterocyclic disulphide radical of formula **(II)** as defined in Claim 1, the radical **A** of the formula (I) contains at least one cationic radical which is carried by or included in at least one of the chromophores and with the provisos that:

- the compound of formula **(I)** cannot comprise fluorescent riboflavin-5-monophosphate chromophore **(A);**
- the compound of formula **(I)** cannot comprise fluorescent chromophore **(A)** which represents an indol-3-yl or 5-methoxyindol-3-yl group; and
- the compound of formula **(I)** cannot represent the compounds **(A), (B), (C)** or **(D)** :

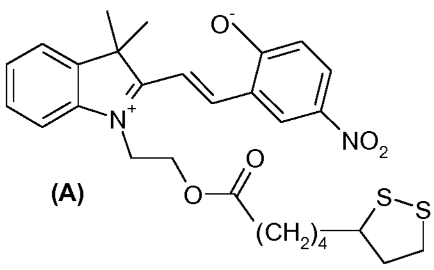

(A)

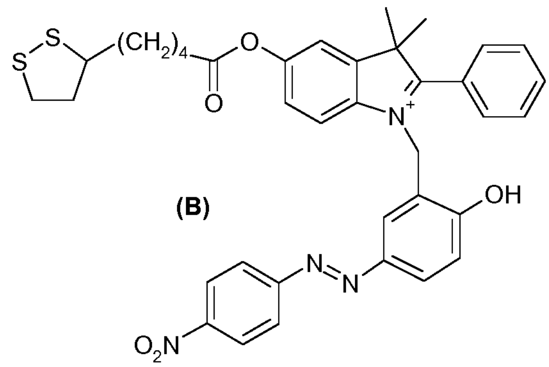

(B)

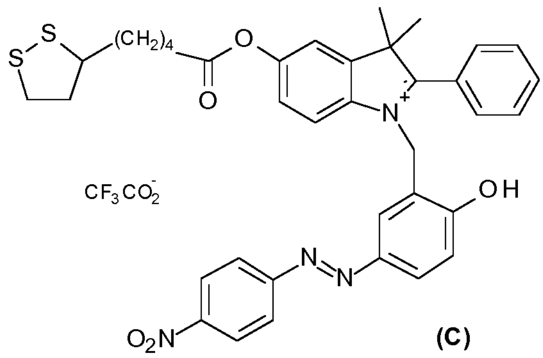

(C)

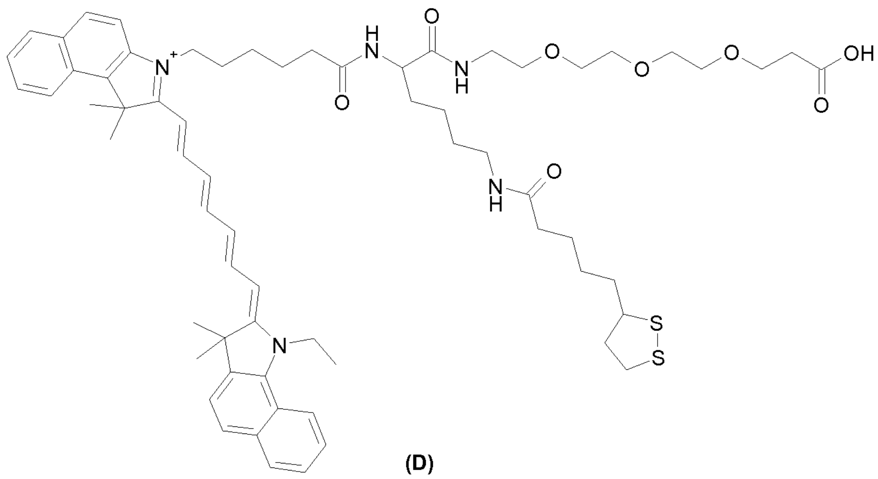

(D)

where **(B)** is combined or not combined with a CdSe-ZnS, CdTe-ZnS, CdSe-ZnSe or CdTe-ZnSe core-shell quantum dot; **(C)** is combined or not combined with a CdSe-ZnS core-shell quantum dot.

**18.** Composition according to the preceding claim, wherein the dye of formula **(I)** is present in an amount of between 0.001 and 50% by weight, relative to the total weight of the composition.

**19.** Dye of formula **(I)** as defined in any of Claims 1 to 12, where **Het** represents a heterocyclic disulphide radical of formula **(II)** as defined in Claim 1, the radical **A** of the formula (I) contains at least one cationic radical which is carried by or included in at least one of the chromophores, and with the provisos that:

- the compound of formula **(I)** cannot comprise fluorescent riboflavin-5-monophosphate chromophore **(A);**
- the compound of formula **(I)** cannot comprise fluorescent chromophore **(A)** which represents an indol-3-yl or 5-methoxyindol-3-yl group; and
- the compound of formula **(I)** cannot represent the compounds **(A), (B), (C)** or **(D)** :

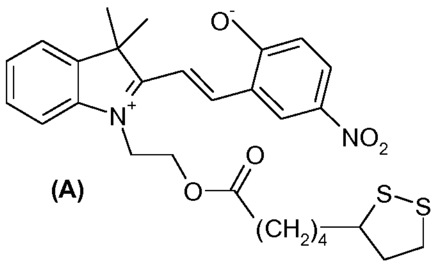

(A)

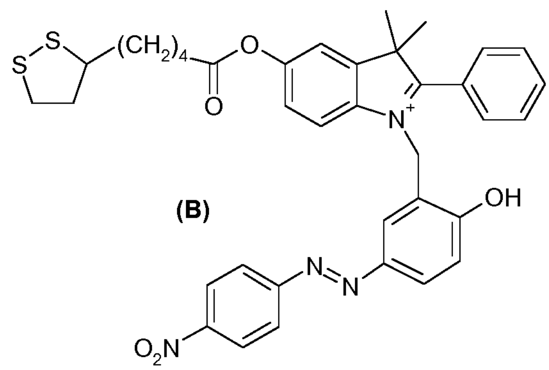

(B)

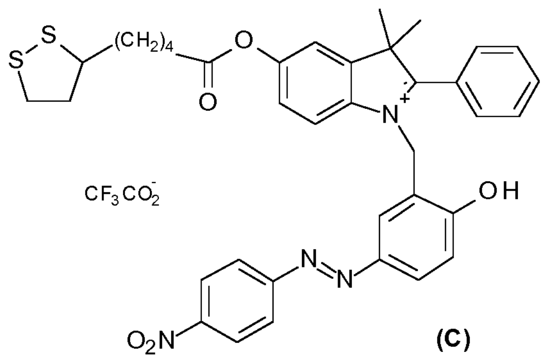

(C)

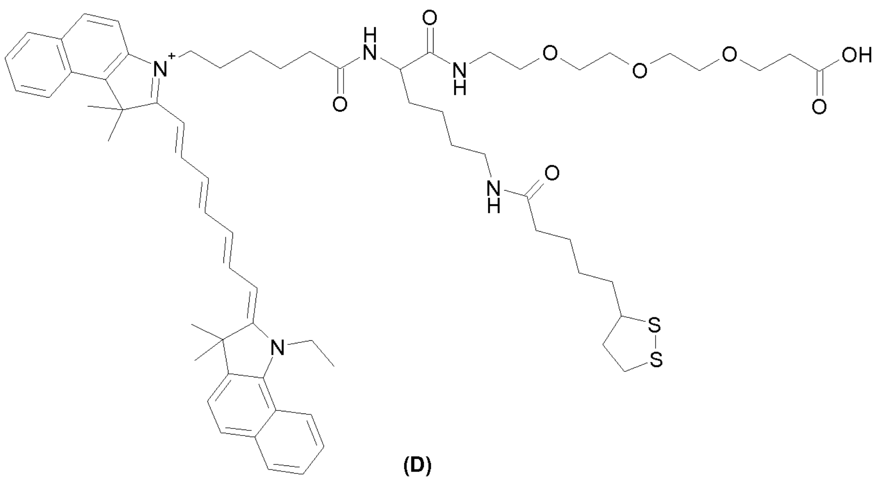

(D)

where **(B)** is combined or not combined with a CdSe-ZnS, CdTe-ZnS, CdSe-ZnSe or CdTe-ZnSe core-shell quantum dot; **(C)** is combined or not combined with a CdSe-ZnS core-shell quantum dot.

**20.** Use of dyes of formula **(I)** as defined in Claims 1 to 12 for dyeing and/or lightening human keratinous materials such as human keratinous fibres, especially dark fibres with a tone level of less than 6, preferably less than or equal to 4.

**Patentansprüche**

**1.** Verfahren zum Färben von Keratinmaterialien, bei dem man auf die Materialien eine geeignete Färbezusammensetzung, die einen oder mehrere Farbstoffe der nachstehenden Formel **(I)**:

$$\mathbf{A\text{-}(X)_p\text{-}C_{sat}\text{-}(X')_{p'}\text{-}Het} \qquad \mathbf{(I)}$$

Salze mit organischen oder anorganischen Säuren, optische Isomere und geometrische Isomere davon und Solvate

davon wie Hydrate umfasst, aufbringt; wobei in Formel **(I):**

- **A** für einen Rest, der mindestens einen kationischen oder nichtkationischen Chromophor enthält, steht;
- **Het** für einen heterocyclischen Disulfidrest wie denjenigen der Formel **(II)** steht, der über einen der Substituenten $R_5$, $R_6$, $R_7$, $R_8$, $R_9$ oder $R_{10}$ an den Rest des Moleküls gebunden ist oder der über eines der Kohlenstoffatome des heterocyclischen Rests in alpha- oder beta-Position oder dann, wenn q für 2 oder 3 steht, in gamma-Position direkt an den Rest des Moleküls gebunden ist, wobei in diesem Fall einer der Substituenten $R_5$, $R_6$, $R_7$, $R_8$, $R_9$ oder $R_{10}$ fehlt:

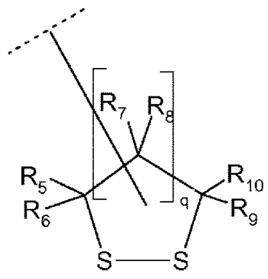

**(II)**

wobei in Formel **(II):**

- **$R_5$, $R_6$, $R_9$** und **$R_{10}$,** die gleich oder verschieden sind, für ein Wasserstoffatom oder eine aus i) ($C_1$-$C_8$)Alkyl, ii) Aryl, iii) Hydroxyl, iv) (Di) ($C_1$-$C_8$) (alkyl) amino, v) ($C_1$-$C_8$) Alkoxy, vi) (Poly) hydroxy ($C_1$-$C_8$)alkyl, vii) (Di) ($C_1$-$C_8$) (alkyl) amino ($C_1$-$C_8$)alkyl, viii) Carboxyl, ix) Carboxy($C_1$-$C_3$)alkyl, x) (Di) ($C_1$-$C_8$) (alkyl) aminocarbonyl ($C_1$-$C_8$)alkyl und xii) ($C_1$-$C_8$) (Alkyl) carbonyl ($C_1$-$C_8$) (alkyl) amino ($C_1$-$C_8$)alkyl ausgewählte Gruppe stehen;
- **$R_7$** und **$R_8$,** die gleich oder verschieden sind, für ein Wasserstoffatom oder eine aus i) ($C_1$-$C_8$)Alkyl, ii) Aryl, iii) Hydroxyl, iv) (Di)($C_1$-$C_8$)(alkyl)amino, v) ($C_1$-$C_8$)Alkoxy, vi) (Poly)hydroxy($C_1$-$C_8$)alkyl, vii) (Di)($C_1$-$C_8$)(alkyl)amino($C_1$-$C_8$)alkyl, viii) Carboxyl, ix) Carboxy($C_1$-$C_8$)alkyl, x) (Di)($C_1$-$C_8$)(alkyl)amino-carbonyl($C_1$-$C_8$)alkyl und xii) ($C_1$-$C_8$)(Alkyl) carbonyl($C_1$-$C_8$)(alkyl)amino($C_1$-$C_8$)alkyl ausgewählte Gruppe stehen;
- **q** für eine ganze Zahl zwischen 1 und 3 inklusive steht; mit der Maßgabe, dass dann, wenn **q** für 2 oder 3 steht, die Gruppen **$R_7$** und **$R_8$** gleich oder voneinander verschieden sein können;

- **X** und **X'**, die gleich oder verschieden sind, für:

  ◦ eine gesättigte oder ungesättigte, lineare oder verzweigte $C_1$-$C_{30}$-Kohlenwasserstoffkette, die gegebenenfalls durch eine oder mehrere zweiwertige Gruppen oder Kombinationen davon, die aus:

  - -N (R)-; $-N^+(R)(R^1)-$, $Q^-$; -O-; -S-; -CO-; $-SO_2-$, wobei R und R', die gleich oder verschieden sein können, aus einem Wasserstoffatom und einem $C_1$-$C_4$-Alkyl-, Hydroxyalkyl- und Aminoalkylrest ausgewählt sind und $Q^-$ für ein anionisches Gegenion steht;
  - einem anellierten oder nichtanellierten, gesättigten oder ungesättigten, aromatischen oder nichtaromatischen (hetero)-cyclischen Rest, der gegebenenfalls ein oder mehrere gleiche oder verschiedene und gegebenenfalls substituierte Heteroatome umfasst;

  ausgewählt sind, unterbrochen und/oder an einem oder beiden ihrer Enden terminiert ist;

  ◦ eine zweiwertige Gruppe oder Kombination davon, die aus: -N(R)-; $-N^+(R)(R')-$, $Q^-$; -O-; -S-; -CO-; $-SO_2-$ ausgewählt ist, wobei R, R' und $Q^-$ wie oben definiert sind; stehen;

- **p** und **p'**, die gleich oder verschieden sind, für eine ganze Zahl mit einem Wert von 0 oder 1 stehen; und
- **$C_{sat}$** für eine gegebenenfalls cyclische, gegebenenfalls substituierte, lineare oder verzweigte $C_1$-$C_{18}$-Alkylenkette steht.

**2.** Färbeverfahren nach dem vorhergehenden Anspruch, wobei der Farbstoff der Formel **(I)** so beschaffen ist, dass **p**

für 1 steht und **X** und **X',** die gleich oder verschieden sein können, für die folgende Sequenz stehen:

$$-(T)_t-(Z)_z-(T')_{t'}-$$

wobei die Sequenz in Formel **(I)** folgendermaßen gebunden ist:

**-$C_{sat}$-(T')t'-$(Z)_z$-(A** oder **Het)**;

wobei in dieser Sequenz:

- **T** und **T'**, die gleich oder verschieden sind, für einen oder mehrere Reste oder Kombinationen davon stehen, die aus: $-S(O)_2-$; -O-; -S-; -N(R)-; $-N^+(R)(R^\circ)-$, $Q^-$; -CO-; wobei R und R°, die gleich oder verschieden sind, für ein Wasserstoffatom, einen $C_1$-$C_4$-Alkyl-, $C_1$-$C_4$-Hydroxyalkyl- oder Aryl($C_1$-$C_4$)alkylrest stehen und $Q^-$ für ein anionisches Gegenion steht; und einem kationischen oder nichtkationischen Heterocycloalkyl- oder Heteroarylrest ausgewählt sind;
- die Indices **t und t'**, die gleich oder verschieden sind, für 0 oder 1 stehen;
- **Z** für:

  - $-(CR_1R_2)_m-$, wobei m für eine ganze Zahl zwischen 1 und 8 steht und $R_1$ und $R_2$, die gleich oder verschieden sind, für ein Wasserstoffatom oder eine $C_1$-$C_4$-Alkyl-, $C_1$-$C_{12}$-Alkoxy-, Hydroxyl-, Cyano-, Carboxyl-, Amino-, $C_1$-$C_4$-Alkylamino- oder $C_1$-$C_4$-Dialkylaminogruppe stehen, wobei die Alkylreste mit dem Stickstoffatom, das sie trägt, einen 5-bis 7-gliedrigen Heterocyclus bilden können, der gegebenenfalls ein weiteres Heteroatom, das von Stickstoff verschieden ist oder nicht, umfasst;
  - $-(CH_2CH_2O)_q-$ oder $-(OCH_2CH_2)_q-$, wobei q für eine ganze Zahl zwischen 1 und 15 steht;
  - einen Aryl-, Alkylaryl- oder Arylalkylrest, wobei der Alkylrest $C_1$-$C_4$ ist und der Arylrest vorzugsweise $C_6$ ist, der gegebenenfalls durch mindestens eine Gruppe $SO_3M$ substituiert ist, wobei M für ein Wasserstoffatom, ein Alkalimetall oder eine Ammoniumgruppe, die durch einen oder mehrere gleiche oder verschiedene, lineare oder verzweigte $C_1$-$C_{18}$-Alkylreste, die gegebenenfalls mindestens ein Hydroxyl tragen, substituiert ist, steht;

  steht; und

- **z** für 0 oder 1 steht.

3. Färbeverfahren nach einem der vorhergehenden Ansprüche, wobei der Farbstoff der Formel **(I)** so beschaffen ist, dass **A** aus den Chromophoren ausgewählt ist, die sich von i) (Poly)azofarbstoffen wie (Di)azofarbstoffen; ii) Hydrazonofarbstoffen; iii) (Poly)methinfarbstoffen wie Styrylen und iv) Anthrachinofarbstoffen ableiten.

4. Färbeverfahren nach einem der vorhergehenden Ansprüche, wobei der Farbstoff der Formel **(I)** so beschaffen ist, dass **A** sich von kationischen Hydrazono-Chromophoren der Formeln **(III)** und **(III'),** Azo-Chromophoren **(IV)** und **(IV')** und Diazo-Chromophoren **(V)** ableitet:

$$Het^+-C(R^a)=N-N(R^b)-Ar,\ Q^- \quad (II),$$

$$Het^+-N(R^a)-N=C(R^b)-Ar,\ Q^- \quad (III)$$

$$Het^+-N=N-Ar,\ Q^- \quad (IV)$$

$$-Ar^+-N=N-Ar'',\ Q^- \quad (IV')$$

und

$$Het^+-N=N-Ar'-N=N-Ar,\ Q^- \quad (V)$$

wobei in den Formeln **(III), (III'), (IV), (IV')** und **(V):**

- **$Het^+$** für einen kationischen Heteroarylrest, insbesondere einen endocyclischen kationischen Rest wie Imidazolium, Indolium oder Pyridinium, der gegebenenfalls insbesondere durch mindestens eine ($C_1$-$C_8$)Alkylgruppe wie Methyl substituiert ist, steht;

- **Ar⁺** für einen Arylrest, wie eine Phenyl- oder Naphthylgruppe, mit einer exocyclischen kationischen Ladung, insbesondere Ammonium, spezieller Tri($C_1$-$C_8$)alkylammonium wie Trimethylammonium, steht;

- **Ar** für eine Arylgruppe, insbesondere Phenyl, die gegebenenfalls substituiert ist, vorzugsweise durch mindestens einen Elektronendonator wie i) gegebenenfalls substituiertes ($C_1$-$C_8$)Alkyl, ii) gegebenenfalls substituiertes ($C_1$-$C_8$)Alkoxy, iii) (Di)($C_1$-$C_8$)(alkyl)amino, das gegebenenfalls an der Alkylgruppe bzw. den Alkylgruppen durch eine oder mehrere Hydroxylgruppen substituiert ist, iv) Aryl($C_1$-$C_8$)alkylamino, v) gegebenenfalls substituiertes *N*-($C_1$-$C_8$)Alkyl-*N*-aryl($C_1$-$C_8$)alkylamino, steht oder **Ar** für eine Julolidingruppe steht;

- **Ar'** für eine gegebenenfalls substituierte zweiwertige (Hetero)arylengruppe wie Phenylen, insbesondere para-Phenylen, oder Naphthalin, das gegebenenfalls substituiert ist, insbesondere durch eine oder mehrere Gruppen, die aus ($C_1$-$C_8$)Alkyl, Hydroxy und ($C_1$-$C_8$)Alkoxy ausgewählt sind, steht;

- **Ar"** für eine gegebenenfalls substituierte (Hetero)arylgruppe wie Phenyl oder Pyrazolyl, das gegebenenfalls substituiert ist, insbesondere durch eine oder mehrere Gruppen, die aus ($C_1$-$C_8$)Alkyl, Hydroxy, (Di)($C_1$-$C_8$)(alkyl)amino, ($C_1$-$C_8$)Alkoxy und Phenyl ausgewählt sind, steht;

- **$R^a$** und **$R^b$,** die gleich oder verschieden sind, für ein Wasserstoffatom oder eine ($C_1$-$C_8$)Alkylgruppe, die gegebenenfalls substituiert ist, insbesondere durch eine Hydroxygruppe, stehen, oder der Substituent **$R^a$** mit einem Substituenten des **Het⁺**-Rests und/oder **$R^b$** mit einem Substituenten des **Ar**-Rests zusammen mit dem Atom, an das sie gebunden sind, ein (Hetero)-cycloalkyl bilden;

insbesondere **$R^a$** und **$R^b$** für ein Wasserstoffatom oder eine ($C_1$-$C_4$)Alkylgruppe, die möglicherweise oder gegebenenfalls durch eine oder mehrere Hydroxygruppen substituiert ist, stehen;

- **$Q^-$** für ein organisches oder anorganisches anionisches Gegenion wie Halogen oder Alkylsulfat steht;

- mit der Maßgabe, dass **(III), (III'), (IV), (IV')** oder **(V)** über **Het⁺, Ar⁺, Ar** oder **Ar'** an den Farbstoff der Formel (I) gebunden ist.

**5.** Färbeverfahren nach einem der Ansprüche 1 bis 3, wobei der Farbstoff der Formel **(I)** so beschaffen ist, dass **p** für 1 steht und **A** für einen fluoreszierenden Chromophor wie: **W-C($R^c$)=C($R^d$)-Ar'-** oder **-W'-C($R^c$)=C($R^d$)-Ar** steht, wobei:

• **W** ein einwertiger Rest ist, der für ein Heterocyclyl oder ein Heteroaryl mit einem quaternären Ammonium, das gegebenenfalls durch ein $C_1$-$C_8$-Alkyl, das gegebenenfalls insbesondere durch eine oder mehrere Hydroxylgruppen substituiert ist, steht;

• **W'** ein zweiwertiger Rest ist, der für ein Heterocyclyl oder Heteroaryl wie für **W** definiert steht;

• **Ar** für einen 5- oder 6-gliedrigen Arylrest vom Phenyltyp oder ein bicyclisches aromatisches System vom Naphthyltyp, das gegebenenfalls i) durch ein oder mehrere Halogenatome; ii) durch eine oder mehrere Alkylgruppen; iii) durch eine oder mehrere Hydroxylgruppen; iv) durch eine oder mehrere Alkoxygruppen; v) durch eine oder mehrere Hydroxyalkylgruppen; vi) durch eine oder mehrere Amino- oder (Di)alkylaminogruppen, wobei die $C_1$-$C_4$-Alkylgruppierung gegebenenfalls durch ein oder mehrere Hydroxylgruppen substituiert ist, wie (Di)hydroxylethylamino; vii) durch eine oder mehrere Acylaminogruppen oder viii) durch eine oder mehrere 5- oder 6-gliedrige Heterocycloalkyl- oder Heteroarylgruppen substituiert ist, steht;

• **Ar'** ein zweiwertiger Rest ist, der für einen Arylrest wie für **Ar** definiert steht; und

• **$R^c$** und **$R^d$,** die gleich oder verschieden sein können, für ein Wasserstoffatom oder eine $C_1$-$C_4$-Alkylgruppe stehen.

**6.** Färbeverfahren nach einem der vorhergehenden Ansprüche, wobei der Farbstoff der Formel **(I)** die Formel **(I')** aufweist:

$$\mathbf{A\text{-}(T)_t\text{-}[(CR_1R_2)_m]_z\text{-}T_a\text{-}C_{sat}\text{-}(T_b)_{t'}\text{-}Het} \qquad \text{(I')}$$

wobei in der Formel **(I'):**

- **Het** für einen heterocyclischen Disulfidrest der Formel **(II)** gemäß Anspruch 1 steht;

- **A** sich von kationischen Hydrazono-Chromophoren der Formeln **(III)** und **(III'),** Azo-Chromophoren **(IV)** und **(IV')** und Diazo-Chromophoren **(V)** gemäß Anspruch 4 ableitet oder für ein fluoreszierendes Chromophor **W-C($R^c$)=C($R^d$)-Ar'-** oder **-W'-C($R^c$)=C($R^d$)-Ar** gemäß Anspruch 5 steht,

- **z** für 0 oder 1 steht;

- **t** und **t',** die gleich oder verschieden sind, für 0 oder 1 stehen; und

- **T** für i) eine Aminogruppe -N(R)-, wobei R für ein Wasserstoffatom oder einen $C_1$-$C_4$-Alkyl-, $C_1$-$C_4$-Hydroxyalkyl-

oder Aryl($C_1$-$C_4$)alkylrest steht, oder ii) eine gegebenenfalls substituierte zweiwertige Heterocycloalkylgruppe steht;

- **$T_a$** und **$T_b$,** die gleich oder verschieden sind, für einen oder mehrere Reste oder Kombinationen davon stehen, die aus: $-S(O)_2-$, -O-, -S-, -N(R)-, $-N^+(R)(R^\circ)-$, $M^-$, -C(O)-; wobei R und R°, die gleich oder verschieden sind, für ein Wasserstoffatom, einen $C_1$-$C_4$-Alkyl- oder $C_1$-$C_4$-Hydroxyalkylrest oder einen Aryl($C_1$-$C_4$)alkylrest stehen und $M^-$ für ein organisches oder anorganisches anionisches Gegenion wie Halogenid steht, ausgewählt sind, wobei vorzugsweise $T_a$ für eine kovalente σ-Bindung oder eine Gruppe -N(R)- steht und $T_b$ für eine kovalente σ-Bindung oder eine Gruppe, die aus einem Rest -O-, -C(O)-, -N(R)- oder einer Kombination davon ausgewählt ist, steht, wobei R für ein Wasserstoffatom oder eine $C_1$-$C_4$-Alkylgruppe wie Methyl steht; und

- wobei **$T_a$** vorzugsweise in para-Position an Ar oder Ar' zu der Olefinfunktion $-C(R^c)=C(R^d)$-steht.

7. Färbeverfahren nach einem der Ansprüche 5 und 6, wobei der Farbstoff der Formel **(I)** so beschaffen ist, dass **W** für eine Gruppe, die aus Imidazolium, Pyridinium, Benzopyridinium, Benzimidazolium, Chinolinium, Indolinium und Pyrazolium, die gegebenenfalls substituiert sind, ausgewählt ist, steht.

8. Färbeverfahren nach einem der vorhergehenden Ansprüche, wobei der Farbstoff der Formel **(I)** aus den nachstehenden Verbindungen **(Ia)** bis **(Ig):**

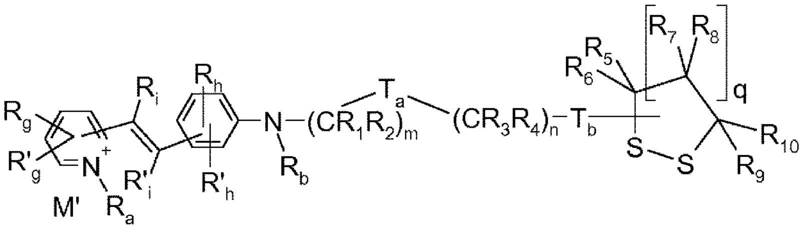

**(Ia)**

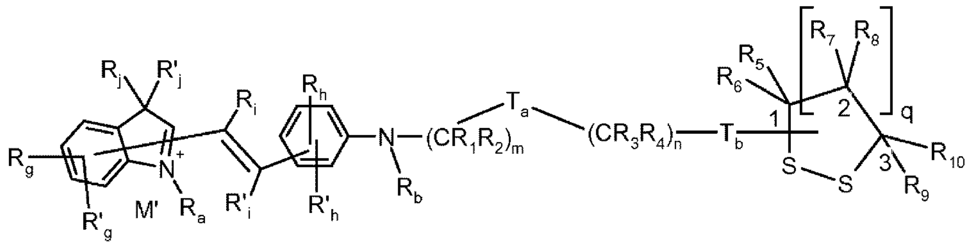

**(Ib)**

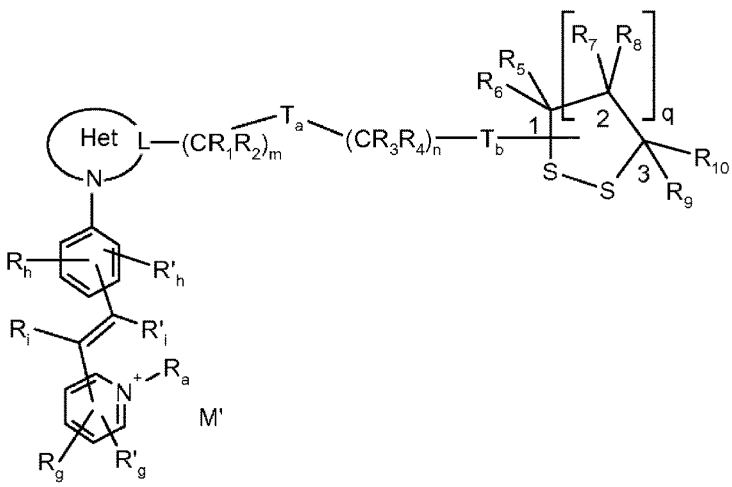

**(Ic)**

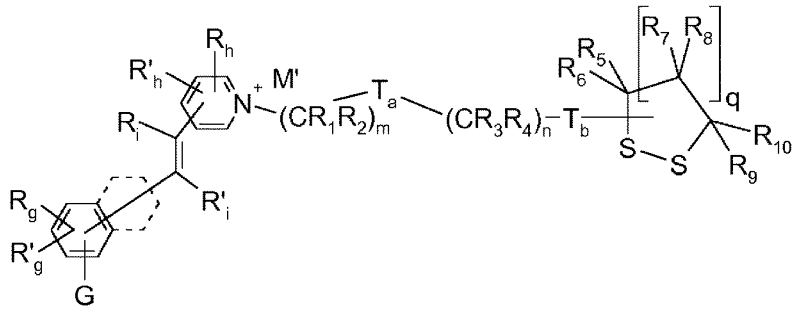

(Id)

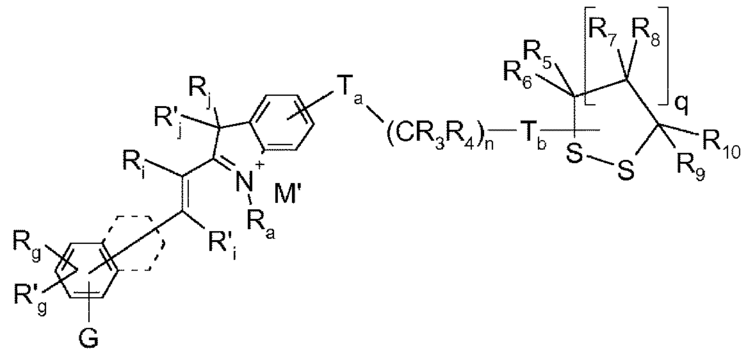

(Ie)

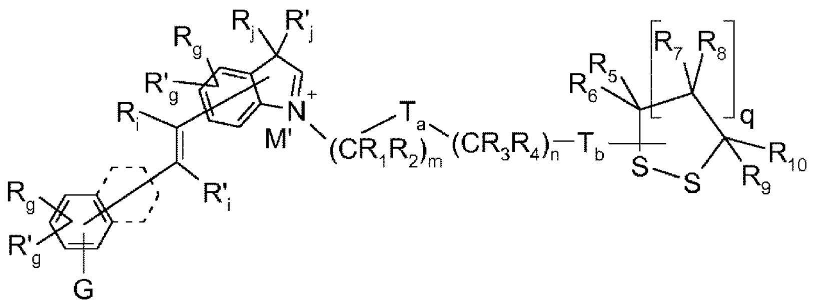

(If)

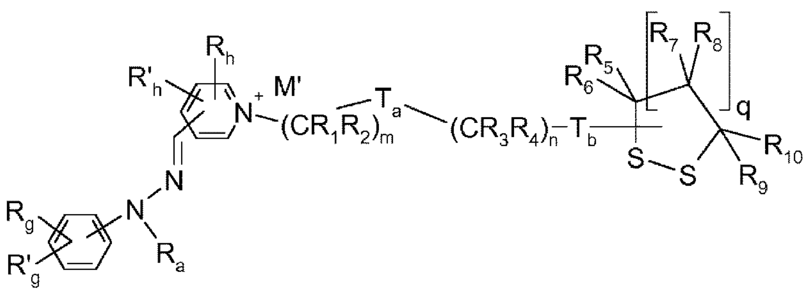

(Ig)

und ihren Salzen mit organischen oder anorganischen Säuren, optischen Isomeren und geometrischen Isomeren und den Solvaten wie Hydraten ausgewählt ist;
wobei in den Formeln **(Ia)** bis **(Ig):**

- **G** für eine Gruppe $-NR_cR_d$ oder eine $C_1$-$C_6$-Alkoxygruppe steht;
- $\mathbf{R^a}$ für eine gegebenenfalls substituierte $C_1$-$C_6$-Alkylgruppe steht;
- $\mathbf{R^b}$ für ein Wasserstoffatom oder eine gegebenenfalls substituierte $C_1$-$C_6$-Alkylgruppe steht;
- $\mathbf{R_c}$ und $\mathbf{R_d}$, die gleich oder verschieden sind, für ein Wasserstoffatom, eine Aryl($C_1$-$C_4$)alkylgruppe, $C_1$-$C_6$-Alkoxy oder eine gegebenenfalls substituierte $C_1$-$C_6$-Alkylgruppe stehen; $R_c$ und $R_d$ vorzugsweise für ein Wasserstoffatom oder eine $C_1$-$C_3$-Alkylgruppe, die gegebenenfalls durch i) eine Hydroxylgruppe, ii) Amino, iii) $C_1$-$C_3$-(Di)alkylamino oder iv) quaternäres Ammonium (R'')(R''')(R'''')$N^+$- substituiert ist, stehen; oder zwei benachbarte Reste $\mathbf{R_c}$ und $\mathbf{R_d}$, die von demselben Stickstoffatom getragen werden, zusammen eine heterocyclische Gruppe oder Heteroarylgruppe bilden;
insbesondere $\mathbf{R_c}$ und $\mathbf{R_d}$ für gleiche Gruppen stehen und vorzugsweise $R_c$ und $R_d$ für ein $C_1$-$C_3$-Alkyl, das gegebenenfalls durch eine Hydroxylgruppe substituiert ist, wie Methyl, Hydroxyethyl und 2-Hydroxypropyl, stehen;

• $\mathbf{R_g}$, $\mathbf{R'_g}$, $\mathbf{R_h}$ und $\mathbf{R'_h}$, die gleich oder verschieden sind, für ein Wasserstoffatom, ein Halogenatom, eine Amino-, $C_1$-$C_4$-Alkylamino-, $C_1$-$C_4$-Dialkylamino-, Cyano-, Carboxyl-, Hydroxyl- oder Trifluormethylgruppe, einen Acylamino-, $C_1$-$C_4$-Alkoxy-, $C_2$-$C_4$-(Poly)hydroxyalkoxy-, Alkylcarbonyloxy-, Alkoxycarbonyl- oder Alkylcarbonylaminorest, einen Acylamino-, Carbamoyl- oder Alkylsulfonylaminorest, einen Aminosulfonylrest oder einen $C_1$-$C_{16}$-Alkylrest, der gegebenenfalls durch eine Gruppe, die aus $C_1$-$C_{12}$-Alkoxy, Hydroxyl, Cyano, Carboxyl, Amino, $C_1$-$C_4$-Alkylamino und $C_1$-$C_4$-Dialkylamino ausgewählt ist, substituiert ist oder die beiden Alkylreste, die von dem Stickstoffatom der Aminogruppe getragen werden, einen 5- bis 7-gliedrigen Heterocyclus bilden, der gegebenenfalls ein weiteres Stickstoff- oder Nichtstickstoff-Heteroatom umfasst, stehen; vorzugsweise $R_g$, $R'_g$, $R_h$ und $R'_h$ für ein Wasserstoff- oder Halogenatom oder eine $C_1$-$C_3$-Alkylgruppe stehen;

• oder zwei Gruppen $\mathbf{R_g}$ und $\mathbf{R'_g}$; $\mathbf{R_h}$ und $\mathbf{R'_h}$; die von zwei benachbarten Kohlenstoffatomen getragen werden, zusammen einen Benzo- oder Indenoring, eine kondensierte Heterocycloalkylgruppe oder eine kondensierte Heteroarylgruppe bilden; wobei der Benzo-, Indeno-, Heterocycloalkyl- oder Heteroarylring gegebenenfalls durch ein Halogenatom, eine Amino-, $C_1$-$C_4$-Alkylamino-, $C_1$-$C_4$-Dialkylamino-, Nitro-, Cyano-, Carboxyl-, Hydroxyl- oder Trifluormethylgruppe, einen Acylamino-, $C_1$-$C_4$-Alkoxy-, $C_2$-$C_4$-(Poly)hydroxy-alkoxy-, Alkylcarbonyloxy-, Alkoxycarbonyl- oder Alkylcarbonylaminorest, einen Acylamino-, Carbamoyl- oder Alkylsulfonylaminorest, einen Aminosulfonylrest oder einen $C_1$-$C_{16}$-Alkylrest, der gegebenenfalls durch eine Gruppe, die aus $C_1$-$C_{12}$-Alkoxy, Hydroxyl, Cyano, Carboxyl, Amino, $C_1$-$C_4$-Alkylamino und $C_1$-$C_4$-Dialkylamino ausgewählt ist, substituiert ist oder die beiden Alkylreste, die von dem Stickstoffatom der Aminogruppe getragen werden, einen 5- bis 7-gliedrigen Heterocyclus bilden, der gegebenenfalls ein weiteres Stickstoff- oder Nichtstickstoff-Heteroatom umfasst, substituiert ist; vorzugsweise $\mathbf{R_g}$ und $\mathbf{R'_g}$ zusammen eine Benzogruppe bilden;

• oder dann, wenn **G** für $-NR_cR_d$ steht, zwei Gruppen $R_c$ und $R'_g$; $R_d$ und $R_g$; zusammen ein einen gesättigten Heterocyclus oder ein gesättigtes Heteroaryl, der bzw. das gegebenenfalls durch eine oder mehrere $C_1$-$C_6$-Alkylgruppen substituiert ist, vorzugsweise einen 5- bis 7-gliedrigen Heterocyclus mit einem oder zwei Heteroatomen, die aus Stickstoff und Sauerstoff ausgewählt sind, bilden;

• $\mathbf{R_i}$, $\mathbf{R_j}$, $\mathbf{R'_j}$ **und** $\mathbf{R'_i}$, die gleich oder verschieden sind, für ein Wasserstoffatom oder eine $C_1$-$C_4$-Alkylgruppe stehen; vorzugsweise $\mathbf{R_i}$, $\mathbf{R_j}$, $\mathbf{R'_j}$ **und** $\mathbf{R'_i}$ für ein Wasserstoffatom stehen;

• $\mathbf{R_1}$, $\mathbf{R_2}$, $\mathbf{R_3}$ und $\mathbf{R_4}$, die gleich oder verschieden sind, für ein Wasserstoffatom oder eine $C_1$-$C_4$-Alkyl-, $C_1$-$C_{12}$-Alkoxy-, Hydroxyl-, Cyano-, Carboxyl-, Amino-, $C_1$-$C_4$-Alkylamino- oder $C_1$-$C_4$-Dialkylaminogruppe stehen, wobei die Alkylreste mit dem Stickstoffatom, das sie trägt, einen 5-bis 7-gliedrigen Heterocyclus, der gegebenenfalls ein weiteres Stickstoff- oder Nichtstickstoff-Heteroatom umfasst, bilden können; vorzugsweise $R_1$, $R_2$, $R_3$ und $R_4$ Wasserstoffatome oder eine Aminogruppe sing; weiter bevorzugt $R_1$, $R_2$, $R_3$ und $R_4$ für ein Wasserstoffatom stehen;

• $\mathbf{T_a}$ und $\mathbf{T_b}$, die gleich oder verschieden sind, für

i) entweder eine kovalente $\sigma$-Bindung;
ii) oder einen oder mehrere Reste oder Kombinationen davon, die aus $-S(O)_2-$, -O-, -S-, -N(R)-, $-N^+(R)(R^\circ)-$, $M^-$, -C(O)-ausgewählt sind, wobei R und R°, die gleich oder verschieden sind, für ein Wasserstoffatom oder einen $C_1$-$C_4$-Alkyl- oder $C_1$-$C_4$-Hydroxyalkylrest oder ein Aryl ($C_1$-$C_4$)alkyl stehen und $M^-$ für ein organisches oder anorganisches anionisches Gegenion wie Halogenid steht; vorzugsweise $T_a$ für eine kovalente $\sigma$-Bindung oder eine Gruppe -N(R)- steht und $T_b$ für eine kovalente $\sigma$-Bindung oder eine Gruppe, die aus einem Rest -O-, -C(O)-, -N(R)- oder einer Kombination davon ausgewählt ist, steht, wobei R für ein Wasserstoffatom oder eine $C_1$-$C_4$-Alkylgruppe wie Methyl steht;

•

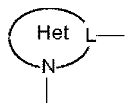

für eine gegebenenfalls substituierte heterocyclische Gruppe steht; L für ein Kohlenstoffatom oder ein Stickstoffatom steht, wobei der Heterocyclus vorzugsweise monocyclisch und gesättigt ist und insgesamt zwei Stickstoffatome umfasst und 5- bis 8-gliedrig ist;

•

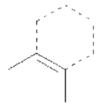

für eine Aryl-Heteroarylgruppe, die an den Phenylring kondensiert ist, steht oder bei dem Phenylring fehlt; dann, wenn der Ring vorliegt, der Ring vorzugsweise ein Benzo ist; weiter bevorzugt der Ring fehlt;

- **m** und **n,** die gleich oder verschieden sind, für eine ganze Zahl zwischen 0 und 10, vorzugsweise zwischen 0 und 7, inklusive stehen, wobei m vorzugsweise zwischen 0 und 5, weiter bevorzugt zwischen 0 und 3, inklusive liegt und wobei n zwischen 0 und 8 inklusive liegt; weiter bevorzugt n für 0, 1, 2, 3 oder 4 steht;
- **$R_5$, $R_6$, $R_9$** und **$R_{10}$,** die gleich oder verschieden sind, für ein Wasserstoffatom oder eine aus: i) ($C_1$-$C_8$)Alkyl, iii) Hydroxyl, iv) (Di)($C_1$-$C_8$)-(alkyl)amino, v) ($C_1$-$C_8$)Alkoxy, vi) (Poly)-hydroxy($C_1$-$C_8$)alkyl, vii) (Di)($C_1$-$C_8$)(alkyl)-amino($C_1$-$C_8$)alkyl, viii) Carboxyl, ix) Carboxy-($C_1$-$C_3$)alkyl, x) (Di)($C_1$-$C_8$)(alkyl)aminocarbonyl-($C_1$-$C_8$)alkyl und xii) ($C_1$-$C_8$)(Alkyl)carbonyl ($C_1$-$C_8$)(alkyl)amino ($C_1$-$C_8$)alkylausgewählte Gruppe stehen;
vorzugsweise **$R_5$** für ein Atom oder eine Alkylgruppe wie Methyl steht und **$R_6$, $R_9$** und **$R_{10}$** für Wasserstoffatome stehen;
- **$R_7$** und **$R_8$,** die gleich oder verschieden sind, für ein Wasserstoffatom oder eine aus: i) ($C_1$-$C_8$)-Alkyl, iii) Hydroxyl, iv) (Di)($C_1$-$C_8$)(alkyl)-amino, v) ($C_1$-$C_8$)Alkoxy, vi) (Poly)hydroxy($C_1$-$C_8$)alkyl, vii) (Di)($C_1$-$C_8$)(alkyl)amino($C_1$-$C_8$)-alkyl, viii) Carboxyl, ix) Carboxy($C_1$-$C_8$)alkyl, x) (Di)($C_1$-$C_8$)(alkyl)amino-carbonyl($C_1$-$C_8$)alkyl und xii) ($C_1$-$C_8$)(Alkyl)carbonyl($C_1$-$C_8$)(alkyl)-amino($C_1$-$C_8$)alkyl ausgewählte Gruppe stehen;
**$R_7$** und **$R_8$** für ein Wasserstoffatom oder eine $C_1$-$C_4$-Alkylgruppe wie Methyl stehen;
- **q** für eine ganze Zahl zwischen 1 und 3 inklusive steht; vorzugsweise q = 1; und
- **M'** für ein organisches oder anorganisches anionisches Gegenion steht;

mit der Maßgabe, dass der Disulfid-Heterocyclus direkt über das Kohlenstoffatom an den Rest des Moleküls gebunden ist, vorzugsweise in der Beta-Position der Disulfid-Funktion des Heterocyclus.

**9.** Färbeverfahren nach einem der Ansprüche 1 bis 3 und 5 bis 8, wobei der fluoreszierende Farbstoff der Formel **(I)** aus den nachstehenden Verbindungen **(I'a), (I'b), (I'd), (I'e)** und **(I'f)** ausgewählt ist:

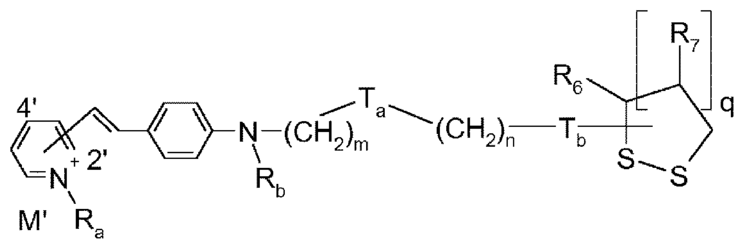

**(I'a)**

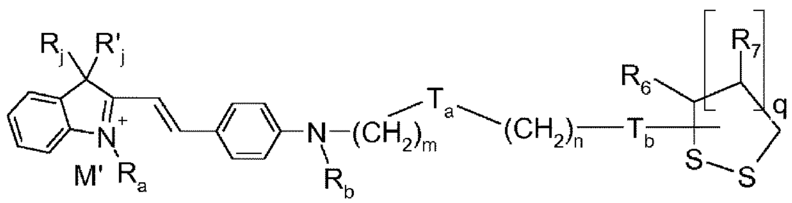

**(I'b)**

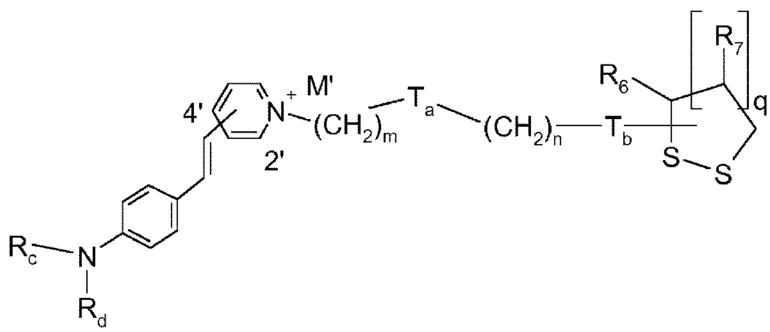

**(I'd)**

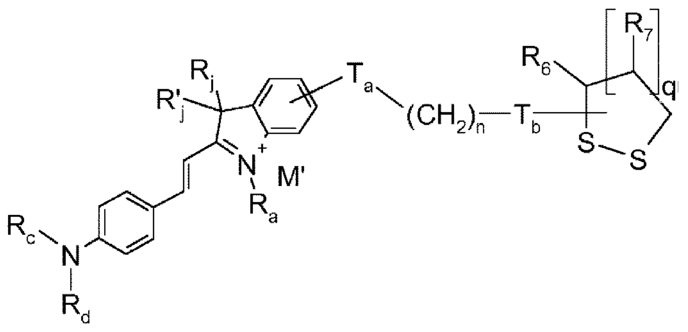

**(I'e)**

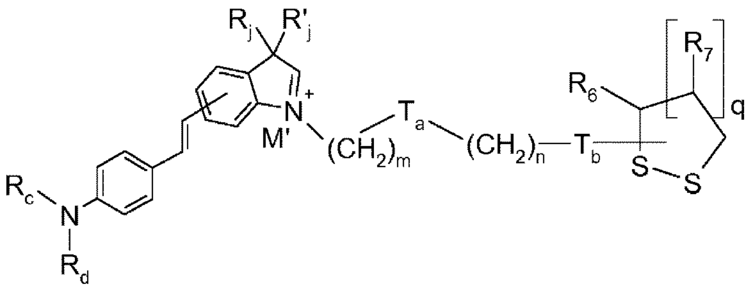

**(I'f)**

wobei in den Formeln **(I'a), (I'b), (I'd), (I'e)** und **(I'f):**

- ◦ $\mathbf{R_a}$, $\mathbf{R_b}$, $\mathbf{R_j}$ und $\mathbf{R'_j}$ für eine $C_1$-$C_4$-Alkylgruppe wie Methyl stehen;
- ◦ $\mathbf{R_6}$ und $\mathbf{R_7}$ für ein Wasserstoffatom oder eine $C_1$-$C_4$-Alkylgruppe wie Methyl stehen, wobei $\mathbf{T_b}$ direkt an das Kohlenstoffatom in alpha- oder beta-Position zur Disulfid-Funktion des Heterocyclus gebunden ist;
- ◦ **m** und **n,** die gleich oder verschieden sind, für eine ganze Zahl zwischen 0 und 7 inklusive stehen, wobei m zwischen 0 und 3 liegt und wobei n für 0, 1, 2, 3 oder 4 steht;
- ◦ $\mathbf{T_a}$ und $\mathbf{T_b}$, die gleich oder verschieden sind, für i) eine kovalente σ-Bindung oder ii) eine Gruppe, die aus einem Rest -O-, -C(O)-, -N(R)- oder einer Kombination davon wie -N(R)-C(O)-, -C(O)-N(R)-, -O-C(O)-, -C(O)-O- oder -C(O)-N(R)-C(O)- ausgewählt ist, steht, wobei R für ein Wasserstoffatom oder eine $C_1$-$C_4$-Alkylgruppe steht; insbesondere R für ein Wasserstoffatom oder ein Methyl steht;
- ◦ **q** für 1, 2 oder 3 steht; insbesondere q für 1 steht;
- ◦ $\mathbf{R_c}$ und $\mathbf{R_d}$ für gleiche $C_1$-$C_3$-Alkylgruppen, die gegebenenfalls durch eine Hydroxylgruppe substituiert sind, wie Methyl, Hydroxyethyl und 2-Hydroxypropyl, stehen;
  und
- ◦ **M'** für ein organisches oder anorganisches anionisches Gegenion steht;

mit der Maßgabe, dass in der Formel **(I'a)** und **(I'd)** die Pyridiniumgruppe über das Kohlenstoffatom in Position 2' (ortho) oder 4' (para), vorzugsweise 4', an das Styryl gebunden ist.

**10.** Färbeverfahren nach einem der Ansprüche 1 bis 4, 6 und 8, wobei der Farbstoff der Formel **(I)** aus nachstehendem **(I'g)** ausgewählt ist:

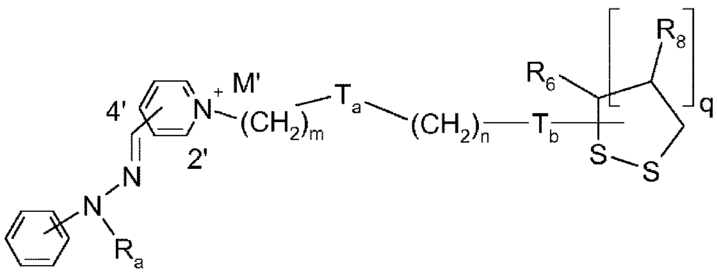

**(I'g)**

wobei in der Formel **(I'g):**

◦ $\mathbf{R_a}$ für eine $C_1$-$C_4$-Alkylgruppe wie Methyl steht;

◦ $\mathbf{T_a}$ und $\mathbf{T_b}$, die gleich oder verschieden sind, für i) eine kovalente σ-Bindung oder ii) eine Gruppe, die aus einem Rest -O-, -C(O)-, -N(R)- oder einer Kombination davon wie -N(R)-C(O)-, -C(O)-N(R), -O-C(O)-, -C(O)-O- oder -C(O)-N(R)-C(O)- ausgewählt ist, steht, wobei R für ein Wasserstoffatom oder eine $C_1$-$C_4$-Alkylgruppe steht; insbesondere R für ein Wasserstoffatom oder ein Methyl steht;

◦ $\mathbf{R_6}$ und $\mathbf{R_7}$ für ein Wasserstoffatom oder eine $C_1$-$C_4$-Alkylgruppe wie Methyl stehen, wobei $\mathbf{T_b}$ direkt an das Kohlenstoffatom in alpha- oder beta-Position zur Disulfid-Funktion des Heterocyclus gebunden ist;

◦ **m** und **n,** die gleich oder verschieden sind, für eine ganze Zahl zwischen 0 und 7 inklusive stehen, wobei m zwischen 0 und 3 liegt und wobei n für 0, 1, 2, 3 oder 4 steht;

◦ **q** für 1, 2 oder 3 steht; insbesondere q für 1 steht;

◦ **M'** für ein organisches oder anorganisches anionisches Gegenion steht;

mit der Maßgabe, dass die Pyridiniumgruppe über das Kohlenstoffatom in Position 2' (ortho) oder 4' (para), vorzugsweise 4', an das Hydrazono gebunden ist.

**11.** Färbeverfahren nach einem der Ansprüche 1 bis 3 und 5 bis 9, wobei der fluoreszierende Farbstoff der Formel **(I)** aus den nachstehenden Verbindungen **(1)** bis **(68)** ausgewählt ist:

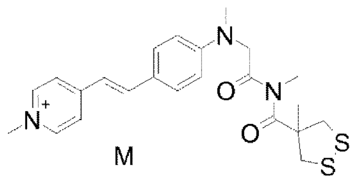

**1**

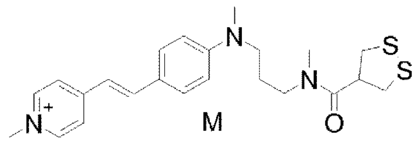

**2**

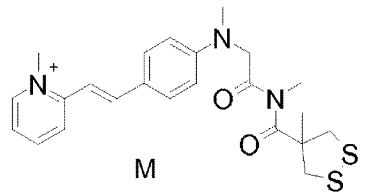

**3**

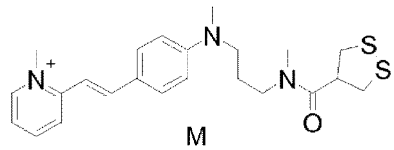

**4**

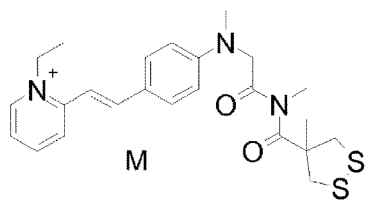

**5**

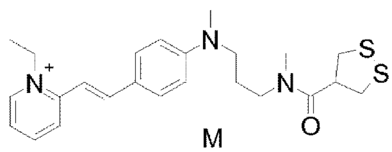

**6**

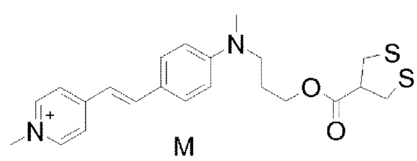

**7**

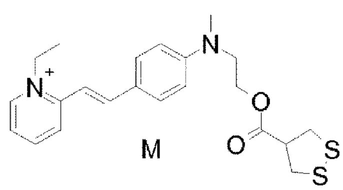

**8**

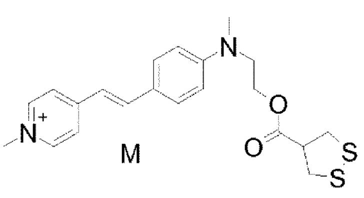

**9**

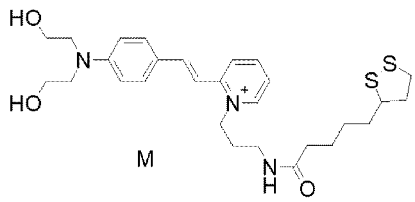

**10**

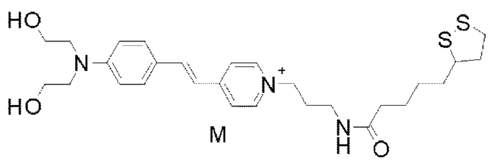

**11**

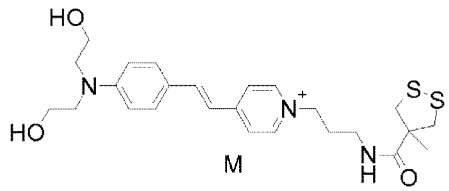

**12**

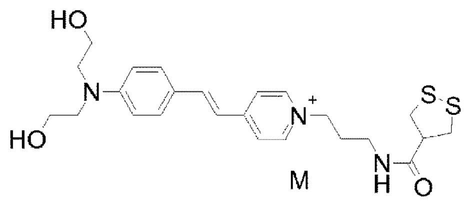

**13**

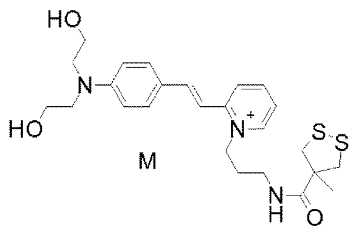

**14**

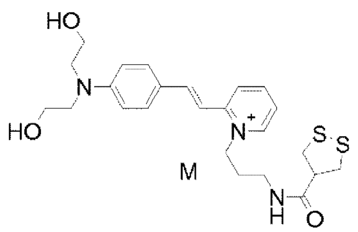

**15**

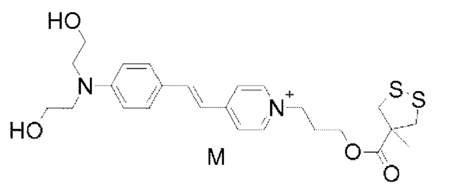

**16**

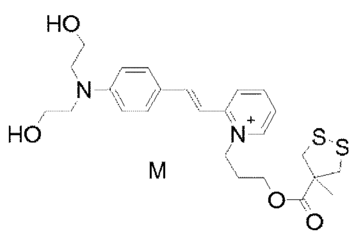

**17**

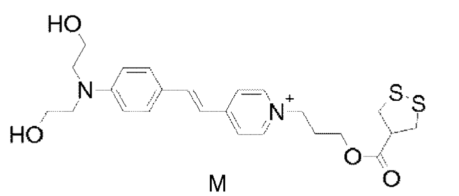

**18**

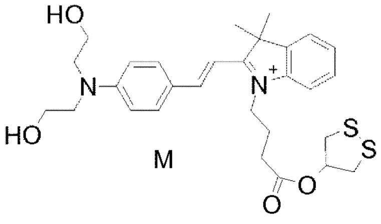

**19**

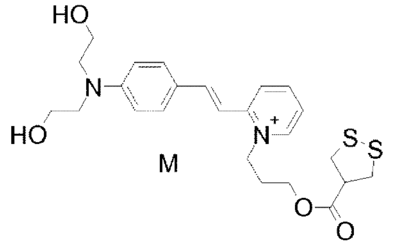

**20**

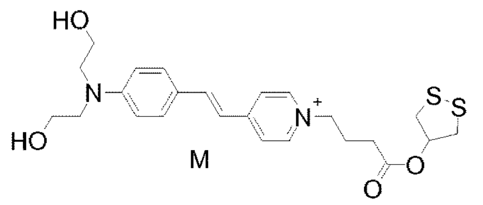

**21**

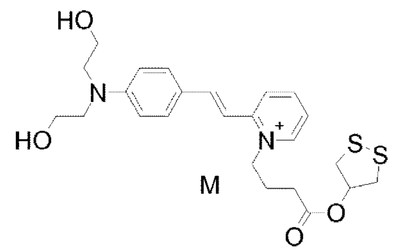

**22**

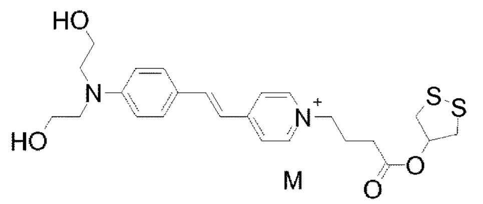

**23**

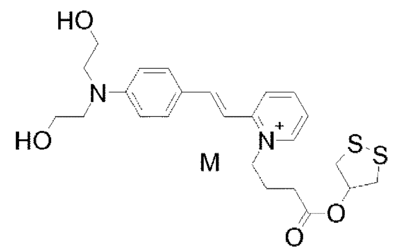

**24**

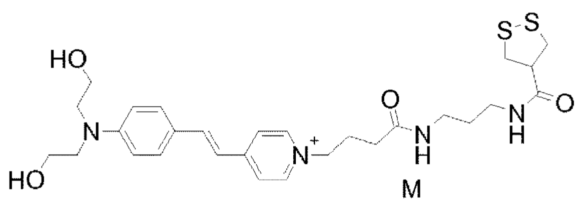

**25**

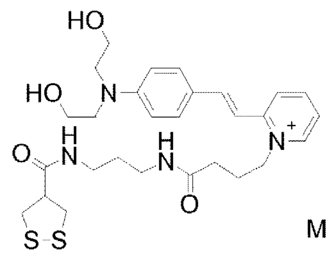

**26**

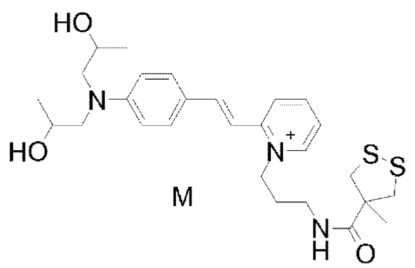

**27**

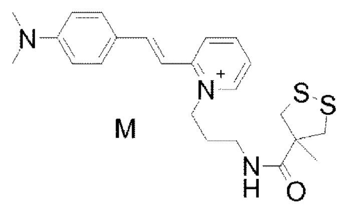

**28**

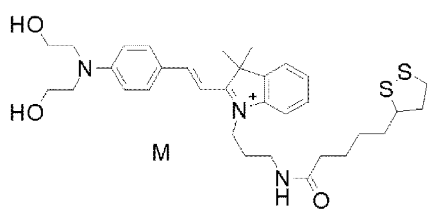

**29**

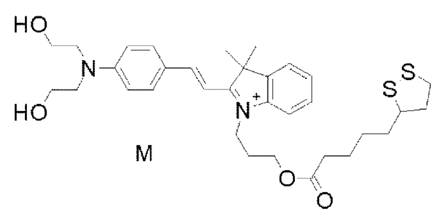

**30**

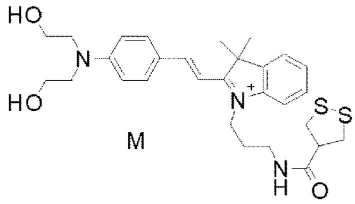

**31**

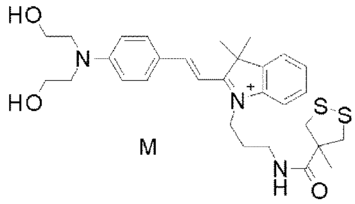

**32**

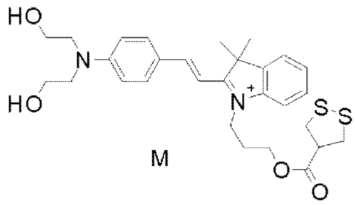

**33**

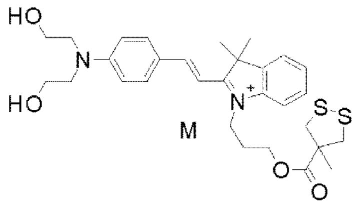

**34**

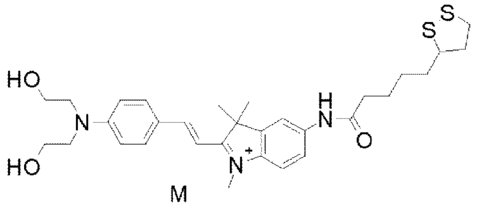

**35**

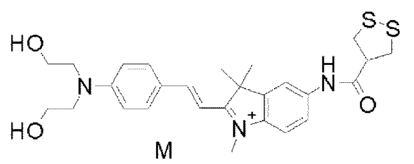

**36**

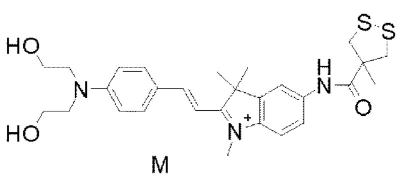

**37**

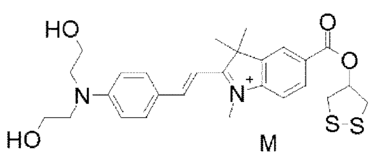

**38**

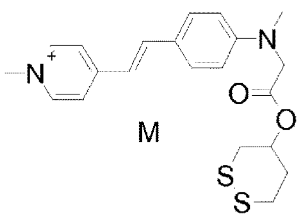

**39**

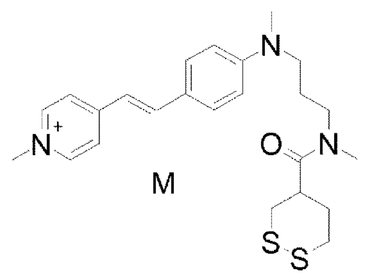

**40**

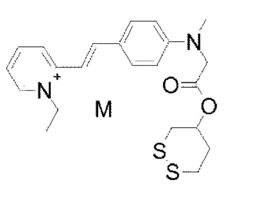

**41**

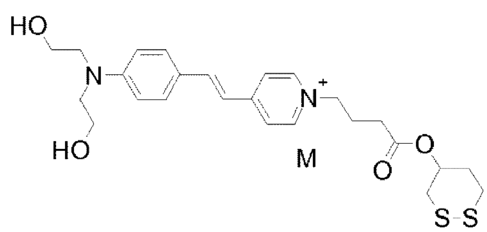

**42**

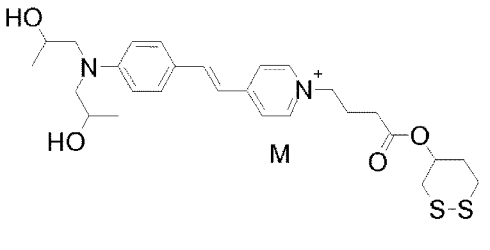

43

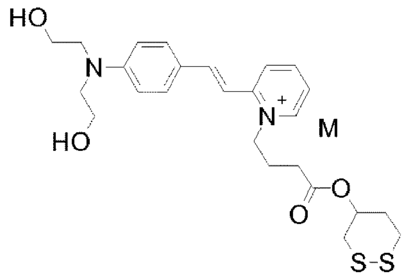

44

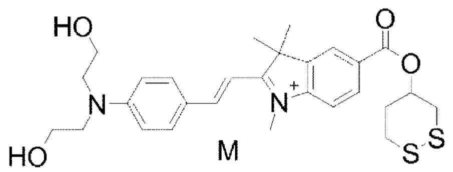

45

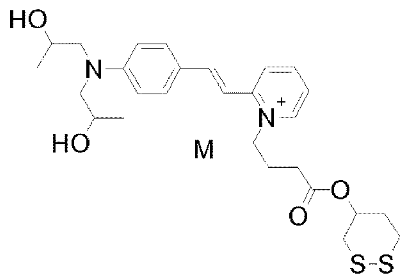

46

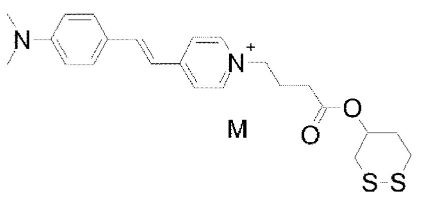

47

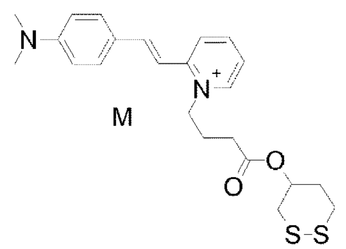

48

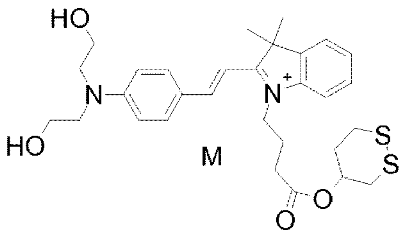

49

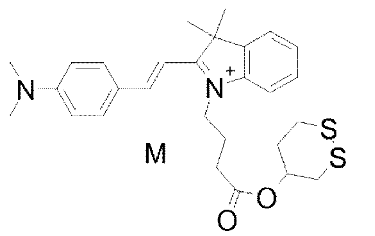

50

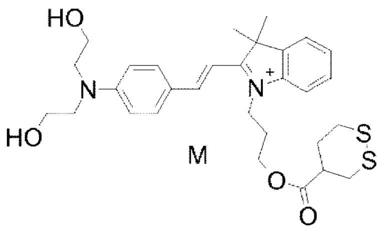

51

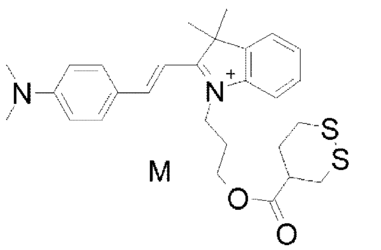

52

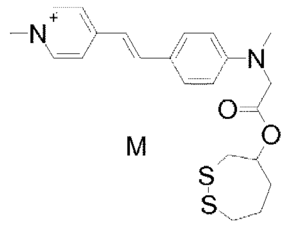

53

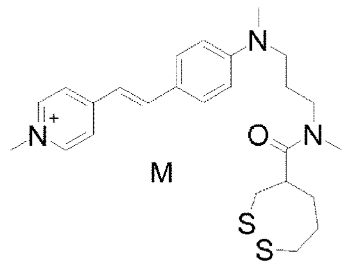

54

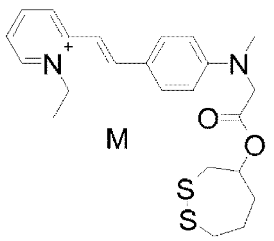

55

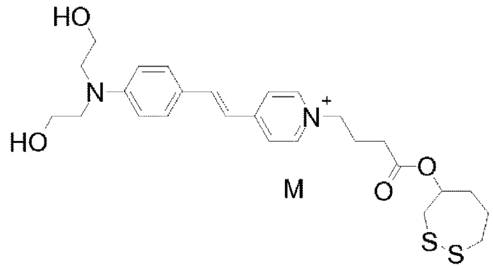

56

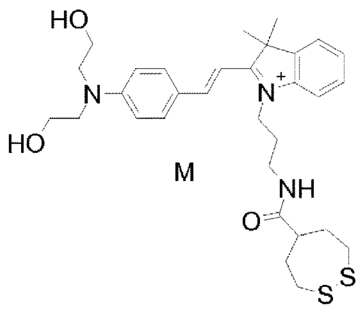

57

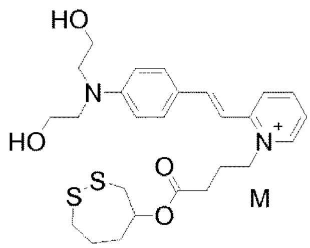

58

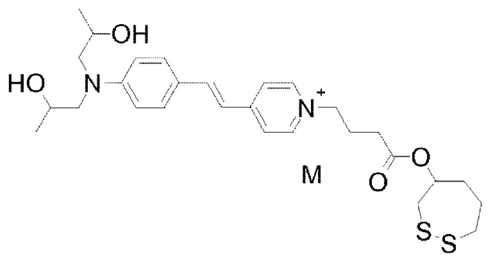

59

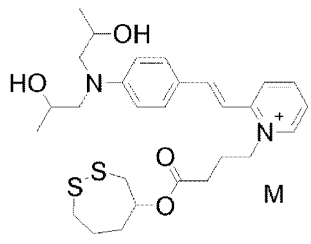

60

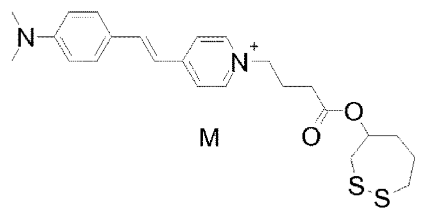

61

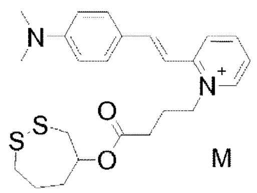

62

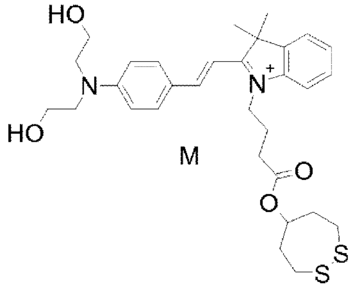

**63**

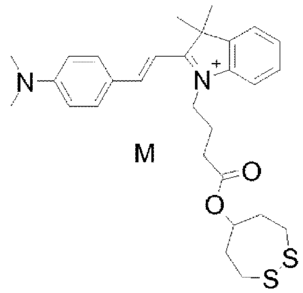

**64**

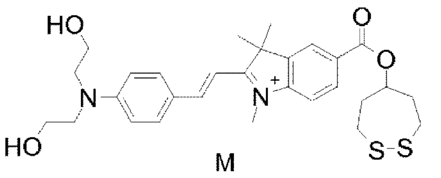

**65**

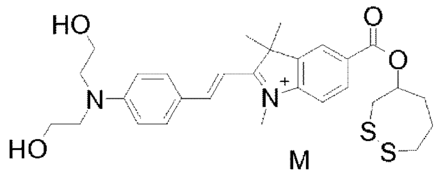

**66**

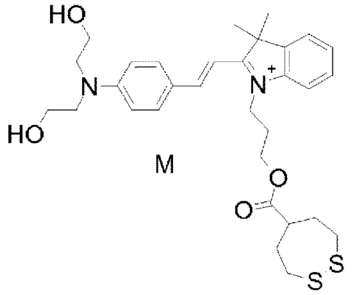

**67**

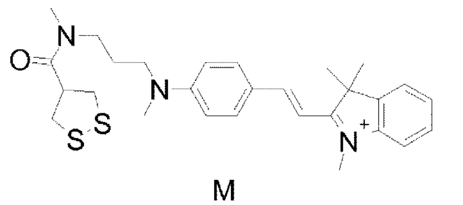

**68**

wobei M für ein anionisches Gegenion steht.

**12.** Färbeverfahren nach einem der Ansprüche 1 bis 4, 6, 8 und 10, wobei der Farbstoff der Formel **(I)** aus nachstehender Verbindung **69** ausgewählt ist:

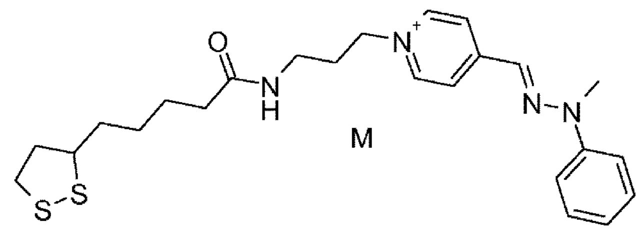

**69**

wobei M für ein anionisches Gegenion steht.

**13.** Verfahren zum Färben von Keratinmaterialien nach einem der vorhergehenden Ansprüche, wobei die Zusammensetzung, die die Verbindung bzw. Verbindungen der Formel **(I)** umfasst, einwirken gelassen wird und die Keratinmaterialien dann gespült und/oder ausgewrungen werden und/oder eine Wärmebehandlung durchlaufen und/oder Ultraviolett(UV)-Licht ausgesetzt werden.

**14.** Verfahren zum Färben von Keratinmaterialien nach dem vorhergehenden Anspruch, wobei die Zusammensetzung, die die Verbindung bzw. Verbindungen der Formel **(I)** umfasst, einwirken gelassen wird und die Keratinmaterialien dann ausgewrungen werden und/oder eine Wärmebehandlung, insbesondere bei einer Temperatur zwischen 50 und 200 °C, vorzugsweise zwischen 80 und 180 °C, durchlaufen.

**15.** Verfahren nach einem der vorhergehenden Ansprüche, bei dem man zusätzlich ein Oxidationsmittel auf die Keratinfasern aufbringt.

**16.** Verfahren zum Färben und/oder Aufhellen nach einem der vorhergehenden Ansprüche, wobei es sich bei den Keratinmaterialien um dunkle Keratinfasern mit einer Tonhöhe kleiner oder gleich 6, insbesondere kleiner oder gleich 4, handelt.

**17.** Färbezusammensetzung, die in einem geeigneten kosmetischen Medium einen Farbstoff der Formel **(I)** gemäß einem der Ansprüche 1 bis 12 umfasst, wobei **Het** für einen heterocyclischen Disulfidrest der Formel **(II)** gemäß Anspruch 1 steht, der Rest **A** der Formel (I) mindestens einen kationischen Rest enthält, der durch mindestens einen der Chromophoren getragen wird oder in mindestens einem der Chromophoren enthalten ist, und mit den Maßgabe, dass:

- die Verbindung der Formel **(I)** keinen fluoreszierenden Riboflavin-5-monophosphat-Chromophor **(A)** umfassen kann;
- die Verbindung der Formel **(I)** keinen fluoreszierenden Chromophor **(A),** der für eine Indol-3-yl- oder 5-Methoxyindol-3-ylgruppe steht, umfassen kann;
- die Verbindung der Formel **(I)** nicht für die Verbindungen **(A), (B), (C)** oder **(D)** stehen kann:

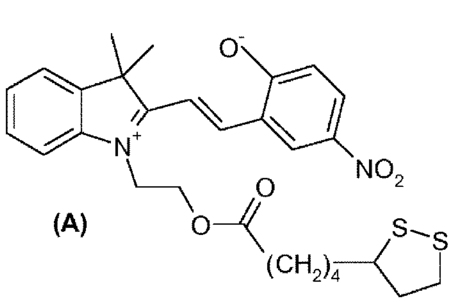

**(A)**

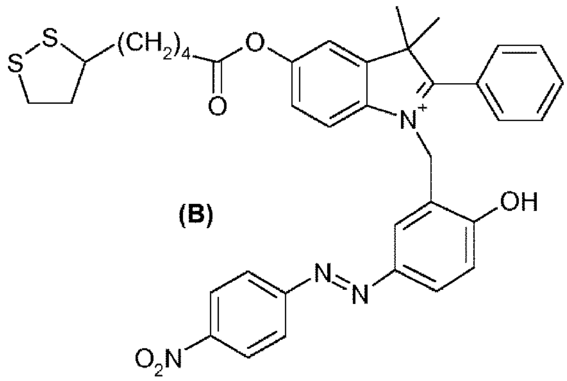

**(B)**

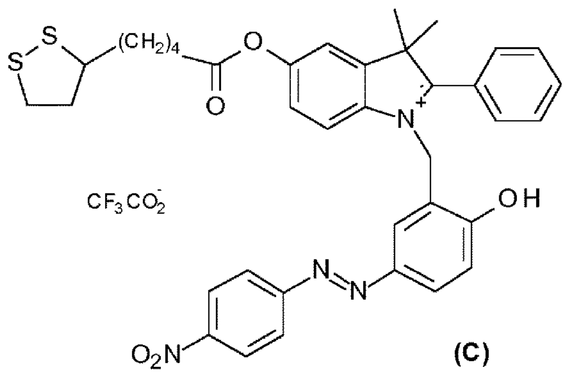

**(C)**

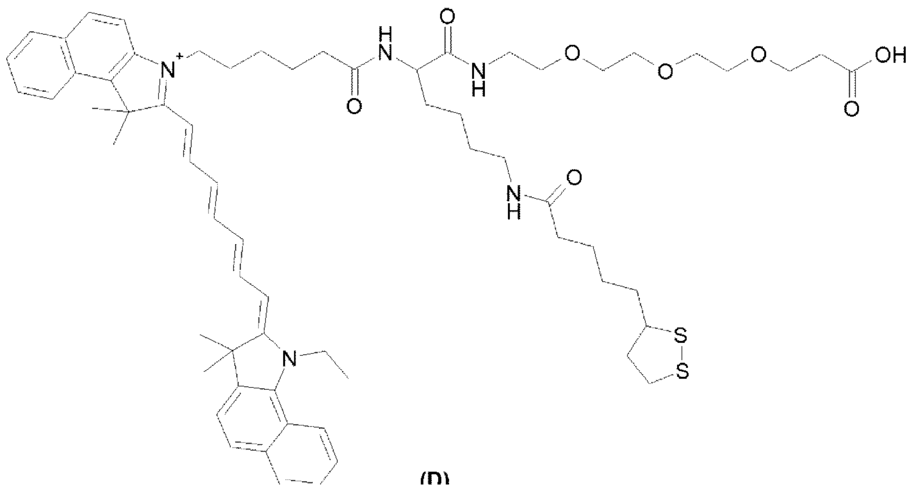

(D)

wobei **(B)** gegebenenfalls mit einem CdSe-ZnS-, CdTe-ZnS-, CdSe-ZnSe- oder CdTe-ZnSe-Kern-Schale-Quantenpunkt kombiniert ist; **(C)** gegebenenfalls mit einem CdSe-ZnS-Kern-Schale-Quantenpunkt kombiniert ist.

**18.** Zusammensetzung nach dem vorhergehenden Anspruch, wobei der Farbstoff der Formel **(I)** in einer Menge zwischen 0,001 und 50 Gew.-%, bezogen auf das Gesamtgewicht der Zusammensetzung, vorliegt.

**19.** Farbstoff der Formel **(I)** gemäß einem der Ansprüche 1 bis 12, wobei **Het** für einen heterocyclischen Disulfidrest der Formel **(II)** gemäß Anspruch 1 steht, der Rest **A** der Formel (I) mindestens einen kationischen Rest enthält, der durch mindestens einen der Chromophoren getragen wird oder in mindestens einem der Chromophoren enthalten ist, und mit den Maßgabe, dass:

- die Verbindung der Formel **(I)** keinen fluoreszierenden Riboflavin-5-monophosphat-Chromophor **(A)** umfassen kann;
- die Verbindung der Formel **(I)** keinen fluoreszierenden Chromophor **(A),** der für eine Indol-3-yl- oder 5-Methoxyindol-3-ylgruppe steht, umfassen kann;
- die Verbindung der Formel **(I)** nicht für die Verbindungen **(A), (B), (C)** oder **(D)** stehen kann:

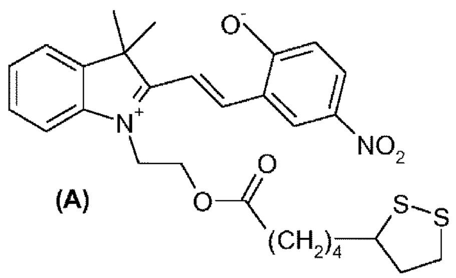

**(A)**

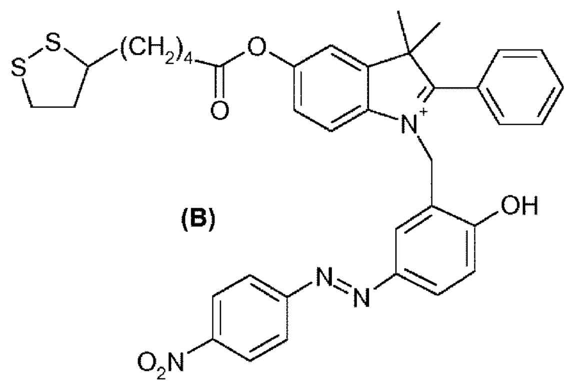

**(B)**

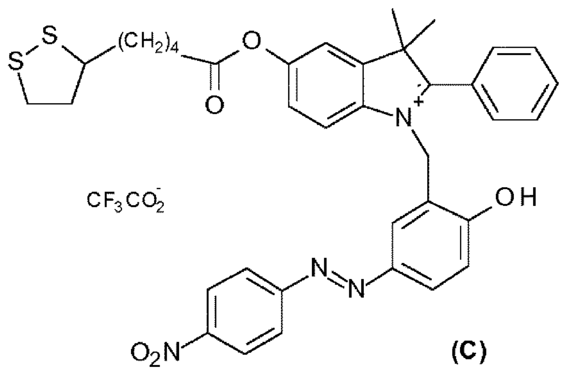

(C)

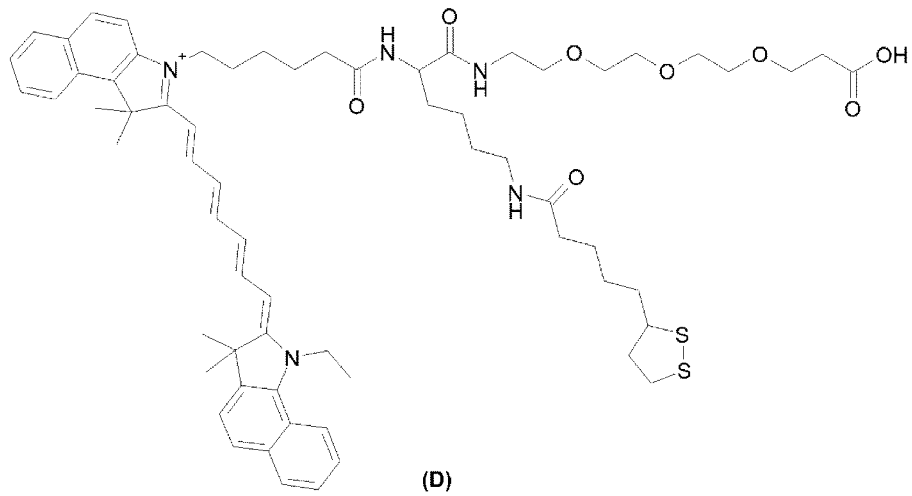

(D)

wobei **(B)** gegebenenfalls mit einem CdSe-ZnS-, CdTe-ZnS-, CdSe-ZnSe- oder CdTe-ZnSe-Kern-Schale-Quantenpunkt kombiniert ist; **(C)** gegebenenfalls mit einem CdSe-ZnS-Kern-Schale-Quantenpunkt kombiniert ist.

**20.** Verwendung von Farbstoffen der Formel **(I)** gemäß den Ansprüchen 1 bis 12 zum Färben und/oder Aufhellen von menschlichen Keratinmaterialien wie menschlichen Keratinfasern, insbesondere dunklen Fasern mit einer Tonhöhe von weniger als 6, vorzugsweise kleiner oder gleich 4.

## Revendications

**1.** Procédé de coloration de matières kératiniques, dans lequel on applique sur lesdites matières une composition colorante appropriée comprenant un ou plusieurs colorants selon la formule (I) ci-dessous :

$$A\text{-}(X)_p\text{-}C_{sat}\text{-}(X')_{p'}\text{-}Het \qquad (I)$$

des sels d'acides organiques ou inorganiques, des isomères optiques et des isomères géométriques de ceux-ci, et des solvates de ceux-ci tels que des hydrates ;
dans laquelle formule (I) :

- ➢ A représente un radical contenant au moins un chromophore cationique ou non cationique ;
- ➢ Het représente un radical disulfure hétérocyclique tel que celui selon la formule (II) qui est relié au reste de la molécule par l'un des substituants $R_5$, $R_6$, $R_7$, $R_8$, $R_9$ ou $R_{10}$, ou ledit radical est relié au reste de la molécule directement par l'un des atomes de carbone du radical hétérocyclique en position alpha ou bêta ou, lorsque q vaut 2 ou 3, en position gamma, auquel cas l'un des substituants $R_5$, $R_6$, $R_7$, $R_8$, $R_9$ ou $R_{10}$ est absent :

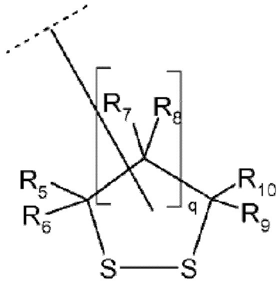

**(II)**

dans laquelle formule (II) :

- $R_5$, $R_6$, $R_9$ et $R_{10}$, qui sont identiques ou différents, représentent un atome d'hydrogène ou un groupe sélectionné parmi des groupes : i) $(C_1\text{-}C_8)$alkyle, ii) aryle, iii) hydroxyle, iv) (di)$(C_1\text{-}C_8)$(alkyl)amino, v) $(C_1\text{-}C_8)$alcoxy, vi) (poly)hydroxy$(C_1\text{-}C_8)$alkyle, vii) (di)$(C_1\text{-}C_8)$(alkyl)amino$(C_1\text{-}C_8)$alkyle, viii) carboxyle, ix) carboxy$(C_1\text{-}C_3)$alkyle, x) (di)$(C_1\text{-}C_8)$(alkyl)aminocarbonyl$(C_1\text{-}C_8)$alkyle, et xii) $(C_1\text{-}C_8)$(alkyl) carbonyl $(C_1\text{-}C_8)$(alkyl) amino-$(C_1\text{-}C_8)$alkyle ;
- $R_7$ et $R_8$, qui sont identiques ou différents, représentent un atome d'hydrogène ou un groupe sélectionné parmi des groupes : i) $(C_1\text{-}C_8)$alkyle, ii) aryle, iii) hydroxyle, iv) (di) $(C_1\text{-}C_8)$(alkyl) amino, v) $(C_1\text{-}C_8)$alcoxy, vi) (poly)hydroxy$(C_1\text{-}C_8)$alkyle, vii) (di)$(C_1\text{-}C_8)$(alkyl)amino$(C_1\text{-}C_8)$alkyle, viii) carboxyle, ix) carboxy$(C_1\text{-}C_8)$alkyle, x) (di)$(C_1\text{-}C_8)$(alkyl)aminocarbonyl$(C_1\text{-}C_8)$alkyle, et xii) $(C_1\text{-}C_8)$(alkyl)carbonyl$(C_1\text{-}C_8)$(alkyl)amino-$(C_1\text{-}C_8)$alkyle ;
- q représente un nombre entier de 1 à 3 inclusivement ;
  sous réserve que lorsque q vaut 2 ou 3, les groupes $R_7$ et $R_8$ puissent être identiques ou différents l'un de l'autre ;

➢ X et X', qui sont identiques ou différents, représentent :

➢ une chaîne hydrocarbonée $C_1\text{-}C_{30}$ saturée ou insaturée, linéaire ou ramifiée, qui est optionnellement interrompue et/ou optionnellement terminée à l'une de ses extrémités ou à ses deux extrémités par un ou plusieurs groupes divalents ou des combinaisons de ceux-ci sélectionnés parmi :

➢ des groupes -N(R)-, $-N^+(R)(R')-$, $Q^-$, -O-, -S-, -C(O)-; $-S(O)_2-$, où R et R', qui sont identiques ou différents, sont sélectionnés parmi un atome d'hydrogène et un radical alkyle $C_1\text{-}C_4$, hydroxyalkyle et aminoalkyle, et $Q^-$ représente un contre-ion anionique ;

➢ un radical (hétéro)cyclique condensé ou non condensé, saturé ou insaturé, aromatique ou non aromatique, comprenant optionnellement un ou plusieurs hétéroatomes identiques ou non identiques et optionnellement substitués ;

➢ un groupe divalent ou une combinaison de ceux-ci sélectionnés parmi des groupes : -N(R)-, $-N^+(R)(R')-$, $Q^-$, -O-, -S-, -C(O)-, $-S(O)_2-$, où R, R' et $Q^-$ sont tels que définis ci-dessus ;

➢ p et p', qui sont identiques ou différents, représentent un nombre entier de 0 ou 1 ; et

➢ $C_{sat}$ représente une chaîne alkylène $C_1\text{-}C_{18}$ optionnellement cyclique, optionnellement substituée, linéaire ou ramifiée.

**2.** Procédé de coloration selon la revendication précédente, dans lequel le colorant selon la formule (I) est tel que p vaut 1, X et X', qui sont identiques ou différents, représentent la séquence suivante :

$$-(T)_t-(Z)_z-(T')t'-$$

ladite séquence étant insérée dans la formule (I) comme il est indiqué ci-après :

$$-C_{sat}-(T')_{t'}-(Z)_z-(T)_t-(A \text{ ou Het}) ;$$

dans laquelle séquence :

➢ T et T', qui sont identiques ou différents, représentent un ou plusieurs radicaux ou des combinaisons de ceux-ci sélectionnés parmi des groupes : $-S(O)_2-$, -O-, -S-, -N(R)-, $-N^+(R)(R°)-$, $Q^-$, -CO-, où R et R°, qui sont identiques ou différents, représentent un atome d'hydrogène ou un radical alkyle $C_1$-$C_4$, hydroxyalkyle $C_1$-$C_4$ ou aryl ($C_1$-$C_4$)alkyle et Q' représente un contre-ion anionique ; et un radical hétérocycloalkyle ou hétéroaryle cationique ou non cationique ;

➢ les indices t et t', qui sont identiques ou différents, valent 0 ou 1 ;

➢ Z représente : un groupe $-(CR_1R_2)_m-$ où m est un nombre entier de 1 à 8 et $R_1$ et $R_2$, qui sont identiques ou différents, représentent un atome d'hydrogène ou un groupe alkyle $C_1$-$C_4$, alcoxy $C_1$-$C_{12}$, hydroxyle, cyano, carboxyle, amino, alkylamino $C_1$-$C_4$ ou dialkylamino $C_1$-$C_4$, lesdits radicaux alkyle pouvant former, avec l'atome d'azote qui les porte, un hétérocycle contenant de 5 à 7 chaînons, comprenant optionnellement un autre hétéroatome différent ou non de l'azote ;
un groupe $-(CH_2CH_2O)_q-$ ou $-(OCH_2CH_2)_q-$ dans lequel q est un nombre entier de 1 à 15, ou
un radical aryle, alkylaryle ou arylalkyle dans lequel le radical alkyle est $C_1$-$C_4$ et le radical aryle est préférablement $C_6$, étant optionnellement substitué par au moins un groupe $SO_3M$, où M représente un atome d'hydrogène, un métal alcalin ou un groupe ammonium qui est substitué par un ou plusieurs radicaux alkyle $C_1$-$C_{18}$, linéaires ou ramifiés, identiques ou non identiques, qui, optionnellement, portent au moins un groupe hydroxyle ; et

➢ z vaut 0 ou 1.

**3.** Procédé de coloration selon l'une ou l'autre des revendications précédentes, dans lequel le colorant selon la formule (I) est tel que A est sélectionné parmi les chromophores dérivés de i) colorants (poly)azoïques tels que les colorants (di)azoïques; ii) colorants hydrazono ; iii) colorants (poly)méthines tels que les styryles et iv) colorants anthraquinones.

**4.** Procédé de coloration selon l'une quelconque des revendications précédentes, dans lequel le colorant selon la formule (I) est tel que A est dérivé de chromophores cationiques hydrazono selon les formules (III) et (III'), azoïques (IV) et (IV'), et diazoïques (V) :

$Hét^+-C(R^a)=N-N(R^b)-Ar, Q^-$ (II),

$Hét^+-N(R^a)-N=C(R^b)-Ar, Q^-$ (III),

$Hét^+-N=N-Ar, Q^-$ (IV)

$Ar^+-N=N-Ar'', Q^-$ (IV')

et

$Hét^+-N=N-Ar'-N=N-Ar, Q^-$ (V)

dans lesquelles formules (III), (III'), (IV), (IV') et (V) :

- $Hét^+$ représentant un radical hétéroaryle cationique, en particulier un radical cationique endocyclique tel que l'imidazolium, l'indolium ou le pyridinium, potentiellement substitué en particulier par au moins un groupe ($C_1$-$C_8$)alkyle tel qu'un groupe méthyle ;
- $Ar^+$ représentant un radical aryle, tel qu'un groupe phényle ou naphtyle, comportant une charge cationique exocyclique en particulier l'ammonium, plus particulièrement le tri($C_1$-$C_8$)alkyl-ammonium tel que le triméthylammonium ;
- Ar représentant un groupe aryle, en particulier un groupe phényle, potentiellement substitué, préférablement par au moins un donneur d'électrons tel que des groupes i) ($C_1$-$C_8$)alkyle potentiellement substitué, ii) ($C_1$-$C_8$)alcoxy potentiellement substitué, iii) (di)($C_1$-$C_8$)(alkyl)amino potentiellement substitué sur le(s) groupe(s) alkyle par un ou plusieurs groupes hydroxyle, iv) aryl ($C_1$-$C_8$)alkylamino, v) *N*-($C_1$-$C_8$)alkyl-*N*-aryl($C_1$-$C_8$)alkylamino potentiellement substitué, ou Ar représentant un groupe julolidine ;
- Ar' représentant un groupe (hétéro)arylène divalent potentiellement substitué tel que le phénylène, en particulier le para-phénylène, ou le naphtalène, potentiellement substitué, en particulier par un ou plusieurs groupes sélectionnés parmi des groupes ($C_1$-$C_8$)alkyle, hydroxy, et ($C_1$-$C_8$)alcoxy ;

- Ar" représentant un groupe (hétéro)aryle potentiellement substitué tel qu'un groupe phényle ou pyrazolyle potentiellement substitué, préférablement par un ou plusieurs groupes sélectionnés parmi des groupes ($C_1$-$C_8$)alkyle, hydroxy, (di) ($C_1$-$C_8$)(alkyl) amino, ($C_1$-$C_8$)alcoxy et phényle ;
- $R^a$ et $R^b$, qui sont identiques ou différents, représentant un atome d'hydrogène ou un groupe ($C_1$-$C_8$)alkyle potentiellement substitué, en particulier par un groupe hydroxy, ou le substituant $R^a$ avec un substituant du radical $Het^+$ et/ou $R^b$ avec un substituant du radical Ar pouvant former, avec l'atome auquel ils sont fixés, un groupe (hétéro)cycloalkyle ;
en particulier, $R^a$ et $R^b$ représentant un atome d'hydrogène ou un groupe ($C_1$-$C_4$)alkyle possiblement ou potentiellement substitué par un ou plusieurs groupes hydroxyle ;
- $Q^-$ représentant un contre-ion anionique organique ou inorganique tel qu'un halogène ou un sulfate d'alkyle ;

sous réserve que (III), (III'), (IV), (IV') ou (V) soit fixé sur le colorant selon la formule (I) par un groupe $Hét^+$, $Ar^+$, Ar ou Ar".

**5.** Procédé de coloration selon l'une quelconque des revendications 1 à 3, dans lequel le colorant selon la formule (I) est tel que p vaut 1, et A représente un chromophore fluorescent tel que :

$$W\text{-}C(R^c)=C(R^d)\text{-}Ar'\text{- ou -}W'\text{-}C(R^c)=C(R^d)\text{-}Ar,$$

où :

- W est un radical monovalent qui représente un hétérocycle ou un groupe hétéroaryle, comprenant un groupe ammonium quaternaire qui est optionnellement substitué par un groupe alkyle $C_1$-$C_8$ qui est optionnellement substitué en particulier par un ou plusieurs groupes hydroxyle ;
- W' est un radical divalent qui représente un hétérocycle ou un groupe hétéroaryle tel que défini pour W ;
- Ar représente un radical aryle à 5 ou 6 chaînons de type phényle ou un système aromatique bicyclique de type naphtyle, optionnellement substitué i) par un ou plusieurs atomes d'halogènes ; ii) par un ou plusieurs groupes alkyle ; iii) par un ou plusieurs groupes hydroxyle ; iv) par un ou plusieurs groupes alcoxy ; v) par un ou plusieurs groupes hydroxyalkyle ; vi) par un ou plusieurs groupes amino ou (di)alkylamino, où la fraction alkyle $C_1$-$C_4$ est optionnellement substituée par un ou plusieurs groupes hydroxyle, tels que des groupes (di)hydroxyléthylamino ; vii) par un ou plusieurs groupes acylamino ; ou viii) par un ou plusieurs groupes hétérocycloalkyle ou hétéroaryle à 5 ou 6 chaînons ;
- Ar' est un radical divalent qui représente un radical aryle tel que défini pour Ar ; et
- $R^c$ et $R^d$, qui sont identiques ou différents, représentent un atome d'hydrogène ou un groupe alkyle $C_1$-$C_4$.

**6.** Procédé de coloration selon l'une quelconque des revendications précédentes, dans lequel le colorant selon la formule (I) est selon la formule (I') :

$$A\text{-}(T)_t\text{-}[(CR_1R_2)_m]_z\text{-}T_a\text{-}C_{sat}\text{-}(T_b)_{t'}\text{-}Het \qquad (I')$$

dans laquelle formule (I') :

- Het représente un radical disulfure hétérocyclique selon la formule (II) tel que défini dans la revendication 1 ;
- A est dérivé de chromophores cationiques hydrazono selon les formules (III) et (III'), azoïques (IV) et (IV'), et diazoïques (V) tels que définis dans la revendication 4, ou représente un chromophore fluorescent W-$C(R^c)=C(R^d)$-Ar'- ou -W'-$C(R^c)=C(R^d)$-Ar, tel que défini dans la revendication 5,
- z vaut 0 ou 1 ;
- t et t', qui sont identiques ou différents, valent 0 ou 1 ; et
- T représente i) un groupe amino -N(R)- où R représente un atome d'hydrogène, un radical alkyle $C_1$-$C_4$, hydroxyalkyle $C_1$-$C_4$ ou aryl ($C_1$-$C_4$)alkyle, ou ii) un groupe hétérocycloalkyle divalent optionnellement substitué ;
- $T_a$ et $T_b$, qui sont identiques ou différents, représentent un ou plusieurs radicaux ou des combinaisons de ceux-ci sélectionnés parmi des groupes $-S(O)_2-$, -O-, -S-, -N(R)-, $-N^+(R)(R^\circ)-$, $M^-$, -C(O)-, où R et $R^\circ$, qui sont identiques ou différents, représentent un atome d'hydrogène, un radical alkyle $C_1$-$C_4$ ou hydroxyalkyle $C_1$-$C_4$ ; ou un radical aryl($C_1$-$C_4$)alkyle, et $M^-$ représente un contre-ion anionique organique ou inorganique tel qu'un halogénure, préférablement, $T_a$ représentant une liaison $\sigma$ covalente ou un groupe -N(R)- et $T_b$ représentant une liaison $\sigma$ covalente ou un groupe sélectionné parmi un radical -O-, -C(O)-, -N(R)-, ou une combinaison de ceux-ci, où R représente un atome d'hydrogène ou un groupe alkyle $C_1$-$C_4$ tel qu'un groupe méthyle ;
et

- $T_a$ étant préférablement en position para sur Ar ou Ar', par rapport à la fonction oléfine $-C(R^c)=C(R^d)-$.

**7.** Procédé de coloration selon l'une quelconque des revendications 5 et 6, dans lequel le colorant selon la formule (I) est tel que W représente un groupe sélectionné parmi l'imidazolium, le pyridinium, le benzopyridinium, le benzimidazolium, le quinolinium, l'indolinium et le pyrazolium, qui sont optionnellement substitués.

**8.** Procédé de coloration selon l'une quelconque des revendications précédentes, dans lequel le colorant selon la formule (I) est sélectionné parmi les composés (Ia) à (Ig) ci-dessous :

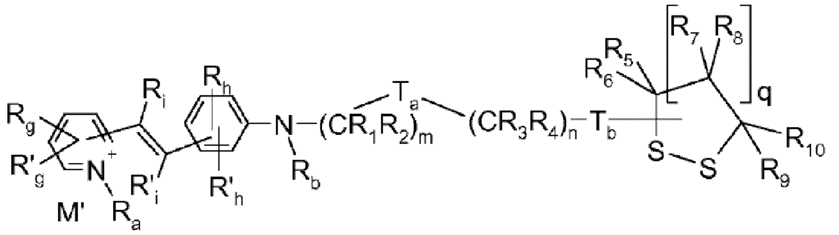

**(Ia)**

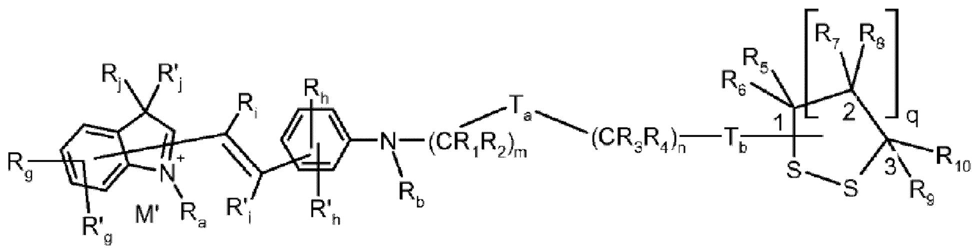

**(Ib)**

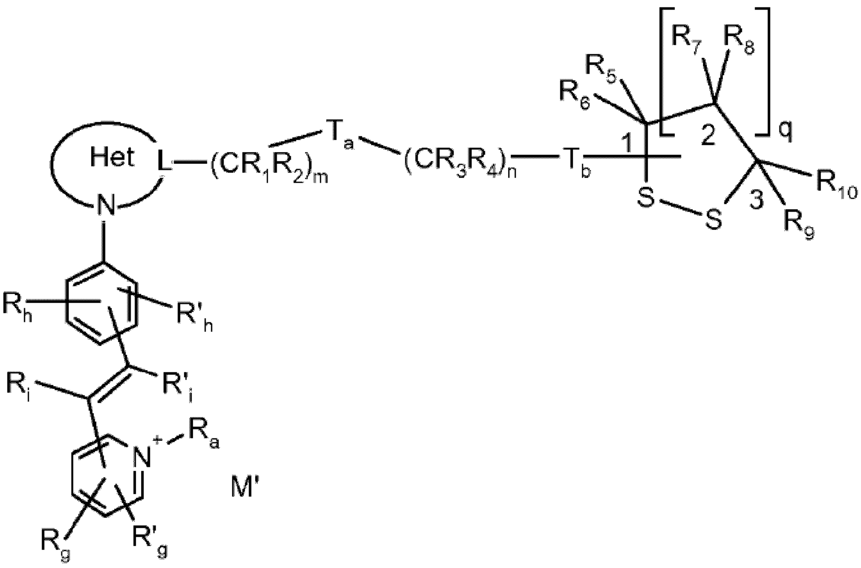

**(Ic)**

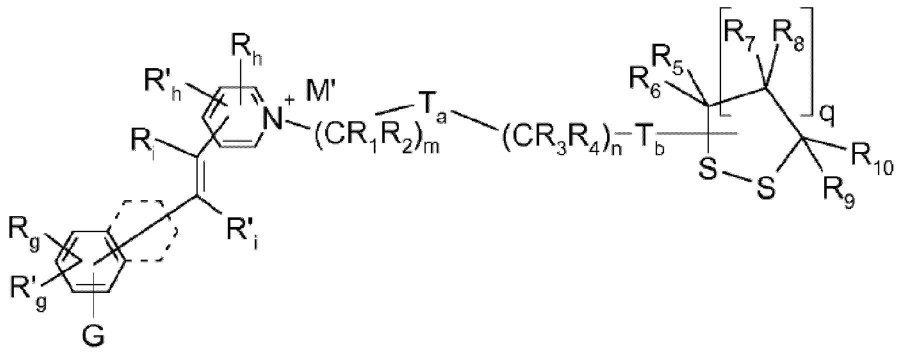

**(Id)**

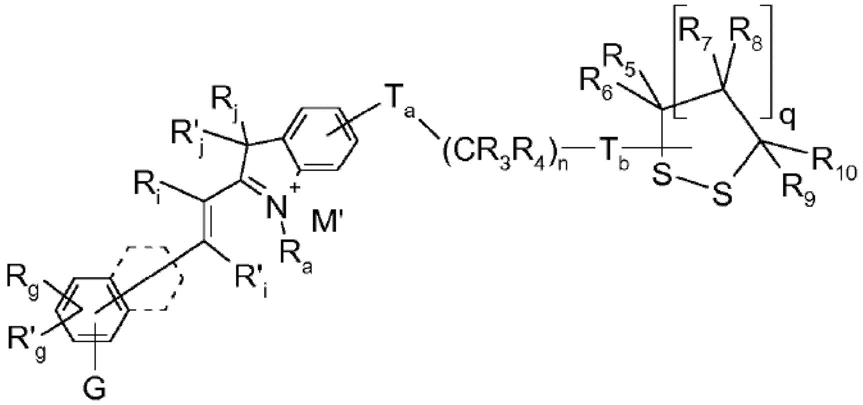

**(Ie)**

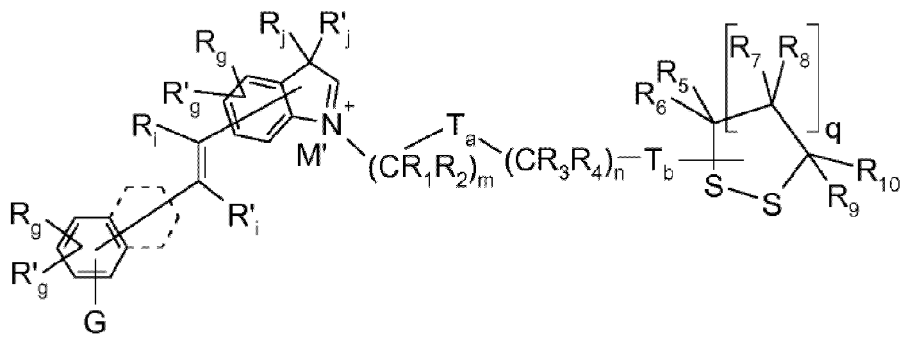

**(If)**

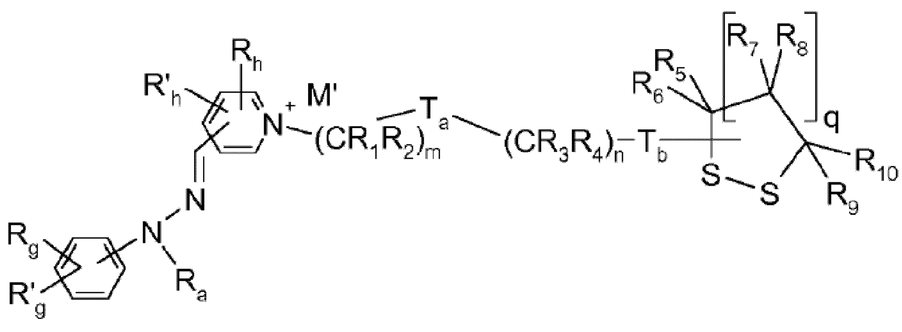

**(Ig)**

et leurs sels d'acides organiques ou inorganiques, leurs isomères optiques et leurs isomères géométriques, et leurs solvates tels que des hydrates ;
dans lesquelles formules (Ia) à (Ig) :

- G représente un groupe $-NR_cR_d$ ou un groupe alcoxy $C_1$-$C_6$;
- $R_a$ représente un groupe alkyle $C_1$-$C_6$ optionnellement substitué ;
- $R_b$ représente un atome d'hydrogène ou un groupe alkyle $C_1$-$C_6$ optionnellement substitué ;

• $R^c$ et $R_d$, qui sont identiques ou différents, représentent un atome d'hydrogène, un groupe aryl ($C_1$-$C_4$)alkyle, un groupe alcoxy $C_1$-$C_6$ ou un groupe alkyle $C_1$-$C_6$ optionnellement substitué ; $R_c$ et $R_d$ représentent préférablement un atome d'hydrogène ou un groupe alkyle $C_1$-$C_3$ qui est optionnellement substitué par i) un groupe hydroxyle, ii) un groupe amino, iii) un groupe (di)alkylamino $C_1$-$C_3$, ou iv) un groupe ammonium quaternaire (R") (R"')(R"")$N^+$-;

ou deux radicaux $R_c$ et $R_d$ adjacents portés par le même atome d'azote forment ensemble un groupe hétérocyclique ou hétéroaryle ;

en particulier, $R_c$ et $R_d$ représentent des groupes identiques, et préférablement, $R_c$ et $R_d$ représentent un groupe alkyle $C_1$-$C_3$ qui est optionnellement substitué par un groupe hydroxyle, tel qu'un groupe méthyle, hydroxyéthyle et 2-hydroxypropyle ;

• $R_g$, $R'_g$, $R_h$ et $R'_h$, qui sont identiques ou différents, représentent un atome d'hydrogène, un atome d'halogène, un groupe amino, alkylamino $C_1$-$C_4$, dialkylamino $C_1$-$C_4$, cyano, carboxyle, hydroxyle ou trifluorométhyle, un radical acylamino, alcoxy $C_1$-$C_4$, (poly) hydroxyalcoxy $C_2$-$C_4$, alkylcarbonyloxy, alcoxycarbonyle ou alkylcarbonylamino, un radical acylamino, carbamoyle ou alkylsulfonylamino, un radical aminosulfonyle, ou un radical alkyle $C_1$-$C_{16}$ qui est optionnellement substitué par un groupe sélectionné parmi un groupe alcoxy $C_1$-$C_{12}$, hydroxyle, cyano, carboxyle, amino, alkylamino $C_1$-$C_4$ et dialkylamino $C_1$-$C_4$, ou les deux radicaux alkyle portés par l'atome d'azote du groupe amino forment un hétérocycle contenant de 5 à 7 chaînons et comprenant optionnellement un autre hétéroatome, qui est identique à ou différent de l'atome d'azote ; préférablement, $R_g$, $R'_g$, $R_h$ et $R'_h$ représentent un atome d'hydrogène ou d'halogène ou un groupe alkyle $C_1$-$C_3$ ;

• ou deux groupes $R_g$ et $R'_g$, $R_h$ et $R'_h$, portés par deux atomes de carbone adjacents, forment ensemble un système cyclique benzo ou indéno ou un groupe hétérocycloalkyle condensé ou hétéroaryle condensé ; le système cyclique benzo, indéno, hétérocycloalkyle ou hétéroaryle étant optionnellement substitué par un atome d'halogène, un groupe amino, alkylamino $C_1$-$C_4$, dialkylamino $C_1$-$C_4$, nitro, cyano, carboxyle, hydroxyle ou trifluorométhyle, un radical acylamino, alcoxy $C_1$-$C_4$, (poly) hydroxyalcoxy $C_2$-$C_4$, alkylcarbonyloxy, alcoxycarbonyle ou alkylcarbonylamino, un radical acylamino, carbamoyle ou alkylsulfonylamino, un radical aminosulfonyle, ou un radical alkyle $C_1$-$C_{16}$ qui est optionnellement substitué par un groupe sélectionné parmi un groupe alcoxy $C_1$-$C_{12}$, hydroxyle, cyano, carboxyle, amino, alkylamino $C_1$-$C_4$, dialkylamino $C_1$-$C_4$, ou les deux radicaux alkyle portés par l'atome d'azote du groupe amino forment un hétérocycle comprenant de 5 à 7 chaînons et comprenant optionnellement un autre hétéroatome, qui est identique à ou différent de l'atome d'azote ; préférablement, $R_g$ et $R'_g$ forment ensemble un groupe benzo ;

• ou, lorsque G représente un groupe $-NR_cR_d$, deux groupes $R_c$ et $R'_g$, $R_d$ et $R_g$ forment ensemble un hétérocycle ou un groupe hétéroaryle saturé qui est optionnellement substitué par un ou plusieurs groupes alkyle $C_1$-$C_6$, préférablement un hétérocycle contenant un ou deux hétéroatomes sélectionnés parmi des atomes d'azote et d'oxygène et comprenant de 5 à 7 chaînons ;

• $R_i$ $R_j$, $R'_j$ et $R'_i$, qui sont identiques ou différents, représentent un atome d'hydrogène ou un groupe alkyle $C_1$-$C_4$ ; préférablement, $R_i$ $R_j$, $R'_j$ et $R'_i$ représentent un atome d'hydrogène ;

• $R_1$, $R_2$, $R_3$ et $R_4$, qui sont identiques ou différents, représentent un atome d'hydrogène ou un groupe alkyle $C_1$-$C_4$, alcoxy $C_1$-$C_{12}$, hydroxyle, cyano, carboxyle, amino, alkylamino $C_1$-$C_4$ ou dialkylamino $C_1$-$C_4$, lesdits radicaux alkyle pouvant former, avec l'atome d'azote qui les porte, un hétérocycle comprenant de 5 à 7 chaînons, comprenant optionnellement un autre hétéroatome, qui est différent ou non de l'azote ; préférablement, $R_1$, $R_2$, $R_3$ et $R_4$ sont des atomes d'hydrogène ou un groupe amino ; plus préférablement, $R_1$, $R_2$, $R_3$ et $R_4$ représentent un atome d'hydrogène ;

• $T_a$ et $T_b$, qui sont identiques ou différents, représentent :

i) soit une liaison $\sigma$ covalente ;

ii) soit un ou plusieurs radicaux ou des combinaisons de ceux-ci sélectionnés parmi des groupes $-S(O)_2-$, -O-, -S-, -N(R)-, $-N^+(R)(R^\circ)-M^-$, -C(O)-, où R et R°, qui sont identiques ou différents, représentent un atome d'hydrogène ou un radical alkyle $C_1$-$C_4$ ou hydroxyalkyle $C_1$-$C_4$ ; ou un groupe aryl ($C_1$-$C_4$)alkyle, et $M^-$ représente un contre-ion anionique organique ou inorganique tel qu'un halogénure ; préférablement, $T_a$ représente une liaison $\sigma$ covalente ou un groupe -N(R)- et $T_b$ représente une liaison $\sigma$ covalente ou un groupe sélectionné parmi un radical -O-, -C(O)-, -N(R)-, ou une combinaison de ceux-ci, où R représente un atome d'hydrogène ou un groupe alkyle $C_1$-$C_4$ tel qu'un groupe méthyle ;

•

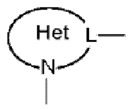

représente un groupe hétérocyclique optionnellement substitué ; L représente un atome de carbone ou un atome d'azote, l'hétérocycle est préférablement monocyclique et saturé et comprend un total de deux atomes d'azote et de 5 à 8 chaînons ;

•

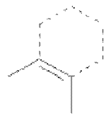

représente un groupe aryle ou hétéroaryle qui est condensé avec le cycle phényle ; ou est absent du cycle phényle ; lorsque le cycle est présent, le cycle est préférablement un groupe benzo ; plus préférablement, le cycle est absent ;

- m et n, qui sont identiques ou différents, représentent un nombre entier de 0 à 10, préférablement de 0 à 7, inclusivement, m valant préférablement de 0 à 5, plus particulièrement de 0 à 3, inclusivement, et n valant de 0 à 8, inclusivement, n valant plus particulièrement 0, 1, 2, 3 ou 4 ;
- $R_5$, $R_6$, $R_9$ et $R_{10}$, qui sont identiques ou différents, représentent un atome d'hydrogène ou un groupe sélectionné parmi des groupes : i) ($C_1$-$C_8$) alkyle, iii) hydroxyle, iv) (di)($C_1$-$C_8$)(alkyl) amino, v) ($C_1$-$C_8$)alcoxy, vi) (poly) hydroxy ($C_1$-$C_8$)alkyle, vii) (di) ($C_1$-$C_8$)(alkyl) amino ($C_1$-$C_8$)alkyle, viii) carboxyle, ix) carboxy($C_1$-$C_3$)alkyle, x) (di) ($C_1$-$C_8$)(alkyl) aminocarbonyl ($C_1$-$C_8$)alkyle et xii) ($C_1$-$C_8$)(alkyl) carbonyl ($C_1$-$C_8$)(alkyl) amino ($C_1$-$C_8$)alkyle ; préférablement, R5 est un atome ou un groupe alkyle tel qu'un groupe méthyle et $R_6$, $R_9$ et $R_{10}$ représentent des atomes d'hydrogène ;
- $R_7$ et $R_8$, qui sont identiques ou différents, représentent un atome d'hydrogène ou un groupe sélectionné parmi des groupes : i) ($C_1$-$C_8$)alkyle, iii) hydroxyle, iv) (di) ($C_1$-$C_8$)(alkyl) amino, v) ($C_1$-$C_8$)alcoxy, vi) (poly) hydroxy ($C_1$-$C_8$)alkyle, vii) (di) ($C_1$-$C_8$)(alkyl) amino ($C_1$-$C_8$)alkyle, viii) carboxyle, ix) carboxy ($C_1$-$C_8$)alkyle, x) (di) ($C_1$-$C_8$)(alkyl) amino-carbonyl ($C_1$-$C_8$)alkyle et xii) ($C_1$-$C_8$)(alkyl) carbonyl ($C_1$-$C_8$)(alkyl) amino ($C_1$-$C_8$)alkyle ; préférablement, $R_7$ et $R_8$ représentent un atome d'hydrogène ou un groupe alkyle $C_1$-$C_4$ tel qu'un groupe méthyle ;
- q représente un nombre entier de 1 à 3 inclusivement ; préférablement q = 1 ; et
- M' représente un contre-ion anionique organique ou inorganique ;

sous réserve que l'hétérocycle disulfure soit relié au reste de la molécule directement par l'atome de carbone, préférablement en position bêta par rapport à la fonction disulfure dudit hétérocycle.

**9.** Procédé de coloration selon l'une quelconque des revendications 1 à 3 et 5 à 8, dans lequel le colorant fluorescent selon la formule (I) est sélectionné parmi les composés (I'a), (I'b), (I'd), (I'e) et (I'f) ci-dessous :

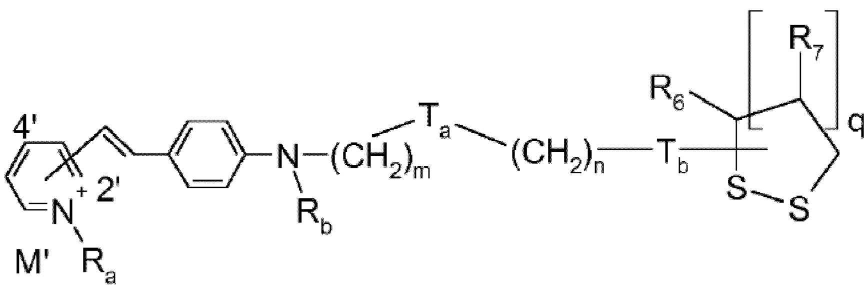

**(I'a)**

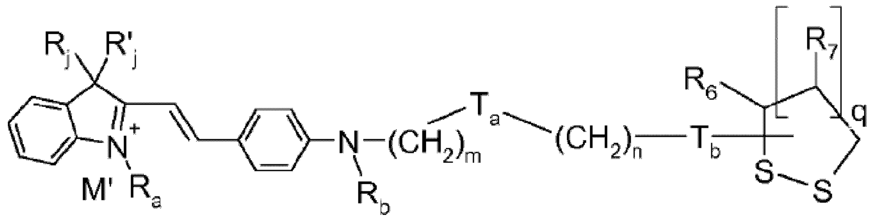

**(I'b)**

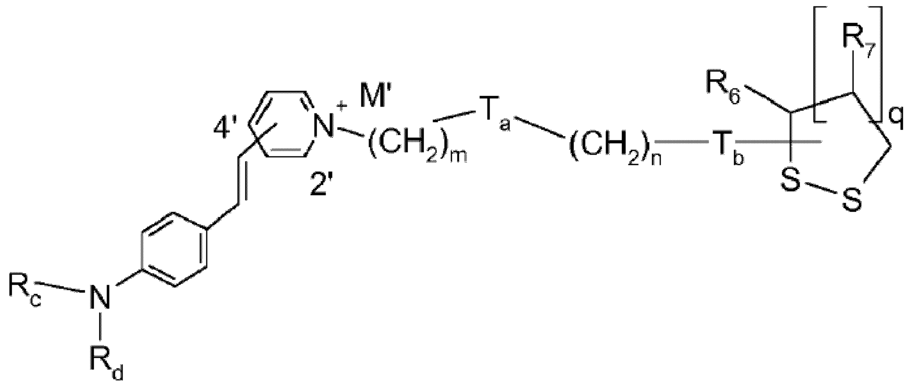

**(I'd)**

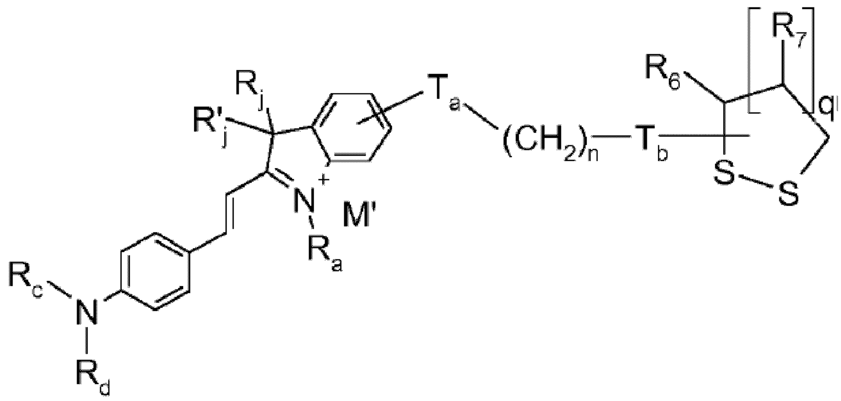

**(I'e)**

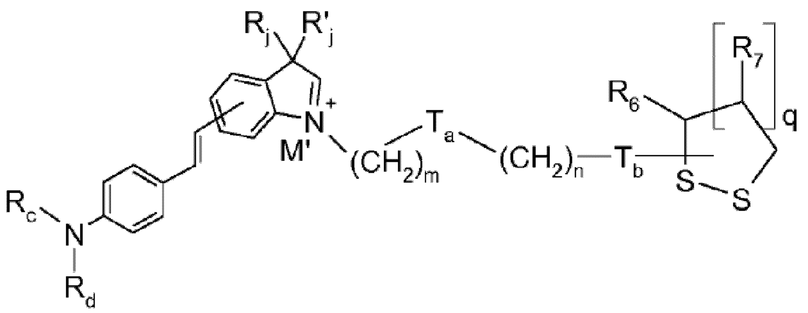

**(I'f)**

dans lesquelles formules (I'a), (I'b), (I'd), (I'e) et (I'f) :

- ◦ $R_a$, $R_b$, $R_j$ et $R'_j$ représentent un groupe alkyle $C_1$-$C_4$ tel qu'un groupe méthyle ;
- ◦ $R_6$ et $R_7$ représentent un atome d'hydrogène ou un groupe alkyle $C_1$-$C_4$ tel qu'un groupe méthyle, $T_b$ étant relié directement sur l'atome de carbone en position alpha ou bêta relativement à la fonction disulfure de l'hétérocycle ;
- ◦ m et n, qui sont identiques ou différents, représentent un nombre entier de 0 à 7, inclusivement, m valant de 0 à 3 et n valant 0, 1, 2, 3 ou 4 ;
- ◦ $T_a$ et $T_b$, qui sont identiques ou différents, représentent i) une liaison σ covalente ; ou ii) un groupe sélectionné parmi un radical -O-, -C(O)-, -N(R)- ou une combinaison de ceux-ci tels que -N(R)-C(O)-, -C(O)-N(R)-, -O-C(O)-,

-C(O)-O- ou -C(O)-N(R)-C(O)-, où R représente un atome d'hydrogène ou un groupe alkyle $C_1$-$C_4$ ; en particulier, R est un atome d'hydrogène ou un groupe méthyle ;

◦ q vaut 1, 2 ou 3 ; en particulier, q vaut 1 ;

◦ $R_c$ et $R_d$ représentent des groupes alkyle $C_1$-$C_3$ identiques, optionnellement substitués par un groupe hydroxyle, tel qu'un groupe méthyle, hydroxyéthyle et 2-hydroxypropyle ;
et

◦ M' représente un contre-ion anionique organique ou inorganique ;

sous réserve que dans les formules (I'a) et (I'd), le groupe pyridinium soit relié au groupe styryle par l'atome de carbone en position 2' (ortho) ou 4' (para).

**10.** Procédé de coloration selon l'une quelconque des revendications 1 à 4, 6 et 8, dans lequel le colorant selon la formule (I) est sélectionné à partir de (I'g) ci-dessous :

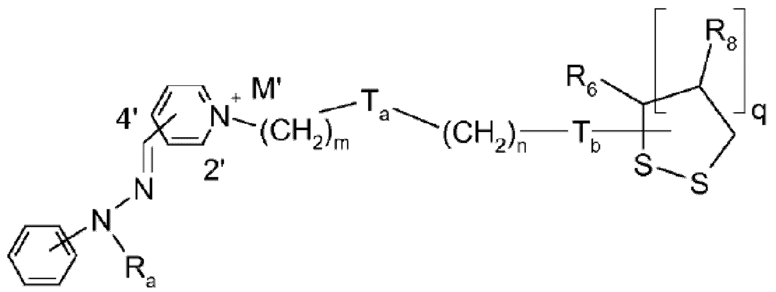

**(I'g)**

dans laquelle formule (I'g) :

◦ $R_a$ représente un groupe alkyle $C_1$-$C_4$ tel qu'un groupe méthyle ;

◦ $T_a$ et $T_b$, qui sont identiques ou différents, représentent i) une liaison σ covalente ; ou ii) un groupe sélectionné parmi un radical -O-, -C(O)-, -N(R)- ou une combinaison de ceux-ci tels que -N(R)-C(O)-, -C(O)-N(R)-, -O-C(O)-, -C(O)-O- ou -C(O)-N(R)-C(O)-, où R représente un atome d'hydrogène ou un groupe alkyle $C_1$-$C_4$ ; en particulier, R est un atome d'hydrogène ou un groupe méthyle ;

◦ $R_6$ et $R_7$ représentent un atome d'hydrogène ou un groupe alkyle $C_1$-$C_4$ tel qu'un groupe méthyle, $T_b$ étant relié directement sur l'atome de carbone en position alpha ou bêta relativement à la fonction disulfure de l'hétérocycle ;

◦ m et n, qui sont identiques ou différents, représentent un nombre entier de 0 à 7, inclusivement, m valant de 0 à 3 et n valant 0, 1, 2, 3 ou 4 ;

◦ q vaut 1, 2 ou 3 ; en particulier, q vaut 1 ;

◦ M' représente un contre-ion anionique organique ou inorganique ;

sous réserve que le groupe pyridinium soit relié au groupe hydrazono par l'atome de carbone en position 2' (ortho) ou 4' (para), préférablement 4' .

**11.** Procédé de coloration selon l'une quelconque des revendications 1 à 3 et 5 à 9, dans lequel le colorant fluorescent selon la formule (I) est sélectionné parmi les composés (1) à (68) ci-dessous :

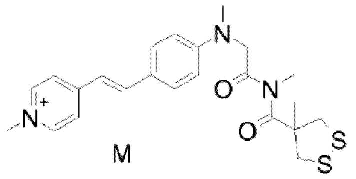

**1**

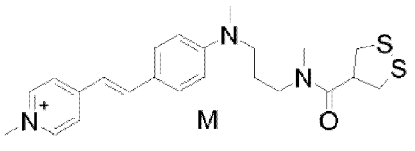

**2**

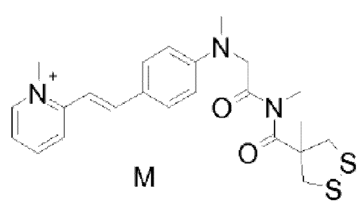

3

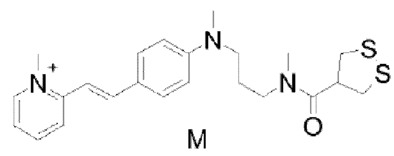

4

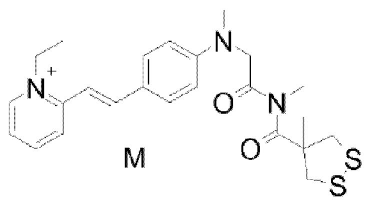

5

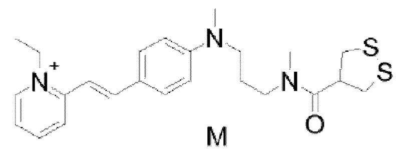

6

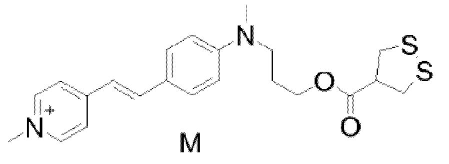

7

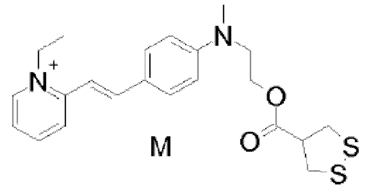

8

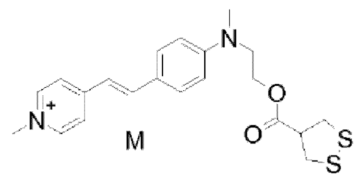

9

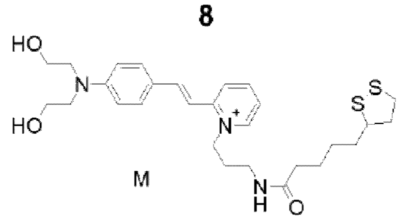

10

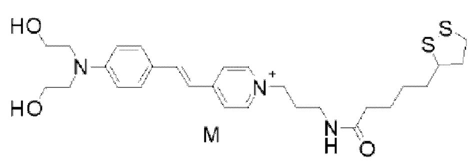

11

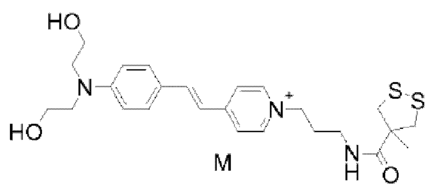

12

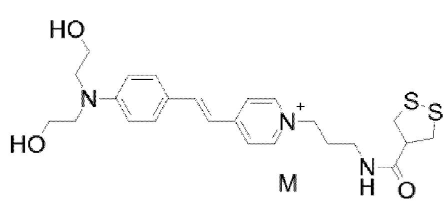

13

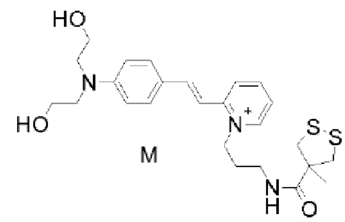

14

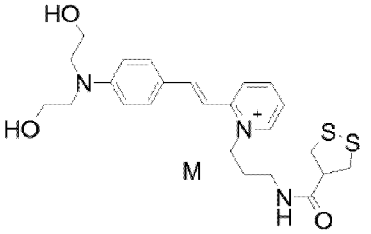

**15**

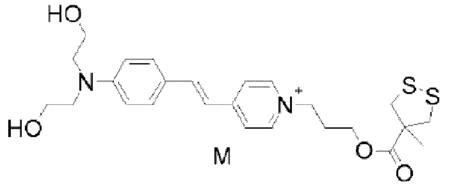

**16**

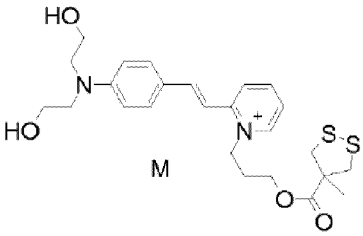

**17**

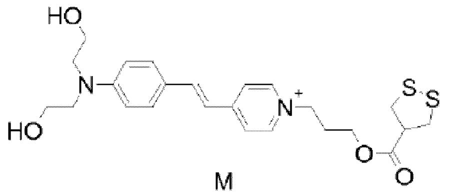

**18**

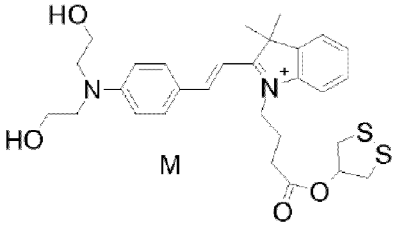

**19**

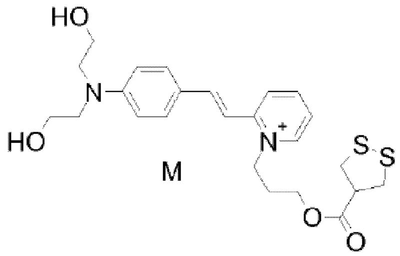

**20**

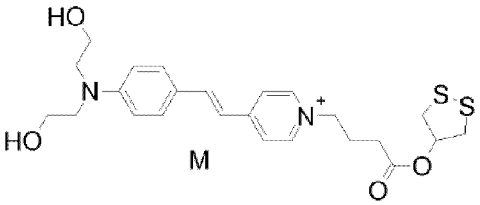

**21**

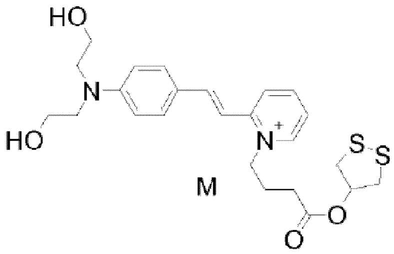

**22**

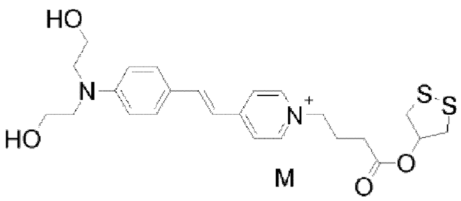

**23**

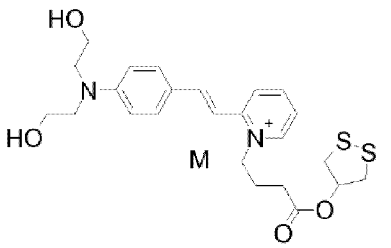

**24**

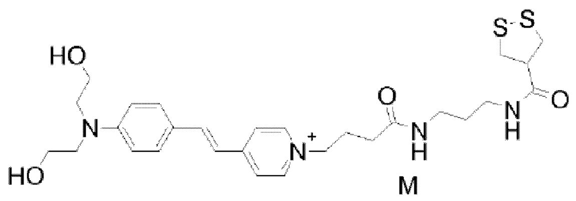

**25**

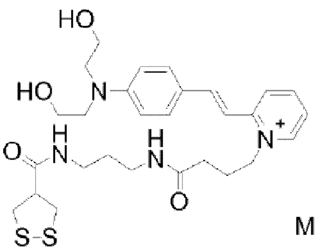

**26**

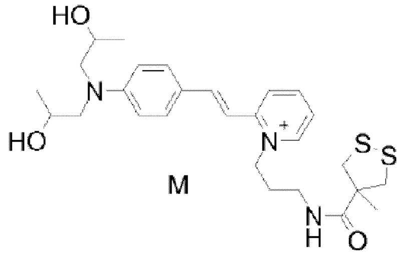

**27**

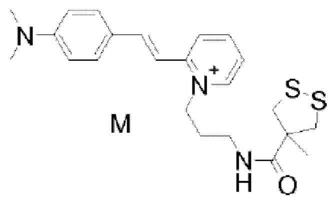

**28**

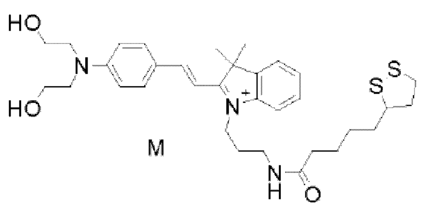

**29**

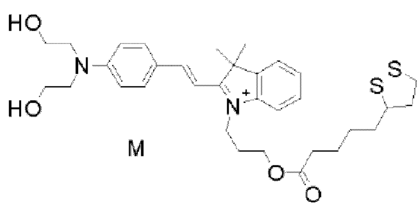

**30**

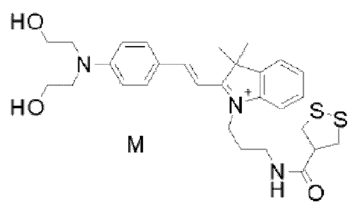

**31**

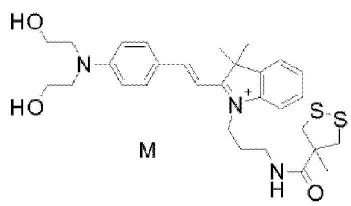

**32**

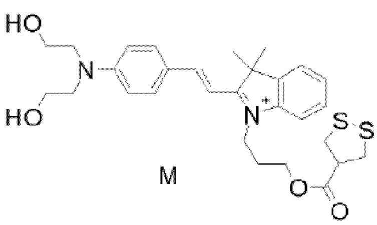

**33**

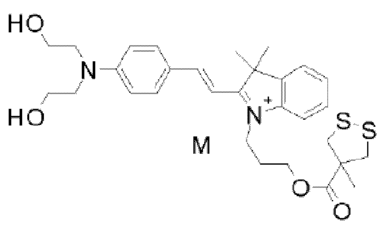

**34**

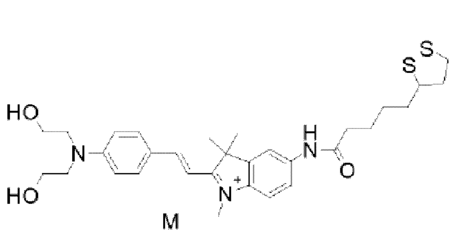

**35**

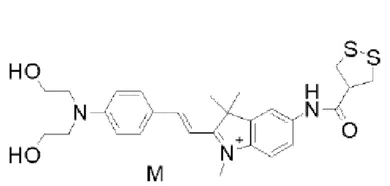

**36**

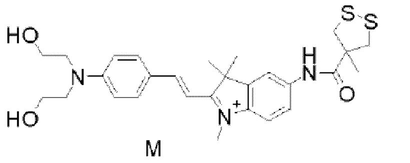

**37**

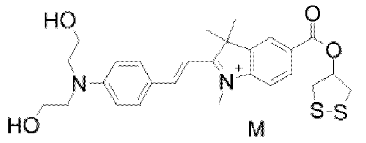

**38**

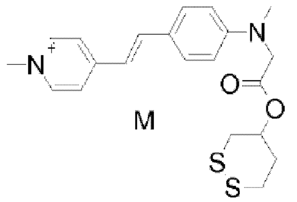

**39**

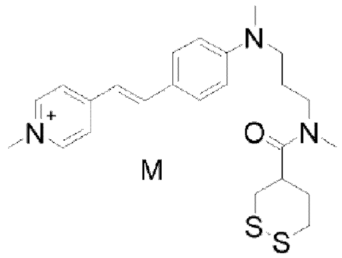

**40**

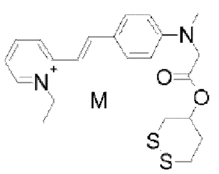

**41**

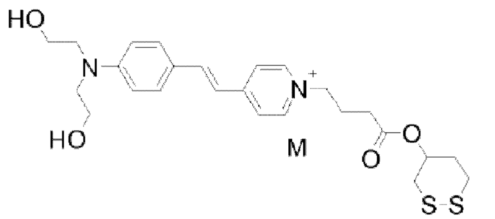

**42**

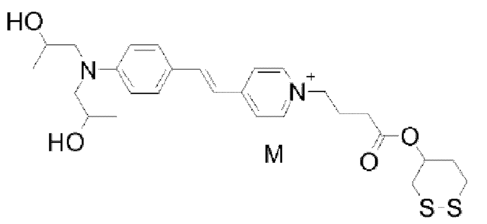

**43**

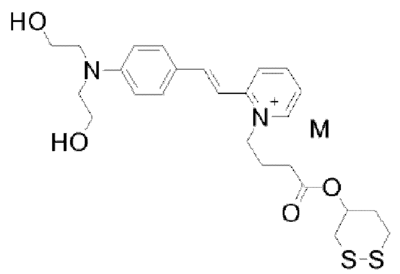

**44**

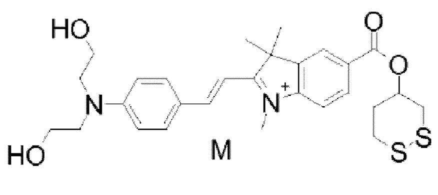

**45**

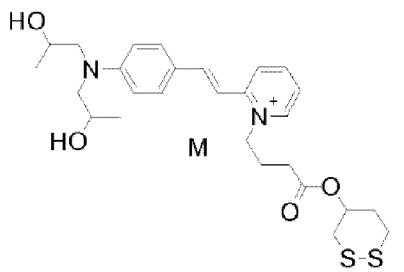

**46**

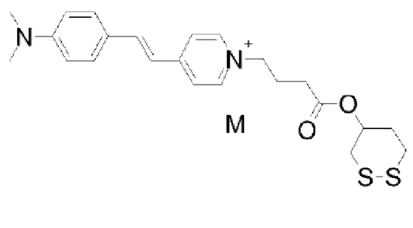

**47**

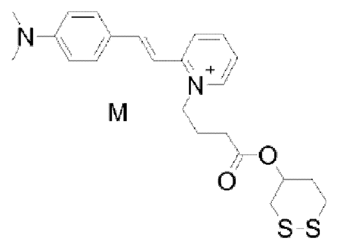

**48**

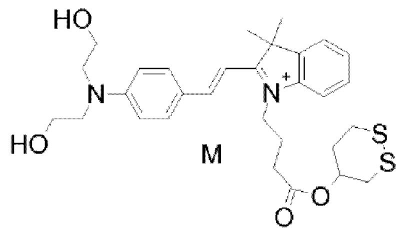

**49**

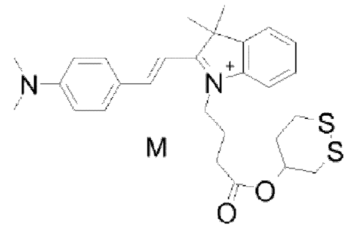

**50**

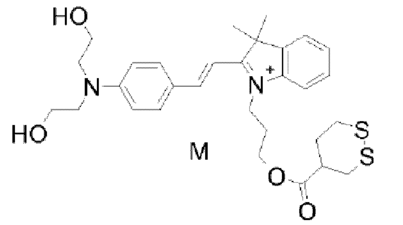

**51**

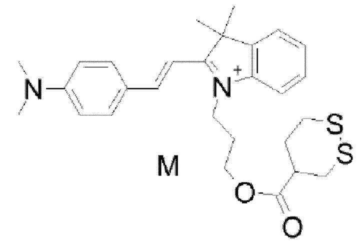

**52**

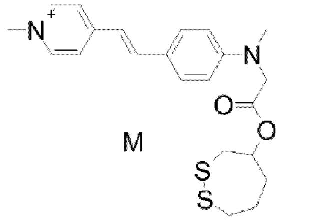

**53**

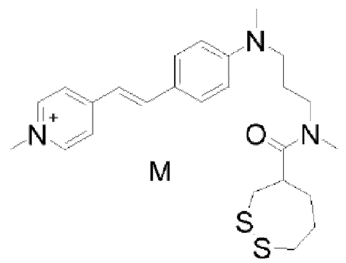

**54**

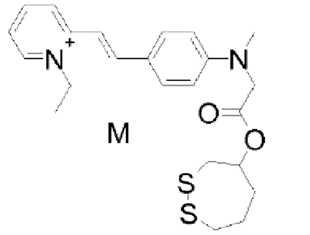

**55**

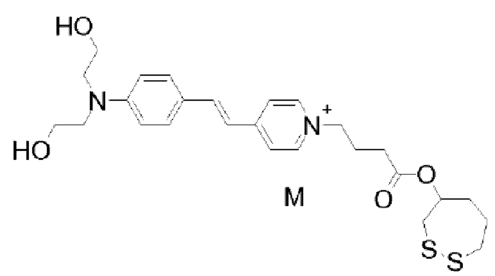

**56**

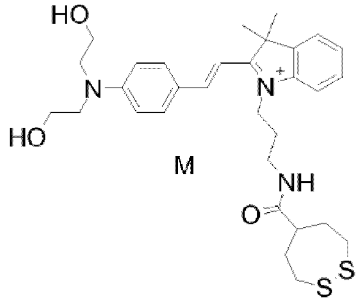

57

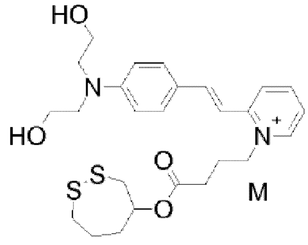

58

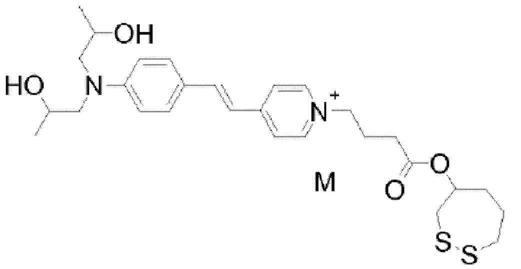

59

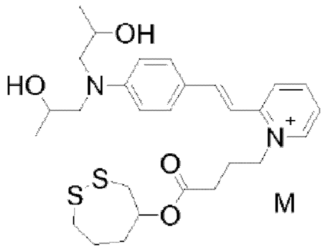

60

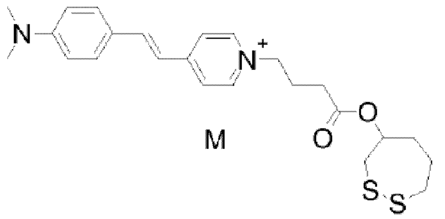

61

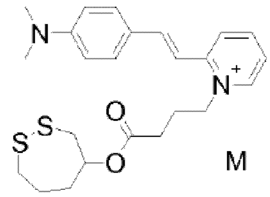

62

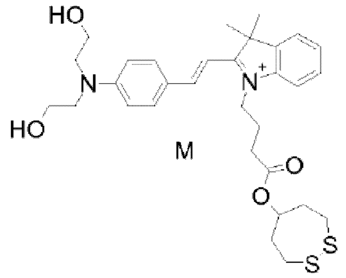

63

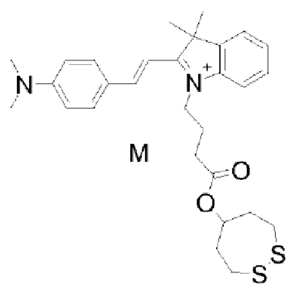

64

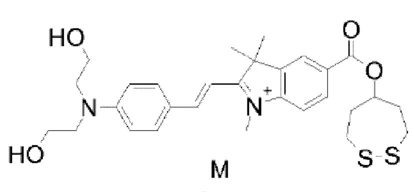

65

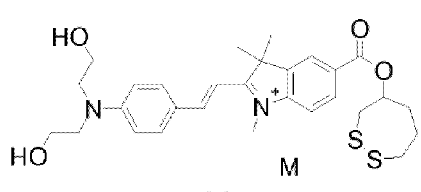

66

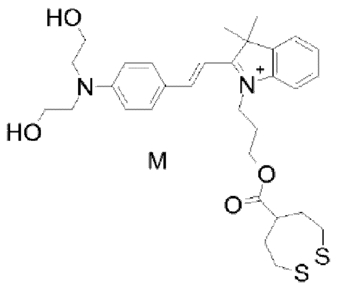

**67**

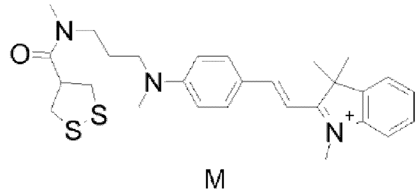

**68**

où M représente un contre-ion anionique.

12. Procédé de coloration selon l'une quelconque des revendications 1 à 4, 6, 8 et 10, dans lequel le colorant selon la formule (I) est sélectionné à partir du composé 69 ci-dessous :

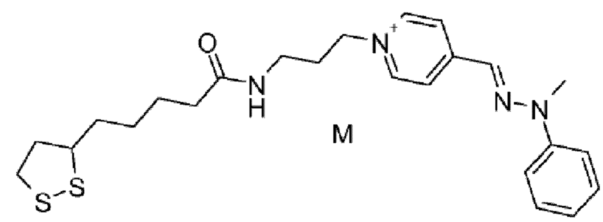

**69**

où M représente un contre-ion anionique.

13. Procédé de coloration de matières kératiniques selon l'une quelconque des revendications précédentes, dans lequel on laisse agir la composition comprenant le composé ou les composés selon la formule (I), puis les matières kératiniques sont rincées, et/ou sont essorées et/ou subissent un traitement thermique et/ou sont exposées à la lumière ultraviolette (UV).

14. Procédé de coloration de matières kératiniques selon la revendication précédente, dans lequel on laisse agir la composition comprenant le composé ou les composés selon la formule (I), puis les matières kératiniques sont essorées et/ou subissent un traitement thermique, en particulier à une température de 50 à 200 °C, préférablement de 80 à 180 °C.

15. Procédé selon l'une quelconque des revendications précédentes, comprenant une étape additionnelle d'application d'un agent oxydant sur les fibres de kératine.

16. Procédé de coloration et/ou d'éclaircissement selon l'une quelconque des revendications précédentes, dans lequel les matières kératiniques sont des fibres de kératine foncées possédant un niveau de tonalité inférieur ou égal à 6, en particulier inférieur ou égal à 4.

17. Composition de coloration comprenant, dans un véhicule cosmétique approprié, un colorant selon la formule (I) tel que défini dans l'une quelconque des revendications 1 à 12, où Het représente un radical disulfure hétérocyclique selon la formule (II) tel que défini dans la revendication 1, le radical A de la formule (I) contenant au moins un radical cationique qui est porté par ou inclus dans au moins l'un des chromophores, et sous réserve que :

- le composé selon la formule (I) ne puisse pas comprendre de chromophore fluorescent de type riboflavine-5-monophosphate (A) ;
- le composé selon la formule (I) ne puisse pas comprendre de chromophore fluorescent (A) qui représente un groupe indo-3-yle ou 5-méthoxyindol-3-yle ; et
- le composé selon la formule (I) ne puisse pas représenter les composés (A), (B), (C) ou (D) :

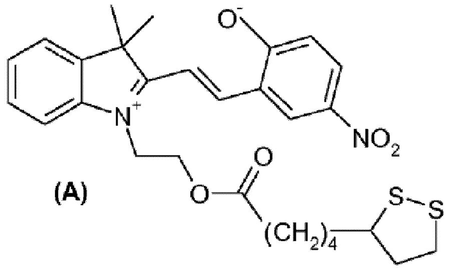

(A)

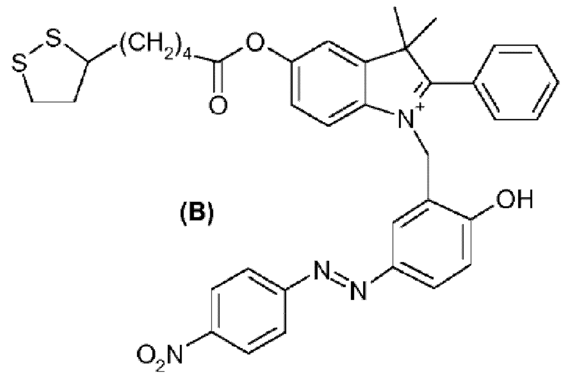

(B)

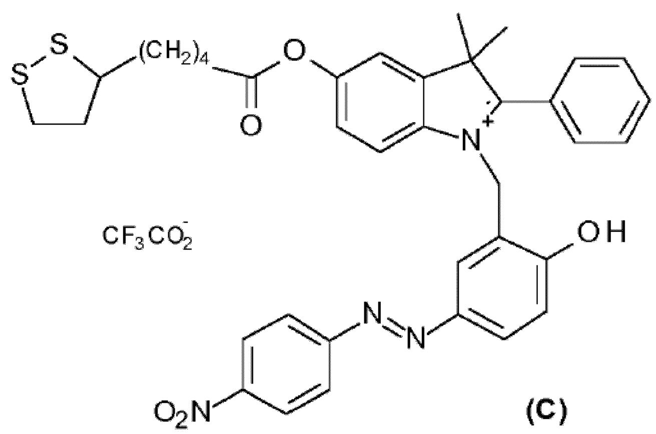

(C)

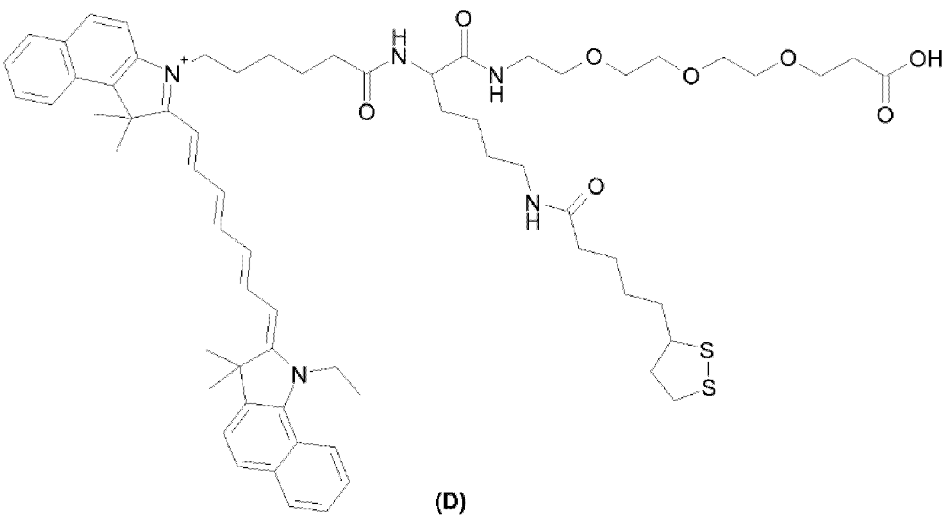

(D)

(B) étant combiné ou non combiné avec un puits quantique à structure noyau-enveloppe CdSe-ZnS, CdTe-ZnS, CdSe-ZnSe ou CdTe-ZnSe ; (C) étant combiné ou non combiné avec un puits quantique à structure noyau-enveloppe CdSe-ZnS.

**18.** Composition selon la revendication précédente, dans laquelle le colorant selon la formule (I) est présent dans une quantité de 0,001 à 50 % en poids, relativement au poids total de la composition.

**19.** Colorant selon la formule (I) tel que défini dans l'une quelconque des revendications 1 à 12, dans lequel Het représente un radical disulfure hétérocyclique selon la formule (II) tel que défini dans la revendication 1, le radical A de la formule (I) contenant au moins un radical cationique qui est porté par ou inclus dans au moins l'un des chromophores, et sous réserve que :

- le composé selon la formule (I) ne puisse pas comprendre de chromophore fluorescent de type riboflavine-5-monophosphate (A) ;
- le composé selon la formule (I) ne puisse pas comprendre de chromophore fluorescent (A) qui représente un groupe indo-3-yle ou 5-méthoxyindol-3-yle ; et
- le composé selon la formule (I) ne puisse pas représenter les composés (A), (B), (C) ou (D) :

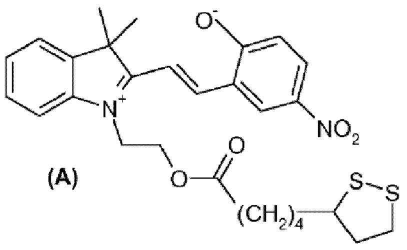
(A)

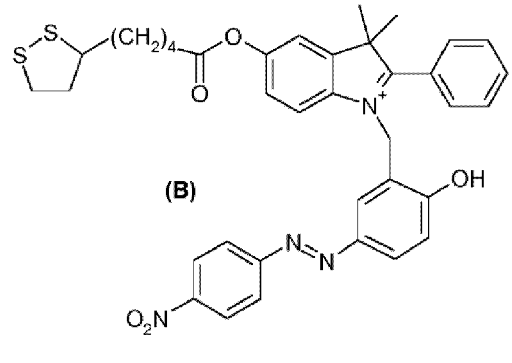
(B)

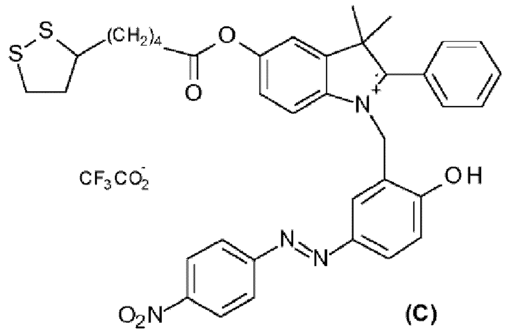
(C)

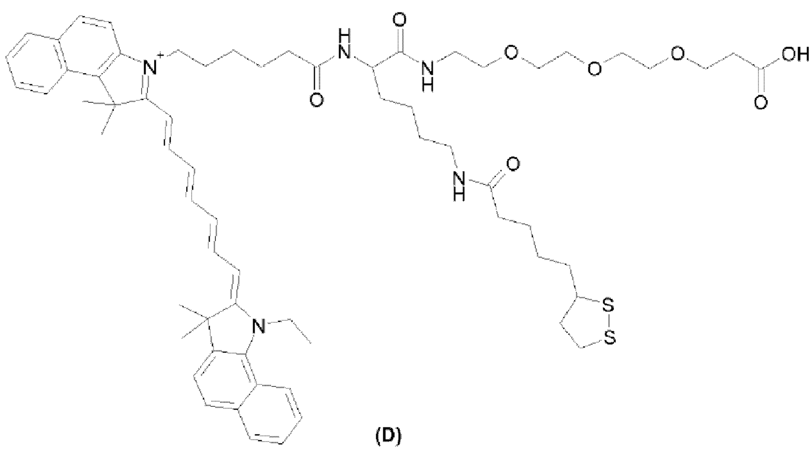
(D)

(B) étant combiné ou non combiné avec un puits quantique à structure noyau-enveloppe CdSe-ZnS, CdTe-ZnS, CdSe-ZnSe ou CdTe-ZnSe ; (C) étant combiné ou non combiné avec un puits quantique à structure noyau-enveloppe CdSe-ZnS.

**20.** Utilisation de colorants selon la formule (I) tels que définis dans les revendications 1 à 12 pour la coloration et/ou l'éclaircissement de matières kératiniques humaines telles que des fibres de kératine humaines, en particulier des fibres foncées dont le niveau de tonalité est inférieur à 6, préférablement inférieur ou égal à 4.

Fig. 1/1

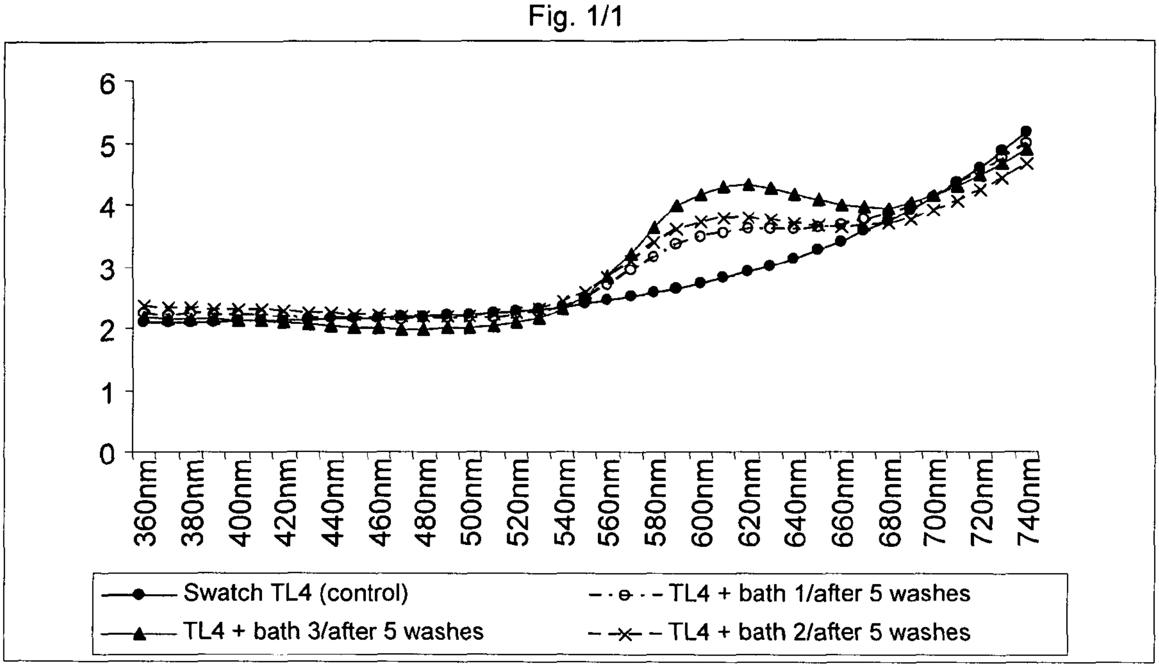
6
5
4
3
2
1
0
360nm
380nm
400nm
420nm
440nm
460nm
480nm
500nm
520nm
540nm
560nm
580nm
600nm
620nm
640nm
660nm
680nm
700nm
720nm
740nm
Swatch TL4 (control)
TL4 + bath 1/after 5 washes
TL4 + bath 3/after 5 washes
TL4 + bath 2/after 5 washes

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- FR 1156407
- WO 2005097051 A
- EP 1647580 A
- WO 03028685 A
- WO 2004091473 A
- WO 2006134043 A
- WO 2006136617 A
- WO 2007110533 A
- WO 2007110534 A
- WO 2007110535 A
- WO 2007110537 A
- WO 2007110542 A
- WO 200818894 A
- WO 9515144 A
- WO 9501772 A
- EP 714954 A
- EP 1133975 A
- WO 03029359 A
- EP 860636 A
- FR 2586913


### Non-patent literature cited in the description


- *Australian Journal of Chemistry,* 2006, vol. 59 (3), 175-178
- *Journal of Materials Chemistry,* 2008, vol. 18 (46), 5577-5584
- *Analyst,* 2009, vol. 134, 549-556
- **CHARLES ZVIAK.** Science des traitements capillaires. Masson, 1988, 215-278
- Dyes, Azo. Kirk Othmer Encyclopedia of Chemical Technology. J. Wiley & sons, 19 April 2010
- **K. VENKATARAMAN.** The chemistry of synthetic dyes. Academic Press, 1952, vol. 1 to 7
- Dyes and Dye Intermediates. **KIRK OTHMER.** Chemical Technology. Wiley and Sons, 1993
- ULLMANN's ENCYCLOPEDIA of Industrial Chemistry. Wiley and Sons
- The Handbook - A Guide to Fluorescent Probes and Labeling Technologies. Molecular Probes/Invitrogen, 2005
- Advanced Organic Chemistry
- Protecting groups in Organic Chemistry
- Comprehensive Heterocyclic Chemistry III. 2008, 893-954
- Comprehensive Heterocyclic Chemistry III. 2008, 299-319
- Comprehensive Heterocyclic Chemistry III. 2008, 893-954, 299-319
- COMPREHENSIVE HETEROCYCLIC CHEMISTRY III. 299-319
- COMPREHENSIVE HETEROLYC CHEMISTRY III. 299-319